(19) Europäisches Patentamt
European Patent Office
Office européen des brevets

(11) EP 2 852 389 B1

(12) EUROPEAN PATENT SPECIFICATION

(45) Date of publication and mention of the grant of the patent:
**18.10.2017 Bulletin 2017/42**

(21) Application number: **13730128.9**

(22) Date of filing: **22.05.2013**

(51) Int Cl.:
*A61K 31/55* (2006.01)
*A61K 47/06* (2006.01)
*A61K 47/32* (2006.01)
*A61K 47/36* (2006.01)
*A61K 47/38* (2006.01)
*A61K 31/5517* (2006.01)
*A61K 9/14* (2006.01)
*A61K 47/12* (2006.01)
*A61K 47/26* (2006.01)
*A61K 47/40* (2006.01)
*A61K 9/19* (2006.01)
*A61K 31/5513* (2006.01)
*C07D 243/16* (2006.01)
*C07D 243/24* (2006.01)
*C07D 401/04* (2006.01)

(86) International application number:
**PCT/EP2013/060543**

(87) International publication number:
**WO 2013/174883 (28.11.2013 Gazette 2013/48)**

(54) **COMPOSITIONS COMPRISING SHORT-ACTING BENZODIAZEPINES**

ZUSAMMENSETZUNGEN MIT KURZZEITWIRKENDEN BENZODIAZEPINEN

COMPOSITIONS COMPRENANT DES BENZODIAZÉPINES À ACTION BRÈVE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **22.05.2012 EP 12168968**

(43) Date of publication of application:
**01.04.2015 Bulletin 2015/14**

(73) Proprietor: **Paion UK Limited**
**Histon, Cambridge CB24 9ZR (GB)**

(72) Inventors:
- **GRAHAM, John Aitken**
**Cambridge**
**Cambridgeshire CB24 5NE (GB)**
- **BAILLIE, Alan James**
**Glasgow**
**Lancashire G84 9HY (GB)**
- **WARD, Kevin Richard**
**Basingstoke**
**Hampshire RG23 7HT (GB)**
- **PEACOCK, Thomas**
**Southampton**
**Hampshire S016 4GB (GB)**

(74) Representative: **von Renesse, Dorothea et al**
**König-Szynka-Tilmann-von Renesse**
**Patentanwälte Partnerschaft mbB**
**Mönchenwerther Str. 11**
**40545 Düsseldorf (DE)**

(56) References cited:
**EP-A1- 1 161 949**
**WO-A1-2008/007071**
**WO-A1-2008/007081**
**WO-A1-2011/054845**
**WO-A1-2013/029431**
**US-A- 3 520 877**
**US-A1- 2006 198 896**

- **GREGORY J. PACOFSKY ET AL: "Relating the Structure, Activity, and Physical Properties of Ultrashort-Acting Benzodiazepine Receptor Agonists", BIOORGANIC & MEDICINAL CHEMISTRY LETTERS, vol. 12, no. 21, 1 November 2002 (2002-11-01), pages 3219-3222, XP055039974, ISSN: 0960-894X, DOI: 10.1016/S0960-894X(02)00513-9**
- **JEFFREY A. STAFFORD ET AL: "Identification and Structure-Activity Studies of Novel Ultrashort-Acting Benzodiazepine Receptor Agonists", BIOORGANIC & MEDICINAL CHEMISTRY LETTERS, vol. 12, no. 21, 1 November 2002 (2002-11-01), pages 3215-3218, XP055039851, ISSN: 0960-894X, DOI: 10.1016/S0960-894X(02)00512-7**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 852 389 B1

- KARYOPHYLLIS C. TSIAKITZIS ET AL: "Novel Compounds Designed as Antistress Agents", JOURNAL OF MEDICINAL CHEMISTRY, vol. 52, no. 22, 26 November 2009 (2009-11-26), pages 7315-7318, XP055039992, ISSN: 0022-2623, DOI: 10.1021/jm901169b
- BAHETI A ET AL: "Excipients used in lyophilization of small molecules", JOURNAL OF EXCIPIENTS AND FOOD CHEMICALS, INTERNATIONAL PHARMACEUTICAL EXCIPIENTS COUNCIL, AMERICAS, UNITED STATES, vol. 1, no. 1, 1 January 2010 (2010-01-01) , pages 41-54, XP002676186, ISSN: 2150-2668
- PATRICK J. CROWLEY, LUIGI G. MARTINI: "Effects of excipients on the stability of medicinal products", chimica Oggi / CHEMISTRY toda , vol. 28, no. 5 1 October 2010 (2010-10-01), pages VII-XIII, XP002684613, Retrieved from the Internet: URL:http://chemistry-today.teknoscienze.com/pdf/crowley_excipents2010.pdf [retrieved on 2012-10-04]
- CROWLEY P J: "Excipients as stabilizers", PHARMACEUTICAL SCIENCE AND TECHNOLOGY TODAY 19990601 GB LNKD- DOI:10.1016/S1461-5347(99)00158-3, vol. 2, no. 6, 1 June 1999 (1999-06-01), pages 237-243, XP002684614, ISSN: 1461-5347
- LEE YUNG-CHI ET AL: "The protective effect of lactose on lyophilization of CNK-20402.", AAPS PHARMSCITECH 2005 LNKD- PUBMED:16353962, vol. 6, no. 1, 2005, pages E42-E48, XP002684615, ISSN: 1530-9932

## Description

The present invention relates to compositions comprising benzodiazepines or pharmaceutically acceptable salts thereof and their use as pharmaceuticals.

Benzodiazepine compounds are known for their capacity to bind to a site on a specific receptor/chloride ion channel complex known as the $GABA_A$ receptor. The binding of the benzodiazepine compound potentiates the binding of the inhibitory neurotransmitter 7-aminobutyric acid (GABA) to the complex, thereby leading to inhibition of normal neuronal function. Therapeutic purposes of the treatment with benzodiazepine compounds are in particular production of sedation or hypnosis, induction of anxiolysis, induction of muscle relaxation, treatment of convulsions or induction and/or maintenance of anesthesia in a mammal. See generally, Goodman and Gilman's The Pharmacological Basis of Therapeutics, Eighth Edition; Gilman, A. G.; Rall, T. W.; Nies, A. S.; Taylor, P., Eds. ; Pergamon Press: New York, 1990; pp. 303-304, 346-358.

Short-acting benzodiazepines that may provide faster recovery profiles have been the subject of clinical investigations (W. Hering et al., Anesthesiology 1996, 189, 85 (Suppl.); J. Dingemanse et al., Br. J. Anaesth 1997, 79, 567-574). Further compounds of interest are disclosed in WO 96/23790, WO 96/20941 and US 5665 718. Other publications that describe benzodiazepinones include E. Manghisi and A. Salimbemi, Boll. Chim. Farm. 1974, 113, 642-644, W. A. Khan and P. Singh, Org. Prep. Proc. Int. 1978, 10,105-111 and J. B. Hester, Jr, et al., J. Med. Chem. 1980, 23, 643-647. Benzodiazepines such as diazepam, lorazepam, and midazolam all undergo metabolism by hepatic-dependent processes. Active metabolites, which are often much more slowly metabolized than the parent drug, can be generated by these hepatic mechanisms in effect prolonging the duration of action of many benzodiazepines (T. M. Bauer et al, Lancet 1995, 346, 145-7). Inadvertent oversedation has been associated with the use of benzodiazepines (A. Shafer, Crit Care Med 1998, 26, 947-956), particularly in the intensive care unit, where benzodiazepines, such as midazolam, enjoy frequent use.

Short-acting benzodiazepines have been further disclosed in WO 2000/69836 A1. The benzodiazepines as disclosed herein comprise a carboxylic acid ester moiety and are inactivated by non-specific tissue esterases.

WO 2008/007071 A1 discloses a highly crystalline besylate salt of a benzodiazepine with a carboxylic acid ester moiety as disclosed in WO 2000/69836 A1. WO 2008/007081 A1 an esylate salt of a benzodiazepine.

Products which are used as sedatives or anesthetic agents are normally stored at room temperature. Therefore there is a need to provide formulations of short-acting benzodiazepines, which exhibit sufficient stability at room temperature. In addition for products that require to be presented as sterile, for example for injection via various routes, the process used to produce these formulations must be capable of being processed in a manner to ensure sterility assurance e.g. aseptic filtration.

It was in particular an object of the present invention to find pharmaceutically acceptable compositions with sufficient stability at room temperature for short-acting benzodiazepines as disclosed in WO 2000/69836 A1, WO 2008/007071 A1 and WO 2008/007081 A1.

Tests which were carried out with these compounds did show that aqueous solutions of the compounds do not have sufficient stability at room temperature and show strong degradation within a short period of time. Therefore alternative approaches had to be found.

A known technique for stabilizing water-labile compounds is the method of lyophilization. However, lyophilising the benzodiazepine of WO 2008/007071 A1 alone did not result in satisfactory stability of this benzodiazepine.

The inventors have now found that stable lyophilized formulations can be obtained, when mixtures of the benzodiazepine with hygroscopic excipients are formulated and/or when the lyophilized formulation is at least in part amorphous. In addition it has been demonstrated that an alternative drying process, namely spray -drying, can be used to get the same effect.

The present invention provides lyophilized or spray-dried compositions comprising a benzodiazepine and at least one pharmaceutically acceptable hygroscopic excipient which is a carbohydrates selected from disaccharides and dextran, a use of a mixture of at least one disaccharide and at least one dextran, and a preparation method has set out in the claims.

The present specification additionally describes a composition comprising a mixture of at least one benzodiazepine or a pharmaceutically acceptable salt thereof and at least one pharmaceutically acceptable hygroscopic excipient, wherein the benzodiazepine comprises at least one carboxylic acid ester moiety.

According to the specification the benzodiazepine is preferably a compound according to formula (I)

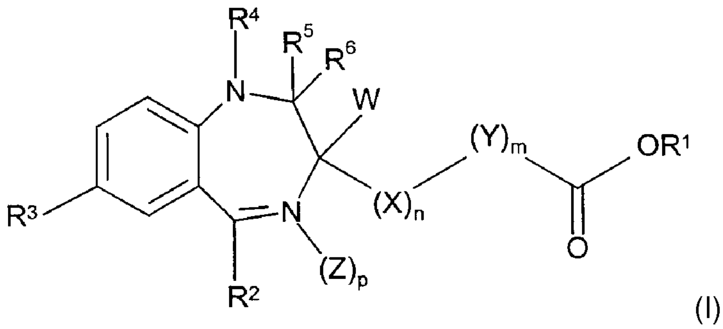

(I)

wherein

W is H, a $C_1$-$C_4$ branched alkyl, or a straight chained alkyl;
X is $CH_2$, NH, or $NCH_3$; n is 1 or 2;
Y is O or $CH_2$; m is 0 or 1;
Z is O; p is 0 or 1;
$R^1$ is a $C_1$-$C_7$ straight chain alkyl, a $C_3$-$C_7$ branched chain alkyl, a $C_1$-$C_4$ haloalkyl, a $C_3$-$C_7$ cycloalkyl, an aryl, a heteroaryl, an aralkyl, or a heteroaralkyl;
$R^2$ is phenyl, 2-halophenyl or 2-pyridyl,
$R^3$ is H, Cl, Br, F, I, $CF_3$, or $NO_2$;

(1) $R^4$ is H, a $C_1$-$C_4$ alkyl, or a dialkylaminoalkyl and $R^5$ and $R^6$ together represent a single oxygen or S atom which is linked to the diazepine ring by a double bond and p is zero or 1; or (2) $R^4$ and $R^5$ together form a double bond in the diazepine ring and $R^6$ represents the group $NHR^7$ wherein $R^7$ is H, $C_{1-4}$ alkyl, $C_{1-4}$ hydroxyalkyl, benzyl or benzyl mono or disubstituted independently with halogen substituents, $C_{1-4}$ alkylpyridyl or $C_{1-4}$ alkylimidazolyl and p is zero; or (3) $R^4$, $R^5$ and $R^6$ form the group $-CR^8$=U-V= wherein $R^8$ is hydrogen, $C_{1-4}$ alkyl or $C_{1-3}$ hydroxyalkyl, U is N or $CR^9$ wherein $R^9$ is H, $C_{1-4}$ alkyl, $C_{1-3}$ hydroxyalkyl or $C_{1-4}$ alkoxy-$C_{1-4}$alkyl, V is N or CH and p is zero.

The term "aryl", alone or in combination, is defined herein as a monocyclic or polycyclic group, preferably a monocyclic or bicyclic group, e.g., phenyl or naphthyl, which can be unsubstituted or substituted, for example, with one or more and, in particular, one to three substituents selected from halogen, $C_{1-4}$ branched or straight chained alkyl, $C_{1-4}$ alkoxy, $C_{1-4}$ haloalkyl, hydroxy, nitro, amino, and the like. The term "heteroaryl" is defined herein as a 5-membered or 6-membered heterocyclic aromatic group which can optionally carry a fused benzene ring and wherein said 5-membered or 6-membered heterocyclic aromatic group can be unsubstituted or substituted, for example, with one or more and, in particular, one to three substituents selected from halogen, $C_{1-4}$ branched or straight chained alkyl, $C_{1-4}$ alkoxy, $C_{1-4}$ haloalkyl, hydroxy, nitro, amino, and the like. The term "alkoxy", alone or in combination, is defined herein to include an alkyl group, which is attached through an oxygen atom to the parent molecular subunit. Exemplary alkoxy groups include but are not necessarily limited to methoxy, ethoxy and isopropoxy. The term "aralkyl" is defined herein as an alkyl group, in which one of the hydrogen atoms is replaced by an aryl group. The term "heteroaralkyl" is defined herein as an alkyl group, in which one of the hydrogen atoms is replaced by a heteroaryl group.

Exemplary branched or straight chained $C_{1-4}$ alkyl groups include but are not necessarily limited to methyl, ethyl, propyl, isopropyl, isobutyl and n-butyl. Exemplary $C_{1-7}$ straight chain alkyl groups include, but are not necessarily limited to, methyl, ethyl, propyl, n-butyl, n-hexyl and n-heptyl. Exemplary $C_{3-7}$ branched chain alkyl groups include, but are not necessarily limited to, isopropyl, isobutyl, sec-butyl, tert-butyl, isopentyl, neopentyl, tert-pentyl and isohexyl. Exemplary $C_{3-7}$ cycloalkyl groups include, but are not necessarily limited to, cyclopropyl, cyclobutyl, cyclopentyl, cyclohexyl and cycloheptyl. Exemplary $C_{1-4}$ haloalkyl groups include, but are not necessarily limited, to methyl, ethyl, propyl, isopropyl, isobutyl and n-butyl substituted independently with one or more halogens, e.g., fluoro, chloro, bromo and iodo.

The compounds of formula (I) where the groups $R^4$ and $R^5$ and $R^6$ together form the group $-CR^8$=U-V= and p is 0 represent a preferred embodiment of the specification and may be conveniently represented by the compound of formula (II):

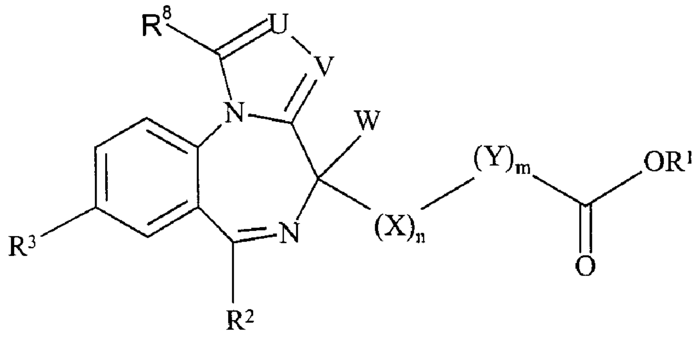

(II)

wherein $R^1$, $R^2$, $R^3$, $R^8$, U, V, W, X, Y, n and m have the meanings given for formula (I).

Further preferred are compounds of formula (I)

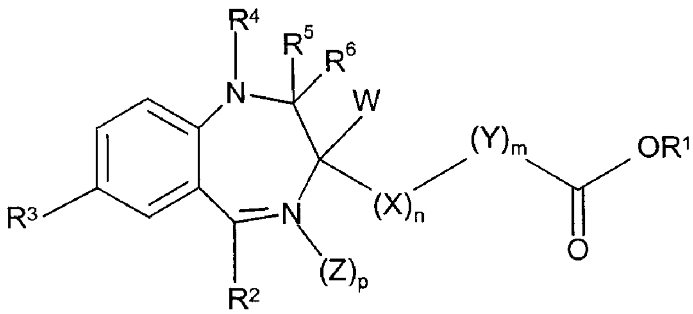

(I)

with

Wis H;
X is $CH_2$; n is 1;
Y is $CH_2$; m is 1;
Z is O; p is 0 or 1;
$R^1$ is $CH_3$, $CH_2CH_3$, $CH_2CH_2CH_3$, $CH(CH_3)_2$ or $CH_2CH(CH_3)_2$;
$R^2$ is 2-fluorohenyl, 2-chlorophenyl or 2-pyridyl;
$R^3$ is Cl or Br;

(1) $R^4$ is H, a $C_1$-$C_4$ alkyl, or a dialkylaminoalkyl and $R^5$ and $R^6$ together represent a single oxygen or S atom which is linked to the diazepine ring by a double bond and p is zero or 1; or (2) $R^4$ and $R^5$ together is a double bond in the diazepine ring and $R^6$ represents the group $NHR^7$ wherein $R^7$ is H, $C_{1-4}$ alkyl, $C_{1-4}$ hydroxyalkyl, benzyl or benzyl mono or disubstituted independently with halogen substituents, $C_{1-4}$ alkylpyridyl or $C_{1-4}$ alkylmidazolyl and p is zero; or (3) $R^4$, $R^5$ and $R^6$ form the group-$CR^8$=U-V= wherein $R^8$ is hydrogen, $C_{1-4}$ alkyl or $C_{1-3}$ hydroxyalkyl, U is N or $CR^9$ wherein $R^9$ is H, $C_{1-4}$ alkyl, $C_{1-3}$ hydroxyalkyl or $C_{1-4}$ alkoxy, V is N or CH and p is zero.

Preferably, in particular in compounds according to formula (II), W is H, X is $CH_2$, n is 1; Y is $CH_2$, m is 1; $R^1$ is $CH_3$, $CH_2CH_3$, $CH_2CH_2CH_3$, $CH(CH_3)_2$ or $CH_2CH(CH_3)_2$; $R^2$ is 2-fluorophenyl, 2-chlorophenyl or 2-pyridyl; $R^3$ is Cl or Br; $R^8$ is H, $CH_3$ or $CH_2OH$; $R^9$ is H, $CH_3$, $CH_2OH$ or $CH_2O$-t-butyl; U is $CR^9$ or N; and V is N or CH.

Particularly preferred amongst these compounds are compounds according to formula (II), wherein in each compound W is H, X is $CH_2$, n is 1, Y is $CH_2$, m is 1 and wherein $R^1$, $R^2$, $R^3$, $R^8$, U and V for each compound are as follows:

| $R^1$ | $R^2$ | $R^3$ | $R^8$ | U | V |
|---|---|---|---|---|---|
| $CH_3$ | 2-fluorophenyl | Cl | H | CH | N |
| $CH_3$ | 2-fluorophenyl | Cl | $CH_3$ | CH | N |
| $CH_3$ | 2-fluorophenyl | Cl | H | C-$CH_3$ | N |

(continued)

| $R^1$ | $R^2$ | $R^3$ | $R^8$ | U | V |
|---|---|---|---|---|---|
| $CH_3$ | 2-fluorophenyl | Cl | H | C-$CH_2OH$ | N |
| $CH_3$ | 2-fluorophenyl | Cl | $CH_2OH$ | CH | N |
| $CH_3$ | 2-pyridyl | Cl | H | CH | N |
| $CH_3$ | 2-pyridyl | Cl | $CH_3$ | CH | N |
| $CH_3$ | 2-pyridyl | Br | $CH_3$ | CH | N |
| $CH_3$ | 2-pyridyl | Br | H | C-$CH_3$ | N |
| $CH_3$ | 2-pyridyl | Cl | H | C-$CH_3$ | N |
| $CH_3$ | 2-pyridyl | Cl | H | C-$CH_2OH$ | N |
| $CH_3$ | 2-pyridyl | Cl | $CH_2OH$ | CH | N |
| $CH_3$ | 2-pyridyl | Cl | $CH_3$ | C-$CH_3$ | N |
| $CH_3$ | 2-chlorophenyl | Cl | $CH_3$ | N | N |
| $CH_3$ | 2-fluorophenyl | Cl | $CH_3$ | N | N |
| $CH_3$ | 2-fluorophenyl | Cl | $CH_3$ | N | N |
| $CH_3$ | 2-fluorophenyl | Cl | H | N | CH |
| $CH_3$ | 2-fluorophenyl | Cl | $CH_3$ | N | CH |
| $CH_3$ | 2-fluorophenyl | Cl | H | C-$CH_2$O-t-butyl | N |
| $CH_3$ | 2-pyridyl | Cl | $CH_3$ | C-$CH_2OH$ | N |

Amongst these compounds the most preferred is remimazolam (INN), wherein W is H, X is $CH_2$, n is 1, Y is $CH_2$, m is 1, $R^1$ is $CH_3$, $R^2$ is 2-pyridyl, $R^3$ is Br, $R^8$ is $CH_3$, U is CH and V is N. According to IUPAC system remimazolam is methyl 3-[(4S)-8-bromo-1-methyl-6-(pyridin-2-yl)-4H-imidazo[1,2-a][1,4]benzodiazepin-4-yl]propanoate. It is clinically developed by PAION AG, Aachen under the internal designation "CNS7056". The besylate form of CNS7056 is also called "CNS7056B" (see the experimental data *infra*).

Compounds according to formula (I) and (II) possess a stereocenter. According to the invention enantiomeric pure forms can be used, which are substantially free of the other enantiomer, but also racemic mixtures can be used.

The composition according to the invention might comprise the free form of the benzodiazepine, but in a preferred embodiment of the invention the benzodiazepine is used in the form of a salt, in particular in the form of an inorganic or organic salt. In a very preferred embodiment the benzodiazepine is used in the salt in a cationic form.

The counter ion of the cationic benzodiazepine is preferably selected from halogenides, in particular fluoride, chloride or bromide, sulfate, organic sulfates, sulfonate, organic sulfonates, nitrate, phosphate, salicylate, tartrate, citrate, maleate, formiate, malonate, succinate, isethionate, lactobionate and sulfamate.

The salts of the invention are obtained by reaction of the benzodiazepine with suitable acids, in particular by reaction with the following acids: hydrochloric, hydrobromic, sulfuric, nitric, phosphoric, salicylic, p-toluenesulfonic, tartaric, citric, methanesulfonic, maleic, formic, malonic, succinic, isethionic, lactobionic, naphtalene-2-sulfonic, sulfamic, ethanesulfonic and benzenesulfonic.

In a preferred embodiment the counter ion is selected from organic sulfates and organic sulfonates, in particular from aromatic sulfates and aromatic sulfonates. In a very preferred embodiment an organic sulfonate is used as counter ion, preferably an aromatic sulfonate, in particular p-toluenesulfonic acid (tosylate), naphthalene-2-sulfonic acid, ethanesulfonic acid (esylate) or benzenesulfonic acid, wherein benzenesulfonic acid (besylate) is the most preferred counter ion.

The most preferred salts according to the invention are the besylate salt (as disclosed in WO 2008/007071 A1) or the esylate salt (as disclosed in WO 2008/007081 A1) of remimazolam. The tosylate of remimazolam is also preferred and is subject matter of WO 2013029431 A1.

The compositions according to one aspect of the specification comprise at least one pharmaceutically acceptable hygroscopic excipient. The hygroscopic excipient might be an organic or inorganic substance, but is preferably an organic substance. The hygroscopic excipient does not include water as such but water can be present in addition to a hygroscopic excipient. The hygroscopic excipient is preferably a compound which is able to form stable hydrates.

The hygroscopic excipient is preferably a substance which under normal conditions (25°C, 1013,25 hPa) binds water molecules reversibly and is preferably further able to release the water molecules, when sufficient vacuum and/or heat is applied. Vice versa the hygroscopic excipient as obtained by application of vacuum and/or heat after dehydration is able to bind water molecules again. Under normal temperature conditions (25°C) the water vapour pressure of the hydrated hygroscopic excipients is preferably less than 23 hPa, more preferably less than 20 hPa, preferentially less than 15 hPa, in particular less than 10 hPa. In a particularly preferred embodiment the water vapour pressure of the hydrated hygroscopic excipient is between 2 and 20 hPa, more preferably between 5 and 15 hPa. The dehydrated hygroscopic excipient preferably has a capacity to bind at least 0.01 g, more preferably at least 0.03 g, in particular at least 0.05 g or at least 1 g water per g of hygroscopic substance. In a preferred embodiment the dehydrated hygroscopic excipient can bind up to 5 g, more preferably up to 10 g, in particular up to 20 g water per g of hygroscopic substance

The organic hygroscopic excipients according to the specification preferably possess a molecular weight of less than 400 kD, preferably less than 350 kD, more preferably less than 100 kD., especially preferably less than 20 kD and further preferably less than1 kD. It is most preferred that the hygroscopic excipient has a molecular weight of less than 0.1 kD.

According to the invention the term "excipient" is defined as an ingredient added intentionally to the drug substance which should not have pharmacological properties in the quantity used. Such excipients can provide some other beneficial purpose be this to aid processing, solubility or dissolution, drug delivery via the target route of administration or aid stability.

Within the context of the present invention, the definition of "pharmaceutically acceptable" is meant to encompass any substance, which does not interfere with effectiveness of the biological activity of the active ingredient and that is not toxic to the host to which it is administered.

In a preferred embodiment of the present specification the organic hygroscopic excipient is selected from carbohydrates and/or organic polymers.

According to the specification a "carbohydrate" is an organic compound with the empirical formula $C_m(H_2O)_n$ (where m could be different from n). Structurally, carbohydrates can be described as polyhydroxy aldehydes and ketones. The term "saccharide" or "sugar" as used hereinafter is a synonym of the term carbohydrate. The carbohydrates are divided into four chemical groups: monosaccharides, disaccharides, oligosaccharides and polysaccharides. The carbohydrates as defined herein encompasses all modifications, derivatives and analogues of carbohydrates such as acidic saccharides containing carboxyl groups, phosphate groups and/or sulfuric ester groups

In a very preferred embodiment of the present specification the organic hygroscopic excipient is a carbohydrate or a mixture of various, at least two types of carbohydrates. Suitable carbohydrates are for example amylose, amylopectin, alginate, dextrans, starches as well as mono-, di- and oligosaccharides.

The inventors have found that the use of hygroscopic carbohydrates or mixtures thereof is especially suitable in order to prepare stable solid formulations/compositions - e.g. lyophilized or spray dried compositions - for benzodiazepines, in particular remimazolam salts, which have a favourable reconstitution time.

Preferably the carbohydrate possesses a molecular weight of less than 150 kD, preferably less than 100 kD, in particular less than 80 kD, especially preferably less than 20 kD and further preferably less than1 kD (e.g. less than 0.5 kD). Preferably oligo- or polysaccharide chains are non-cyclic, i.e. they are not cyclic hemiacetals or hemiketals.

In a particularly preferred embodiment of the present invention dextrans with a molecular weight of less than 150 kD, preferably less than 100 kD, in particular less than 80 kD are used as hygroscopic excipient. Very preferred dextrans possess a molecular weight of between 5 and 80 kD, in particular of between 10 and 40 kD.

In another particularly preferred embodiment of the present specification mono- or oligosaccharides are used as hygroscopic excipients - either as the sole hygroscopic excipient or in a mixture with at least one further hygroscopic excipient, such as dextran - wherein the oligosaccharides are preferably non-cyclic. In this embodiment the carbohydrate is preferably selected from monosaccharides and $C_{2-6}$-oligosaccharides, in particular from disaccharides. The disaccharide is preferably selected from lactose, maltose, sucrose and trehalose and is most preferably lactose. In yet another embodiment two or more disaccharides can be used, in particular including lactose. These disaccharides can be combined with further excipients, e.g. dextran.

In another very preferred embodiment of the present specification, the organic hygroscopic excipient is a polymer, preferably a polyacrylate or a vinylpolymer, more preferably a polyvinylpyrrolidone.

Preferably the polyvinylpyrrolidone possesses a molecular weight of less than 150 kD, preferably less than 100 kD, in particular less than 50 kD. Very preferred polyvinylpyrrolidones possess a molecular weight of between 5 and 40 kD, in particular of between 10 and 30 kD.

In a further embodiment of the specification the hygroscopic excipient is a mixture of at least two different hygroscopic excipients, in particular exactly two, three, four, five or more excipients. These at least two different excipients can be of the same chemical nature e.g. both are carbohydrates or both are organic polymers. Alternatively they can be of different chemical nature, e.g. one or more are carbohydrates and one or more are organic polymers. Preferably the composition comprises a mixture of at least two carbohydrates.

These at least two carbohydrates can be from the same type of carbohydrates, e.g. they all represent monosaccharides, disaccharides, oligosaccharides or polysaccharides, respectively. The carbohydrates can alternatively represent different types of carbohydrates, e.g. one or more monosaccharide combined with one or more disaccharide etc. Preferred is a combination of at least one disaccharide with at least one or more polysaccharide.

Particularly preferred is the combination of one disaccharide and one polysaccharide. The disaccharide preferably is lactose and the polysaccharide is preferably dextran, in particular with a molecular weight of 80 KD or less. The composition preferably contains remimazolam, preferably in besylate, esylate or tosylate salt. Of particular relevance is the besylate salt thereof.

Particular preferred carbohydrate mixtures of the invention comprise or consist of the combinations given in the following table:

| Disaccharide 1 | Disaccharide 2 | Polysaccaride |
|---|---|---|
| Lactose | - | Dextran 40 |
| Trehalose | - | Dextran 40 |
| Sucrose | - | Dextran 40 |
| Lactose | - | Dextran 70 |
| Trehalose | - | Dextran 70 |
| Sucrose | - | Dextran 70 |
| Lactose | Trehalose | Dextran 40 |
| Lactose | Sucrose | Dextran 40 |
| Lactose | Trehalose | Dextran 70 |
| Lactose | Sucrose | Dextran 70 |
| Trehalose | Sucrose | Dextran 40 |
| Trehalose | Sucrose | Dextran 70 |

A composition with the above listed disaccharide/dextran mixture preferably comprise remimazolam, either in its besylate, esylate or tosylate salt. Especially preferred is the besylate salt.

As outlined above the inventors have found that the composition according to the invention, in particular a composition with a mixture of at least one disaccharide such as lactose and at least one dextran can form stable solid, in particular lyophilized or spray dried, formulations with an acceptable lyophilisation (also called "total cycle duration") and/or reconstitution time. According to the invention, a favourable reconstitution time is 5 min or less, preferably 3 minutes or less, more preferably 2 or even 1 minute. A reconstitution time of 1 min is further preferred and a reconstitution time of less than 1 minute is most preferred.

The lyophilisation time for the composition of the invention favourably is less than 120 hours, preferably less than 100 hours, more preferably less than 80 hours and even more preferably less than 70 hours, and specifically 66 hours.

This reduction in lyophilisation time in particular applies when the primary drying step is performed at -25 °C and below 100 mTorr (e.g. between 90 and 100 mTorr) or at - 15 °C or above and 350 to 750 mTorr.

The inventors have found a correlation between the amount of polymer, in particular polysaccharide, more particular dextran, and the time required for the lyophilisation: an increasing amount of polysaccharide in the mixture of carbohydrate excipients increases the collapse temperature of the composition and therewith reduces the time required for lyophilisation. For example a composition with remimazolam salt further comprising lactose and dextran in a weight ratio of 1:1 shows a lyophilisation time of 99 hours, whereas the same composition with a lactose and dextran weight ratio of 1:4 requires a lyophilisation time of only 66 hours or less.

Within the mixtures of excipients (excipients of different chemical nature or of different types, *supra*), the wt.-% ratio between the first excipient (e.g. the disaccharide) and the second excipient (e.g. the dextran) can range from 1:1 to 1:10, more preferably from 1:1 to 1:6, even more preferably from 1:1 to 1:5 and particularly preferably from 1:1.0 to 1:4.5. In a specific embodiment said wt-%ratio is 1:1.5 or 1:4. The first excipient is in particular lactose and the second excipient preferably is dextran, in particular dextran 70 or dextran 40.

Lactose can be used as a hydrate. However, unless otherwise explicity mentioned the weight ratios and concentrations provided herein relate to lactose. The same applies to other excipients suitable according to the invention.

It is especially preferred when the relative amount of polysaccharide in the mixture exceeds the relative amount

of disaccharides therein. Hence 50 wt.-% or more of the mixture of carbohydrates can be a polysaccharide, more preferably 60 wt.-% or more, even more preferred 80 wt.- % or more. In a binary mixture the rest preferably is disaccharide. In these embodiments of the specification it is possible to improve the lyophilisation time, i.e. to obtain higher collapse temperatures. Preferably the polysaccharide is dextran.

The composition of the invention can comprises the benzodiazepine or a salt thereof, being preferably the besylate or tosylate salt of remimazolam, in a relative amount between 5 and 50 wt.%, more preferably in a relative amount between 8 and 25 wt.-%, even more preferably in a relative amount between 10 and 20 wt.%, and specifically in relative amounts of 10 or 19 wt.%. Notably, all relative amounts, weight ratios etc. of the benzodiazepine, in particular remimazolam, in the compositions of the invention are calculated for the free base; unless otherwise explicitly outined.

The composition of the specification can comprises the total amount of hygroscopic excipients, being preferably a carbohydrate or a mixture of carbohydrates, in a relative amount between 50 and 95 wt.%, more preferably in a relative amount between 75 and 92 wt.%, even more preferably in a relative amount between 80 and 90 wt.-%, and specifically in relative amounts of 81 or 90 wt.%.

The wt.% ratio between the total amount of hygroscopic excipients and total amount of benzodiazepines or salts thereof in the composition - calculated for the free base - is preferably at least 1:1, more preferably at least 2:1, 3:1 or 4:1, in particular at least 5:1, 6:1, 7:1 or 9:1. In particularly preferred embodiments the wt.% ratio between the total amount of hygroscopic excipients and the total amount of benzodiazepines or salts thereof, calculated for the free base, in the composition is between 1:1 and 100:1, particularly between 3:1 and 50:1, more preferably between 5:1 and 25:1, most preferably between 7:1 and 15:1, and in the most preferred embodiment at 13:1.

In one embodiment of the specification the composition contains only hygroscopic excipients.

In one aspect the composition of the invention has a collapse temperature above - 20.5°C, preferably above -18° and more preferably above -15.5°C.

In a further aspect the composition of the invention the collapse temperature of the composition is increased by the addition of at least one compound with a collapse temperature above -20°C (collapse temperature modifier).

In a further aspect the composition of the invention further comprises at least one compound with a collapse temperature above -20°C (hereinafter called "collapse temperature modifier"). This component is added to the composition which is then further dried (in particular by lyophilization) to form a solid composition.

The collapse temperature modifier according to the invention can be selected from the group consisting of a sucrose-epichlorhydrin-copolymer (such as Ficoll®), gelatine and hydroxyethyl starch (HES) or dextran. In a preferred embodiment of the invention the collapse temperature modifier is HES. In yet another preferred embodiment of the invention the collapse temperature modifier is dextran.

The collapse temperature modifier can be present within the composition of the invention in a relative amount from 1 to 75 wt.%, more preferably in a relative amount from 5 to 50 wt.%, even more preferably in a relative amount from 10 to 40wt.%. The collapse temperature modifier can be identical with the hygroscopic excipient.

The term "collapse temperature" as used in the context of the present invention relates to the temperature at which softening of a solid composition (the "cake") progresses to structural "collapse", a phenomenon that can be observed by Freeze Drying Microscopy (FDM). For a crystallizing system, collapse occurs if the lowest eutectic melting temperature (Teu) is exceeded. For a non-crystallizing system, the collapse temperature is determined by the glass transition temperature (Tg), which can be e.g. measured using differential scanning calorimetry (DSC). The determination of the collapse temperature is common knowledge of the skilled person. Hence, for an amorphous substance the collapse temperature is given by its glass transition temperature.

Hence the term "collapse" in particular relates to a loss of the integral structure of the solid composition (cake) and/ or to a reduction of its volume of at least about 10%, 25%, 50%, 75%, 85%, 95% or 100%. The reduction in volume and the loss of structural integrity can be measured using known methodologies, including but not limited to visual inspection or Brunauer-Emmett-Teller (BET) surface area analysis.

In one embodiment of the specification the counter ion can render the salt hygroscopic. Hence in this embodiment the salt of the benzodiazepine constitutes also the excipient.

In another embodiment of the invention the compositions according to the invention might comprise besides the at least one hygroscopic excipient further pharmaceutically acceptable carriers and/or excipients. The further carriers and/or excipients must, if used, of course be acceptable in the sense of being compatible with the other ingredients of the formulation and must not be deleterious to the patient. Accordingly, the present invention provides in a further embodiment a composition as hereinbefore defined and further pharmaceutically acceptable carriers and/or excipients. The further carrier and/or excipient for example might be selected from ascorbic acid, glycine, glycine hydrochloride, sodium chloride, sugar alcohols, and mixtures thereof. In a preferred embodiment the further excipient is selected from sugar alcohols, in particular $C_{3-6}$ sugar alcohols, more preferably $C_6$ sugar alcohols.

In the context of the invention a sugar alcohol (also known as a polyol, polyhydric alcohol or polyalcohol) is defined as a hydrogenated form of carbohydrate, whose carbonyl group (aldehyde or ketone, reducing sugar) has been reduced to a primary or secondary hydroxal group).

The present specification also describes a composition comprising at least one benzodiazepine or a pharmaceutically acceptable salt thereof, wherein at least parts of the composition are amorphous and wherein the benzodiazepine comprises at least one carboxylic acid ester moiety. The composition can also contain crystalline parts/compounds.

The present specification further describes a composition which comprises a mixture of said benzodiazepine with at least one hygroscopic excipient, wherein said composition is at least in parts amorphous, but it can also contain crystalline parts.

The compositions according to the specification are in a preferred embodiment solid composition, in particular obtained by lyophilization or spray drying. The dried composition contains at least one compound (e.g. the excipient) in amorphous form. In a preferred embodiment the lyophilized composition consists of a mixture of amorphous and crystalline, in particular microcrystalline, parts/compounds. In a preferred embodiment the crystalline part of the lyophilized solids comprises or preferentially substantially consists of the benzodiazepine compounds or salts thereof.

In a further embodiment of the invention, at least 50% (w/w), preferably at least 75% (w/w), more preferably at least 90% (w/w) and most preferably at least 95% (w/w) of the benzodiazepine within the composition is in an amorphous state. In preferred embodiment of the invention, at least 96%, 97%, 88% or 99% (w/w) of the benzodiazepine within the composition is in an amorphous state. In a preferred embodiment of the invention, the composition is amorphous for at least 96%, 97%, 88% or 99%.

In one embodiment of the invention the composition contains a mixture of crystalline and amorphous benzodiazepine or the benzodiazepine salt. In one embodiment at least 25 %, 50-75 % or greater than 90% (w/w) of the total benzodiazepine or the benzodiazepine salt of the composition is crystalline.

In the most preferred embodiment of the invention the benzodiazepine salt is remimazolam besylate. When the composition contains crystalline remimazolam besylate, in one embodiment, the crystalline polymorph (herein designated besylate Form 1) exhibits an X-ray powder diffraction (XRPD) pattern which comprises a characteristic peak at about 7.3, 7.8, 9.4, 12.1, 14.1, 14.4, 14.7, or 15.6 degrees two-theta.

Preferably the besylate Form 1 of remimazolam crystalline polymorph exhibits an XRPD pattern which comprises characteristic peaks at about 7.3, 7.8, 9.4, 12.1, 14.1, 14.4, 14.7, and 15.6 degrees two-theta.

More preferably the besylate Form 1 crystalline polymorph exhibits an XRPD pattern which comprises characteristic peaks at: 7.25 (10.60), 7.84 (72.60), 9.36 (12.10), 12.13 (32.50), 14.06 (48.50), 14.41 (74.30), 14.70 (50.70), 15.60 (26.90) [angle two-theta degrees (percentage relative intensity)].

Preferably the besylate Form 1 crystalline polymorph has a differential scanning calorimetry (DSC) onset melting temperature in the range 187-204°C, preferably about 191-192°C.

The structure of a 2-methoxyethanol:pentyl acetate grown needle habit crystal of Form 1 has been resolved at 190K (R factor of 6.3, example 9 of WO2008/007071 A1). Form I has a stoichiometry of 1:1 compound:besylate. Its crystallographic asymmetric unit contains two independent compound molecules and two besylate molecules. The two independent compound molecules are singly protonated on the imidazole ring. The crystal structure has unit cell dimensions of a = 7.6868 Å, b = 29.2607 Å, c = 12.3756 Å, $\alpha$ = 90°, $\beta$ = 97.7880°, $\gamma$= 90°, and a space group of $P2_1$. The crystal structure further features the following parameters: system: monoclinic, volume: 2757.86 Å, density: 1.439 g $cm^{-3}$, absorption: 1.610 $\mu$ [MoK$\alpha$] ($mm^{-1}$), F(000): 1224. The Flack "Enantiopole" parameter was determined as 0.03. The crystal structure is also described in more detail in Example 9 of WO2008/007071 A1, and crystallographic coordinates are given in Figure 5A to 5D (corresponding to table 17 of WO2008/007071 A1). Bond lengths and angles for Form 1 are given in Figures 7A-B and 8A-C, respectively (corresponding to Tables 19 and 20 of WO2008/007071 A1).

According to the invention the composition can comprise a besylate salt of remimazolam which is a crystalline polymorph comprising a crystal with unit cell dimensions of a = 7.6868 Å, b = 29.2607 Å, c = 12.3756 Å, $\alpha$ = 90°, $\beta$ = 97.7880°, $\gamma$= 90°.

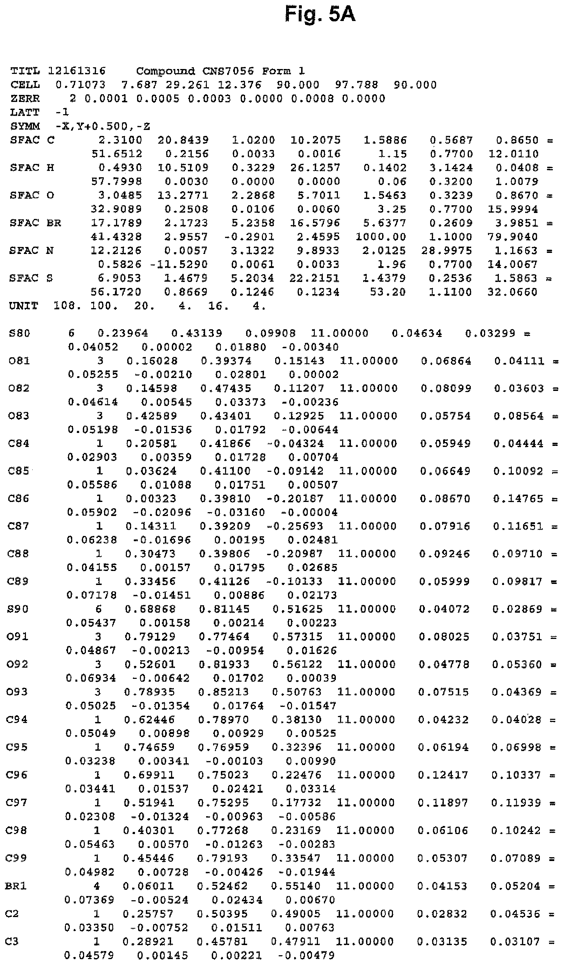

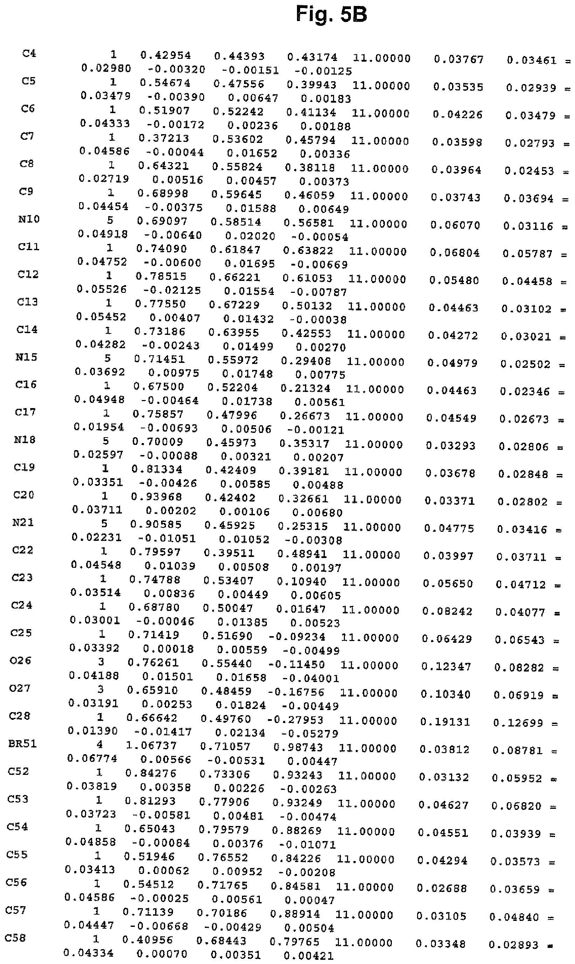

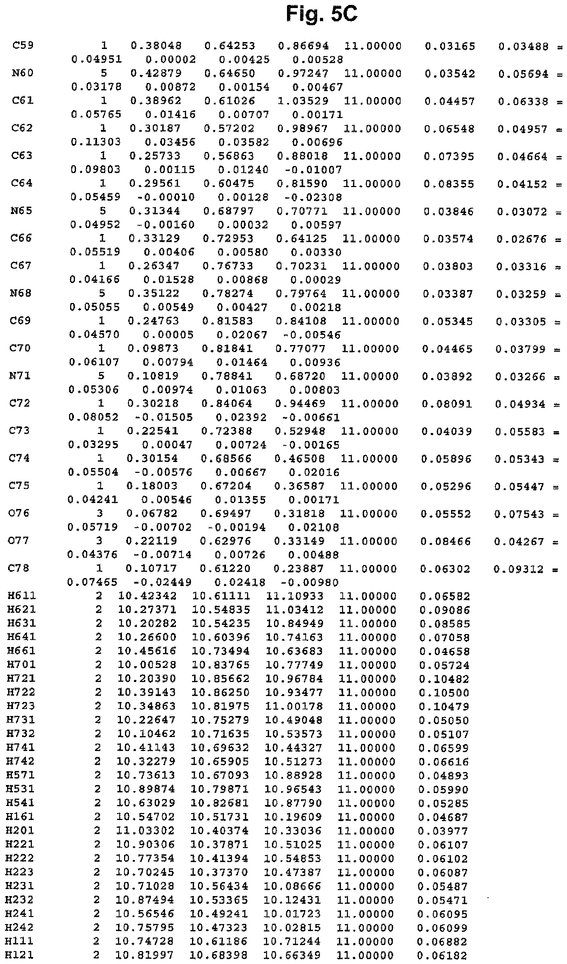

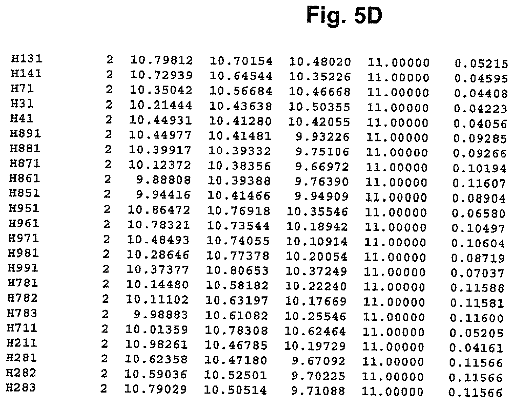

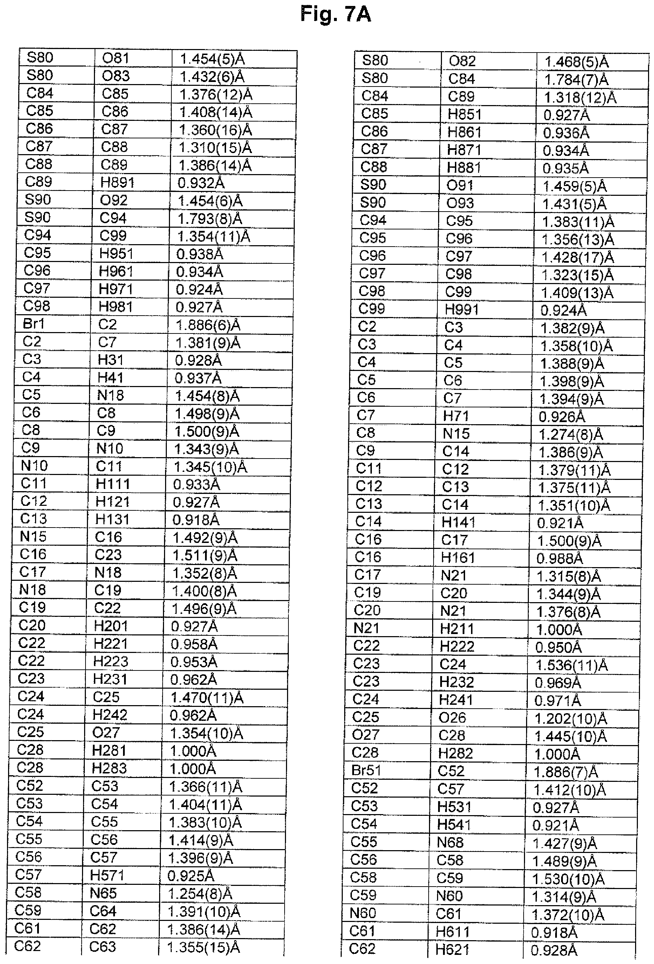

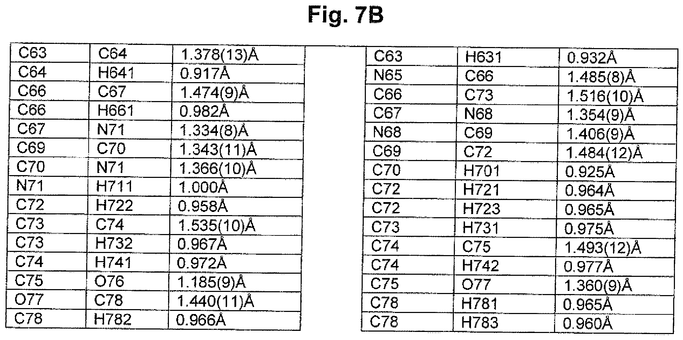

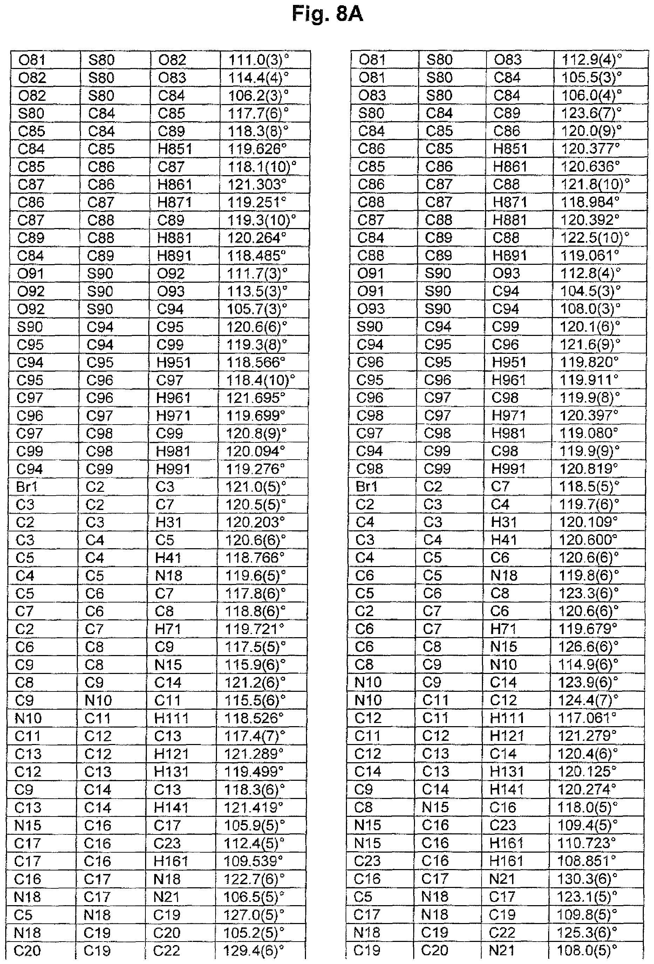

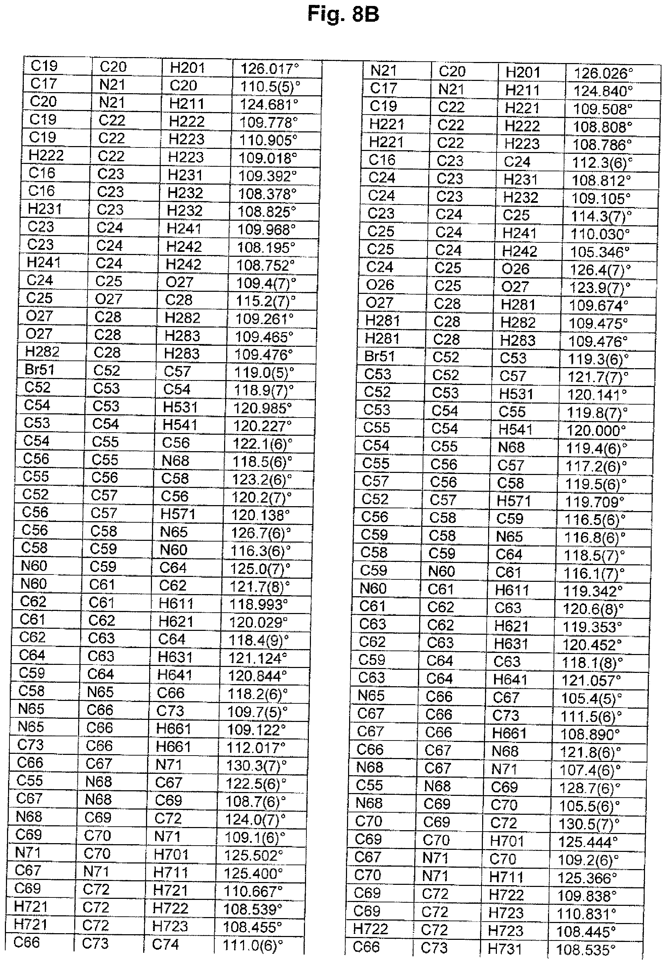

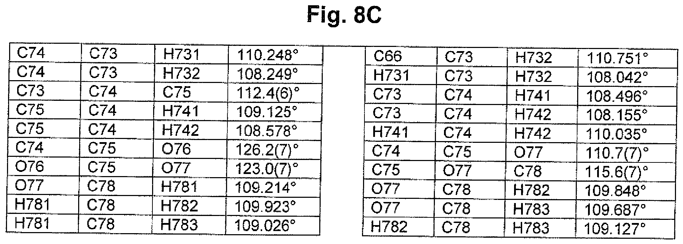

There is also provided according to the invention a composition with a besylate salt of remimazolam which is a crystalline polymorph having a crystal structure defined by the structural coordinates as shown in Figure 5A-D. The crystalline form preferably has a bond lengths and angles as shown in Figures 7A-B and 8A-C, respectively.

In a further embodiment the composition according to the invention comprises a polymorph of the besylate salt of remimazolam (herein designated besylate Form 2), that exhibits an XRPD pattern which comprises a characteristic peak at about 8.6, 10.5, 12.0, 13.1, 14.4, or 15.9 degrees two-theta. Preferably the besylate Form 2 crystalline polymorph exhibits an XRPD pattern which comprises characteristic peaks at about 8.6, 10.5, 12.0, 13.1, 14.4, and 15.9 degrees two-theta.

More preferably the besylate Form 2 crystalline polymorph exhibits an XRPD pattern which comprises characteristic peaks at: 8.64 (17.60), 10.46 (21.00), 12.03 (22.80), 13.14 (27.70), 14.42 (11.20), 15.91 (100.00) [angle two-theta degrees (percentage relative intensity)].

Preferably the besylate Form 2 crystalline polymorph has a differential scanning calorimetry (DSC) onset melting temperature in the range 170-200°C, preferably about 180°C.

The structure of an ethanol:ethyl acetate grown plate habit crystal of Form 2 has been resolved at 190K (R

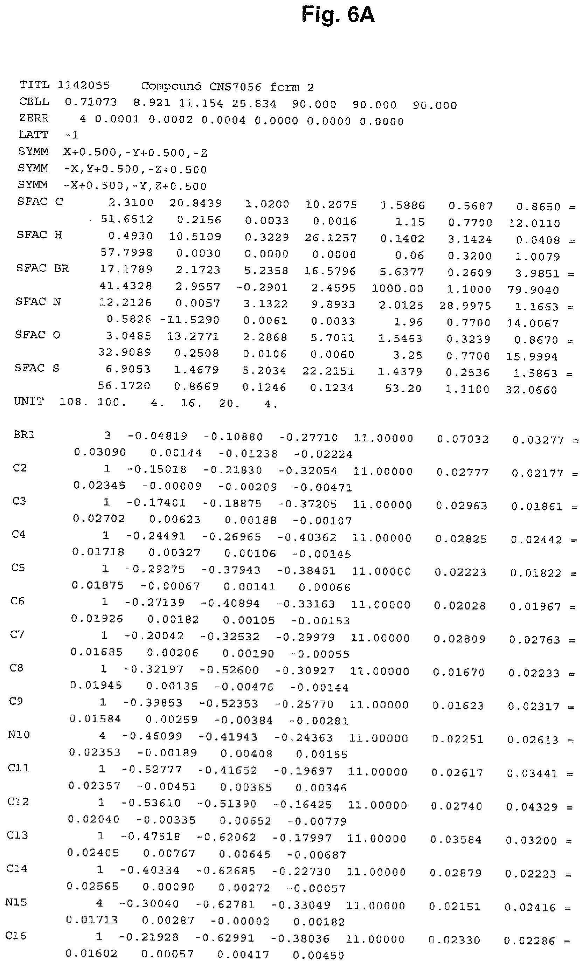

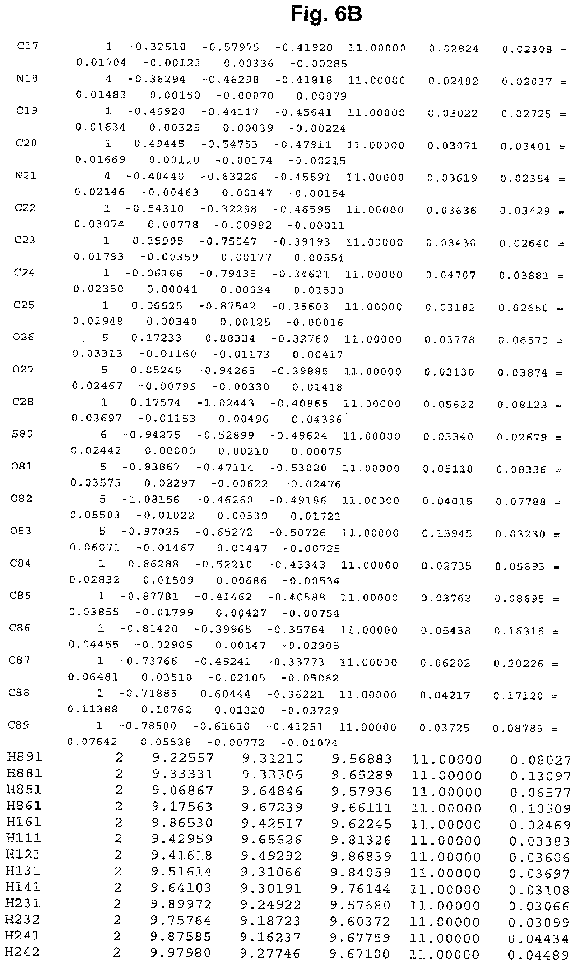

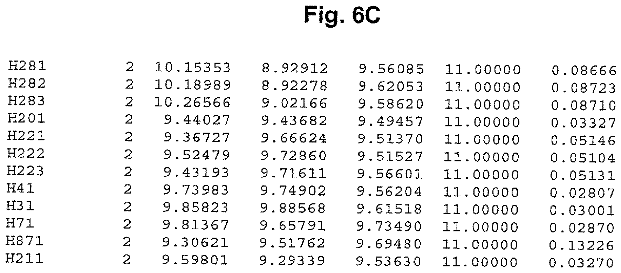

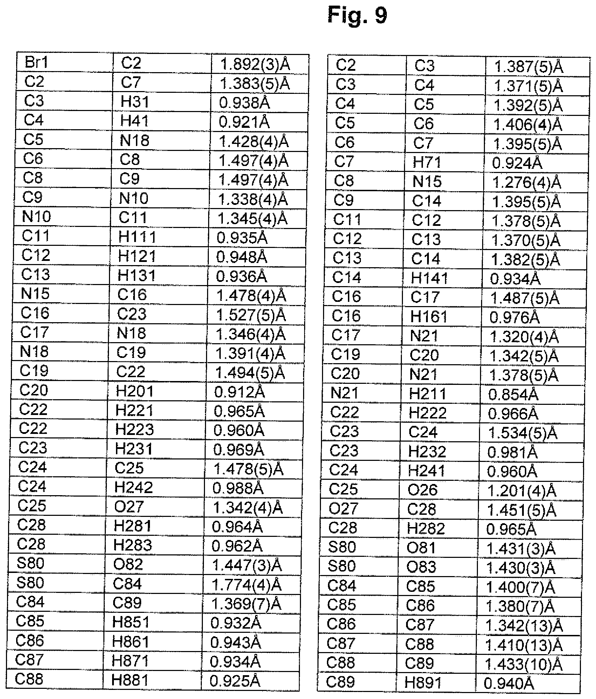

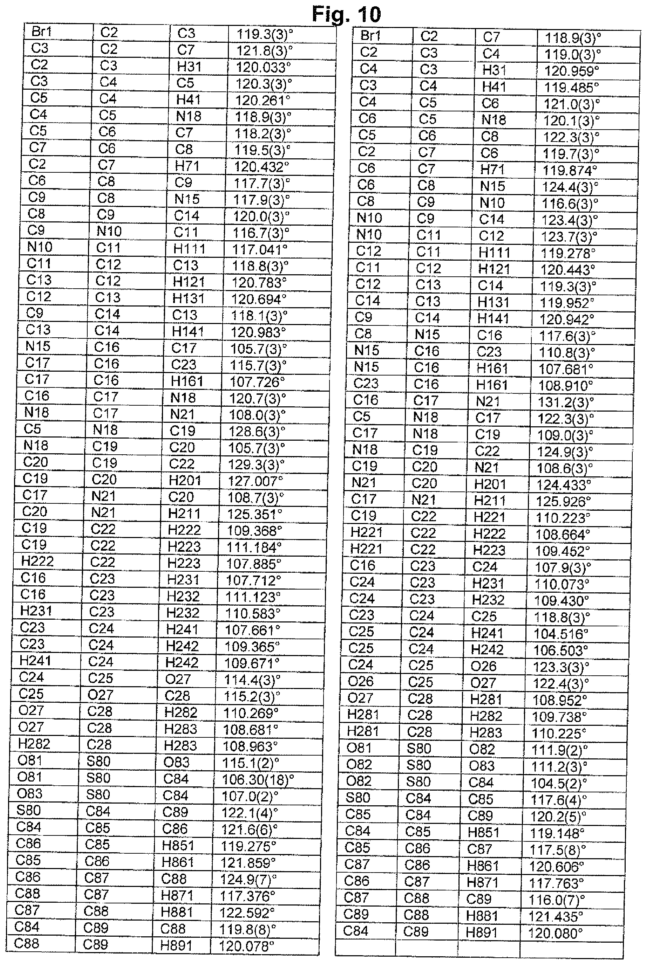

factor of 3.8, example 10 of WO2008/007071 A1). Form 2 has stoichiometry of 1:1 compound:besylate. Its crystallographic asymmetric unit contains one compound molecule and one besylate molecule. The compound molecule is singly protonated on the imidazole ring. The crystal structure has unit cell dimensions of a = 8.92130 Å, b = 11.1536 Å, c = 25.8345 Å, $\alpha$ = 90°, $\beta$ = 90°, $\gamma$= 90°, and a space group of $P2_12_12_1$. The crystal structure further features the following parameters: system: orthorhombic, volume: 2570.65 Å, density: 1.544 g $cm^{-3}$, absorption: 1.727 $\mu$ [MoK$\alpha$] ($mm^{-1}$), F(000): 1224. The Flack "Enantiopole" parameter was determined as 0,011. The crystal structure is described in more detail in Example 10 of WO2008/007071 A1, and crystallographic coordinates are given in Figure 6A-C (corresponding to Table 18 of WO2008/007071 A1). Bond lengths and angles for Form 2 are given in Figures 9 and 10, respectively (corresponding to Tables 21 and 22 of WO2008/007071 A1).

According to the invention there is provided a composition with a besylate salt of remimazolam, which is a crystalline polymorph comprising a crystal with unit cell dimensions of a = 8.92130 A, b = 11.1536 Å, c = 25.8345 A, $\alpha$ = 90°, $\beta$ = 90°, $\gamma$= 90°.

There is also provided according to the invention a composition with a besylate salt of remimazolam which is a crystalline polymorph having a crystal structure defined by the structural coordinates as shown in Figure 6A-C. There is further provided according to the invention a composition with a besylate salt of remimazolam with bond lengths and angles as shown in Figures 9 and 10, respectively.

There is further provided according to the invention a composition with a crystalline polymorph of a besylate salt of remimazolam (herein designated besylate Form 3), that exhibits an X-ray powder diffraction (XRPD) pattern which comprises a characteristic peak at about 7.6, 11.2, 12.4, 14.6, 15.2, 16.4, or 17.7 degrees two-theta. Preferably the besylate Form 3 crystalline polymorph exhibits an XRPD pattern which comprises characteristic peaks at about: 7.6, 11.2, 12.4, 14.6, 15.2, 16.4, and 17.7 degrees two-theta.

More preferably the besylate Form 3 crystalline polymorph exhibits an XRPD pattern which comprises characteristic peaks at: 7.61 (65.70), 11.19 (33.20), 12.38 (48.70), 14.63 (30.60), 15.18 (33.20), 16.40 (29.60), 17.68 (51.30) [angle two-theta degrees (percentage relative intensity)].

Preferably the besylate Form 3 crystalline polymorph has a differential scanning calorimetry (DSC) onset melting temperature in the range 195-205°C, preferably about 200-201°C.

There is further provided according to the invention a composition with a crystalline polymorph of a besylate salt of remimazolam (herein designated besylate Form 4), that exhibits an XRPD pattern which comprises a characteristic peak at about 7.6, 10.8, 15.2, 15.9, or 22.0 degrees two-theta. Preferably the besylate Form 4 crystalline polymorph exhibits an XRPD pattern which comprises characteristic peaks at about: 7.6, 10.8, 15.2, 15.9, and 22.0 degrees two-theta.

Preferably the besylate Form 4 crystalline polymorph exhibits an XRPD pattern which comprises characteristic peaks at: 7.62 (83.50), 10.75 (14.70), 15.17 (37.80), 15.85 (28.70), 22.03 (100) [angle two-theta degrees (percentage relative intensity)].

Preferably the besylate Form 4 crystalline polymorph has a differential scanning calorimetry (DSC) onset melting temperature in the range 180-185°C, preferably about 182°C.

The besylate Forms 1 to 4 may be prepared and crystallised by using the methods and solvents disclosed in WO 2008/007071 A1.

A preferred salt is the besylate Form 1 based on the robustness of formation, yield, purity and chemical and solid form stability.

In one embodiment of the invention the composition comprises a mixture of Forms 1, 2, 3 and 4. However compositions with only one of the Forms 1 to 4 are possible.

In another preferred embodiment of the invention the benzodiazepine salt is remimazolam esylate. When the composition contains crystalline remimazolam esylate, in one embodiment, the crystalline polymorph (herein designated esylate Form 1) exhibits an X-ray powder diffraction (XRPD) pattern which comprises a characteristic peak at about 6.2, 9.2, 12.3, 15.0, 17.2, or 20.6 degrees two-theta.

Preferably the esylate Form 1 crystalline polymorph exhibits an XRPD pattern which comprises characteristic peaks at about 6.2, 9.2, 12.3, 15.0, 17.2, and 20.6 degrees two-theta.

More preferably the esylate Form 1 crystalline polymorph exhibits an XRPD pattern which comprises characteristic peaks at: 6.17 (19.30), 9.21 (20.50), 12.28 (16.40), 14.97 (23.40), 17.18 (52.80), 20.63 (100.00) [angle two-theta degrees (percentage relative intensity)].

Preferably the esylate Form 1 crystalline polymorph has a differential scanning calorimetry (DSC) onset melting temperature in the range 195-205°C, preferably about 201-202°C.

There is further provided according to the invention a crystalline polymorph of an esylate salt of a compound of formula (I) (herein designated esylate Form 2) that exhibits an X-ray powder diffraction (XRPD) pattern which comprises a characteristic peak at about 3.6, 6.4, 7.1, 12.3, 14.1, or 17.1 degrees two-theta.

Preferably the esylate Form 2 crystalline polymorph exhibits an XRPD pattern which comprises characteristic peaks at about 3.6, 6.4, 7.1, 12.3, 14.1, and 17.1 degrees two-theta.

More preferably the crystalline polymorph exhibits an XRPD pattern which comprises characteristic peaks at:

3.57 (15.60), 6.42 (21.10), 7.13 (58.30), 12.29 (51.50), 14.10 (58.90), 17.13 (68.00) [angle two-theta degrees (percentage relative intensity)].

Preferably the esylate Form 2 crystalline polymorph has a differential scanning calorimetry (DSC) onset melting temperature in the range 185-195°C, preferably about 190-191°C.

The esylate Forms 1 and 2 may be prepared and crystallised by using the methods and solvents disclosed in WO 2008/007081 A1

A preferred salt is the esylate Form 1 based on the robustness of formation, yield, purity and chemical and solid form stability.

In one embodiment of the invention the composition comprises a mixture of Forms 1, and 2. However compositions with only one of the Forms 1 or 2 are possible.

The lyophilized form of the composition according to the invention is preferably used for storage of the compositions.

The solid form of the compositions, in particular the lyophilized or spray dried solids, preferably show very good storage stability. In a preferred embodiment, they show degradation of the benzodiazepine, in particular hydrolysis of the carboxylic ester moiety, of less than 1 % during storage for 13 weeks, in particular at storage conditions of 40°C/75%RH.

The solid, in particular lyophilized or spray dried, compositions according to the invention in a preferred embodiment maintain a room temperature shelf life of at least one year, more preferably of at least two years, in particular of at least three years. They further comprise in a preferred embodiment less than 5 wt.% of water, preferably less than 2 wt.% of water, more preferably less than 1 wt.% of water.

In the solid, in particular lyophilized or spray dried, compositions according to the specification the total amount of benzodiazepines or salts thereof and hygroscopic excipients preferably sums up to at least 50 wt.%, more preferably at least 70 wt.%, in particular at least 90 wt.% of the composition.

In another preferred embodiment the compositions according to the specification are in liquid form, more preferably aqueous solutions. The liquid form is on the one hand used for the preparation of the lyophilized or spray dried solids, and on the other hand obtained by solubilization of the lyophilized or spray dried solids when transforming the lyophilized composition into a suitable pharmaceutically applicable solution.

The liquid contains the reconstituted solid benzodiazepine as free base preferably in an amount of between 0.5 and 30 mg/ml, more preferably in an amount of between 1 and 20 mg/ml, in particular in an amount of between 2 and 10 mg/ml.

Further subject of the present specification is a pharmaceutical comprising a composition according to the invention.

Subject of the specification is therefore also a method of manufacturing a composition or pharmaceutical composition according to the specification, wherein the composition or pharmaceutical composition is in the solid state, comprising the following steps:

a) providing a solution comprising at least one benzodiazepine with at least one carboxylic acid ester moiety or a pharmaceutically acceptable salt thereof (in particular remimazolam salt) as hereinbefore described and at least one pharmaceutically acceptable hygroscopic excipient or mixtures of at least two hygroscopic excipients as hereinbefore described, wherein the solution is preferably an aqueous solution and wherein the solution preferably possesses a pH of between 2 and 7, preferably 2 and 5 and more preferably 2 and 4;
b) lyophilizing the solution according to (a).

Preferably the lyophilisation time of step b) is less than 120 hours, preferably less than 100 hours, more preferably less than 80 hours and even more preferably less than 70 hours, and specifically 66 hours or even lower.

Preferably the solid composition resulting from step b) is reconstituted to a liquid pharmaceutical composition in a further step c). Reconstituting the solid composition as of step b) favourably is possible in less than 5 min, less than 3 min, most favourably is less than 1 minute. For reconstitution physiological saline (0.9 wt% sodium chloride) can be used.

In yet another embodiment of the specification the lyophilisation of step b) can be replaced by spray-drying.

Further subject of the specification is therefore also a method of providing a composition or pharmaceutical according to the specification, wherein the composition or pharmaceutical is in the liquid state, comprising the step of solubilizing a composition according to the specification, wherein the starting composition is in the solid, preferably lyophilized or spray dried, state and wherein the starting composition is preferably at least in part amorphous. Solubilization of the solid, preferably lyophilized or spray dried, composition is preferably carried out with water, an aqueous solution of dextrose or saline solutions.

The composition according to the invention, in particular the pharmaceutical, is preferably presented in unit dosage forms such as ampoules or disposable injection devices like syringes. It may also be presented in multi-dose forms such as a bottle or vial, from which the appropriate dose may be withdrawn. All such formulations should be sterile.

In a preferred embodiment of the specification the ampoules, injection devices and multi-dose forms contain the composition according to the specification, in particular the pharmaceutical, in solid, preferably lyophilized or spray dried, form, and the compositions are transformed into ready-to-use pharmaceuticals by solubilization of the compositions only shortly before their use.

The formulations according to the invention include those suitable for oral, rectal, topical, buccal (e.g. sublingual) and parenteral (e.g. subcutaneous, intramuscular, intradermal or intravenous) administration. It is preferred to present compositions of the present invention in the form of a pharmaceutical formulation for parenteral administration, most preferable for any type of injection, in particular for intravenous, intraarterial, intralumbar, intraperitoneal, intramuscular, intradermal, subcutaneous or intraosseal injection.

Where the pharmaceutical formulation is for parenteral administration, the formulation may be an aqueous or non-aqueous solution or mixture of liquids, which may contain bacteriostatic agents, antioxidants, buffers or other pharmaceutically acceptable additives. The preferred formulation of compositions of the present invention is either an aqueous acidic medium of pH 2-7, preferably 2-5 and more preferably 2-4 or an aqueous solution of a cyclodextrin. Cyclodextrins that can be used for these formulations are either the negatively charged sulfobutylether (SBE) derivatives of β-CD, specifically SBE7-β-CD, marketed under the tradename Captisol by CyDex, Inc. (Critical Reviews in Therapeutic Drug Carrier Systems, 14 (1), 1-104 (1997)), or the hydroxypropyl CD's. The preferred method of formulation (i.e., acid buffer or CD-based) may depend on the physicochemical properties (e.g., aqueous solubility, pKa, etc.) of a particular composition. When the composition is in the solid, in particular lyophilized, state, the solid is correspondingly preferably solubilized before its application in either an aqueous acidic medium preferably resulting in a pH 2-4 of the solution or in an aqueous solution of a cyclodextrin.

According to one embodiment of the specification a solid pharmaceutical composition is provided. This composition can comprise 5 to 25% wt.% of remimazolam salt, preferably besylate salt, preferably 8 to 23 wt.%, even more preferred 10 to 19 wt.%.

This composition can further comprise 75 to 95 wt.% of one or more hygroscopic excipients, preferably 77 to 92 wt.% and more preferably 81 to 90 wt.%. The hygroscopic excipients preferably is a mixture of carbohydrates, comprising at up to 40% lactose, 38 wt.%, more preferably up to 33 wt.% disaccharide, preferably lactose. The rest of the mixture can be dextran.

In one embodiment of the specification the solid composition as outlined above contains no further excipients. In yet another embodiment the solid composition consists of remimazolam salt, dextran and a dissaccharide (e.g. lactose) only. In yet another embodiment the sormulation consits only of remimazolam salt and lactose (this might be presented as a hydrate).

In another embodiment of the specification the composition is a liquid composition consisting of remimazolam, dextran, a disaccharide and a solvent, which preferably is physilogical saline (0.9 wt.% sodium chloride). The pH value of such liquid (aqueous) composition, being preferably reconstituted from the solid composition, can range from about 3 to about 4, preferably from about 3.2 to about 3.3 and more preferably from 3.21 to 3.28.

Accordingly, the present specification also provides a method for producing sedation or hypnosis in a mammal, which comprises administering to the mammal an effective sedative or hypnotic amount of a pharmaceutical of the present invention as hereinbefore defined. The present specification also provides a method for inducing anxiolysis in a mammal, which comprises administering to the mammal an effective anxiolytic amount of a pharmaceutical of the present invention as hereinbefore defined. The present specification also provides a method for inducing muscle relaxation in a mammal, which comprises administering to the mammal an effective muscle relaxant amount of a pharmaceutical of the present invention as hereinbefore defined. The present specification also provides a method for treating convulsions in a mammal, which comprises administering to the mammal an effective anticonvulsant amount of a pharmaceutical of the present invention as hereinbefore defined. The present specification also provides a method for inducing or maintaining anesthesia in a mammal, which comprises administering to the mammal an effective anesthetic amount of a pharmaceutical of the present invention as hereinbefore defined.

The present specification also provides the use of a sedative or hypnotic amount of a composition of the present invention as hereinbefore defined in the manufacture of a medicament for producing sedation or hypnosis in a mammal, including in a human. The present specification also provides the use of an anxiolytic amount of a composition of the present invention as hereinbefore defined in the manufacture of a medicament for producing anxiolysis in a mammal, including in a human. The present specification also provides the use of a muscle relaxant amount of a composition of the present invention as hereinbefore defined in the manufacture of a medicament for producing muscle relaxation in a mammal, including in a human. The present specification also provides the use of an anticonvulsant amount of a composition of the present invention as hereinbefore defined in the manufacture of a medicament for treating convulsions in a mammal, including in a human. The present specification also provides the use of an anesthetic amount of a composition of the present invention as hereinbefore defined in the manufacture of a medicament for inducing or maintaining anesthesia in a mammal, including in a human.

The present specification also provides the use of a pharmaceutical according to the invention for producing

sedation or hypnosis and/or inducing anxiolysis and/or inducing muscle relaxation and/or treating convulsions and/or inducing or maintaining anaesthesia in a mammal.

Intravenous administration can take the form of bolus injection or, more appropriately, continuous infusion. The dosage for each subject may vary, however, a suitable intravenous amount or dosage of the compounds of the present specification to obtain sedation or hypnosis in mammals would be 0.01 to 5.0 mg/kg of body weight, and more particularly, 0.02 to 0.5 mg/kg of body weight, the above being based on the weight of the compound which is the active ingredient (i.e. the weight of the benzodiazepine). A suitable intravenous amount or dosage of the compounds of the present specification to obtain anxiolysis in mammals would be 0.01 to 5.0 mg/kg of body weight, and more particularly, 0.02 to 0.5 mg/kg of body weight, the above being based on the weight of the compound which is the active ingredient. A suitable intravenous amount or dosage of the compounds of the present specification to obtain muscle relaxation in mammals would be 0.01 to 5.0 mg/kg of body weight, and more particularly, 0.02 to 0.5 mg/kg of body weight, the above being based on the weight of the compound which is the active ingredient. A suitable intravenous amount or dosage of the compounds of the present specification to treat convulsions in mammals would be 0.01 to 5.0 mg/kg of body weight, and more particularly, 0.02 to 0.5 mg/kg of body weight, the above being based on the weight of the compound which is the active ingredient. Thus a suitable pharmaceutical parenteral preparation for administration to humans will preferably contain 0.1 to 20 mg/ml of a compound of the present specification in solution or multiples thereof for multi-dose vials.

The present specification also describes the use of a mixture of at least one disaccharide and at least dextran for preparing a solid composition comprising at least one benzodiazepine comprising at least one carboxylic acid ester moiety or a pharmaceutically acceptable salt thereof, which is preferably a remimazolam salt (particularly its besylate or tosylate salt). Preferably the mixture contains or consists of lactose and dextran, preferably a dextran with 80 kD or less (e.g. dextran 40 or dextran 70). The solid composition has a favourable reconstitution time.

Particularly, the specification relates to the following embodiments:

In the embodiment 1, the specification relates to a composition comprising at least one benzodiazepine comprising at least one carboxylic acid ester moiety or a pharmaceutically acceptable salt thereof, wherein the composition

a) comprises at least one pharmaceutically acceptable hygroscopic excipient, and /or
b) the composition is at least in part amorphous.

Embodiment 2 relates to a composition according to embodiment 1, wherein the benzodiazepine is a compound according to formula (I)

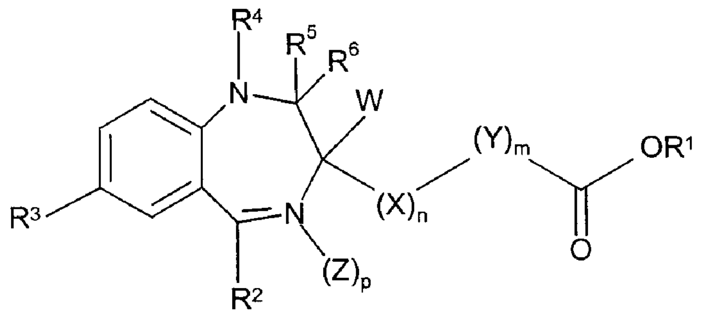

(I)

wherein

W is H, a $C_1$-$C_4$ branched or straight chain alkyl;
X is $CH_2$, NH, or $NCH_3$; n is 1 or 2;
Y is O or $CH_2$; m is 0 or 1;
Z is O; p is 0 or 1;
$R^1$ is a $C_1$-$C_7$ straight chain alkyl, a $C_3$-$C_7$ branched chain alkyl, a $C_1$-$C_4$ haloalkyl, a $C_3$-$C_7$ cycloalkyl, an aryl, a heteroaryl, an aralkyl, or a heteroaralkyl;
$R^2$ is phenyl, 2-halophenyl or 2-pyridyl,
$R^3$ is H, Cl, Br, F, I, $CF_3$, or $NO_2$;

(1) $R^4$ is H, a $C_1$-$C_4$ alkyl, or a dialkylaminoalkyl and $R^5$ and $R^6$ together represent a single oxygen or S atom which is linked to the diazepine ring by a double bond and p is zero or 1; or (2) $R^4$ and $R^5$ together

is a double bond in the diazepine ring and $R^6$ represents the group $NHR^7$ wherein $R^7$ is H, $C_{1-4}$ alkyl, $C_{1-4}$ hydroxyalkyl, benzyl or benzyl mono or disubstituted independently with halogen substituents, $C_{1-4}$alkylpyridyl or $C_{1-4}$ alkylmidazolyl and p is zero; or (3) $R^4$, $R^5$ and $R^6$ form the group-$CR^8$=U-V= wherein $R^8$ is hydrogen, $C_{1-4}$ alkyl or $C_{1-3}$ hydroxyalkyl, U is N or $CR^9$ wherein $R^9$ is H, $C_{1-4}$ alkyl, $C_{1-3}$ hydroxyalkyl or $C_{1-4}$ alkoxy, V is N or CH and p is zero.

Embodiment 3 relates to a composition according to embodiment 2, wherein p is zero and $R^4$, $R^5$ and $R^6$ form the group -$CR^8$=U-V= wherein $R^8$ is hydrogen, $C_{1-4}$ alkyl or $C_{1-3}$ hydroxyalkyl, U is N or $CR^9$ wherein $R^9$ is H, $C_{1-4}$ alkyl, $C_{1-3}$ hydroxyalkyl or $C_{1-4}$ alkoxy, V is N or CH.

Embodiment 4 relates to a composition according to embodiment 2 or 3, wherein W is H; X is $CH_2$, n is 1; Y is $CH_2$, m is 1;
$R^1$ is $CH_3$, $CH_2CH_3$, $CH_2CH_2CH_3$, $CH(CH_3)_2$ or $CH_2CH(CH_3)_2$;
$R^2$ is 2-fluorophenyl, 2-chlorophenyl or 2-pyridyl;
$R^3$ is Cl or Br.

Embodiment 5 relates to a composition according to any of embodiments 2 to 4, wherein p is zero and $R^4$, $R^5$ and $R^6$ form the group -$CR^8$=U-V= wherein $R^8$ is methyl, U is $CH_2$, V is N;
W is H; X is $CH_2$, n is 1; Y is $CH_2$, m is 1;
R1 is $CH_3$; R2 is 2-pyridyl; R3 is Br.

Embodiment 6 relates to a composition according to any of the embodiments 1 to 5, wherein the benzodiazepine is in the form of a pharmaceutically acceptable salt.

Embodiment 7 relates to a composition according to any of the embodiments 1 to 6, wherein in the pharmaceutically acceptable salt the benzodiazepine is formulated in cationic form and the counter ion is selected from halogenides, in particular fluoride, chloride or bromide, sulfate, organic sulfates, sulfonate, organic sulfonates, nitrate, phosphate, salicylate, tartrate, citrate, maleate, formiate, malonate, succinate, isethionate, lactobionate and sulfamate.

Embodiment 8 relates to a composition according to embodiment 7, wherein the counter ion is selected from organic sulfates and sulfonates, in particular aromatic sulfates and sulfonates.

Embodiment 9 relates to a composition according to embodiment 8, wherein the counter ion is benzene sulfonate (besylate).

Embodiment 10 relates to a composition according to embodiment 9, wherein the benzodiazepine salt is crystalline remimazolam besylate.

Embodiment 11 relates to a composition according to any of the embodiments 1 to 10, wherein the hygroscopic excipient is a compound which is able to form stable hydrates.

Embodiment 12 relates to a composition according to any of the embodiments 1 to 11, wherein the hygroscopic excipient is an organic substance, preferably selected from carbohydrates and/or organic polymers.

Embodiment 13 relates to a composition according to embodiment 12, wherein the hygroscopic excipient possesses a molecular weight of less than 150 kD.

Embodiment 14 relates to a composition according to embodiment 12 or 13, wherein the carbohydrate is a dextran molecule.

Embodiment 15 relates to a composition according to embodiment 12 or 13, wherein the carbohydrate is selected from monosaccharides and $C_{2-6}$-oligosaccharides.

Embodiment 16 relates to a composition according to embodiment 15, wherein the carbohydrate is a disaccharide, preferably selected from the group consisting of lactose, maltose, sucrose and trehalose.

Embodiment 17 relates to a composition according to embodiment 12, wherein the organic polymer is a polyvinylpyrrolidone and preferably possesses a molecular weight of between 5 and 40 kD.

Embodiment 18 relates to a composition according to any of the embodiments 1 to 17, wherein the wt.% ratio between the total amount of hygroscopic excipients and the total amount of benzodiazepines or salts thereof in the composition is at least 1:1, preferably at least 2:1, most preferably at least 5:1.

Embodiment 19 relates to a composition according to any of the embodiments 1 to 18, wherein the composition is in the solid state and is preferably a lyophilized solid.

**I. STABILITY OF CNS7056; FOMULATIONS WITH SELECTED EXCIPIENTS**

**1. Formulations**

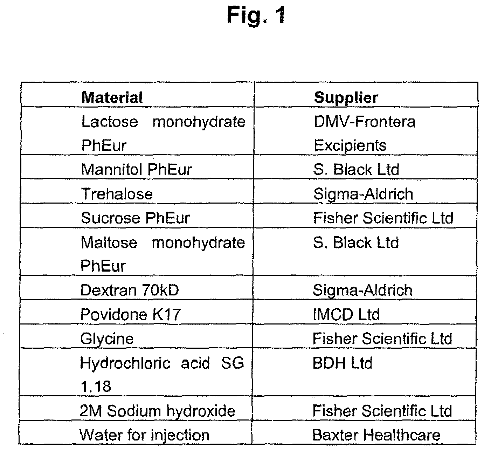

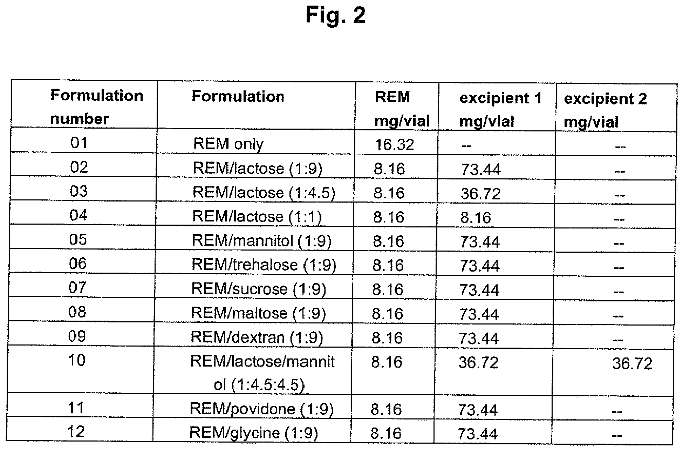

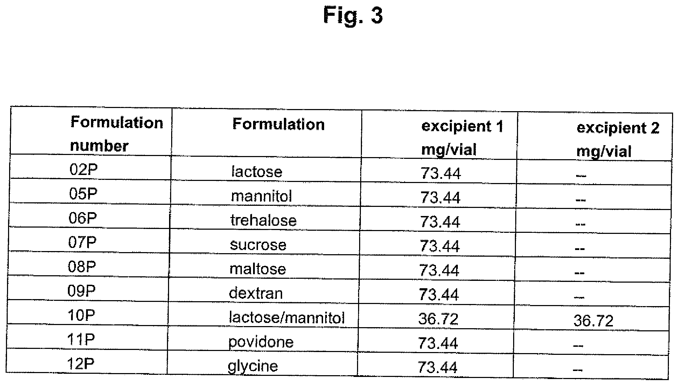

A total of 11 formulations of the besylate salt of remimazolam with a selection of suitable excipients as detailed in Figure 1 and 2 were lyophilized. In addition a formulation containing the besylate salt of remimazolam alone and matching placebos for each formulation were also prepared (see Figure 3). In the following the abbreviation "REM" is used for the besylate salt of remimazolam.

Each formulation was prepared as follows and filled into vials prior to freeze drying: Exicipient was dissolved in approximately 50 ml water. REM was added and stirred to dissolve. Once dissolved the pH of the solutions was adjusted to 3.10 ± 0.05 with 0.5 M hydrochloric acid/ 2 M sodium hydroxide. Placebo solutions and the solution containing REM alone were prepared in the same manner. Each solution was made up to 100 ml and 1.2 ml of each solution was aliquoted into 2 ml vials. The formulations were lyophilized using a Virtis Genesis 25 EL freeze dryer according to the following cycle:

Freezing steps

| Step | Temperature (°C) | Time (minutes) | Pressure (mTorr) | Hold/Ramp |
|---|---|---|---|---|
| 1 | 4 | 10 | -- | H |
| 2 | -45 | 490 | -- | R |
| 3 | -45 | 170 | -- | H |

Drying steps

| Step | Temperature (°C) | Time (minutes) | Pressure (mTorr) | Hold/Ramp |
|---|---|---|---|---|
| 1 | -45 | 10 | 100 | H |
| 2 | -25 | 200 | 100 | R |
| 3 | -25 | 3640 | 100 | H |
| 4 | 30 | 275 | 70 | R |
| 5 | 30 | 1300 | 70 | H |

After freeze drying the samples were stored in storage cabinets at 25°C/60%RH and at 40°C/75%RH for 13 weeks, respectively. ("RH" means relative humidity.)

Analysis

a) Reconstitution time

After 13 weeks of storage the vials were reconstituted in duplicate with 1.2 ml of water for irrigation/injection and swirled gently to mix. The time taken for complete dissolution was recorded.

b) HPLC

For HPLC each vial was reconstituted with sample solvent (50/50 % v/v acetonitrile/water) and the contents transferred to a 25 ml volumetric flask (with exception of REM only vial, which was transferred to a 50 ml volumetric flask) with several rinsings. The dextran formulation was insoluble in sample solvent and was diluted in 100% water.

For each formulation a placebo was also analysed in the same way. Analyses were performed in duplicate, unless otherwise stated.

Results

a) Reconstitution time

Reconstitution time was acceptable for all samples.

b) HPLC

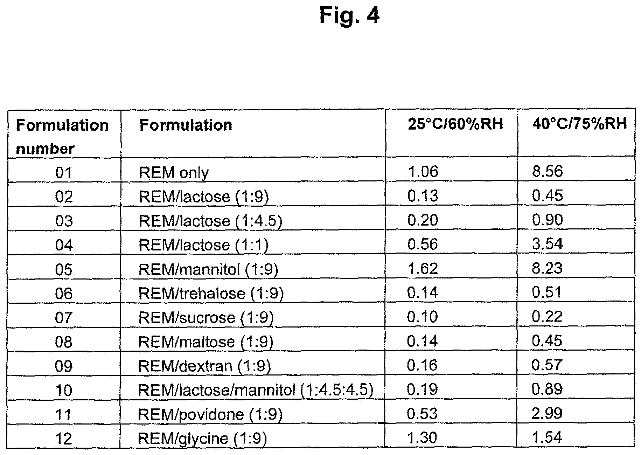

The investigation of the emergence of the hydrolysis degradant of REM (results are summarized in Figure 4), which is formed by hydrolysis of the ester bond, revealed that the sample with REM only as well as the sample containing mannitol, which is a commonly used excipient for lyophilization of pharmaceuticals, exhibited only poor stability of REM, showing a degradation after 13 weeks at storage conditions of 40°C/75%RH of more than 8 %.

Samples containing glycine showed moderate degradation, whereas all samples containing hygroscopic excipients (carbohydrates or polyvinylpyrrolidone) showed good or excellent stability. In particular the samples containing carbohydrates (disaccharides or dextran) showed excellent stability, i.e., a degradation after 13 weeks at storage conditions of 40°C/75%RH of less than 1 %.

The samples with differing amounts of lactose revealed that the greater the amount of carbohydrate relative to the amount of REM, the better the stability of the REM. Further by incorporating a carbohydrate (e.g. lactose) as a component of a formulation of CNS 7056 that is inherently unstable e.g. mannitol it is possible to improve the stability of this formulation.

**2. Stability data of CNS 7056: lactose based formulation batches after storage for up to 36 months**

**2.1 Introduction**

CNS 7056 is presented for clinical use as a sterile lyophilized powder for reconstitution in 20 mL vials with a Bromobutyl stopper, suitable for intravenous injection. Each vial contains 26 mg of CNS 7056. During development further batches with 25, 23 and 26 mg of CNS 7056 have been prepared. On reconstitution with a defined volume of Water for Injection, the concentration of the dose solution is 5mg/mL CNS7056. All these products contain the same CNS 7056 to lactose ratio in the lyophilized product (i.e. 1:13 CNS 7056: lactose monohydrate). Stability data were collected for all intervals through the month shown in bold in the following Table 1:

Table 1: Summary of Stability studies for CNS 7056 batches

| **Summary of CNS 7056 for Injection on Stability** | | | |
|---|---|---|---|
| **Batch** | **Storage Conditions** | **Type of Batch** | **Test Interval (Months)** |
| A01P310 | 25 °C/60% RH | GMP clinical batch, stability | 0, 1, 3, 6, 9, 12, **18**, 24, 36[c], 48[c] |
| | 30 °C/65% RH | | 0, 1, 3, 6, 9, 12 |
| | 40 °C/75% RH | | 0, 1, 3, **6** |
| P310-01 (B) | 25 °C/60% RH | Lab development batch, stability | 0, 1, 3, 6, 9, 12, **18** |
| | 30 °C/65% RH | | 0, 6, 9, 12 |
| | 40 °C/75% RH | | 0, 1, 3, 6 9, **12** |
| 025CNS27 | 25 °C/60% RH | Lab development batch, stability | 0, 9, 2, 3, 6, 9, 12, **18**[ab] |
| | 40 °C/75% RH | | 0, 1, 2, 3, 6, **12**[b] |
| 026CNS27 | 25 °C/60% RH | Lab development batch, stability | 0, 1, 2, 3, 6, 9, 12, **18**[ab] |
| | 40 °C/75% RH | | 0, 1, 2, 3, **6**[b] |
| G384 | 25 °C/60% RH | Lab development batch, stability | 0, 1, 2, 3, 6, 9, 12, **18**[ab] |
| | 40 °C/75% RH | | 0, 1, 2, 3, **6**[b] |

(continued)

| Summary of CNS 7056 for Injection on Stability | | | |
|---|---|---|---|
| **Batch** | **Storage Conditions** | **Type of Batch** | **Test Interval (Months)** |
| P02308 | 25 °C/60% RH | GMP clinical batch, stability | 0, 1, 2, 3, 6, 9, 12, 18, 24, **36**[c], 48[c] |
| | 40 °C/75% RH | | 0, 1, 2, 3, 6, 9[a], **12** |
| [a] = Stability test interval added. Test vials taken from remaining spare vials at specified temperature.<br>[b] = Stability studies on 025CNS27, 026C0NS27 and G384 are now complete<br>[c] = Optional time points | | | |

**2.1.1 CNS 7056 batch composition**

Table 2 Composition of the different CNS7056 batches

| Substance | Batch # | | | | | |
|---|---|---|---|---|---|---|
| | 025CNS27 | 026CNS27 | G384 | P02308 | P310-01 (B) | A01P310 |
| CNS7056 base | 25 mg | 25 mg | 25 mg | 23 mg | 26 mg | 26 mg |
| Lactose monohydrate | 433 mg | 433 mg | 433 mg | 398 mg | 450.3 mg | 450.3 mg |
| 0.12 M NaOH/0.12 M HCl | qs to pH 3.1 | qs to pH 3.1 | qs to pH 3.1 | qs to pH 3.1 | qs to pH 3.1 | qs to pH 3.1 |
| qs = to add sufficiently quantity to | | | | | | |

**2.1.2 Freeze drying conditions**

The freeze drying conditions for the batches are given in the following tables 3 to 7:

Table 3: Freeze drying cycle for batch 025CNS27:

| Step | Process | Shelf temperature (°C) | Ramp rate (°C/min) | Hold time (min) | Pressure (mTorr) |
|---|---|---|---|---|---|
| 1 | Load | 4 | 0 | 30 | n/a |
| 2 | Freezing | -45 | 0.1 | 180 | n/a |
| 3 | Primary drying | -25 | 0.1 | 1700 | 100 |
| 4 | Secondary drying | 30 | 0.2 | 1300 | 75 |
| 5 | Finish | Vials stoppered under 95% pure nitrogen | | | |

Table 4: Freeze drying cycle for batch 026CNS27:

| Step | Process | Shelf temperature (°C) | Ramp rate (°C/min) | Hold time (min) | Pressure (mTorr) |
|---|---|---|---|---|---|
| 1 | Load | 4 | 0 | 120 | n/a |
| 2 | Freezing | -45 | 0.1 | 300 | n/a |
| 3 | Primary drying | -30 | 0.1 | 2885 | 100 |
| 4 | Primary drying | -25 | 0.2 | 4100 | 100 |
| 4 | Secondary drying | 30 | 0.2 | 1580 | 75 |
| 5 | Finish | Vials stoppered under 95% pure nitrogen | | | |

Table 5: Freeze drying cycle for batch G384:

| Step | Process | Shelf temperature (°C) | Ramp rate (°C/min) | Hold time (min) | Pressure (mTorr) |
|---|---|---|---|---|---|
| 1 | Load | 4 | 0 | 10 | n/a |
| 2 | Freezing | -45 | 0.1 | 300 | n/a |
| 3 | Primary drying | -25 | 0.1 | 3640 | 100 |
| 4 | Secondary drying | 30 | 0.2 | 1125 | 70 |
| 5 | Finish | Vials stoppered under 95% pure nitrogen | | | |

Table 6: Freeze drying cycle for batch P02308:

| Step | Process | Shelf temperature (°C) | Ramp rate (°C/min) | Hold time (min) | Pressure (mTorr) |
|---|---|---|---|---|---|
| 1 | Load | 4 | 0 | 60 | n/a |
| 2 | Freezing | -45 | 0.1 | 180 | n/a |
| 3 | Primary drying | -25 | 0.1 | 3640 | 100 |
| 4 | Secondary drying | 30 | 0.2 | 1300 | 75 |
| 5 | Finish | Vials stoppered under 95% pure nitrogen | | | |

Table 7: Freeze drying cycle for batch A01 P310:

| Step | Process | Shelf temperature (°C) | Ramp rate (°C/min) | Hold time (min) | Pressure (mTorr) |
|---|---|---|---|---|---|
| 1 | Load | 4 | 0 | 60 | n/a |
| 2 | Freezing | -45 | 0.1 | 210 | n/a |
| 3 | Primary drying | -25 | 0.1 | 3640 | 100 |
| 4 | Secondary drying | 30 | 0.2 | 1300 | 75 |
| 5 | Finish | Vials stoppered under 95% pure nitrogen | | | |
| [a] = Includes 30 min for condenser preparation | | | | | |

**2.2 Methods of analysis**

**2.2.1 Appearance of Lyophilized Product**

The same CNS 7056 vials (6 at each storage condition) were inspected visually, the appearance recorded and the vials placed back on storage. A comparison was also made to a set of vials that had been stored in the same secondary packaging at 2 to 8°C to assess whether thee were any differences (in particular in colour) between these controls and those stored at elevated temperatures.

**2.2.2 CNS7056 vial content, concentration on reconstitution and related substances**

The CNS7056 assay and related substances determination was determined by HPLC). For this purpose, the appropriate volume of WFI was added to each vial and swirled until complete dissolution was achieved. The seal and the stopper were carefully removed and the stopper was rinsed thoroughly into a 100 ml volumetric flask. The contents of the vial with washings of diluent were transferred to a volumetric flask. The diluent was added to reach a volume of 100 mL (equals a concentration of 0.23, 0.25 or 0.26 mg/mL, respectively). The sample were analysed by HPLC by using the following conditions:

| | |
|---|---|
| Column: | YMC ODS-AQ, 250 x 4.6 mm, 3 μm particle size |
| Mobile phase: | Res A: 0.01% trifluoroacetic acid in water |

(continued)

Res B: 0.01% trifluoroacetic acid in acetonitrile

Gradient:

| Time (mins) | %A | %B |
|---|---|---|
| 0 | 75 | 25 |
| 20.0 | 60 | 40 |
| 30.0 | 20 | 80 |
| 32.0 | 20 | 80 |
| 32.5 | 75 | 25 |
| 40.0 | 75 | 25 |

| | |
|---|---|
| Flow rate: | 1.0 ml/min |
| Column temperature: | 40°C |
| Autosampler: | ambient |
| Detection: | UV at 230 nm |
| Injection volume: | 10μl |
| Run time: | 40 min |

The retention time for CNS7056 is about 15 minutes. The CNS 7056 content was assayed by comparison with similarly chromatographed reference solutions. Related substances were determined by normalised area %.

The concentration of the reconstituted solution is calculated by the following equation:

$$\text{CNS7056 base (mg)} = \left(\frac{\text{sample peak area}}{\text{mean peak area std}_1\text{ throughout run}}\right) \times \frac{\text{Wt std}_1}{50} \times \frac{\text{MWt CNS 7056 base}}{\text{MWt CNS 7056B}} \times \frac{P}{100} \times DF$$

where

Wt std l is the weight of CNS 7056B reference material used to prepared standard 1 (mg)
MWt CNS 7056 base is the molecular weight of the free base of CNS 7056 = 439.3
MWt CNS 7056B is the molecular weight of CNS7056 besylate salt = 597.5
P is the declared assay as per the C of A for the reference standard
DF is the dilution factor

The vial content is calculated according to the following formula:

$$\text{CNS7056 base (mg)} = \left(\frac{\text{sample peak area}}{\text{mean peak area std}_1\text{ throughout run}}\right) \times \frac{\text{Wt std}_1}{50} \times \frac{\text{MWt CNS 7056 base}}{\text{MWt CNS 7056B}} \times \frac{P}{100} \times DF$$

where

Wt std 1 is the weight of CNS 7056B reference material used to prepared standard 1 (mg)
MWt CNS 7056 base is the molecular weight of the free base of CNS 7056 = 439.3
MWt CNS 7056B is the molecular weight of CNS7056 besylate salt = 597.5
P is the declared assay as per the C of A for the reference standard
DF is the dilution factor

For determination of related substances CNS7056 is identified by comparison of the retention time to that of CNS7056 in the reference standard chromatograms. The amount of each individual detected related substance is calculated as area percent for each sample injection according to the following formula:

$$\text{Area\%} = \left(\frac{A}{T}\right) \times 100$$

Where,

A = area of the related substance peak
T = total area of all peaks in the chromatogram

### 2.2.3 Chiral purity

The chiral purity of CNS 7056 was determined by HPLC by using the following conditions:

| | |
|---|---|
| Column: | Chiralpak IC, 250 x 4.6 mm, 5μm particle size |
| Mobile phase: | phosphate buffer pH 7.0/water/acetonitrile 10/40/50, v/v/v |
| Sample solvent: | water/acetonitrile 50/50, v/v |
| Flow rate: | 0.7 ml/min |
| Column temperature: | 40°C |
| Autosampler: | ambient |
| Detection: | UV at 250 nm |
| Injection volume: | 10 μl |
| Run time: | 35 minutes |

The retention time for the CNS 7056 S-enantiomer is about 21.3 min and for CNS 7056 R-enantiomer is about 17.8 min (RRT = 0.84).The chiral purity is calculated according to the following formula:

$$\text{Area\%} = \left(\frac{A}{T}\right) \times 100$$

where

A = area of R - CNS 7056 peak
T = total area of the CNS 7056 and the R - CNS 7056 peak

### 2.2.4 Volume of solution in vial following reconstitution (Ph.Eur 2.9.17)

A single vial was reconstituted with 5.0 ml of Water for Injection (WFI), Ph. Eur. using a 5 ml BD syringe fitted with a suitable needle. When fully reconstituted, the contents were removed using a syringe and 21 gauge needle and transferred to a calibrated 10 ml measuring cylinder.

### 2.2.5 Appearance of reconstitution

The appearance of the solution following reconstitution was recorded.

### 2.2.6 Reconstitution time

Two vials were reconstituted with 5.0 mL of Water for Injection (WFI), Ph. Eur., using a 5 ml BD syringe and appropriate needle, and the time taken to fully dissolve recorded.

### 2.2.7 pH value

The pH was determined on two reconstituted solutions following addition of 5.0 mL Water for Injection (WFI), Ph. Eur., using a 5 ml BD syringe fitted with a suitable needle. The pH was measured on one aliquot from each of the two vials.

### 2.2.8 Osmolality

The osmolality was determined on the two reconstituted solutions following addition of 5.0 mL Water for Injection (WFI), Ph. Eur., using a 5 ml BD syringe fitted with a suitable needle. Osmolality was measured on one aliquot from each of the two vials by freezing point depression with reference to a solution of known Osmolality. For this purpose 100μl of the reconstituted CNS 7056 solution is measured in a freezing point depression osmometer.

### 2.2.9 Water content

The water content was determined by coloumetric Karl Fischer titration. The moisture content of vials of CNS 7056 drug product is determined by dissolving the entire contents of a vial of CNS 7056 lyophilised powder in anhydrous dimethylformamide (DMF) and injecting a known volume of the solution into the anolyte of a coloumetric Karl Fischer apparatus. In the Karl Fischer reaction, water reacts in a 1:1 ratio with iodine. The amount of water is determined by measuring the number of coulombs of electricity required to oxidise iodide ions to the iodine required for the Karl Fischer reaction. The number of Coulombs is used to calculate the amount of water titrated in μg, which is displayed by the apparatus.

The following equipment and reagents were used:

| | |
|---|---|
| Karl-Fischer titratus apparatus: | Mitsubishi CA-100 |
| Anolyte: | Hydranal Coulomat AG |
| Catholyte: | Hydranal Coulomat CG |

The water content of CNS7056 lyophilised powder is calculated according the following formula:

$$\text{Moisture per vial (mg)} = \frac{(\text{Msample} - \text{Msolvent})}{1000} \times \frac{(\text{Wsolvent/DSolvent})}{\text{Vtitration}}$$

$$\text{Moisture, \%w/w} = \frac{\text{Moisture per vial} \times 100}{(\text{Svial})}$$

Where:

| | |
|---|---|
| Msample | = amount of water in the sample solution added to the titration vessel (μg) |
| Msolvent | = mean amount of water in the sample blanks added to the titration vessel (μg) |
| Wsolvent | = weight of DMF added to the vial (g) |
| Dsolvent | = density of the solvent (g/ml) |
| | For DMF d=0.944 g/ml, source CRC handbook $81^{st}$ edition |
| Vtitration | = volume of solution added to the titration vessel (ml) |
| Svial | = Calculated total weight of solid per vial, including water (mg) |

### 2.2.10 ID by UV

Analysis was performed in duplicate on a single vial. The identification by UV was confirmed by comparison of the drug product spectra to reference spectra.

### 2.2.11 Sub visible particles (EP 2.9.19)

Ten vials were reconstituted with 5 mL WFI using an appropriate sterile syringe and needle. The vials were

pooled together under aseptic conditions and analysed according to European Pharmacopeia 2.9.19.

### 2.2.12 Sub visible particles (EP 2.9.19)

Ten vials were reconstituted with 5 mL WFI using an appropriate sterile syringe and needle. The vials were pooled together under aseptic conditions and analysed according to European Pharmacopeia 2.9.19.

### 2.2.13 Bacterial Endotoxin

Bacterial Endotoxin was determined by the Limulus amebocyte lysate (LAL) gel-clot method as a limit test with a limit of < 0.5 EU/mg. For this purpose a LAL with declared sensitivity equal to 0.03 EU/ml is used. The Endotoxins are quantified using the following formula:

$$\textit{Endotoxin concentration in the sample (EU/mg)} = \frac{\text{lysate sensitivity } (\lambda) \text{ x test dilution factor}}{\textit{sample concentration}}$$

where:

lysate sensitivity = 0.03 EU/ml

sample concentration = 5 mg/ml

### 2.2.14 Sterility

Sterility was determined by reconstituting the lyophilised CNS 7056 with 5 ml of sterile peptonate water (0.1%) each and incubating the samples in 100 ml of thioglycollate medium THG) at 30 to 35°C and 100 ml of tryptic soy broth (TSB) at 20 to 25°C. The incubations were performed for not less than 14 days. The media are visually inspected every 2 to 3 days for the presence of microbiological proliferation. If there is no microbial growth, the examined sample meets the test requirements (sterile).

### 2.3 Results

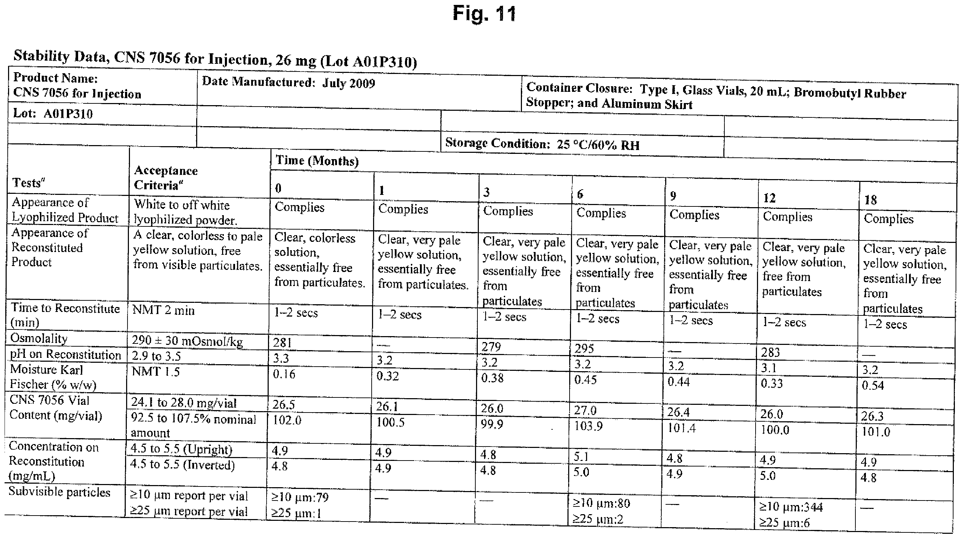

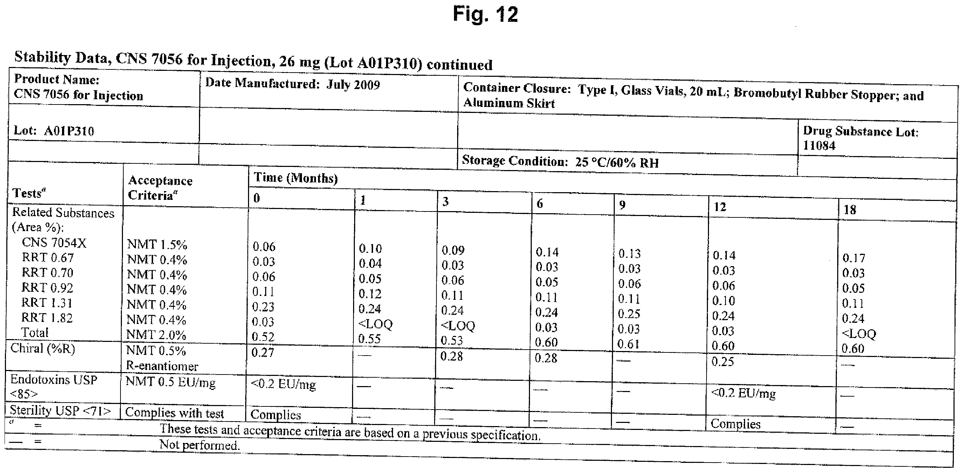

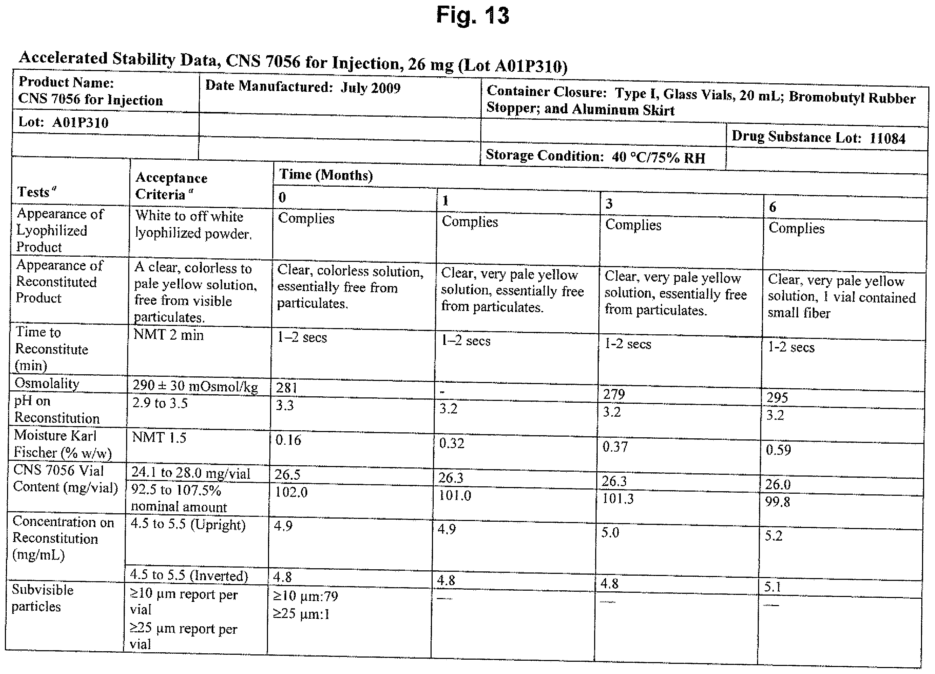

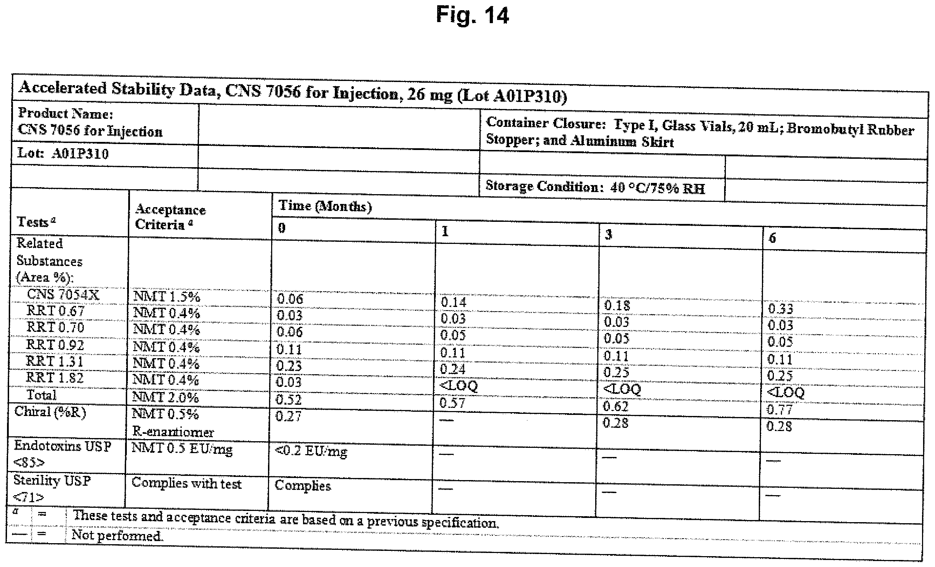

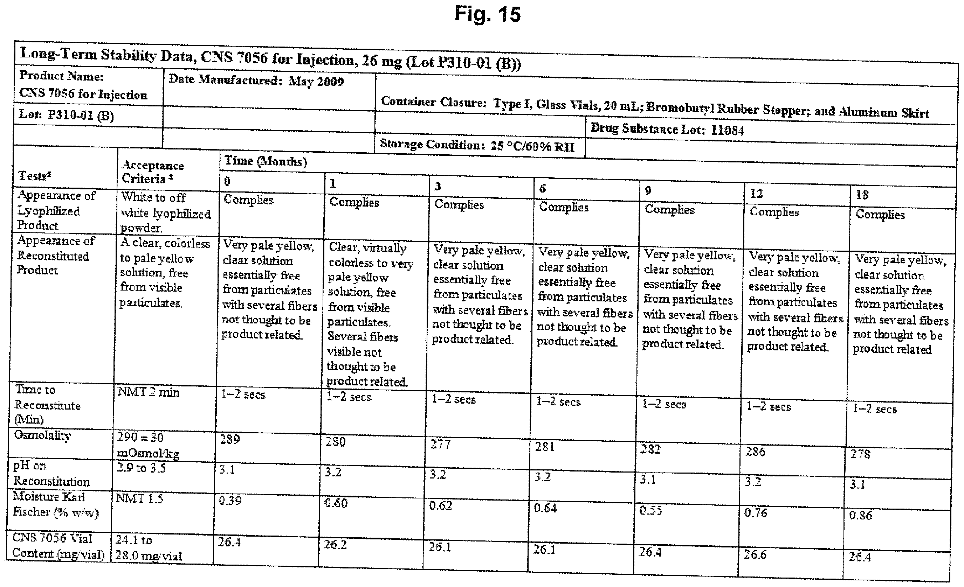

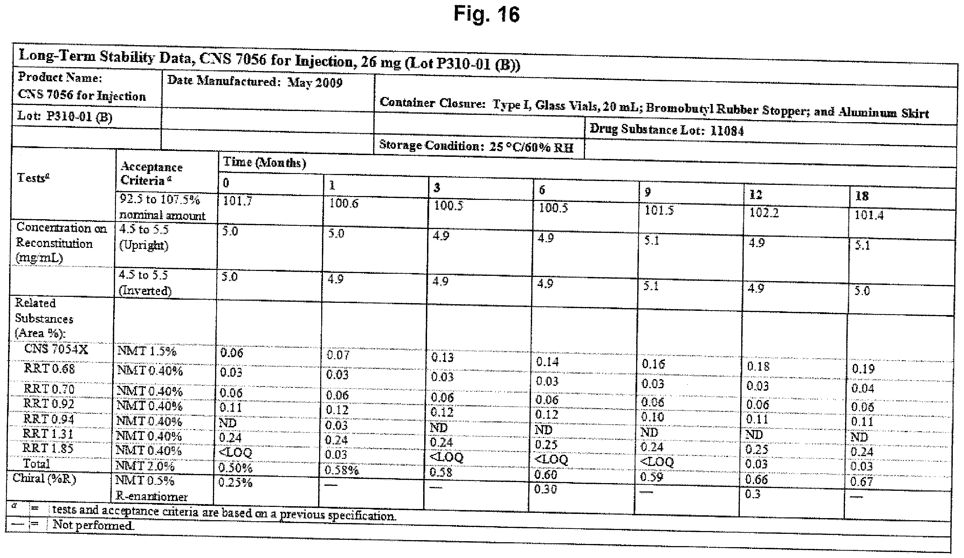

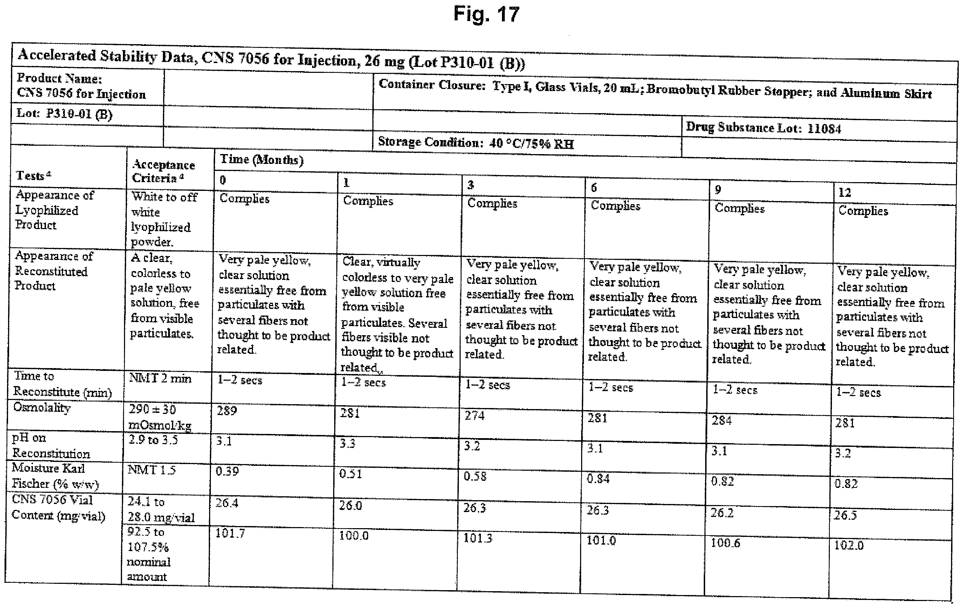

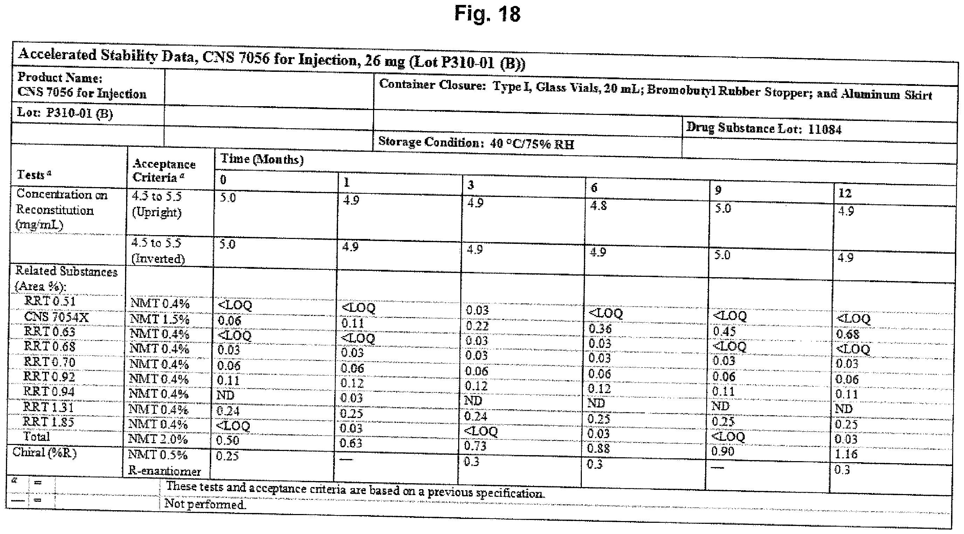

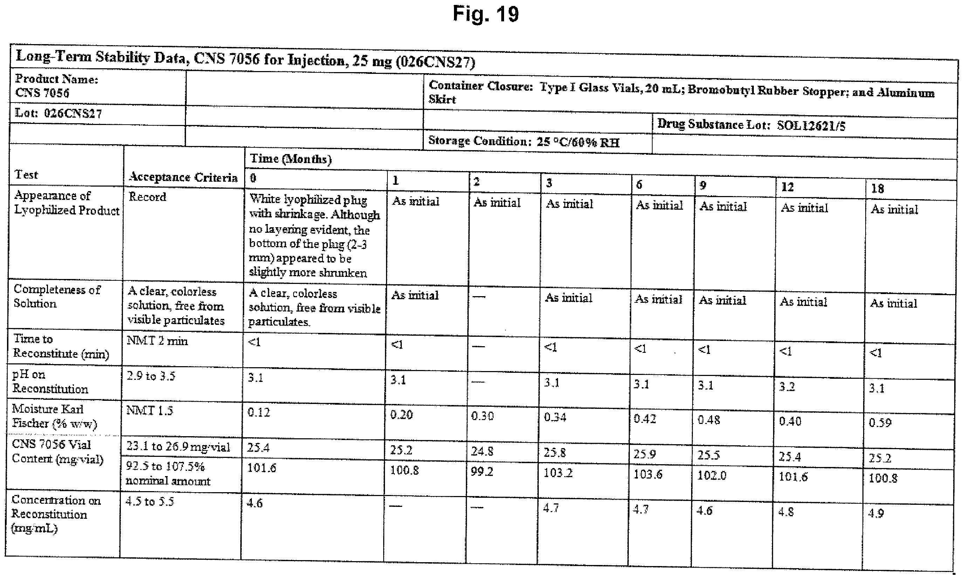

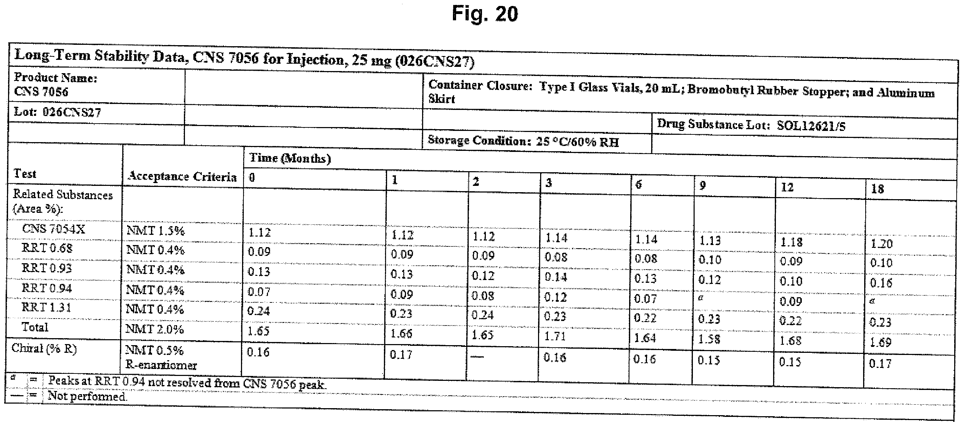

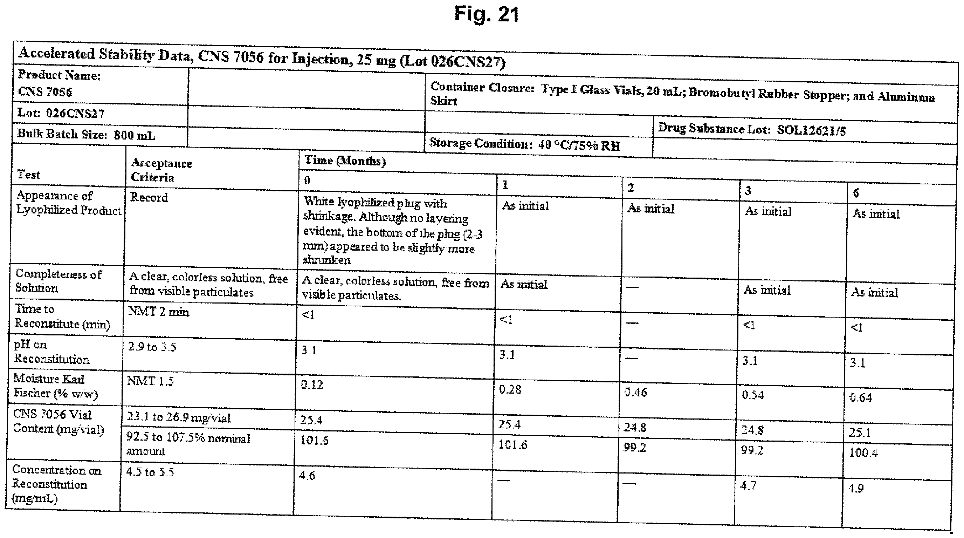

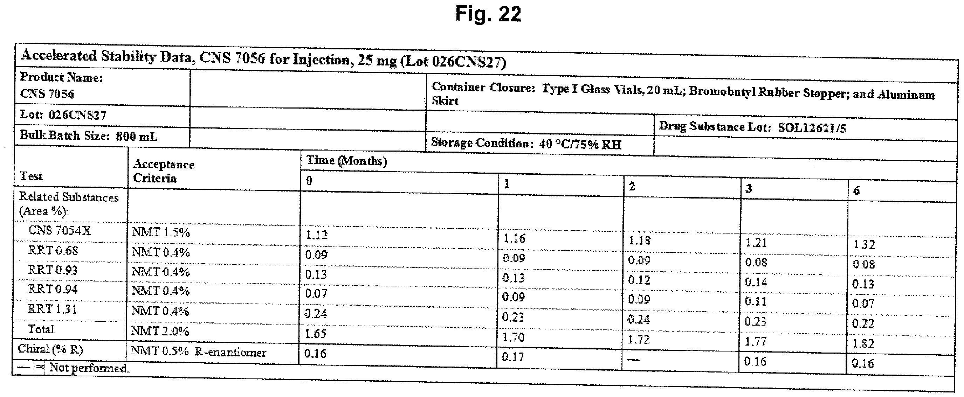

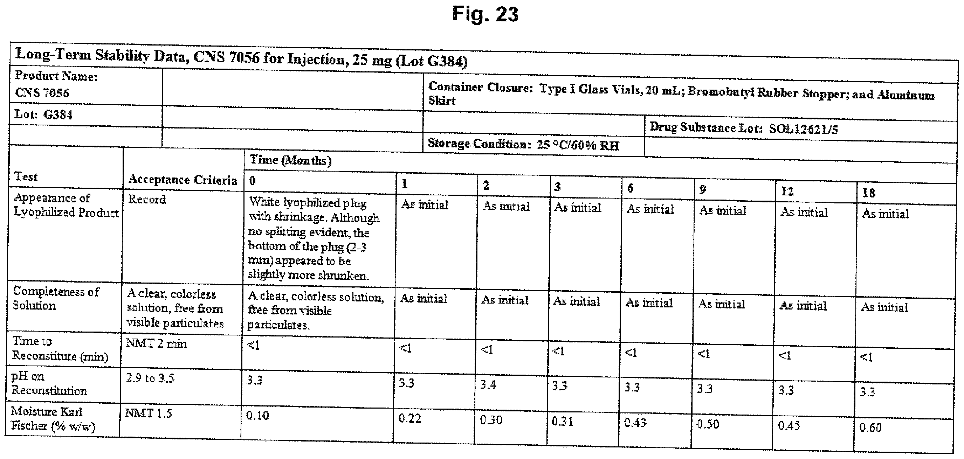

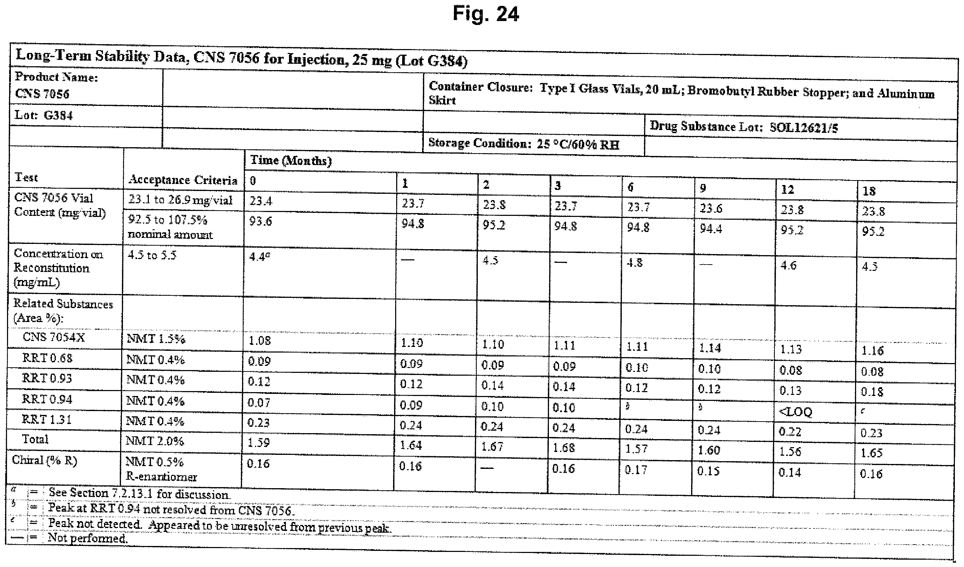

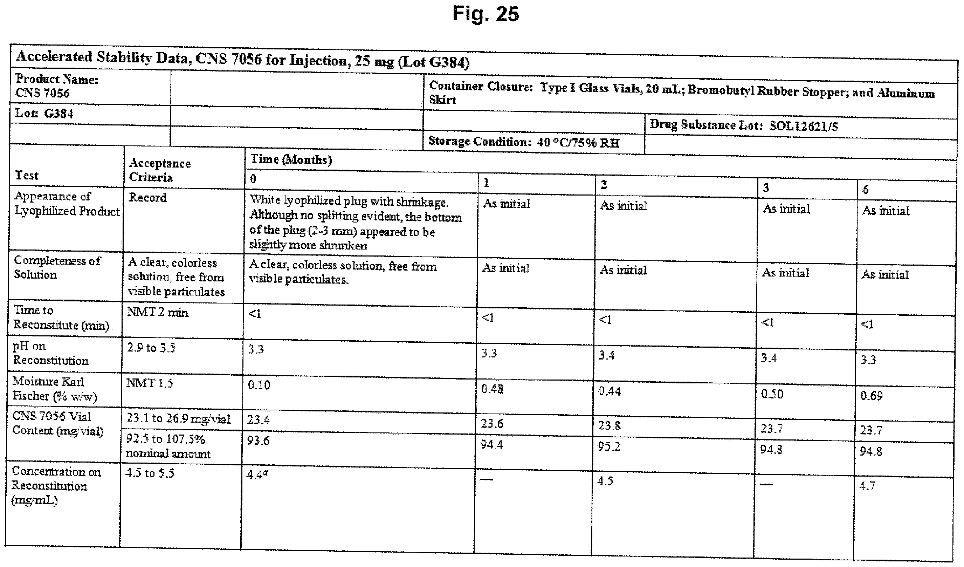

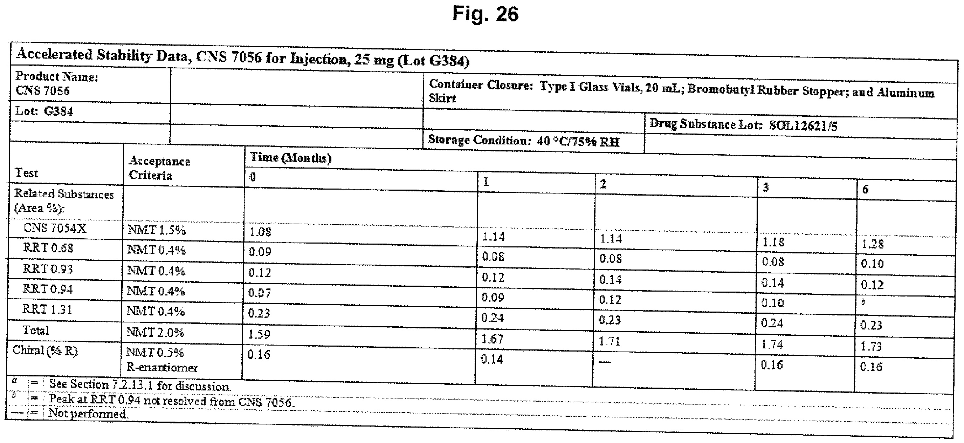

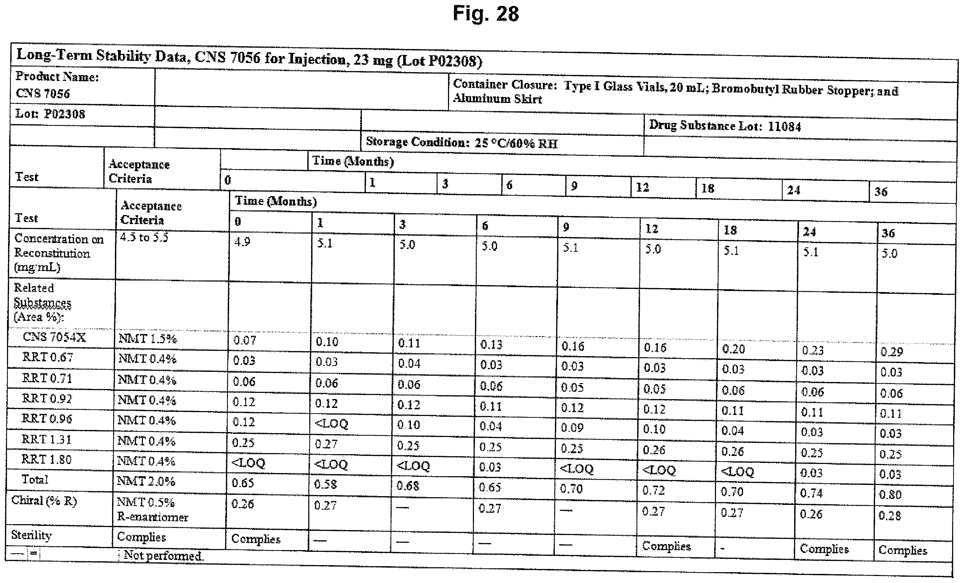

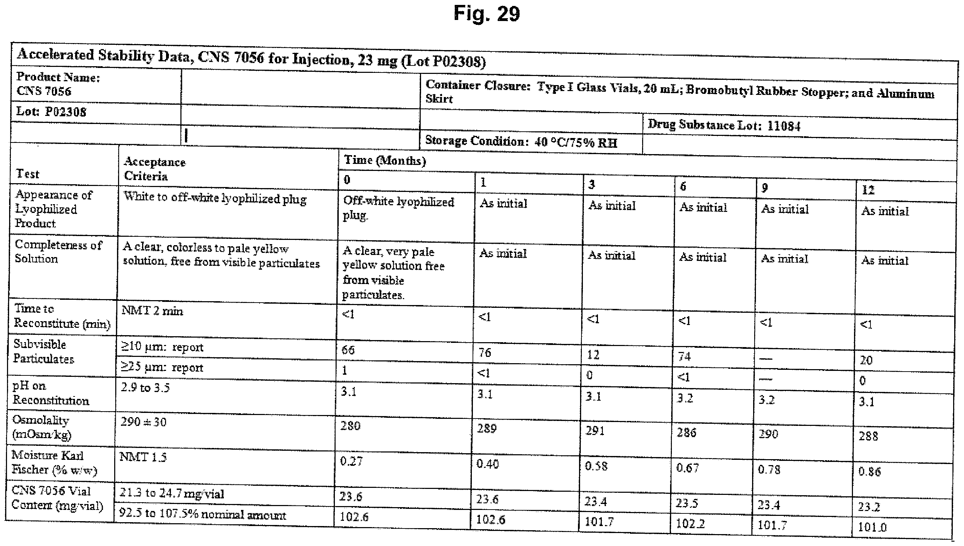

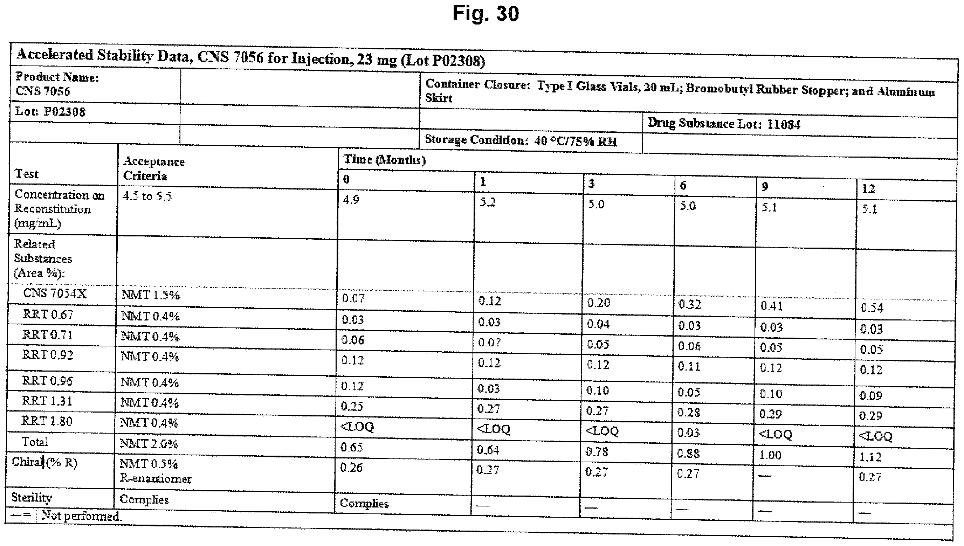

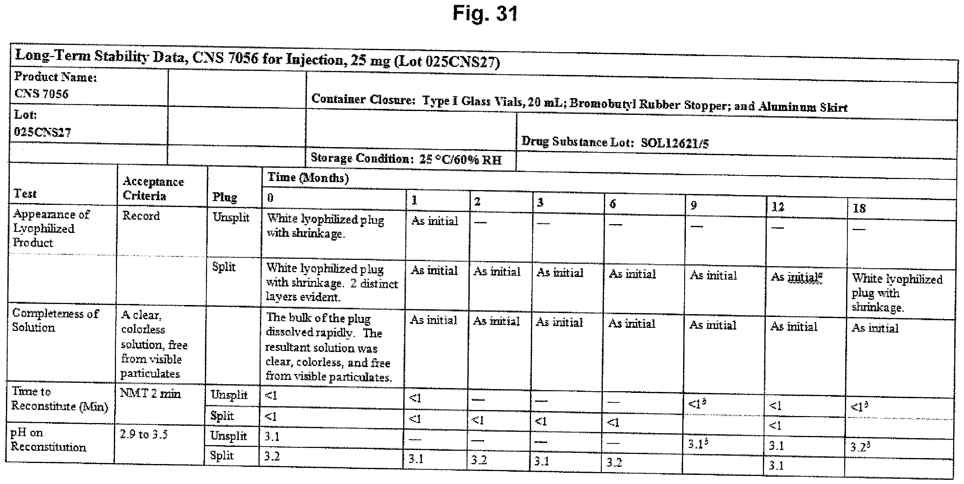

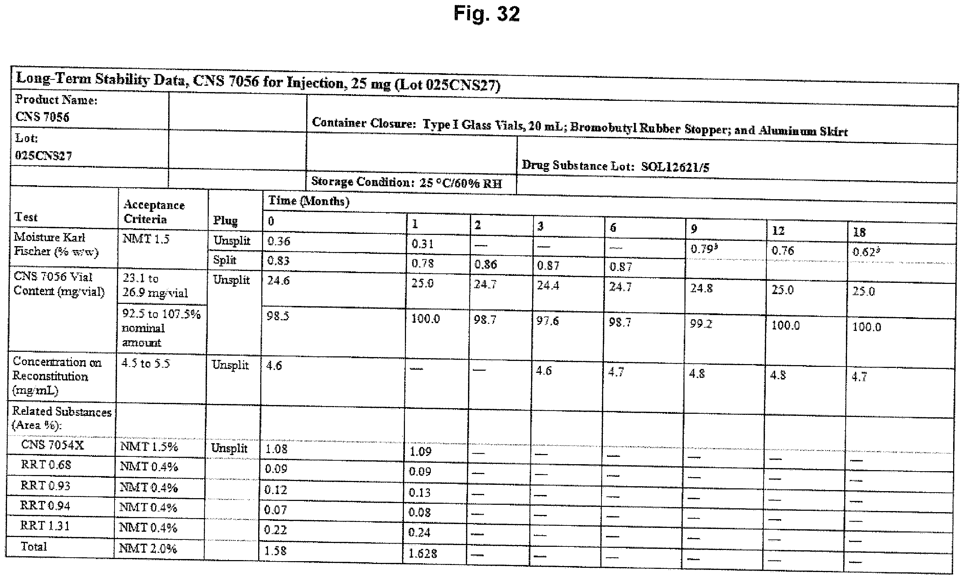

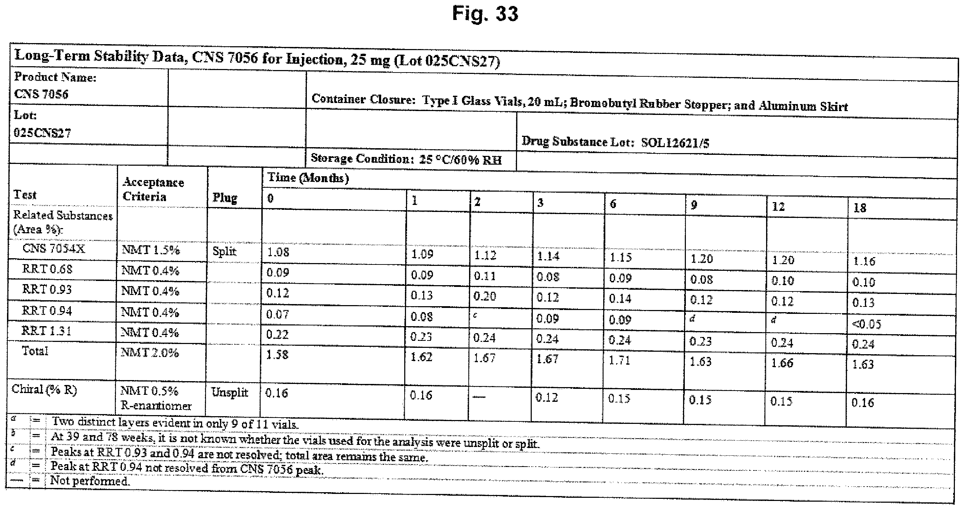

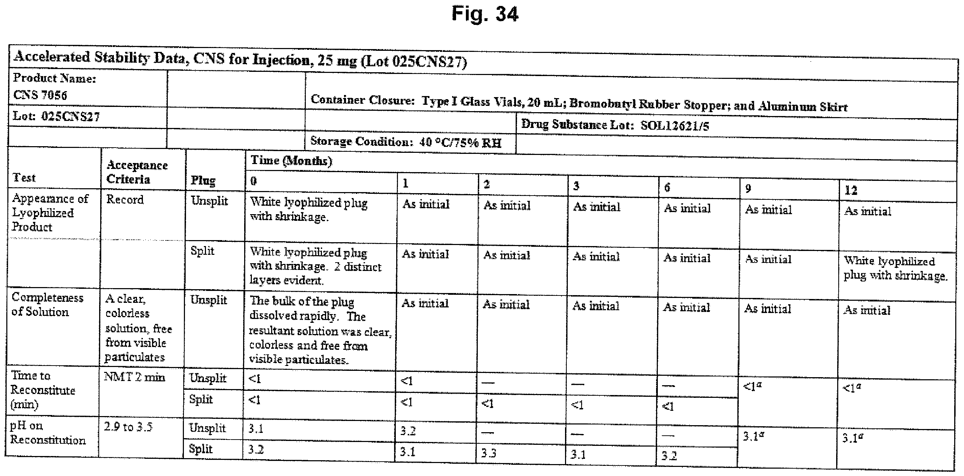

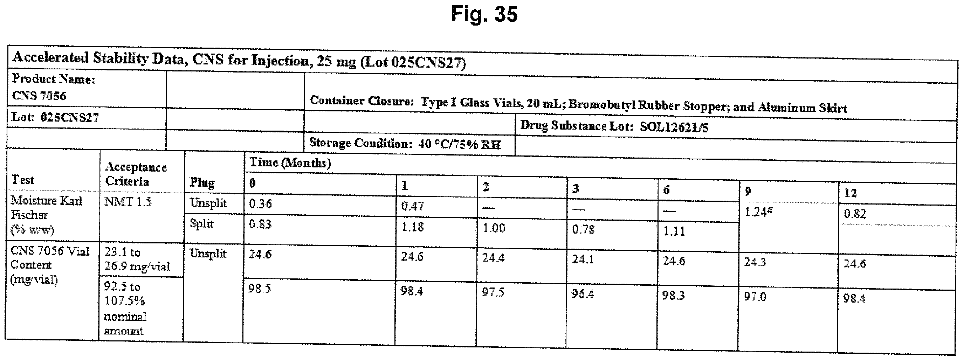

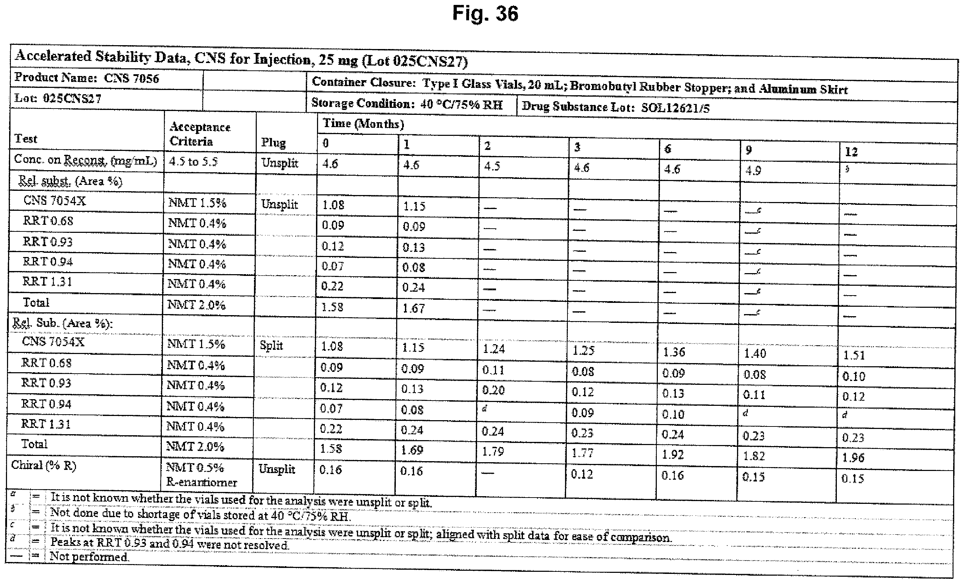

The results of the stability analyses for the above described batches after storage at 25°C/60% relative humidity(RH) or at 40°C/75%RH (so called "accelerated stability" analysis) are summarized in the Figures 11 to 36.

### 2.4 Summary

The tested formulations for CNS 7056 exhibit an excellent long term stability which already supports a shelf life of 36 month for the drug product.

## 3. Stability data after storage for 36 month

The CNS 7056 batch P02308 was subjected to a stability study whereby the vials were stored for 36 months at 25°C/60%RH.
For batch composition and freeze drying conditions see chapter 2.1. For description of the analytical methods see chapter 2.2.

### 3.1 Results

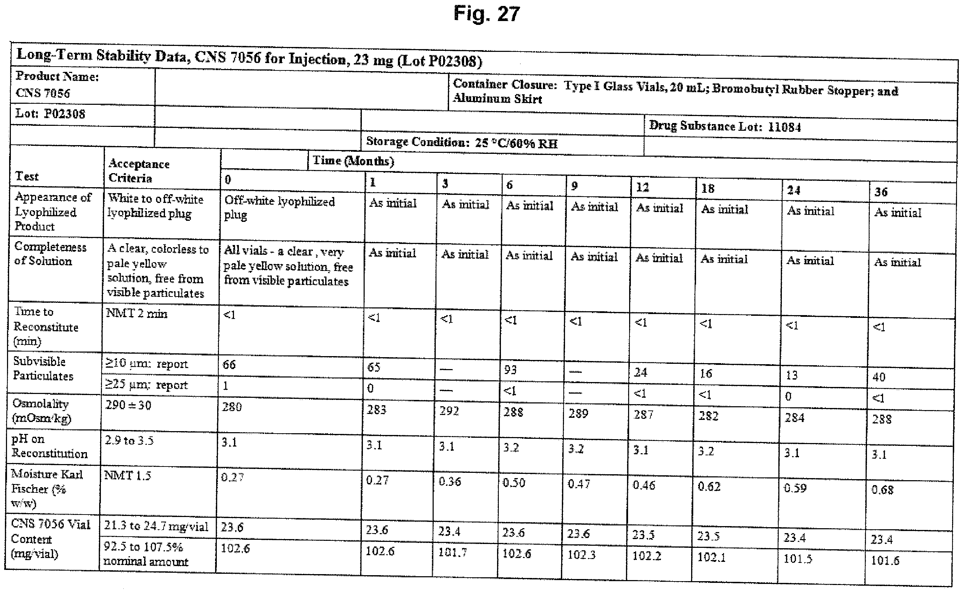

The results of the stability analysis for the batch P02308 after storage at 25°C/60% relative humidity (RH) up to and including 36 months are summarized in figures 27-30.

### 3.2 Summary

All tests performed on batch P02308 after storage at 25°C/60%RH (T=36 months) were within the specified acceptance criteria. Appearance of Lyophilised Product, Completeness of Solution, Time to Reconstitute, pH, and Osmolality of all samples at T=36 months were well within specification.

CNS7056B Vial Content at 25°C/60%RH is 23.4 mg/vial, which is in keeping with all previous results. These results are well within specification. The main CNS 7056 hydrolysis product CNS7054X (RRT 0.59) has increased to 0.29% at 25°C/60%RH from 0.07% at initial months.

A total impurities result of 0.80% was observed at the T=36 month time point, compared to 0.65% at initial.. These results, together with the supporting data from storage over 12 months at the acclearted stability storage condition of 40°C/75%RH, reflect only a slight increase in degradation over this significant period of time and demonstrate the stabilising effect of CNS 7056 in combination with lactose..

Moisture content at T=36 months 25°C/60%RH is 0.68% which shows an increase from 0.27%at initial. This increase is thought to be due to water desorption from the stopper, which will occur over time. These results are well within specification.

### 3.3 Conclusion

All parameters are within specification and the only noticeable trends are the expected increase in the hydrolysis product CNS 7054X and moisture content. The rate of increase of CNS 7054X is similar to previous laboratory non GMP development batches of CNS 7056 for injection produced/tested.

## 4. Evaluation of crystalline material in a lyophilised lactose formulation of CNS 7056 by Raman mapping

### 4.1 Introduction

XRPD studies showed that the lyophilised formulation of CNS 7056 is amorphous, however when material is examined under polarised light microscopy there is evidence of crystalline material present in the amorphous matrix. In order to reveal if this crystalline material is due to CNS 7056 or some other component e.g. lactose monohydrate a Raman mapping analysis was performed. This study makes use of a confocal Raman dispersive microscope to study the physical form of CNS 7056 within the lyophilized lactose formulation using Raman mapping. In Raman mapping experiments, once the first Raman spectrum collection is completed from the in focus sample surface, the sample stage is moved at a predefined step (normally a few to few tens micron) and another spectrum is taken. This is continued until the chosen analytical area has been covered and a hyperspectral data set has been constructed. The sample is prepared to ensure its surface is flat as this avoids the need to refocus the microscope during the data collection from one point to another. The hyperspectral data cube is then processed to generate chemical images based on the distinguishable specific Raman peak (fingerprint) of each component of the sample under study. The chemical images thus generated can then establish each component variation over the chosen area of the studied sample. Crystalline (Form I polymorph), amorphous (lyophilized) CNS 7056B and the lyophilized (amorphous) lactose were characterized by Raman, and the characteristic Raman peak of crystalline CNS7056B were used to generate the chemical images of crystalline CNS7056B. The chemical images of the lactose were also generated based on its own characteristic peak. One area of a lyophilized formulation of CNS 7056B was mapped to determine, if present, the content (based on area ratio without calibration) and distribution of crystalline (Form I) CNS 7056B within the mapping area of the lyophilized lactose formulation. The aim of this study was to establish whether crystalline material within the lactose formulation is due to CNS 7056B or some other component eg. lactose monohydrate.

### 4.2. Material and methods

The following samples were tested in the Raman mapping study:

<u>CNS 7056B (Form I)</u>

- Item/Lot Number: SOL 12621/5
- Appearance: white powder
- Pharmaterials Ref no: PMO553/08

CNS 7056 for Injection (received from Paion)

- Item/Lot Number: PO2308
- Appearance: white lyophilised powder
- Pharmaterials Ref no: PM0554/08

Lyophilized CNS 7056 (amorphous)

- Item/Lot Number: 05/CNS/06
- Appearance: white lyophilised powder
- Pharmaterials Ref no: PM0555/O8

Lyophilized lactose (received from Paion)

- Item/Lot Number: O28/CNS/27
- Appearance: white lyophilized powder
- Pharmaterials Ref no: PM0548/08

Raman spectra of supplied materials

Raman spectroscopy on crystalline (Form I) and amorphous (lyophilized) CNS7056B and amorphous lactose (lyophilised) as supplied was performed using a confocal Nicolet Almega XR dispersive Raman microscope. A distinguishable Raman peak of crystalline (Form I) CNS7056B and amorphous lactose (lyophilised) was respectively selected for generating chemical images and establishing each variation in the examined area of a lyophilized formulation as shown later.

Raman mapping using a confocal dispersive Raman microscope

Raman mapping was performed on one area of a lyophilised formulation. For each measurement, Raman mapping was performed on one area (e.g. 300x300 $\mu m^2$). The chemical images were then produced, respectively based on the distinguishable Raman peak of crystalline CNS7056B and amorphous lactose (lyophilised). These operations allowed the identification of crystalline CNS7056B (potentially recrystallized from the lyophilized lactose formulation) and amorphous lactose (lyophilized) in the selected area of the sample. Subsequently, thus produced chemical images were used to indicate the distribution of crystalline CNS7056B and lactose (lyophilised) in the mapping area respectively.

**Raman technique**

Raman spectra

Samples were analysed by a confocal Nicolet Almega XR Dispersive Raman Microscope for its Raman spectrum using the following conditions:

- Exposure Time: 1.0s
- Exposure Times of each spectrum: 10
- Pinhole Size: 100um
- Spectral range: whole (single grating)
- Laser: He Ne 633nm at 100% power
- Objective: 50x/0.75 (magnifier/numerical aperture number)

Afterwards, the measured Raman spectra were corrected by baseline subtraction (BS) using the software OMNICTM v7.3.

Raman mapping

Each sample was gently pressed by hand so that the mapping area has an approximately flat surface. Raman spectra data for mapping were collected using following conditions:

- Exposure Time: 5.0s
- Exposure Times: 10
- Pinhole Size: 100pm
- Wavelength range: 1700~300 $cm^{-1}$ (multiple gratings)
- Laser: He Ne 633nm (100% power)
- Objective:50x/0.75
- Area: circa 300 x 300 µm
- Scanning step: 10pm

Then the measured Raman spectra data from mapping were modified by baseline correction and normalization using the software OMNICTM v7.3.

**4.3 Results and Discussion**

**4.3.1 Raman spectra of each component in a lyophilized formulation**

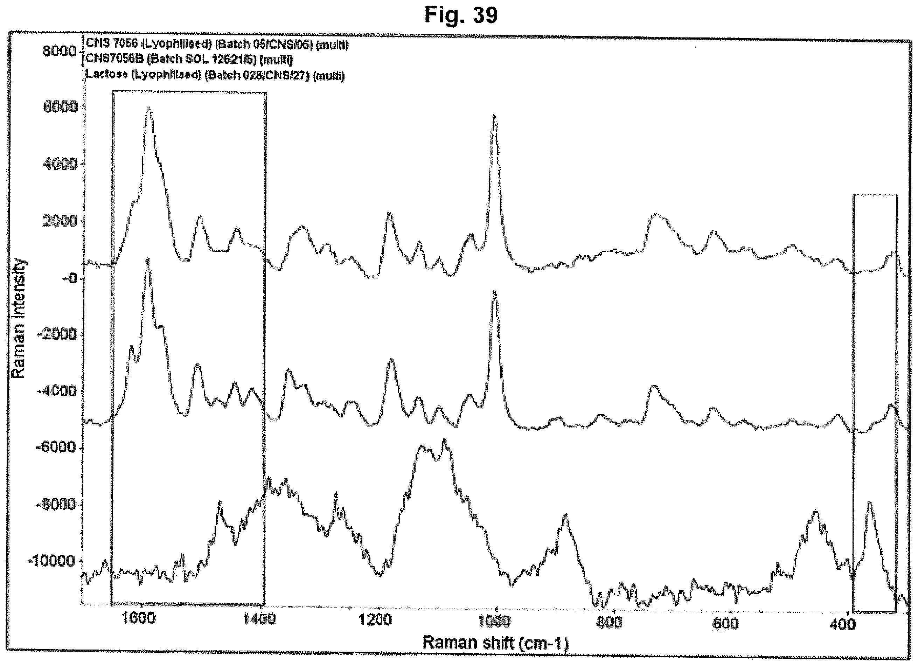

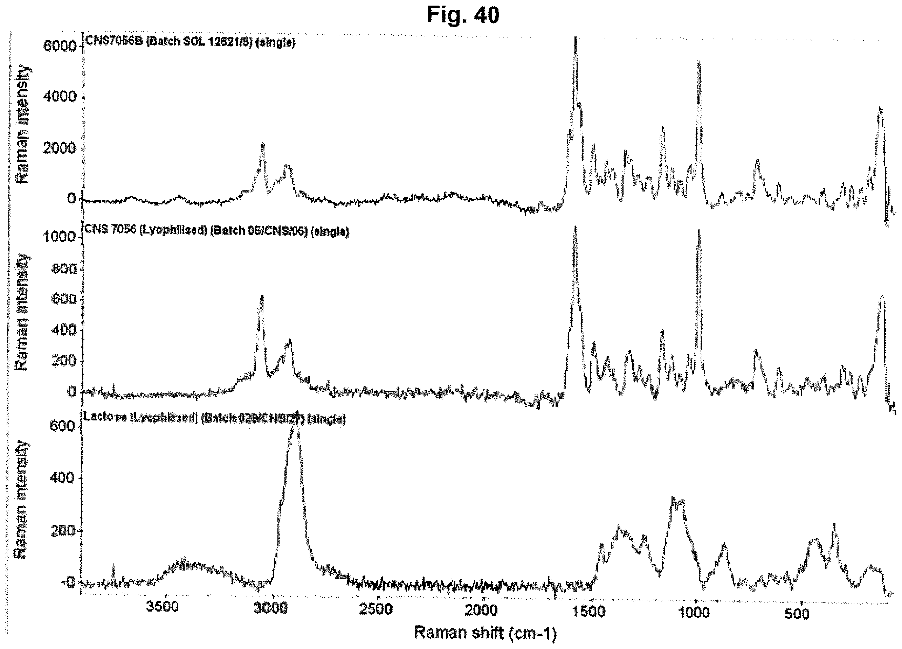

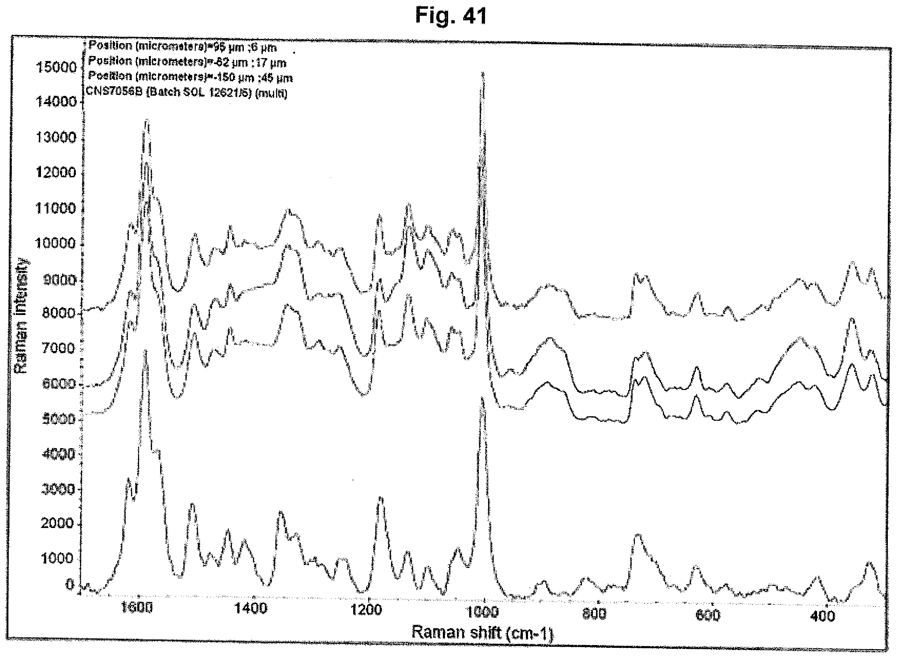

The Raman spectra of crystalline (Form I polymorph) and amorphous (lyophilized) CNS7056B and amorphous lactose (lyophilised) as supplied were collected using the procedure described under Material and Methods. As seen in Figure 39, the Raman peak was then selected respectively: circa 1620 $cm^{-1}$ for crystalline CNS7056B and circa 365 $cm^{-1}$ for lyophilized lactose. These peaks are unique to both materials so that the chemical images of crystalline CNS7056B and lactose (lyophilized) can respectively be generated. The whole range of the Raman spectrum for each component contained in a lyophilized formulation is given in Figures 40 and 41.

**4.4 Summary**

The obtained data demonstrate the presence of crystalline CNS7056B in this lyophilized formulation comprising mostly of amorphous CNS7056B. Furthermore a uniform distribution of CNS7056B and excipient in this lyophilized formulation could be shown.

In the lyophilised product tested approximately 9% of the data points contained a signal corresponding to crystalline CNS 7056B. The actual w/w (%) presence of the crystalline CNS 7056B in the amorphous lactose matrix, however, cannot be concluded from these results as calibration was not performed.

**II. PREPARATION AND STABILITY ASSESSMENTS ANALYSIS OF LYOPHILIZED AND SPRAY-DRIED FORMULATIONS**

A lyophilised and a spray-dried formulation having the same formulation were prepared and tested for stability.

**1. Manufacture of spray-dried CNS7056B formulation (with lactose)**

CNS7056B (Form 2 bn 10201126, 5.1g) and Emprove Lactose Monohydrate, (139.2g) were dissolved in 750ml deionized (DI) water with heating to ~50°C, and then filtered and cooled to room temperature. The pH was checked and not adjusted as it was at 3.1. This solution was spray-dried using the following parameters: Inlet temperature 150°C. Pump rate = 10% (20ml in 7 mins), Fan setting = 50%. Yield 59.5g. The water content was measured via Karl-Fischer and used to calculate the fill weight per vial (997mg). 58 vials were filled with 997mg of spray-dried formulation. 30 vials were placed in the vacuum oven with lids slightly open. These vials were dried under vacuum (~250psi) with a nitrogen bleed at 50°C for 24 hours. The chamber was then flushed with nitrogen, and the vials were then closed quickly under nitrogen. These samples were called 12PM529-8-2. 28 vials were closed without drying. These samples were called 12PM529-8-1. All vials were crimped with aluminium seals.

**2. Scale up of spray dried API (CNS7056B)**

CNS7056B (Form 2 bn 10201126, 20g) was dissolved in 2900ml DI water. This solution was filtered and then spray-dried using the following parameters: Inlet temperature 130°C, outlet temp. 82-79°C. Pump rate = 10% (20ml in

7 minutes), Fan setting = 30%. Yield not noted. The process was repeated with CNS7056B (Form 2 bn 10201126, 5.6g) was dissolved in 812ml DI water, to give 2.2g overall yield (from both runs) of a white powder. The samples were called 12PM529-9-1.

**3. Manufacture of freeze dried (lyophilized) API (CNS7056B)**

A solution of CNS7056B in water was prepared (2.2g of bn 10201126, Form 2, PM0232/12 in 230ml water). This was placed in a round bottomed flask (rbf) and 'shell-frozen' in liquid nitrogen and then lyophilised over 5 days. The resulting fluffy white solid was scraped out and broken up (~2g). The samples were designated as 12PM529-10-1.

**4. Accelerated stability study on lyophilized and spray dried formulation and spray dried API**

The spray-dried CNS7056B formulation, both dried (12PM529-8-2) and non-dried (12PM529-8-1), stored in crimped vials was placed on an accelerated stability study, along with the lyophilized CNS7056B formulation (CNS2501A) as reference, and with the spray-dried amorphous API (12PM529-9-1). Samples were stored at 40°C/75%RH for 4 and 13 weeks, and at 55°C for 4 weeks, and analysed for appearance, assay, related substances, moisture, XRPD, reconstitution time, and appearance following reconstitution.

**4.1. Results**

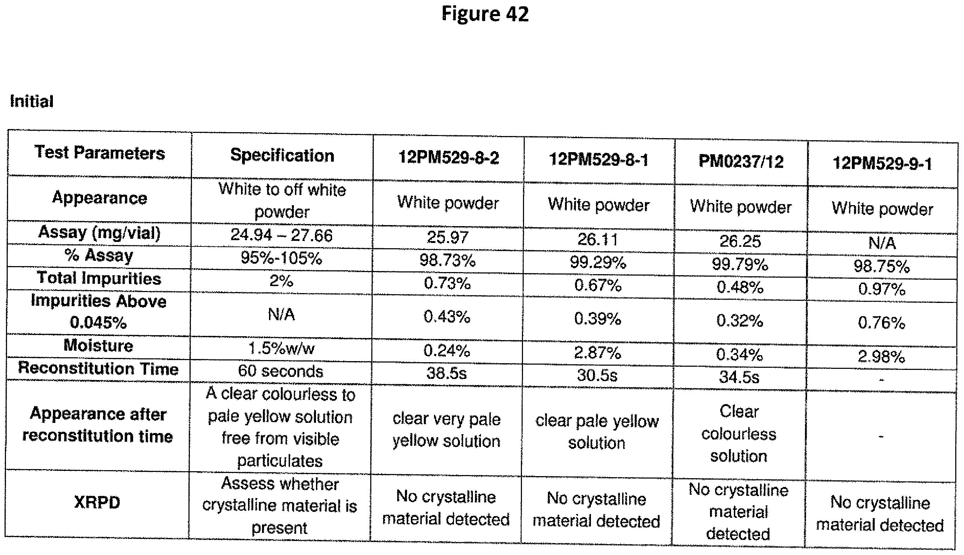

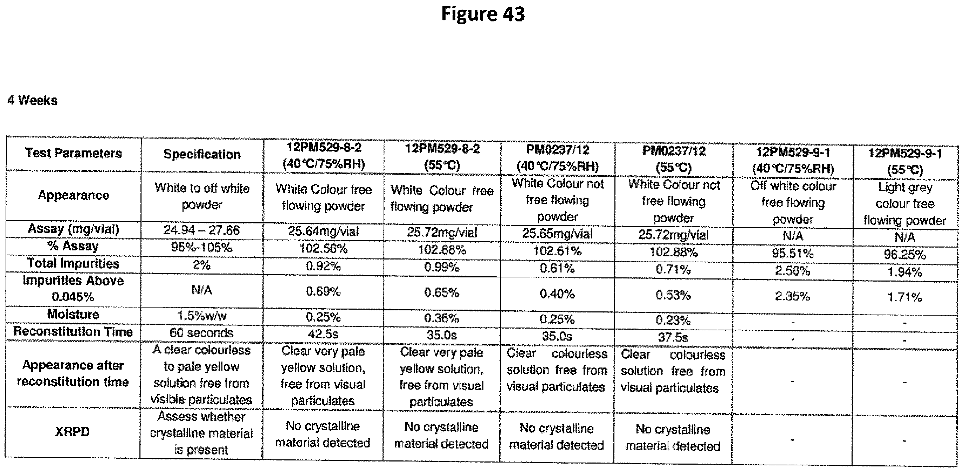

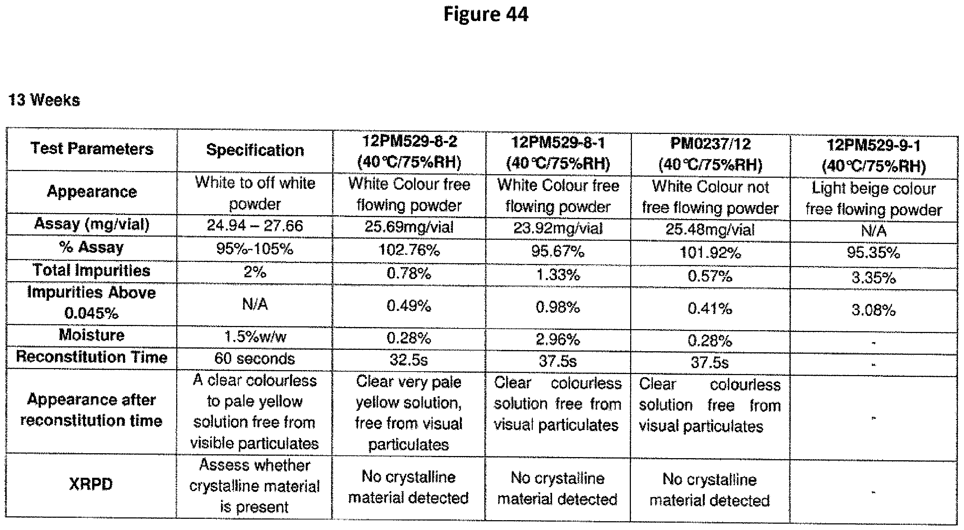

The results from the stability study are presented in the Figures 42 to 44 and can be summarized as follows:

> The spray-dried formulation (sealed prior to additional drying) had a slightly higher initial total impurity level at t=0 than the lyophilised formulation CNS 2501A i.e. batch (~0.73/0.67% vs 0.48%). This is potentially due to the manufacturing process involving higher temperature, and could be optimised on scale up.

The vacuum dried spray-dried formulation sample (12PM529-8-2) had similar water content to the lyophilised sample supplied (0.24% vs 0.34%). The non-dried spray-dried formulation sample (12PM529-8-1) had significantly higher water content (2.87%), as did the amorphous spray-dried API (CNS7056B, 12PM529-9-1).

The 'dry' spray-dried formulation (12PM529-8-2) showed similar stability to the lyophilized formulation. The total impurities increased ~0.2% for both samples after 4 weeks (slightly more at 55°C than at 40/75), and actually only increased ~0.05% for both samples after 13 weeks at 40/75.

The 'wet' spray-dried formulation (12PM529-8-1) had slightly inferior stability than the other formulation samples, but was still within specification for impurities after 13 weeks at 40/75 (total impurities increased from 0.67% at t=0, to 1.33% at t=13 weeks).

The spray-dried API (CNS7056B, 12PM529-9-1) showed significant instability, with increase of total impurities to 1.94% (4 weeks at 55°C), 2.56% (4 weeks at 40/75) and 3.35% (13 weeks at 40/75). This confirms that the lactose formulation is stabilising the API significantly during the stability trial, even when there are similar levels of water present in the formulation to the API sample.

As expected the major impurity that was observed was the hydrolysis product CNS7054X.

**5. Investigation of API distribution and form in the spray-dried and lyophilized formulations using Raman mapping**

A vial of lyophilized formulated product (CNS7056B in lactose, batch number CNS2501A) was opened and sampled randomly four times. Each sampled portion was then presented on a microscope slide and Raman mapping was carried out over a small area of the surface of the formulation sample (~300x300μm). The data was processed in comparison with reference samples of lyophilized (amorphous) and crystalline API (CNS7056B, forms 1 and 2) and lyophilized (amorphous) and crystalline (monohydrate) lactose. The mapping was analysed to determine the distribution of the API within the formulation, and then if any phase separation (regions of API) was detected, these would be analysed to assess the physical form of the API. A second experiment was carried out where a new vial of lyophilized formulated product (CNS7056B in lactose, batch number CNS2501A) was opened and sampled from top, middle and bottom of the cake. These three samples were again analysed by Raman mapping as above. Also, two regions from the top and bottom samples were mapped over a smaller region (~20x20μm) in more detail. Three more batches of lyophilized formulated product (CNS7056B in lactose) were also analysed by Raman mapping: batches P02308, A01P301 and P301-02N. The size of region mapped in these experiments was ~120x100μm.

### 5.1 Results for batch CNS2501 A

The Raman mapping data was processed to give a 'chemical image' which shows the similarity of the Raman spectra detected at each point on the map with:

a) the excipient main peak at 355cm-1 (i.e. lactose)
b) the API (CNS7056B) main peak at 1580cm-1
c) correlation with the whole excipient spectra (lactose).

The data showed that no phase separation and re-crystallization of API was found in batch CNS2501A as supplied after analysis of 7 different grab-samples from 2 different vials. The distribution of API and lactose was uniform and no separate regions or particles of API were found. This suggests that a true molecular dispersion of the API in lactose has been formed in the lyophilised formulation batch CNS2501A.

### 5.2 Results for batches P02308, A01P301 and P301-02N

The Raman mapping data was processed as described in chapter 5.1.

The data revealed no phase separation and re-crystallization of API in batches P02308, A01P301 and P301-02N as supplied based on one set of mapping data for each batch. The distribution of API and lactose was uniform and no separate regions or particles of API were found. This suggests that a molecular dispersion of the API in lactose has been formed in the lyophilised formulation in batches P02308, A01P301 and P301-02N. (Note: some phase separation was found in previous mapping performed on batch P02308. This suggests that the distribution of separated (crystalline) API in this batch is not uniform.

### 6. Summary

An equivalent spray dried formulation to the current lyophilised (freeze-dried) product could be successfully developed and tested.

Both the spray dried and lyophilised formulations were shown to be fully amorphous and single phase by XRPD and Raman analysis (i.e. no detectable separated crystalline API). The spray dried formulation had a slightly higher impurity level (~0.7% total impurities vs. ~0.5% for the lyo product). This is presumed to be formed during spray-drying manufacture and could be reduced with process optimisation.

The fully dried spray-dried formulation showed equivalent stability to the lyo product over 13 weeks at 40°C/75%RH and 4 weeks at 55°C. The non-dried spray-dried formulation (3% water) showed slightly worse stability, but stayed within the specification over 13 weeks at 40°C/75%RH.

The spray-dried formulation showed similar colour change to the lyo product in the light stability trial, both turning grey/blue. Physical analysis of the light stressed samples of API and formulation showed some recrystallisation and absorption of water, but no evidence of changes in physical form contributing to the colour changes.

Raman mapping analysis of the current lyo batch (CNS2501A) and previous lyo batches (P02308, A01P301 and P301-02N) of formulated product showed uniform distribution of API and excipients, with no evidence of separation of API and subsequent crystallisation.

## III. PREPARATION AND STABILITY ANALYSIS OF DISACCHARIDE BINARY EXCIPIENT CONTAINING FORMULATIONS

### 1. Purpose and study outline

The purpose of the present study was to evaluate the stability of selected formulations. Several lyophilized formulations of CNS 7056 were prepared containing lactose monohydrate and the pH adjusted to 3.1, the API is present as besylate salt. Two fill concentrations of CNS 7056 were investigated: 5 mg/ml and 10 mg/ml. The formulations were filled in ISO 10R and ISO 6R clear glass vials. Fill volume was reduced to 4 mL/vial (current fill volume is 5.2 mL). The existing formulation was filled in ISO 10R vials that were stoppered with both West 4023/50 art. 1346 stoppers and West S87 J 4416/50 stoppers. The stability of the new formulations manufactured in ISO 10R vials were evaluated together with the existing formulation. In addition to this, the existing formulation lyophilized in the frame of the last clinical batch manufacturing (batch number A01 P31 0, fill volume 5.2 mL, ISO 20R clear glass vial) was tested to generate comparative stability data.

### 2. Methods

The following tests were performed on the stability samples:

- Appearance of the lyophyilisate.
- Reconstitution time.
- Appearance of the reconstituted solution.
- Moisture content by Karl Fischer titration.
- HPLC Assay/Related Substances.
- Osmolality (only at time 0)

### 3. Batch description

The product composition of the batches submitted to stability is summarised here below.

| Formulation (Excipient weight ratio) | CNS 7056 concentration | Vials | Fill volume | Stoppers | Product Reference |
|---|---|---|---|---|---|
| Lactose Reference (Current) Formulation | 5 mg/mL | 6R | 4mL | West 4023/50 art. 1346 | L6R5 |
| | | 10R | 4mL | West 4023/50 art. 1346 | L10R5 |
| | | | | West S87 J 4416/50 | L10R5S87 |
| | | 20R | 5.2mL | West 4023/50 art. 1346 | L20R5 |
| | 10 mg/mL | 6R | 4mL | West 4023/50 art. 1346 | L6R10 |
| | | 10R | 4mL | West 4023/50 art. 1346 | L10R10 |
| Lactose : Mannitol (4:1) | 5 mg/mL | 6R | 4mL | West 4023/50 art. 1346 | L4M16R5 |
| | | 10R | 4mL | West 4023/50 art. 1346 | L4M110R5 |
| | 10 mg/mL | 6R | 4mL | West 4023/50 art. 1346 | L4M16R10 |
| | | 10R | 4mL | West 4023/50 art. 1346 | L4M110R10 |
| Lactose : Mannitol (2:1) | 5 mg/mL | 6R | 4mL | West 4023/50 art. 1346 | L2M16R5 |
| | | 10R | 4mL | West 4023/50 art. 1346 | L2M110R5 |
| | 10 mg/mL | 6R | 4mL | West 4023/50 art. 1346 | L2M16R10 |
| | | 10R | 4mL | West 4023/50 art. 1346 | L2M110R10 |

### 4. Stability program

The stability program is summarized in the following table:

| Formulation (Excipient weight ratio) | Product Reference | Stability |
|---|---|---|
| Lactose Reference (Current) Formulation | L6R5 | 1 month |
| | L10R5 | 3 months |
| | L10R5S87 | 3 months |
| | L20R5 | 3 months |
| | L6R10 | 1 month |
| | L10R10 | 1 month |
| Lactose 4 : Mannitol 1 | L4M16R5 | 1 month |
| | L4M110R5 | 1 month |
| | L4M16R10 | 1 month |
| | L4M110R10 | 1 month |
| Lactose 2 : Mannitol 1 | L2M16R5 | 1 month |
| | L2M110R5 | 1 month |
| | L2M16R10 | 1 month |
| | L2M110R10 | 1 month |

**5. Stability schedules**

The stability schedules are summarized in the following tables:

| Storage conditions 40°C±2°C / 75%±5% RH and 55°C±5°C | | |
|---|---|---|
| **Tests for 1 month study** | **Time 0** | **1M** |
| Lyo appearance (to be noted on all 5 vials) | √ | √ |
| Reconstitution time | √ | √ |
| Appearance of reconstituted solution | √ | √ |
| Moisture content (KF titration) | √ | √ |
| HPLC (Assay/Related Substances) | √ | √ |
| Osmolality | √ | - |

| Storage conditions 55°C±5°C | | |
|---|---|---|
| **Tests for 3 months study** | **Time 0** | **1M** |
| Lyo appearance (to be noted on all 5 vials) | √ | √ |
| Reconstitution time | √ | √ |
| Appearance of reconstituted solution | √ | √ |
| Moisture content (KF titration) | √ | √ |
| HPLC (Assay/Related Substances) | √ | √ |
| Osmolality | √ | - |

| Storage conditions 25°C±2°C / 60%±5% RH | | | |
|---|---|---|---|
| **Tests for 3 months study** | **Time 0** | **1M** | **3M** |
| Lyo appearance (to be noted on all 5 vials) | √ | - | √ |
| Reconstitution time | √ | - | √ |
| Appearance of reconstituted solution | √ | - | √ |
| Moisture content (KF titration) | √ | - | √ |
| HPLC (Assay/Related Substances) | √ | - | √ |
| Osmolality | √ | - | - |

| Storage conditions 40°C±2°C / 75%±5% RH | | | |
|---|---|---|---|
| **Tests for 3 months study** | **Time 0** | **1M** | **3M** |
| Lyo appearance (to be noted on all 5 vials) | √ | √ | √ |
| Reconstitution time | √ | √ | √ |
| Appearance of reconstituted solution | √ | √ | √ |
| Moisture content (KF titration) | √ | √ | √ |
| HPLC (Assay/Related Substances) | √ | √ | √ |
| Osmolality | √ | - | - |

**6. Stability results**

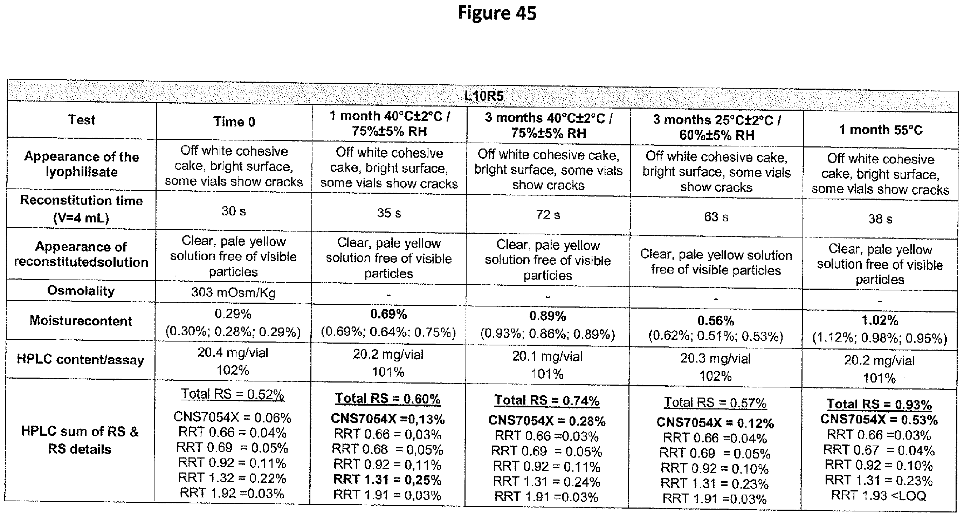

The results collected in the frame of the present study are presented in Figures 45 to 51 and can be summarised as follows:

Samples stored at 40°C 75% RH (1 month).

- Some changes in the appearance of the lyo cake in the formulations L2M110R5 and L2M110R10. Some vials of L20R5 (clinical batch vials rejected after visual inspection) showed a different lyo cake appearance
- Expected increase of moisture content (not observed in L20R5; L2M110R10)
- Small increase in total impurities (not observed in L10R10; L10R5S87; L20R5). The HPLC assay kept practically constant.
- Increase of known impurity CNS7054X.

Samples after 3 months storage at 40°C 75% RH (only L10R5; L10R5S87 and L20R5 formulations).

The appearance of both the cake and the reconstituted solutions didn't undergo any variation (some of the cakes of the L20R5 samples were found to be shrunk).

The assay remained unvaried.

L10R5

- Further increase of moisture content (anyway the % $H_2O$<1.0%).
- Further slight increase of the impurities due to the CNS7054X.

L10R5S87

- Further increase of moisture content (anyway the % $H_2O$<1.0%).
- Further slight increase of the impurities content due to CNS7054X.

L20R5 (visual inspection)

- Further increase of moisture content (anyway the % H2O<1.0%).
- Increase of the impurities content mainly due to CNS7054X.

Samples stored at 55°C (1 month).

- The lyo cake of the formulations L2M110R5, L2M110R10, L4M110R5 and L4M110R10 was found to be shrunk and yellowish colored. Some vials of L20R5 (clinical batch vials rejected after visual inspection) showed a charred (insoluble) lyo cake.
- Increase of moisture content (not observed in L20R5)
- Increase in total impurities (total impurities below 1.00% in L10R5; L10R10). Concurrently negligible reduction of the HPLC assay.
- Increase of known impurity CNS7054X.
- Additional impurities exceeding the LOQ in L20R5, L4M110R5; L4M110R10; L2M110R5; L2M110R10
- Slight presence of foam (not persistent) upon reconstitution of.
- L10R5; L10R5S87 and L20R5 formulations after 1 month storage at 25°C/60%RH.
- The appearance of both the cake and the reconstituted solutions didn't undergo any variation.
- The assay remained unvaried.

L10R5

Slight increase of moisture content (anyway the % $H_2O$<1.0%).
Slight increase of the impurities due to the CNS7054X.

L10R5S87

The moisture content didn't increase.
The impurities content remained practically unvaried.

L20R5 (visual inspection)

Increase of moisture content (anyway the % $H_2O$<1.0%).
Increase of the impurities content mainly due to CNS7054X.

**IV. Preparation and stability analysis of disaccharide/dextran containing formulations, as a means to reduce lyophilization time**

**1. Purpose**

Within this study several CNS7056 lyophylisate formulations containing lactose and dextran were studied. The ratio of the disaccharide to dextran was changed in order to manipulate the glass transition temperature (Tg') and collapse temperature Tc and therefore reduce the lyophylisation time. Compared to the disaccharide lactose the dextran possesses a higher Tg' and therefore can act as a collapse temperature modifier.
Altogether 10 formulations were prepared and tested in different lyophilization protocols.

## 2. Formulations

### 2.1. Formulation compositions

Two CNS7056 formulations were prepared containing dextran only (001/PAN/13) or a mixture of lactose and dextran (002/PAN/13) as summarized in the following table:

| Name | Formulation |
|---|---|
| 001/PAN/13 | 50: 440, 7056:Dextran |
| 002/PAN/13 | 50:220:220, 7056:Lactose:Dextran |

### 2.2. Formulation preparation

#### 2.2.1. Preparation of the solution

The solutions containing 12 mg/mL CNS7056 were prepared according to the following protocol:

- API (CNS 7056 besylate salt) added with magnetic stirring to 85% final volume
- Stirred for 3 hours at ambient, light protected
- Checked pH, nominal pH 3.2 for all formulations, and adjusted to pH 3.0
- Stirred for further 20 minutes: no significant change in appearance
- Made to 90% final volume
- Stirred for further 20 minutes
- Formulations 1-2 appeared light yellow, slightly turbid.
- Checked pH, all nominal pH 3
- Made to final volume and stirred for further 20 minutes
- No change in formulation 1-2 appearance, less undissolved material in the concentrated formulation
- Filtered (0.22 μm PVDF) formulations 1- 2
- Further 25 minutes stirring of concentrated formulationFiltered (0.22 μm PVDF) concentrated formulation
- All filtrates clear, light yellow, free from visible particles
- Filtrates filled in 4.2 mL volumes and lyophilised with protocol as described in 2.1.2

#### 2.2.2. Lyophilisation protocol

The samples were lyophilized according to the following protocol:

| Step | Cycle stage | Temperature (ºC) | Pressure (mTorr) | Time (min) |
|---|---|---|---|---|
| 1 | Load | 25 | n/a | 0 |
| 2 | Ramp | 0 | n/a | 25 |
| 3 | Ramp | -45 | n/a | 225 |
| 4 | Freezing | -45 | n/a | 180 |
| 5 | Hold | -45 | 93 | 0 |
| 6 | Ramp | -25 | 93 | 30 |
| 7 | Primary drying | -25 | 93 | 4890 |
| 8 | Ramp | 30 | 20 | 120 |
| 9 | Secondary drying | 30 | 20 | 480 |
| 10 | Finish | 30 | Vials stoppered to 722000 mTorr with (purse) nitrogen. | |
| Total cycle duration ~ 99 hours (~ 4.1 days) | | | | |

### 2.3 Analysis of the lyophilized samples

The lyophylisate showed a good appearance and a rapid reconstitution time for the carbohydrate:dextran-containing lyophilisates. Both formulations exhibit a purity above 99.72%.

| Name | Formulation | Appearance | Recon time in saline | pH | 7054X (%) | Purity (%) |
|---|---|---|---|---|---|---|
| 001/PAN/13 | 50: 440, 7056:Dextran | Off white plug | 1 m 50 s | 3.241 | 0.11 | 99.72 |
| 002/PAN/13 | 50:220:220, 7056:Lactose:Dextran | Off white plug | 35 s | 3.229 | 0.09 | 99.74 |

### 2.3.1 Appearance

The appearance of the lyophilized samples was determined. The results are listed in the following table:

| Sample details | | | Appearance |
|---|---|---|---|
| **001/PAN/13** | **Initial (T = 0)** | | Off white plug |
| | **40°C/75%RH** | **T = 1 m** | Off white plug with signs of shrinkage |
| **002/PAN/13** | **Initial** | **Initial (T = 0)** | Off white plug |
| | **40°C/75%RH** | **T = 1 m** | Off white plug with signs of shrinkage |

### 2.3.2 Moisture

The moisture content of the lyophilized samples was determined. The results are listed in the following table:

| Sample details | | | Vial 1 | Vial 2 | Vial 3 | Mean moisture (% w/w) |
|---|---|---|---|---|---|---|
| **001/PAN/13** | **Initial (T = 0)** | | 0.04 | 0.12 | 0.19 | **0.12** |
| | **40°C/75%RH** | **T = 1 m** | 0.19 | 0.19 | - | **0.19** |
| **002/PAN/13** | | **Initial (T = 0)** | 0.16 | 0.16 | 0.38 | **0.23** |
| | **40°C/75%RH** | **T = 1 m** | 0.36 | 0.38 | - | **0.37** |

### 2.3.3 Reconstitution time and pH of reconstituted solution

Each vial was reconstituted with 10 mL 0.9% saline. The results regarding reconstitution time and pH are listed in the following table.

| Samples details | | | Recon. time (seconds) | pH |
|---|---|---|---|---|
| **001/PAN/13** | **Initial (T = 0)** | | 110 | 3.24 |
| | **40°C/75%RH** | **T = 1 m** | 153 | 3.21 |
| **002/PAN/13** | **Initial (T = 0)** | | 35 | 3.23 |
| | **40°C/75%RH** | **T = 1 m** | 85 | 3.26 |

### 2.3.4 Vial content

The vial content for the samples at T=1m 40°C/75%RH was determined after each vial was reconstituted with 10 mL 0.9% saline. The results are given in the following table:

| Details | [7056] (mg/vial) | | |
|---|---|---|---|
| | vial 1 | Vial 2 | Mean |
| **001/PAN/13** | 49.1136 | 49.6891 | 49.401 |
| **002/PAN/13** | 49.0496 | 49.2652 | 49.157 |

**2.3.4 Impurities**

The impurities for the different formulations at T=1m 40°C/75%RH were determined. The results are given in the following tables:

001/PAN/13:

| | | | **Initial*[2] (T=0)** | **40°C/75%RH** |
|---|---|---|---|---|
| | **RRT** | **Name** | | **T= 1m** |
| **Impurity profile (area%)** | **0.27** | n.a. | N.D. | 0.03 |
| | **0.42** | n.a. | N.D. | <LOQ |
| | **0.47** | n.a. | <LOQ | <LOQ |
| | **0.51** | n.a. | 0.07 | 0.07 |
| | **0.57** | n.a. | <LOQ | <LOQ |
| | **0.59** | 7054X | 0.11 | 0.17 |
| | **0.64** | n.a. | N.D. | <LOQ |
| | **0.68** | n.a. | <LOQ | <LOQ |
| | **0.71** | n.a. | N.D. | <LOQ |
| | **0.89** | n.a. | 0.10 | N.D. |
| | **0.93** | n.a. | N.D. | 0.10 |
| | **1.00** | 7056B | 99.59 | 99.45 |
| | **1.13** | n.a. | N.D. | 0.03 |
| | **1.31** | n.a. | <LOQ | <LOQ |
| | **1.46** | n.a. | N.D. | <LOQ |
| | **1.73** | n.a. | N.D. | <LOQ |
| | **1.78** | n.a. | <LOQ | <LOQ |
| | **1.84** | n.a. | <LOQ | <LOQ |
| | **1.91** | n.a. | <LOQ | N.D. |
| | **Total imps*[1] (area%)** | | **0.3** | **0.4** |

*[1] Sum of all impurities ≥ 0.03% by area
*[2] Impurities from post lyo samples
Impurities are mean of 2 determinations
n.d. = not detected

002/PAN/13:

| | RRT | Name | Initial (T=0) | 40°C/75%RH T= 1m |
|---|---|---|---|---|
| Impurity profile (area%) | 0.27 | n.a. | N.D. | <LOQ |
| | 0.47 | n.a. | <LOQ | N.D. |
| | 0.51 | n.a. | 0.07 | 0.07 |
| | 0.56 | n.a. | N.D. | <LOQ |
| | 0.59 | 7054X | 0.09 | 0.14 |
| | 0.64 | n.a. | N.D. | <LOQ |
| | 0.68 | n.a. | <LOQ | <LOQ |
| | 0.71 | n.a. | N.D. | <LOQ |
| | 0.83 | n.a. | N.D. | <LOQ |
| | 0.89 | n.a. | <LOQ | N.D. |
| | 0.93 | n.a. | 0.10 | 0.10 |
| | 1.00 | 7056B | 99.62 | 99.49 |
| | 1.10 | n.a. | N.D. | 0.03 |
| | 1.31 | n.a. | <LOQ | <LOQ |
| | 1.73 | n.a. | N.D. | <LOQ |
| | 1.78 | n.a. | <LOQ | <LOQ |
| | 1.84 | n.a. | <LOQ | <LOQ |
| | Total imps* (area%) | | 0.3 | 0.3 |

* Sum of all impurities ≥ 0.03% by area
Impurities are mean of 2 determinations
n.d. = not detected

### 3. Formulations - Potential Process Improvement with Dextran 40 Based Formulations

#### 3.1. Formulation compositions

Two CNS7056 formulations were prepared containing dextran (007/PAN/13), or a mixture of lactose and dextran 40(009/PAN/13) as summarized in the following table:

| Batch | Formulation | 7056:excipient(s) (mg) |
|---|---|---|
| 007/PAN/13 | 7056:dextran 40 | 50:440 |
| 009/PAN/13 | 7056:lactose monohydrate:dextran 40 | 50:88:352 |

#### 3.2. Formulation preparation

##### 3.2.1. Preparation of the solution

Dissolution of CNS7056B was facilitated by overhead stirring under ambient laboratory conditions, protected from light. A summary of the salient points for the preparation of each formulation are presented below.

007/PAN/13

- API addition (<5 minutes) to ~85% final volume
- Stirring started at 500 rpm and increased to 700 rpm by 120 minutes
- Adjusted to pH 3.0 after 70 minutes
- Increased to ~90% after 120 minutes
- Adjusted to pH 2.8 after 150 minutes
- After 180 minutes, checked pH (2.9), adjusted to pH 3.0, and made to final volume

009/PAN/13

- API addition (<5 minutes) to ~95% final volume
- Following API (500 rpm) stirring immediately increased to 700 rpm and then
- increased to 800 rpm by 30 minutes
- Adjusted to pH 3.0 after 10 minutes
- After 75 minutes, checked pH (pH 3.1), adjusted to pH 3.0, and made to final volume

Following preparation all formulations were filtered through a 0.22μm PVDF membrane filter

### 3.2.2. Lyophilisation protocol

Filtrates were filled in 4.2 mL volumes into 20 mL clear Type glass vials and lyophilised, directly from the shelf, with the cycle shown in the following table:

| Step | Cycle stage | Temperature (°C) | Pressure (mTorr) | Time (min) |
|---|---|---|---|---|
| 1 | Load | 25 | n/a | 0 |
| 2 | Ramp | 0 | n/a | 25 |
| 3 | Ramp | -45 | n/a | 225 |
| 4 | Freezing | -45 | n/a | 180 |
| 5 | Hold | -45 | 350 | 30 |
| 6 | Ramp | -15 | 350 | 60 |
| 7 | Primary drying | -15 | 350 | 2861 |
| 8 | Ramp | 30 | 20 | 112 |
| 9 | Secondary drying | 30 | 20 | 459 |
| 10 | Finish | 30 | Vials stoppered to 722000 mTorr with (pure) nitrogen. | |
| Total cycle duration ~ 66 hours (~ 2.8 days) | | | | |

### 3.3 Sample analysis

There was no significant difference in the appearance of the lyophilised plugs among batches 007 and 009/PAN/13. Lyophilised plugs appeared white/off-white, homogeneous and well formed.

Duplicate vials of each product were used for initial T = 0 testing, a summary of the analytical results is presented below.

### 3.3.1 Analysis after reconstitution

10 mL normal saline was added to a lyophilised sample, the vial was swirled and observed. The reconstitution time, reconstitution solution appearance, pH and purity (HPLC) were determined. The results are shown in the following table:

| Batch | Reconstitution time (s) | Reconstituted solution | | | |
|---|---|---|---|---|---|
| | | Appearance | pH | 7056 (%area) | 7054X (%area) |
| 007/PAN/13 | 91 | Clear, colourless, free from visible particulates | 3.3 | 99.58 | 0.10 |

(continued)

| Batch | Reconstitution time (s) | Reconstituted solution | | | |
|---|---|---|---|---|---|
| | | Appearance | pH | 7056 (%area) | 7054X (%area) |
| 009/PAN/13 | 81 | Clear, colourless, free from visible particulates | 3.2 | 99.60 | 0.08 |

### 3.3.2 Appearance

The appearance of the lyophilized samples was determined. The results are listed in the following table:

| Sample details | | | Appearance: |
|---|---|---|---|
| 007/PAN/13 | Initial (T = 0) | | Off white plug |
| | 40°C/75%RH | T = 1 m | Off white plug with signs of shrinkage |
| | 55°c | T = 1 m | Off white plug with signs of shrinkage |
| 009/PAN/13 | Initial (T = 0) | | Off white plug |
| | 40°C/75%RH | T = 1 m | Off white plug with signs of shrinkage |
| | 55°C | T = 1 m | Off white plug with signs of shrinkage |

### 3.3.3 Moisture

The moisture content of the lyophilized samples was determined. The results are listed in the following table:

| Sample details | | | Vial 1 | Vial 2 | Vial 3 | Mean moisture (% w/w) |
|---|---|---|---|---|---|---|
| 007/PAN/13 | Initial (T = 0) | | 0.00 | 0.00 | | **0.00** |
| | 40°C/75%RH | T = 1 m | 0.14 | 0.12 | - | **0.13** |
| | 55°C | T = 1 m | 0.24 | 0.32 | - | **0.28** |
| 009/PAN/13 | Initial (T = 0) | | 0.00 | 0.00 | | **0.00** |
| | 40°C/75%RH | T = 1 m | 0.20 | 0.21 | - | **0.21** |
| | 55°C | T = 1 m | 0.35 | 0.38 | - | **0.37** |

### 3.3.4 Reconstitution time and pH of reconstituted solution

Each vial was reconstituted with 10 mL 0.9% saline. The results regarding reconstitution time and pH are listed in the following table.

| Sample details | | | Recon. time (seconds) | pH |
|---|---|---|---|---|
| 007/PAN/13 | Initial (T = 0) | | 165* | 3.25 |
| | 40°C/75%RH | T = 1 m | 79 | 3.21 |
| | 55°C | T = 1 m | 62 | 3-28 |
| 009/PAN/13 | Initial (T = 0) | | 95* | 3.22 |
| | 40°C/75%RH | T = 1 m | 59 | 3.21 |
| | 55°C | T = 1 m | 50 | 3.21 |

### 3.3.5 Vial content

The vial content for the samples at T=1m 40°C/75%RH was determined after each vial was reconstituted with 10 mL 0.9% saline. The results are given in the following table:

| Sample details | | | Vial 1 | Vial 2 | Mean [7056] (mg/vial) | Recovery[1] (%) |
|---|---|---|---|---|---|---|
| 007/PAN/13 | Initial (T = 0) | | 48.6048 | 48.5855 | 48.595 | - |
| | 40°C/75%RH | T = 1 m | 48.0353 | 47.9755 | 48.005 | 98.8 |
| | 55°C | T = 1 m | 47.9019 | 48.6901 | 48.296 | 99.4 |
| 009/PAN/13 | Initial (T = 0) | | 48.9599 | 48.9696 | 48.965 | - |
| | 40°C/75%RH | T = 1 m | 48.4752 | 49.4228 | 48.949 | 100.0 |
| | 55°C | T = 1 m | 49.0987 | 48.3462 | 48.722 | 99.5 |
| Recovery is calculated as a percentage of T = 0 result. | | | | | | |

### 3.3.6 Impurities

The impurities for the different formulations at T=1m 40°C/75%RH were determined. The results are given in the following tables:

**007/PAN/13:**

| | | | Initial (T=0) | 40°C/75%RH | 55°c |
|---|---|---|---|---|---|
| | RRT | Name | T= 1m | T= 1m | T= 1m |
| Impurity profile (area%) | 0.36 | n.a. | N.D. | N.D. | <LOQ |
| | 0.41 | n.a. | <LOQ | N.D. | N.D. |
| | 0.46 | n.a. | <LOQ | <LOQ | <LOQ |
| | 0.51 | n.a. | 0.07 | 0.07 | 0.07 |
| | 0.57 | n.a. | N.D. | <LOQ | <LOQ |
| | 0.59 | 7054X | 0.10 | 0.16 | 0.43 |
| | 0.63 | n.a. | <LOQ | <LOQ | <LOQ |
| | 0.67 | n.a. | <LOQ | <LOQ | <LOQ |
| | 0.70 | n.a. | <LOQ | <LOQ | <LOQ |
| | 0.88 | n.a. | <LOQ | <LOQ | <LOQ |
| | 0.92 | n.a. | 0.11 | 0.11 | 0.11 |
| | 1.00 | 7056B | 99.58 | 99.54 | 99.23 |
| | 1.31 | n.a. | <LOQ | <LOQ | <LOQ |
| | 1.46 | ONO | N.D. | N.D. | <LOQ |
| | 1.73 | n.a. | <LOQ | <LOQ | <LOQ |
| | 1.77 | n.a. | <LOQ | N.D. | N.D. |
| | 1.79 | n.a. | <LOQ | <LOQ | <LOQ |
| | 1.84 | n.a. | <LOQ | N.D. | N.D. |
| | 1.86 | n.a. | N.D. | <LOQ | <LOQ |
| | Total imps* (area%) | | 0.3 | 0.3 | 0.6 |

* Sum of all impurities ≥ 0.03% by area
Impurities are mean of 2 determinations
n.d. = not detected

## 009/PAN/13:

| | | | Initial (T=0) | 40°C/75%RH | 55°c |
|---|---|---|---|---|---|
| | RRT | Name | | T= 1m | T= 1m |
| Impurity profile (area%) | 0.27 | n.a. | N.D. | N.D. | <LOQ |
| | 0.36 | n.a. | N.D. | N.D. | <LOQ |
| | 0.41 | n.a. | <LOQ | N.D. | N.D. |
| | 0.46 | n.a. | <LOQ | <LOQ | <LOQ |
| | 0.51 | n.a. | 0.07 | 0.07 | 0.07 |
| | 0.57 | n.a. | N.D. | <LOQ | <LOQ |
| | 0.59 | 7054X | 0.08 | 0.12 | 0.38 |
| | 0.63 | n.a. | <LOQ | <LOQ | <LOQ |
| | 0.67 | n.a. | <LOQ | <LOQ | <LOQ |
| | 0.70 | n.a. | <LOQ | <LOQ | <LOQ |
| | 0.88 | n.a. | <LOQ | <LOQ | <LOQ |
| | 0.92 | n.a. | 0.11 | 0.11 | 0.10 |
| | 1.00 | 7056B | 99.60 | 99.57 | 99.27 |
| | 1.31 | n.a. | <LOQ | <LOQ | <LOQ |
| | 1.47 | ONO | N.D. | N.D. | <LOQ |
| | 1.73 | n.a. | <LOQ | N.D. | N.D. |
| | 1.75 | n.a. | N.D. | <LOQ | <LOQ |
| | 1.77 | n.a. | <LOQ | N.D | N.D. |
| | 1.79 | n.a. | <LOQ | <LOQ. | <LOQ |
| | 1.84 | n.a. | <LOQ | N.D. | N.D. |
| | 1.86 | n.a. | N.D. | <LOQ | <LOQ |
| | Total imps* (area%) | | 0.3 | 0.3 | 0.6 |

* Sum of all impurities ≥ 0.03% by area
Impurities are mean of 2 determinations
n.d. = not detected

### 4. Further formulations

#### 4.1 Parameters:

- Fill concentration of CNS 7056 base = 12 mg/mL
- Vial size = 20R (30mm diameter)
- Fill solution volume 4.2 mL
- Approx 50 vials of each product to be prepared
- Also prepare placebos

#### 4.2 Formulation Compositions (Vial equivalent)

| | CNS 7056 (base equivalent) | Total Excipient | Dextran 40 | Lactose Monohydrate | Approx Ratio CNS7056: Lactose monohydrate |
|---|---|---|---|---|---|
| | | | **80** | **20** | |
| 012/PAN/13 | 50 mg | 440 mg | 352 mg | 88 mg | 1:9 |
| 011/PAN/13 | 50 mg | 330 mg | 264 mg | 66 mg | 1:6 |
| 010/PAN/13 | 50 mg | 220 mg | 176 mg | 44 mg | 1:4.5 |

Predicted Tc of formulations from Thermal Assessments = -15°C

### 4.3 Parameters

- Freezing to -30°C @ 0.2°C/min. Held
- Primary Drying -7°C @ 700-750m Torr
- Secondary Drying 30 °C

### 4.4 Test Criteria for analysis of lyo samples

- Appearance
- Recon time - addition of 10 mL saline
- Moisture
- Related substances
- Stability - 1m 55C + 1, 3m 40C/75%RH

#### 4.4.1 Appearance

The appearance of the lyophilized samples was determined. The results are listed in the following table:

| Sample details | | Appearance |
|---|---|---|
| **010/PAN/13** | **Initial (T = 0)** | Off white plug |
| **011/PAN/13** | **Initial (T = 0)** | Off white plug with material on vial will |
| **012/PAN/13** | **Initial (T = 0)** | Off white plug |

#### 4.4.2 Moisture

The moisture content of the lyophilized samples was determined. The results are listed in the following table:

| Sample details | | Vial 1 | Vial 2 | Vial 3 | Mean moisture (% w/w) |
|---|---|---|---|---|---|
| **010/PAN/13** | **Initial (T = 0)** | 0.08 | 0.02 | - | **0.05** |
| **011/PAN/13** | **Initial (T = 0)** | 0.05 | 0.05 | - | **0.05** |
| **012/PAN/13** | **Initial (T = 0)** | 0.11 | 0.06 | - | **0.09** |

#### 4.4.3 Reconstitution time and pH of reconstituted solution

Each vial was reconstituted with 10 mL 0.9% saline. The results regarding reconstitution time and pH are listed in the following table.

| Sample details | | | Recon. time (seconds) | pH |
|---|---|---|---|---|
| **010/PAN/13** | **Initial (T = 0)** | | 48 | 3.23 |
| | **40°C/75%RH** | **T = 1 m** | - | - |
| | **55°C** | **T = 1 m** | - | - |
| **011/PAN/13** | **Initial (T = 0)** | | 63 | 3.20 |
| | **40°C/75%RH** | **T = 1 m** | - | - |
| | **55°C** | **T = 1 m** | - | - |

(continued)

| Sample details | | | Recon. time (seconds) | pH |
|---|---|---|---|---|
| 012/PAN/13 | Initial (T = 0) | | 64 | 3.23 |
| | 40°C/75%RH | T = 1 m | - | - |
| | 55°C | T = 1 m | - | - |

#### 4.4.4 Vial content

The vial content for the samples at T=1m 40ºC/75%RH was determined after each vial was reconstituted with 10 mL 0.9% saline. The results are given in the following table:

| Sample details | | Vial 1 | Vial 2 | Mean [7056] (mg/vial) |
|---|---|---|---|---|
| 010/PAN/13 | Initial (T = 0) | 49.8316 | 49.4836 | 49.658 |
| 011/PAN/13 | Initial (T = 0) | 49.9339 | 49.1202 | 49.527 |
| 012/PAN/13 | Initial (T = 0) | 49.0690 | 47.6608 | 48.365 |

#### 4.4.5 Impurities

The impurities for the different formulations at T=1m 40ºC/75%RH were determined. The results are given in the following tables:

**010/PAN/13:**

| | RRT | Name | Initial (T=0) |
|---|---|---|---|
| Impurity profile (area%) | 0.31 | n.a. | <LOQ |
| | 0.47 | n.a. | <LOQ |
| | 0.52 | n.a. | 0.07 |
| | 0.57 | n.a. | <LOQ |
| | 0.59 | 7054X | 0.10 |
| | 0.65 | n.a. | <LOQ |
| | 0.68 | n.a. | <LOQ |
| | 0.72 | n.a. | <LOQ |
| | 0.89 | n.a. | <LOQ |
| | 0.93 | n.a. | 0.11 |
| | 1.00 | 7056B | 99.57 |
| | 1.14 | n.a. | 0.03 |
| | 1.31 | n.a. | <LOQ |
| | 1.74 | n.a. | <LOQ |
| | 1.80 | n.a. | <LOQ |
| | 1.86 | n.a. | <LOQ |
| | 2.00 | n.a. | <LOQ |
| | Total imps* (area%) | | 0.3 |

* Sum of all impurities ≥ 0.03% by area
Impurities are mean of 2 determinations
n.d. = not detected

011/PAN/13:

| | RRT | Name | Initial (T=0) |
|---|---|---|---|
| Impurity profile (area%) | 0.37 | n.a. | <LOQ |
| | 0.52 | n.a. | 0.07 |
| | 0.59 | 7054X | 0.10 |
| | 0.68 | n.a. | <LOQ |
| | 0.89 | n.a. | <LOQ |
| | 0.93 | n.a. | 0.11 |
| | 1.00 | 7056B | 99.56 |
| | 1.10 | n.a. | 0.03 |
| | 1.74 | n.a. | <LOQ |
| | 1.80 | n.a. | <LOQ |
| | 1.86 | n.a. | <LOQ |
| | 2.00 | n.a. | <LOQ |
| | Total imps* (area%) | | 0.3 |

* Sum of all impurities ≥ 0.03% by area
Impurities are mean of 2 determinations
n.d. = not detected

012/PAN/13:

| | RRT | Name | Initial (T=0) |
|---|---|---|---|
| Impurity profile (area%) | 0.31 | n.a. | <LOQ |
| | 0.47 | n.a. | <LOQ |
| | 0.52 | n.a. | 0.07 |
| | 0.59 | 7054X | 0.08 |
| | 0.64 | n.a. | <LOQ |
| | 0.68 | n.a. | <LOQ |
| | 0.71 | n.a. | <LOQ |
| | 0.89 | n.a. | <LOQ |
| | 0.93 | n.a. | 0.11 |
| | 1.00 | 7056B | 99.58 |
| | 1.14 | n.a. | 0.03 |
| | 1.31 | n.a. | <LOQ |
| | 1.74 | n.a. | <LOQ |
| | 1.80 | n.a. | <LOQ |
| | 1.86 | n.a. | <LOQ |
| | Total imps* (area%) | | 0.3 |

* Sum of all impurities ≥ 0.03% by area
Impurities are mean of 2 determinations
n.d. = not detected

### 5. Further formulations

### 5.1 Parameters:

- Fill concentration of CNS 7056 base = 12 mg/mL
- Vial size = 20R (30mm diameter)
- Fill solution volume 4.2 mL
- Approx 50 vials of each product to be prepared

- Also prepare placebos

**5.2 Formulation Compositions (Vial equivalent)**

| | CNS 7056 (base equivalent) | Total Excipient | Dextran 40 | Lactose Monohydrate | Approx Ratio CNS7056: Lactose monohydrate |
|---|---|---|---|---|---|
| | | | **60** | **40** | |
| 015/PAN/13 | 50 mg | 440 mg | 264 mg | 176 mg | 1:9 |
| 014/PAN/13 | 50 mg | 330 mg | 198 mg | 132 mg | 1:6 |
| 013/PAN/13 | 50 mg | 220 mg | 132 mg | 88 mg | 1:4.5 |

Predicted Tc of formulations from Thermal Assessments = -19°C

**5.3 Parameters**

- Freezing to -30°C @ 0.2°C/min
- Primary Drying -15°C @ 400mTorr
- Secondary Drying 30 °C

**5.4 Test Criteria for analysis of lyo samples**

- Appearance
- Recon time - addition of 10 mL saline
- Moisture
- Related substances
- Stability - 1m 55C + 1, 3m 40C/75%RH

**5.4.1 Appearance**

The appearance of the lyophilized samples was determined. The results are listed in the following table:

| Sample details | | Appearance |
|---|---|---|
| **013/PAN/13** | **Initial (T = 0)** | Off white plug some Material on walls of vial |
| **014/PAN/13** | **Initial (T = 0)** | Off white plug some material on walls of vial |
| **0152/PAN/13** | **Initial (T = 0)** | Off white plug some material on walls of vial |

**5.4.2 Moisture**

The moisture content of the lyophilized samples was determined. The results are listed in the following table:

| Details | | Vial 1 | Vial 2 | Vial 3 | Mean Moisture (% w/w) |
|---|---|---|---|---|---|
| **013/PAN/13** | **Initial (T = 0)** | 0.06 | 0.05 | - | **0.06** |
| **014TAN/13** | **Initial (T =0)** | 0.00 | 0.09 | | **0.09** |
| **015/PAN/13** | **Initial (T = 0)** | 0.09 | 0.00 | - | **0.09** |

### 5.4.3 Reconstitution time and pH of reconstituted solution

Each vial was reconstituted with 10 mL 0.9% saline. The results regarding reconstitution time and pH are listed in the following table.

| Sample details | | Recon. time (seconds) | PH |
|---|---|---|---|
| **013/PAN/13** | **Initial (T = 0)** | 39 | 3.21 |
| **014/PAN/13** | **Initial (T = 0)** | 35 | 3.22 |
| **015PAN/13** | **Initial (T = 0)** | 43 | 3.24 |

### 5.4.4 Vial content

The vial content for the samples at T=1m 40ºC/75%RH was determined after each vial was reconstituted with 10 mL 0.9% saline. The results are given in the following table:

| Sample details | | Vial 1 | Vial 2 | Mean [7056] (mg/vial) |
|---|---|---|---|---|
| **013/PAN/13** | **Initial (T = 0)** | 48.1356 | 48.5325 | 48.334 |
| **014/PAN/13** | **Initial (T = 0)** | 49.9574 | 49.7535 | 49.855 |
| **015/PAN/13** | **Initial (T = 0)** | 48.3542 | 47.9459 | 48.150 |

### 5.4.5 Impurities

The impurities for the different formulations at T=1m 40ºC/75%RH were determined. The results are given in the following tables:

**013/PAN/13:**

| | RRT | Name | Initial (T=0) |
|---|---|---|---|
| Impurity profile (area%) | 0.31 | n.a. | <LOQ |
| | 0.47 | n.a. | <LOQ |
| | 0.52 | n.a. | 0.07 |
| | 0.59 | 7054X | 0.09 |
| | 0.64 | n.a. | <LOQ |
| | 0.68 | n.a. | <LOQ |
| | 0.71 | n.a. | <LOQ |
| | 0.89 | n.a. | <LOQ |
| | 0.93 | n.a. | 0.11 |
| | 1.00 | 7056B | 99.60 |
| | 1.31 | n.a. | <LOQ |
| | 1.74 | n.a. | <LOQ |
| | 1.77 | n.a. | <LOQ |
| | 1.79 | n.a. | <LOQ |
| | 1.85 | n.a. | <LOQ |
| | **Total imps* (area%)** | | **0.3** |

* Sum of all impurities ≥ 0.03% by area
Impurities are mean of 2 determinations
n.d. = not detected

**014/PAN/13:**

| | RRT | Name | Initial (T=0) |
|---|---|---|---|
| Impurity profile (area%) | 0.31 | n.a. | <LOQ |
| | 0.47 | n.a. | <LOQ |
| | 0.51 | n.a. | 0.07 |
| | 0.58 | n.a. | <LOQ |
| | 0.59 | 7054X | 0.09 |
| | 0.64 | n.a. | <LOQ |
| | 0.68 | n.a. | <LOQ |
| | 0.71 | n.a. | <LOQ |
| | 0.89 | n.a. | <LOQ |
| | 0.92 | n.a. | 0.11 |
| | 1.00 | 7056B | 99.61 |
| | 1.31 | n.a. | <LOQ |
| | 1.74 | n.a. | <LOQ |
| | 1.79 | n.a. | <LOQ |
| | 1.85 | n.a. | <LOQ |
| | Total imps* (area%) | | 0.3 |

* Sum of all impurities ≥ 0.03% by area
Impurities are mean of 2 determinations
n.d. = not detected

**015/PAN/13:**

| | RRT | Name | Initial (T=0) |
|---|---|---|---|
| Impurity profile (area%) | 0.31 | n.a. | <LOQ |
| | 0.47 | n.a. | <LOQ |
| | 0.51 | n.a. | 0.07 |
| | 0.56 | n.a. | <LOQ |
| | 0.59 | 7054X | 0.08 |
| | 0.64 | n.a. | <LOQ |
| | 0.68 | n.a. | <LOQ |
| | 0.71 | n.a. | <LOQ |
| | 0.89 | n.a. | <LOQ |
| | 0.92 | n.a. | 0.11 |
| | 1.00 | 7056B | 99.61 |
| | 1.31 | n.a. | <LOQ |
| | 1.74 | n.a. | <LOQ |
| | 1.79 | n.a. | <LOQ |
| | 1.85 | n.a. | <LOQ |
| | Total imps* (area%) | | 0.3 |

* Sum of all impurities ≥ 0.03% by area
Impurities are mean of 2 determinations
n.d. = not detected

## V. THERMAL ANALYSIS OF REMIMAZOLAM FORMULATIONS

### 1. Purpose of the study

In order to increase the lyophilisation temperature, the relative amount of dextran of the lactose:dextran mixture was increased and the critical temperature was determined by differential scanning calorimetry (DSC) and freeze drying microscopy (FDM), for CNS7056B formulations 1 - 6 as shown in the following Table.

| Formulation | $T_g$' by DSC (°C) | $T_g$ by FDM (°C) |
|---|---|---|
| 001 | -13 | -11 |
| 002 | -23 | -21 |
| 003 | -28 | -27 |
| 004 | -24 | -20 |
| 005 | -29 | -28 |
| 006 | -29 | -27 |

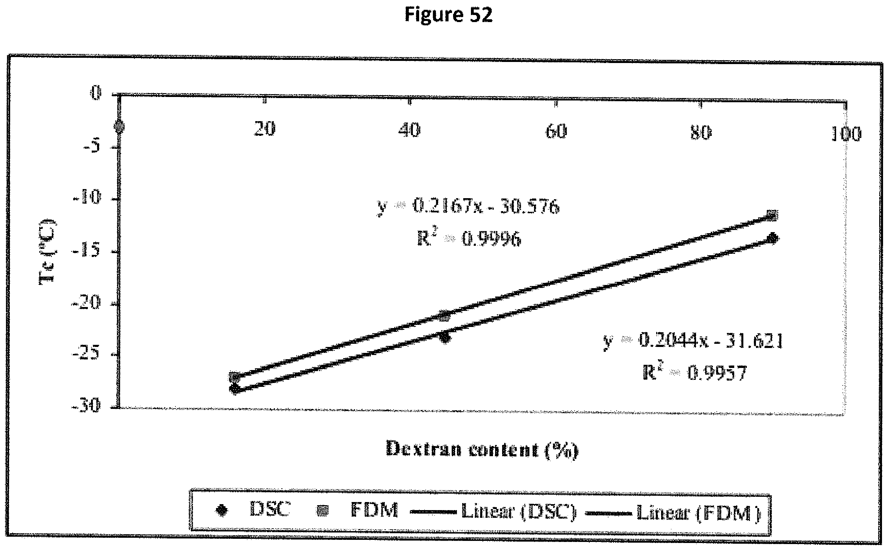

Critical temperatures were plotted with respect to dextran, relative to total formulation solute content and shown in Figure. 52.

From the linear equations (Figure 52), for a given critical temperature, the theoretical dextran content of a CNS7056B formulation can be calculated. As shown in the following table, there was a good correlation between data generated for formulations containing lactose.

| Target Tc (°C) | 2nd excipient | Technique | Theoretical dextran (%) |
|---|---|---|---|
| -20 | Lactose | DSC | 48.8 |
| | | FDM | 56.8 |
| | | | |
| | DSC mean | | 49 |
| | FDM mean | | 58 |
| -17.5 | Lactose | DSC | 60.3 |
| | | FDM | 69.1 |
| | | | |
| | DSC mean | | 60 |
| | FDM mean | | 70 |
| -15 | Lactose | DSC | 71.9 |
| | | FDM | 81.3 |
| | | | |
| | DSC mean | | 71 |
| | FDM mean | | 82 |

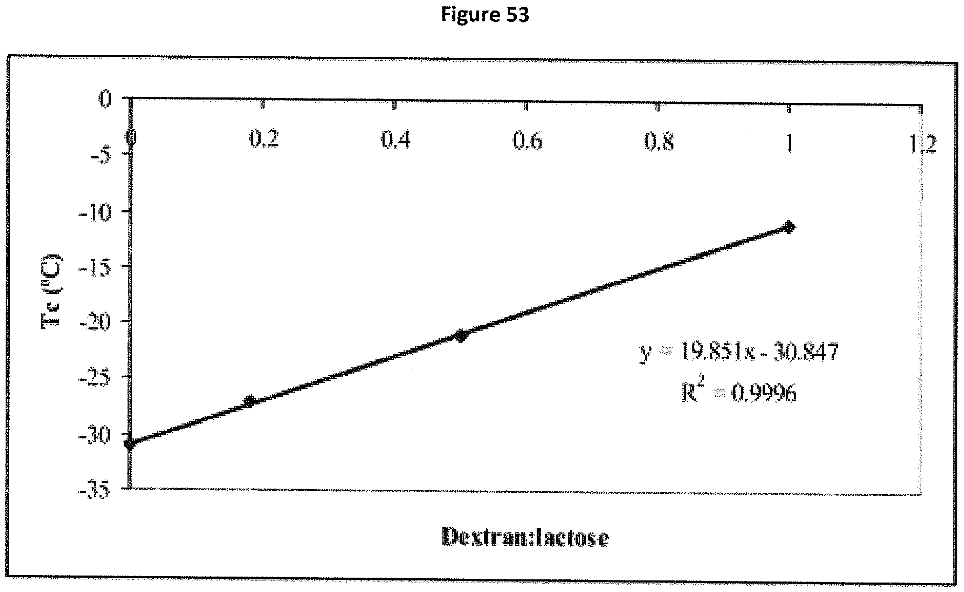

An alternative presentation of the data, expressing collapse temperature relative to the dextran:lactose ratio in each formulation is shown Figure 53. The Phase I/II sedation formulation was used to represent a formulation containing no dextran (zero on the abscissa). The collapse temperature onset for this formulation has been reported as -31ºC.

Similarly, the linear equation from Figure 53 may be used to calculate the theoretical dextran:lactose composition of CNS7056B formulations for given collapse temperatures as shown in the following table:

| Target Tc (°C) | Theoretical excipient composition (%) | | Example formulation API:lactose:dextran (mg/vial) |
|---|---|---|---|
| | Lactose | Dextran | |
| -20 | 45.4 | 54.6 | 50:200:240 |
| -17.5 | 32.8 | 67.2 | 50:145:295 |
| -15 | 20.2 | 79.8 | 50:90:350 |

**Figure Legends**

| | |
|---|---|
| Fig. 1: | Excipients |
| Fig. 2: | Active formulations |
| Fig. 3: | Placebo formulations |
| Fig. 4: | Formulation of hydrolysis degradant of remimazolam (REM) given in % after storage for 13 weeks at 25°C/60% relative humidity (RH) or 40 °C/75% RH. |
| Fig. 5A-D: | Crystallographic co-ordinates and other relevant data tabulated in the form of a SHELX File for Compound of formula (I) besylate Form 1 of WO2008/007071 A1 |
| Fig. 6A-C: | Crystallographic co-ordinates and other relevant data tabulated in the form of a SHELX File for Compound of formula (I) besylate Form 2 of WO2008/007071 A1. |
| Fig. 7A-B: | Bond lengths for Compound of formula (I) besylate Form 1 of WO2008/007071 A1 |
| Fig. 8A-C: | Bond angles for Compound of formula (I) besylate Form 1 of WO2008/007071 A1 |
| Fig. 9: | Bond Lengths for Compound of formula (I) besylate Form 2 of WO2008/007071 A1 |
| Fig. 10: | Bond angles for Compound of formula (I) besylate Form 2 of WO2008/007071 A1 |
| Fig. 11: | Stability data for lot A01P310 |
| Fig. 12: | Stability data for lot A01P310, continued |
| Fig. 13: | Accelerated stability data for lot A01P310 |
| Fig. 14: | Accelerated stability data for lot A01P310, continued |
| Fig. 15: | Long term stability data for lot P310-01 |
| Fig. 16: | Long term stability data for lot P310-01, continued |
| Fig. 17: | Accelerated stability data for lot P310-01 |
| Fig. 18: | Accelerated stability data for lot P310-01, continued |
| Fig. 19: | Long term stability data for lot 026CNS27 |
| Fig. 20: | Long term stability data for lot 026CNS27, continued |
| Fig. 21: | Accelerated stability data for lot 026CNS27 |
| Fig. 22: | Accelerated stability data for lot 026CNS27, continued |
| Fig. 23: | Long term stability data for lot G384 |
| Fig. 24: | Long term stability data for lot G384, continued |
| Fig. 25: | Accelerated stability data for lot G384 |
| Fig. 26: | Accelerated stability data for lot G384, continued |
| Fig. 27: | Long term stability data for lot P02308 |
| Fig. 28: | Long term stability data for lot P02308, continued |
| Fig. 29: | Accelerated stability data for lot P02308 |
| Fig. 30: | Accelerated stability data for lot P02308, continued |
| Fig. 31: | Long term stability data for lot 25CNS27 |
| Fig. 32: | Long term stability data for lot 25CNS27, continued |
| Fig. 33: | Long term stability data for lot 25CNS27, continued |
| Fig. 34: | Accelerated stability data for lot 25CNS27 |
| Fig. 35: | Accelerated stability data for lot 25CNS27, continued |
| Fig. 36: | Accelerated stability data for lot 25CNS27, continued |
| Fig. 37: | Long term stability data (T=36M) for lot P02308 |
| Fig. 38: | Long term stability data (T=36M) for lot P02308, continued |
| Fig. 39: | Raman spectra of each component in a lyophilized formulation: each rectangle range showing distinctive peak(s) of crystalline and lyophilized CNS7056B (L) and lyophilized lactose (R). |
| Fig. 40: | Raman spectra of each component in a lyophilized formulation of CNS7056B in lactose: crystalline CNS7056B (top), lyophilized (amorphous) CNS7056B (middle) and lyophilized lactose (amorphous) (bottom). |
| Fig. 41: | Representative Raman spectra of crystalline CNS7056B (top 3) selected within the Raman mapping area of the lyophilized formulation and pure crystalline (Form I) CNS7056B (bottom) as a reference. |
| Fig. 42: | Table summarizing the results for the stability study of the Lots 12PM529-8-1, 12PM529-8-2, 12PM529-9-1 and PM0232/12 at initial time point t=0 |
| Fig. 43: | Table summarizing the results for the stability study of the 12PM529-8-2, 12PM529-9-1 and PM0232/12 at time point t = 4 weeks |
| Fig. 44: | Table summarizing the results for the stability study of the Lots 12PM529-8-1, 12PM529-8-2, 12PM529-9-1 and PM0232/12 at time point t = 13 weeks |

Fig. 45 Table summarizing the results for the stability study of the Lot L10R5

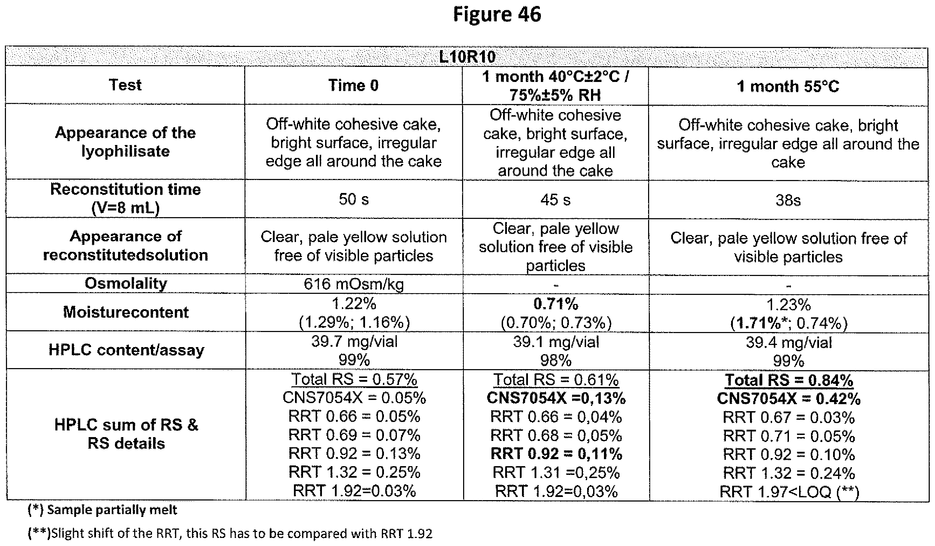

Fig. 46 Table summarizing the results for the stability study of the Lot L10R10

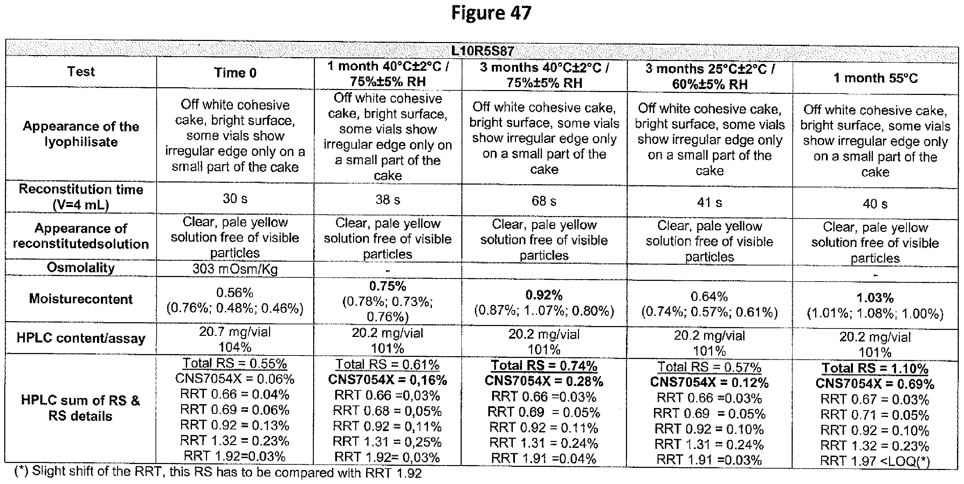

Fig. 47 Table summarizing the results for the stability study of the Lot L10R5S87

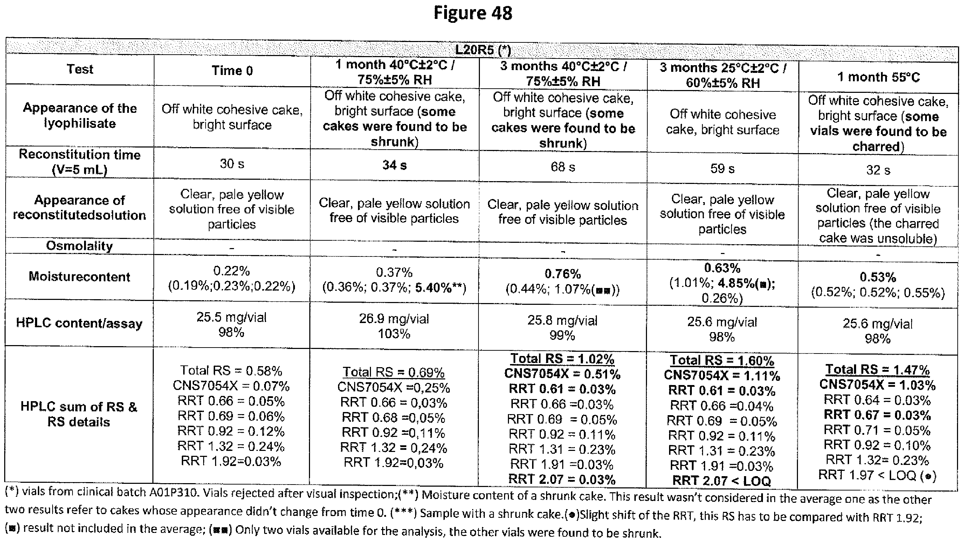

Fig. 48 Table summarizing the results for the stability study of the Lot L20R5

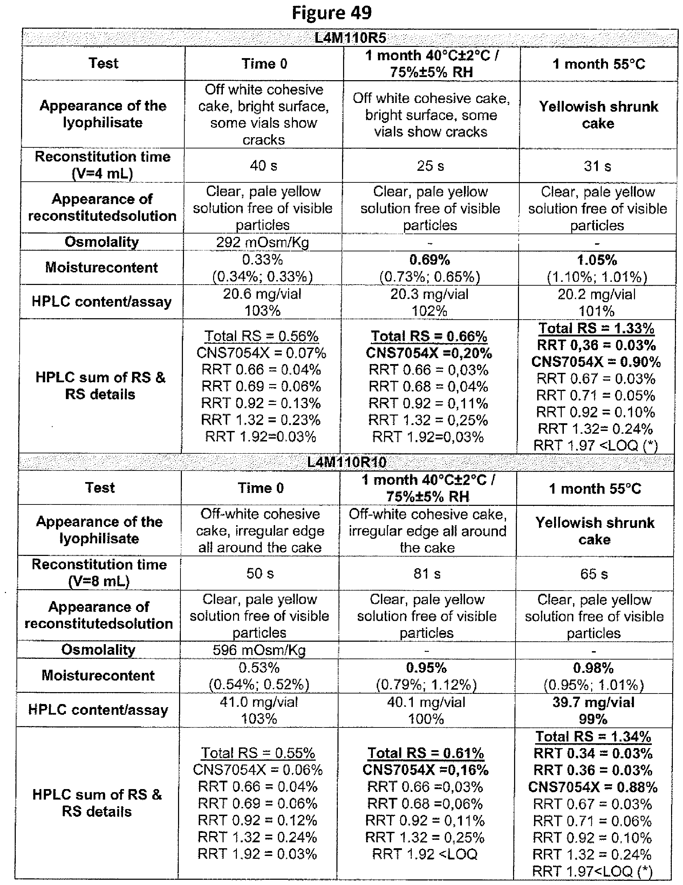

Fig. 49 Table summarizing the results for the stability study of the Lots L4M110R5 and L4M110R10

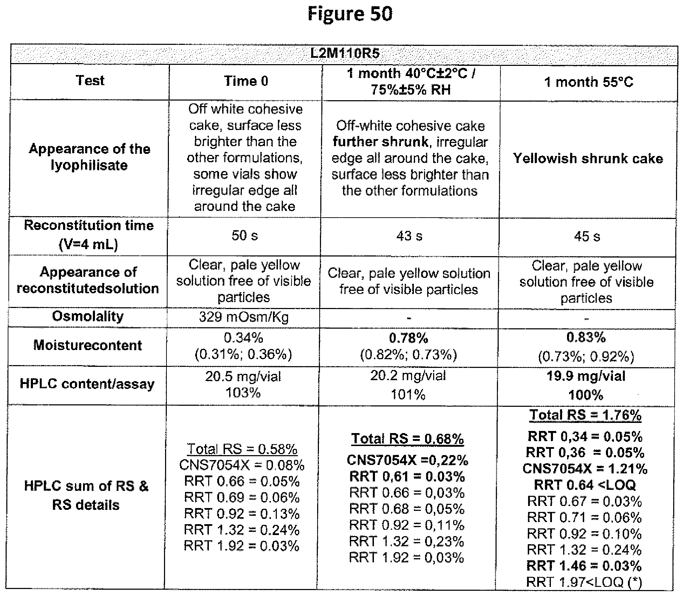

Fig. 50 Table summarizing the results for the stability study of the Lot L2M110R5

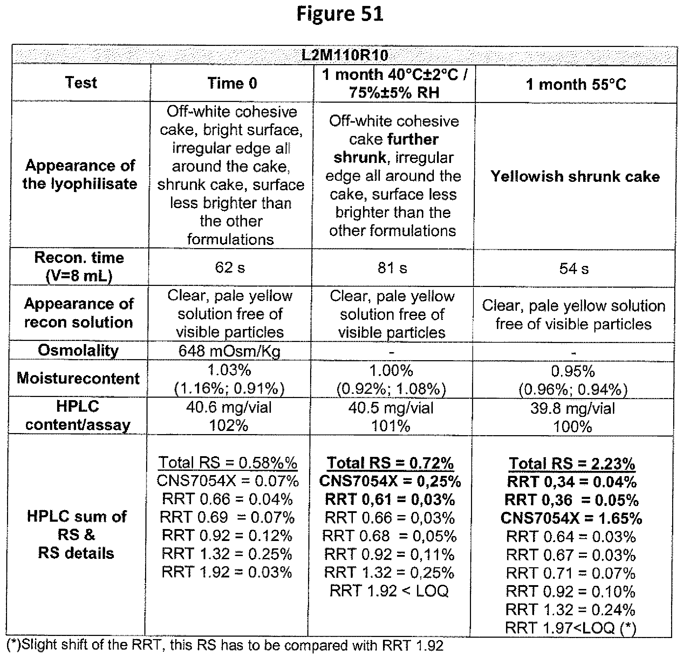

Fig. 51 Table summarizing the results for the stability study of the Lot L2M110R10
Fig. 52 Critical temperature as a function of dextran content for CNS7057B:lactose:dextran formulations
Fig. 53 Collapse temperature relative to the dextran:lactose ratio for CNS7056B formulations

**Claims**

**1.** A lyophilized or spray-dried composition comprising at least one benzodiazepine according to formula (I)

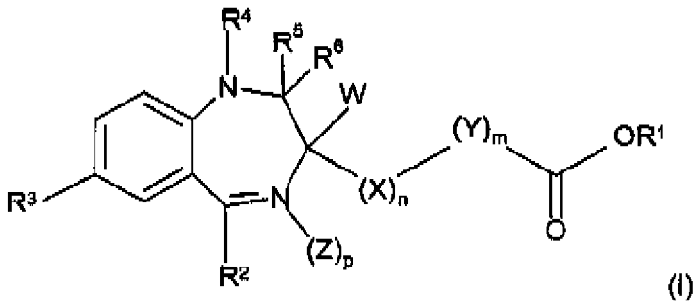

(I)

with

W is H;
X is $CH_2$; n is 1;
Y is $CH_2$; m is 1;
Z is O;
$R^1$ is $CH_3$, $CH_2CH_3$, $CH_2CH_2CH_3$, $CH(CH_3)_2$ or $CH_2CH(CH_3)_2$;
$R^2$ is 2-fluorophenyl, 2-chlorophenyl or 2-pyridyl;
$R^3$ is Cl or Br;
$R^4$, $R^5$ and $R^8$ form the group-$CR^8$=U-V= wherein $R^8$ is hydrogen, $C_{1-4}$ alkyl or $C_{1-3}$ hydroxyalkyl, U is N or $CR^9$ wherein $R^9$ is H, $C_{1-4}$ alkyl, $C_{1-3}$ hydroxyalkyl or $C_{1-4}$ alkoxy, V is N or CH and p is zero

or a pharmaceutically acceptable salt thereof, wherein the composition

comprises at least one pharmaceutically acceptable hygroscopic excipient, wherein the hygroscopic excipient is a carbohydrate selected from the group consisting of disaccharides and dextran and optionally the composition is at least in part amorphous.

**2.** A composition according to claim 1, wherein the benzodiazepine according to formula (I) is methyl 3-[(4S)-8-bromo-1-methyl-6-(pyridin-2-yl)-4H-imidazo[1,2-a][1,4]benzadiazepin-4-yl]prapanoate (remimazolam).

**3.** A composition according to claim 2, wherein in the pharmaceutically acceptable salt of the benzodiazepine is formulated in cationic form and the counter ion is benzene sulfonate (besylate).

**4.** A composition according to any of claims 1 to 3, wherein the dextran possesses a molecular weight of less than 150 kD.

**5.** A composition according to any of claims 1 to 3, wherein the disaccharide is selected from the group consisting of lactose, maltose, sucrose and trehalose.

**6.** A composition according to any of claims 1 to 3, wherein the composition comprises a mixture of a first hygroscopic excipient and a second hygroscopic excipient.

7. A composition according to claim 6, wherein the composition comprises a mixture of a disaccharide and dextran, preferably a mixture of lactose and dextran.

8. A composition according to claim 7, wherein the wt% ratio between lactose and dextran is from 1:1.0 to 1:10, preferably from 1:1.2 to 1:5, and more preferably is 1:1.5 or 1:4.

9. A composition according to any of claims 1 to 3, wherein the wt% ratio between the total amount of hygroscopic excipients and the total amount of benzodiazepines or salts thereof, calculated for the base, in the composition is from 20:1 to 1:1, preferably from 12:1 to 3:1, most preferably from 9: 1 to 3:1.

10. A composition according to any of claims 1 to 3, which is a pharmaceutical formulation.

11. Use of a mixture of at least one disaccharide and at least one dextran for preparing a lyophilized or spray dried pharmaceutical composition comprising a benzodiazepine with a carboxylic acid ester moiety as referred to in claim 1 or a pharmaceutically acceptable salt thereof, in particular remimazolam.

12. Method of preparing a pharmaceutical composition comprising the steps of

a) providing a solution comprising a benzodiazepine as referred to in claim 1 or a pharmaceutically acceptable salt thereof, in particular remimazolam;
b) adding at least one hygroscopic excipient, which is a carbohydrate selected from the group consisting of disaccharides and dextran or a mixture thereof with at least another hygroscopic excipient;
c) drying the solution from step b) by lyophilization or spray-drying.

## Patentansprüche

1. Lyophilisierte oder sprühgetrocknete Zusammensetzung, umfassend wenigstens ein Benzodiazepin gemäß der Formel (I)

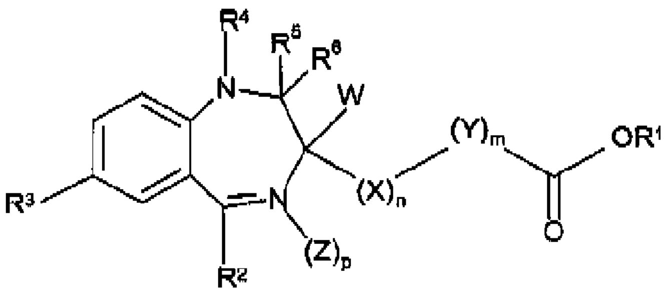

(I)

,

wobei

W H ist;
X $CH_2$ ist; n 1 ist;
Y $CH_2$ ist; m 1 ist;
Z O ist;
$R^1$ $CH_3$, $CH_2CH_3$, $CH_2CH_2CH_3$, $CH(CH_3)_2$ oder $CH_2CH(CH_3)_2$ ist;
$R^2$ 2-Fluorphenyl, 2-Chlorphenyl oder 2-Pyridyl ist;
R3 Cl oder Br ist;
$R^4$, $R^5$ und $R^6$ die Gruppe -$CR^8$=U-V= bildet, wobei $R^8$ Wasserstoff, $C_{1-4}$-Alkyl oder $C_{1-3}$-Hydroxyalkyl ist, U N oder $CR^9$ ist, wobei $R^9$ H, $C_{1-4}$-Alkyl, $C_{1-3}$-Hydroxyalkyl oder $C_{1-4}$-Alkoxy ist, V N oder CH ist, und p Null ist;
oder ein pharmazeutisch akzeptables Salz davon, wobei die Zusammensetzung

wenigstens einen pharmazeutisch akzeptablen hygroskopischen Arzneiträger umfasst, wobei der hygroskopische Arzneiträger ein Kohlenhydrat ist, das ausgewählt ist aus der Gruppe bestehend aus Disacchariden und Dextran, und wobei die Zusammensetzung optional wenigstens teilweise amorph ist.

2. Zusammensetzung nach Anspruch 1, wobei das Benzodiazepin gemäß Formel (I) Methyl 3-[(4S)-8-brom-1-methyl-6-(pyridin-2-yl)-4H-imidazol[1,2-a][1,4]benzodiazepin-4-yl]propanoat (Remimazolam) ist.

3. Zusammensetzung nach Anspruch 2, wobei das pharmazeutisch akzeptable Salz des Benzodiazepins in kationischer Form formuliert ist, und wobei das Gegenion Benzolsulfonat (Besylat) ist.

4. Zusammensetzung nach einem der Ansprüche 1 bis 3, wobei das Dextran ein Molekulargewicht von 150 kD besitzt.

5. Zusammensetzung nach einem der Ansprüche 1 bis 3, wobei das Disaccharid ausgewählt ist aus der Gruppe bestehend aus Laktose, Maltose, Saccharose, und Trehalose.

6. Zusammensetzung nach einem der Ansprüche 1 bis 3, wobei die Zusammensetzung ein Gemisch aus einem ersten hygroskopischen Arzneiträger und einem zweiten hygroskopischen Arzneiträger umfasst.

7. Zusammensetzung nach Anspruch 6, wobei die Zusammensetzung ein Gemisch aus Disaccharid und Dextran umfasst, vorzugsweise ein Gemisch aus Laktose und Dextran.

8. Zusammensetzung nach Anspruch 7, wobei das Gewichtsverhältnis zwischen Laktose und Dextran zwischen 1:1,0 und 1:10 liegt, vorzugsweise zwischen 1:1,2 und 1:5 und darüber hinaus bevorzugt 1:1,5 oder 1:4.

9. Zusammensetzung nach einem der Ansprüche 1 bis 3, wobei das Gewichtsverhältnis zwischen der Gesamtmenge hygroskopischer Arzneiträger und der Gesamtmenge an Benzodiazepinen oder Salzen davon, berechnet für die Base, in der Zusammensetzung zwischen 20:1 und 1:1 liegt, vorzugsweise zwischen 12:1 und 3:1, wobei ein Wert zwischen 9:1 und 3:1 am meisten bevorzugt ist.

10. Zusammensetzung nach einem der Ansprüche 1 bis 3, bei der es sich um eine pharmazeutische Formulierung handelt.

11. Verwendung eines Gemischs aus wenigstens einem Disaccharid und wenigstens einem Dextran für die Herstellung einer lyophilisierten oder sprühgetrockneten pharmazeutischen Zusammensetzung, umfassend ein Benzodiazepin mit einem Carbonsäureesteranteil gemäß Anspruch 1 oder ein pharmazeutisch akzeptables Salz davon, insbesondere Remimazolam.

12. Verfahren zur Herstellung einer pharmazeutischen Zusammensetzung, die folgenden Schritte umfassend:

a) Bereitstellen einer Lösung, umfassend ein Benzodiazepin nach Anspruch 1 oder ein pharmazeutisch akzeptables Salz davon, insbesondere Remimazolam;
b) Zugabe wenigstens eines hygroskopischen Arzneiträgers, bei dem es sich um ein Kohlenhydrat handelt, das ausgewählt ist aus der Gruppe bestehend aus Disacchariden und Dextran oder einem Gemisch davon mit wenigstens einem weiteren hygroskopischen Arzneiträger;
c) Trocknen der Lösung aus Schritt b) durch Lyophilisation oder Sprühtrocknen.

## Revendications

1. Composition lyophilisée ou séchée par atomisation comprenant au moins une benzodiazépine répondant à la formule (I)

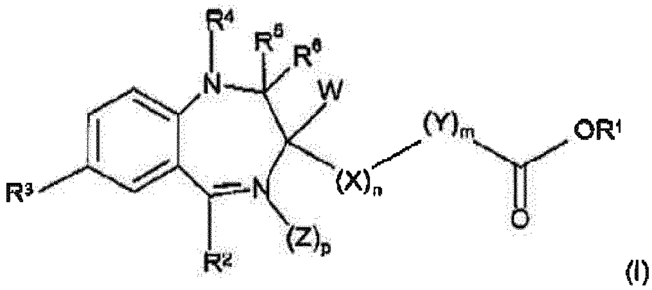

(I)

où

W représente H ;
X représente $CH_2$; n est égal à 1 ;
Y représente $CH_2$; m est égal à 1 ;
Z représente 0 ;
$R^1$ représente $CH_3$, $CH_2CH_3$, $CH_2CH_2CH_3$, $CH(CH_3)_2$ ou $CH_2CH(CH_3)_2$ ;
$R^2$ représente un groupement 2-fluorophényle, 2-chlorophényle ou 2-pyridyle ;
$R^3$ représente Cl ou Br ;
$R^4$, $R^5$ et $R^6$ forment le groupement $-CR^8=U-V=$ où $R^6$ représente un atome d'hydrogène ou un groupement alkyle en $C_{1-4}$ ou hydroxyalkyle en $C_{1-3}$, U représente N ou $CR^9$ où $R^9$ représente H ou un groupement alkyle en $C_{1-4}$, hydroxyalkyle en $C_{1-3}$ ou alkoxy en $C_{1-4}$, V représente N ou CH et p est égal à zéro ou sel pharmaceutiquement acceptable de celui-ci, où la composition comprend au moins un excipient hygroscopique pharmaceutiquement acceptable, où l'excipient hygroscopique est un hydrate de carbone choisi dans le groupe constitué par les disaccharides et le dextrane, et éventuellement, la composition est au moins en partie amorphe.

**2.** Composition selon la revendication 1, où la benzodiazépine répondant à la formule (I) est le 3-[(4S)-8-bromo-1-méthyl-6-(pyridin-2-yl)-4H-imidazo[1,2-a][1,4]benzodiazépin-4-yl]propanoate de méthyle (rémimazolam).

**3.** Composition selon la revendication 2, où le sel pharmaceutiquement acceptable de la benzodiazépine est formulé sous forme cationique et le contre-ion est le benzènesulfonate (bésylate).

**4.** Composition selon l'une quelconque des revendications 1 à 3, où le dextrane présente une masse moléculaire de moins de 150 kD.

**5.** Composition selon l'une quelconque des revendications 1 à 3, où le disaccharide est choisi dans le groupe constitué par le lactose, le maltose, le sucrose et le tréhalose.

**6.** Composition selon l'une quelconque des revendications 1 à 3, où la composition comprend un mélange d'un premier excipient hygroscopique et d'un second excipient hygroscopique.

**7.** Composition selon la revendication 6, où la composition comprend un mélange d'un disaccharide et de dextrane, préférentiellement d'un mélange de lactose et de dextrane.

**8.** Composition selon la revendication 7, où le rapport massique en % entre le lactose et le dextrane est compris entre 1:1,0 et 1:10, préférentiellement entre 1:1,2 et 1:5, et plus préférentiellement est de 1:1,5 ou de 1:4.

**9.** Composition selon l'une quelconque des revendications 1 à 3, où le rapport massique en % entre la quantité totale d'excipients hygroscopiques et la quantité totale de benzodiazépines ou de sels de celui-ci, calculée pour la base, dans la composition, est compris entre 20:1 et 1:1, préférentiellement entre 12:1 et 3:1, le plus préférentiellement entre 9:1 et 3:1.

**10.** Composition selon l'une quelconque des revendications 1 à 3, qui est une formule pharmaceutique.

**11.** Utilisation d'un mélange d'au moins un disaccharide et d'au moins un dextrane pour la préparation d'une composition pharmaceutique lyophilisée ou séchée par atomisation comprenant une benzodiazépine avec une entité ester d'acide carboxylique selon la revendication 1 ou un sel pharmaceutiquement acceptable de celle-ci, en particulier le rémimazolam.

**12.** Procédé d'élaboration d'une composition pharmaceutique comprenant les étapes suivantes :

a) obtention d'une solution comprenant une benzodiazépine selon la revendication 1 ou un sel pharmaceutiquement acceptable de celle-ci, en particulier le rémimazolam ;
b) ajout d'au moins un excipient hygroscopique, qui est un hydrate de carbone choisi dans le groupe constitué par les disaccharides et le dextrane ou un mélange de ceux-ci avec au moins un autre excipient hygroscopique ;
c) séchage de la solution de l'étape b) par lyophilisation ou séchage par atomisation.

# Fig. 1

| Material | Supplier |
|---|---|
| Lactose monohydrate PhEur | DMV-Frontera Excipients |
| Mannitol PhEur | S. Black Ltd |
| Trehalose | Sigma-Aldrich |
| Sucrose PhEur | Fisher Scientific Ltd |
| Maltose monohydrate PhEur | S. Black Ltd |
| Dextran 70kD | Sigma-Aldrich |
| Povidone K17 | IMCD Ltd |
| Glycine | Fisher Scientific Ltd |
| Hydrochloric acid SG 1.18 | BDH Ltd |
| 2M Sodium hydroxide | Fisher Scientific Ltd |
| Water for injection | Baxter Healthcare |

# Fig. 2

| Formulation number | Formulation | REM mg/vial | excipient 1 mg/vial | excipient 2 mg/vial |
|---|---|---|---|---|
| 01 | REM only | 16.32 | -- | -- |
| 02 | REM/lactose (1:9) | 8.16 | 73.44 | -- |
| 03 | REM/lactose (1:4.5) | 8.16 | 36.72 | -- |
| 04 | REM/lactose (1:1) | 8.16 | 8.16 | -- |
| 05 | REM/mannitol (1:9) | 8.16 | 73.44 | -- |
| 06 | REM/trehalose (1:9) | 8.16 | 73.44 | -- |
| 07 | REM/sucrose (1:9) | 8.16 | 73.44 | -- |
| 08 | REM/maltose (1:9) | 8.16 | 73.44 | -- |
| 09 | REM/dextran (1:9) | 8.16 | 73.44 | -- |
| 10 | REM/lactose/mannitol (1:4.5:4.5) | 8.16 | 36.72 | 36.72 |
| 11 | REM/povidone (1:9) | 8.16 | 73.44 | -- |
| 12 | REM/glycine (1:9) | 8.16 | 73.44 | -- |

Fig. 3

| Formulation number | Formulation | excipient 1 mg/vial | excipient 2 mg/vial |
|---|---|---|---|
| 02P | lactose | 73.44 | -- |
| 05P | mannitol | 73.44 | -- |
| 06P | trehalose | 73.44 | -- |
| 07P | sucrose | 73.44 | -- |
| 08P | maltose | 73.44 | -- |
| 09P | dextran | 73.44 | -- |
| 10P | lactose/mannitol | 36.72 | 36.72 |
| 11P | povidone | 73.44 | -- |
| 12P | glycine | 73.44 | -- |

# Fig. 4

| Formulation number | Formulation | 25°C/60%RH | 40°C/75%RH |
|---|---|---|---|
| 01 | REM only | 1.06 | 8.56 |
| 02 | REM/lactose (1:9) | 0.13 | 0.45 |
| 03 | REM/lactose (1:4.5) | 0.20 | 0.90 |
| 04 | REM/lactose (1:1) | 0.56 | 3.54 |
| 05 | REM/mannitol (1:9) | 1.62 | 8.23 |
| 06 | REM/trehalose (1:9) | 0.14 | 0.51 |
| 07 | REM/sucrose (1:9) | 0.10 | 0.22 |
| 08 | REM/maltose (1:9) | 0.14 | 0.45 |
| 09 | REM/dextran (1:9) | 0.16 | 0.57 |
| 10 | REM/lactose/mannitol (1:4.5:4.5) | 0.19 | 0.89 |
| 11 | REM/povidone (1:9) | 0.53 | 2.99 |
| 12 | REM/glycine (1:9) | 1.30 | 1.54 |

# Fig. 5A

```
TITL 12161316     Compound CNS7056 Form 1
CELL  0.71073  7.687 29.261 12.376  90.000  97.788  90.000
ZERR    2 0.0001 0.0005 0.0003 0.0000 0.0008 0.0000
LATT  -1
SYMM  -X,Y+0.500,-Z
SFAC C       2.3100  20.8439   1.0200  10.2075   1.5886   0.5687   0.8650 =
            51.6512   0.2156   0.0033   0.0016     1.15   0.7700  12.0110
SFAC H       0.4930  10.5109   0.3229  26.1257   0.1402   3.1424   0.0408 =
            57.7998   0.0030   0.0000   0.0000     0.06   0.3200   1.0079
SFAC O       3.0485  13.2771   2.2868   5.7011   1.5463   0.3239   0.8670 =
            32.9089   0.2508   0.0106   0.0060     3.25   0.7700  15.9994
SFAC BR     17.1789   2.1723   5.2358  16.5796   5.6377   0.2609   3.9851 =
            41.4328   2.9557  -0.2901   2.4595  1000.00   1.1000  79.9040
SFAC N      12.2126   0.0057   3.1322   9.8933   2.0125  28.9975   1.1663 =
             0.5826 -11.5290   0.0061   0.0033     1.96   0.7700  14.0067
SFAC S       6.9053   1.4679   5.2034  22.2151   1.4379   0.2536   1.5863 =
            56.1720   0.8669   0.1246   0.1234    53.20   1.1100  32.0660
UNIT  108. 100.  20.   4.  16.   4.

S80     6   0.23964   0.43139   0.09908  11.00000   0.04634   0.03299 =
        0.04052   0.00002   0.01880  -0.00340
O81         3   0.16028   0.39374   0.15143  11.00000   0.06864   0.04111 =
        0.05255  -0.00210   0.02801   0.00002
O82         3   0.14598   0.47435   0.11207  11.00000   0.08099   0.03603 =
        0.04614   0.00545   0.03373  -0.00236
O83         3   0.42589   0.43401   0.12925  11.00000   0.05754   0.08564 =
        0.05198  -0.01536   0.01792  -0.00644
C84         1   0.20581   0.41866  -0.04324  11.00000   0.05949   0.04444 =
        0.02903   0.00359   0.01728   0.00704
C85         1   0.03624   0.41100  -0.09142  11.00000   0.06649   0.10092 =
        0.05586   0.01088   0.01751   0.00507
C86         1   0.00323   0.39810  -0.20187  11.00000   0.08670   0.14765 =
        0.05902  -0.02096  -0.03160  -0.00004
C87         1   0.14311   0.39209  -0.25693  11.00000   0.07916   0.11651 =
        0.06238  -0.01696   0.00195   0.02481
C88         1   0.30473   0.39806  -0.20987  11.00000   0.09246   0.09710 =
        0.04155   0.00157   0.01795   0.02685
C89         1   0.33456   0.41126  -0.10133  11.00000   0.05999   0.09817 =
        0.07178  -0.01451   0.00886   0.02173
S90         6   0.68868   0.81145   0.51625  11.00000   0.04072   0.02869 =
        0.05437   0.00158   0.00214   0.00223
O91         3   0.79129   0.77464   0.57315  11.00000   0.08025   0.03751 =
        0.04867  -0.00213  -0.00954   0.01626
O92         3   0.52601   0.81933   0.56122  11.00000   0.04778   0.05360 =
        0.06934  -0.00642   0.01702   0.00039
O93         3   0.78935   0.85213   0.50763  11.00000   0.07515   0.04369 =
        0.05025  -0.01354   0.01764  -0.01547
C94         1   0.62446   0.78970   0.38130  11.00000   0.04232   0.04028 =
        0.05049   0.00898   0.00929   0.00525
C95         1   0.74659   0.76959   0.32396  11.00000   0.06194   0.06998 =
        0.03238   0.00341  -0.00103   0.00990
C96         1   0.69911   0.75023   0.22476  11.00000   0.12417   0.10337 =
        0.03441   0.01537   0.02421   0.03314
C97         1   0.51941   0.75295   0.17732  11.00000   0.11897   0.11939 =
        0.02308  -0.01324  -0.00963  -0.00586
C98         1   0.40301   0.77268   0.23169  11.00000   0.06106   0.10242 =
        0.05463   0.00570  -0.01263  -0.00283
C99         1   0.45446   0.79193   0.33547  11.00000   0.05307   0.07089 =
        0.04982   0.00728  -0.00426  -0.01944
BR1         4   0.06011   0.52462   0.55140  11.00000   0.04153   0.05204 =
        0.07369  -0.00524   0.02434   0.00670
C2          1   0.25757   0.50395   0.49005  11.00000   0.02832   0.04536 =
        0.03350  -0.00752   0.01511   0.00763
C3          1   0.28921   0.45781   0.47911  11.00000   0.03135   0.03107 =
        0.04579   0.00145   0.00221  -0.00479
```

# Fig. 5B

| | | | | | | | | |
|---|---|---|---|---|---|---|---|---|
| C4 | 1 | 0.42954 | 0.44393 | 0.43174 | 11.00000 | 0.03767 | 0.03461 | = |
| | 0.02980 | -0.00320 | -0.00151 | -0.00125 | | | | |
| C5 | 1 | 0.54674 | 0.47556 | 0.39943 | 11.00000 | 0.03535 | 0.02939 | = |
| | 0.03479 | -0.00390 | 0.00647 | 0.00183 | | | | |
| C6 | 1 | 0.51907 | 0.52242 | 0.41134 | 11.00000 | 0.04226 | 0.03479 | = |
| | 0.04333 | -0.00172 | 0.00236 | 0.00188 | | | | |
| C7 | 1 | 0.37213 | 0.53602 | 0.45794 | 11.00000 | 0.03598 | 0.02793 | = |
| | 0.04586 | -0.00044 | 0.01652 | 0.00336 | | | | |
| C8 | 1 | 0.64321 | 0.55824 | 0.38118 | 11.00000 | 0.03964 | 0.02453 | = |
| | 0.02719 | 0.00516 | 0.00457 | 0.00373 | | | | |
| C9 | 1 | 0.68998 | 0.59645 | 0.46059 | 11.00000 | 0.03743 | 0.03694 | = |
| | 0.04454 | -0.00375 | 0.01588 | 0.00649 | | | | |
| N10 | 5 | 0.69097 | 0.58514 | 0.56581 | 11.00000 | 0.06070 | 0.03116 | = |
| | 0.04918 | -0.00640 | 0.02020 | -0.00054 | | | | |
| C11 | 1 | 0.74090 | 0.61847 | 0.63822 | 11.00000 | 0.06804 | 0.05787 | = |
| | 0.04752 | -0.00600 | 0.01695 | -0.00669 | | | | |
| C12 | 1 | 0.78515 | 0.66221 | 0.61053 | 11.00000 | 0.05480 | 0.04458 | = |
| | 0.05526 | -0.02125 | 0.01554 | -0.00787 | | | | |
| C13 | 1 | 0.77550 | 0.67229 | 0.50132 | 11.00000 | 0.04463 | 0.03102 | = |
| | 0.05452 | 0.00407 | 0.01432 | -0.00038 | | | | |
| C14 | 1 | 0.73186 | 0.63955 | 0.42553 | 11.00000 | 0.04272 | 0.03021 | = |
| | 0.04282 | -0.00243 | 0.01499 | 0.00270 | | | | |
| N15 | 5 | 0.71451 | 0.55972 | 0.29408 | 11.00000 | 0.04979 | 0.02502 | = |
| | 0.03692 | 0.00975 | 0.01748 | 0.00775 | | | | |
| C16 | 1 | 0.67500 | 0.52204 | 0.21324 | 11.00000 | 0.04463 | 0.02346 | = |
| | 0.04948 | -0.00464 | 0.01738 | 0.00561 | | | | |
| C17 | 1 | 0.75857 | 0.47996 | 0.26673 | 11.00000 | 0.04549 | 0.02673 | = |
| | 0.01954 | -0.00693 | 0.00506 | -0.00121 | | | | |
| N18 | 5 | 0.70009 | 0.45973 | 0.35317 | 11.00000 | 0.03293 | 0.02806 | = |
| | 0.02597 | -0.00088 | 0.00321 | 0.00207 | | | | |
| C19 | 1 | 0.81334 | 0.42409 | 0.39181 | 11.00000 | 0.03678 | 0.02848 | = |
| | 0.03351 | -0.00426 | 0.00585 | 0.00488 | | | | |
| C20 | 1 | 0.93968 | 0.42402 | 0.32661 | 11.00000 | 0.03371 | 0.02802 | = |
| | 0.03711 | 0.00202 | 0.00106 | 0.00680 | | | | |
| N21 | 5 | 0.90585 | 0.45925 | 0.25315 | 11.00000 | 0.04775 | 0.03416 | = |
| | 0.02231 | -0.01051 | 0.01052 | -0.00308 | | | | |
| C22 | 1 | 0.79597 | 0.39511 | 0.48941 | 11.00000 | 0.03997 | 0.03711 | = |
| | 0.04548 | 0.01039 | 0.00508 | 0.00197 | | | | |
| C23 | 1 | 0.74788 | 0.53407 | 0.10940 | 11.00000 | 0.05650 | 0.04712 | = |
| | 0.03514 | 0.00836 | 0.00449 | 0.00605 | | | | |
| C24 | 1 | 0.68780 | 0.50047 | 0.01647 | 11.00000 | 0.08242 | 0.04077 | = |
| | 0.03001 | -0.00046 | 0.01385 | 0.00523 | | | | |
| C25 | 1 | 0.71419 | 0.51690 | -0.09234 | 11.00000 | 0.06429 | 0.06543 | = |
| | 0.03392 | 0.00018 | 0.00559 | -0.00499 | | | | |
| O26 | 3 | 0.76261 | 0.55440 | -0.11450 | 11.00000 | 0.12347 | 0.08282 | = |
| | 0.04188 | 0.01501 | 0.01658 | -0.04001 | | | | |
| O27 | 3 | 0.65910 | 0.48459 | -0.16756 | 11.00000 | 0.10340 | 0.06919 | = |
| | 0.03191 | 0.00253 | 0.01824 | -0.00449 | | | | |
| C28 | 1 | 0.66642 | 0.49760 | -0.27953 | 11.00000 | 0.19131 | 0.12699 | = |
| | 0.01390 | -0.01417 | 0.02134 | -0.05279 | | | | |
| BR51 | 4 | 1.06737 | 0.71057 | 0.98743 | 11.00000 | 0.03812 | 0.08781 | = |
| | 0.06774 | 0.00566 | -0.00531 | 0.00447 | | | | |
| C52 | 1 | 0.84276 | 0.73306 | 0.93243 | 11.00000 | 0.03132 | 0.05952 | = |
| | 0.03819 | 0.00358 | 0.00226 | -0.00263 | | | | |
| C53 | 1 | 0.81293 | 0.77906 | 0.93249 | 11.00000 | 0.04627 | 0.06820 | = |
| | 0.03723 | -0.00581 | 0.00481 | -0.00474 | | | | |
| C54 | 1 | 0.65043 | 0.79579 | 0.88269 | 11.00000 | 0.04551 | 0.03939 | = |
| | 0.04858 | -0.00084 | 0.00376 | -0.01071 | | | | |
| C55 | 1 | 0.51946 | 0.76552 | 0.84226 | 11.00000 | 0.04294 | 0.03573 | = |
| | 0.03413 | 0.00062 | 0.00952 | -0.00208 | | | | |
| C56 | 1 | 0.54512 | 0.71765 | 0.84581 | 11.00000 | 0.02688 | 0.03659 | = |
| | 0.04586 | -0.00025 | 0.00561 | 0.00047 | | | | |
| C57 | 1 | 0.71139 | 0.70186 | 0.88914 | 11.00000 | 0.03105 | 0.04840 | = |
| | 0.04447 | -0.00668 | -0.00429 | 0.00504 | | | | |
| C58 | 1 | 0.40956 | 0.68443 | 0.79765 | 11.00000 | 0.03348 | 0.02893 | = |
| | 0.04334 | 0.00070 | 0.00351 | 0.00421 | | | | |

# Fig. 5C

| | | | | | | | | |
|---|---|---|---|---|---|---|---|---|
| C59 | 1 | 0.38048 | 0.64253 | 0.86694 | 11.00000 | 0.03165 | 0.03488 | = |
| | 0.04951 | 0.00002 | 0.00425 | 0.00528 | | | | |
| N60 | 5 | 0.42879 | 0.64650 | 0.97247 | 11.00000 | 0.03542 | 0.05694 | = |
| | 0.03178 | 0.00872 | 0.00154 | 0.00467 | | | | |
| C61 | 1 | 0.38962 | 0.61026 | 1.03529 | 11.00000 | 0.04457 | 0.06338 | = |
| | 0.05765 | 0.01416 | 0.00707 | 0.00171 | | | | |
| C62 | 1 | 0.30187 | 0.57202 | 0.98967 | 11.00000 | 0.06548 | 0.04957 | = |
| | 0.11303 | 0.03456 | 0.03582 | 0.00696 | | | | |
| C63 | 1 | 0.25733 | 0.56863 | 0.88018 | 11.00000 | 0.07395 | 0.04664 | = |
| | 0.09803 | 0.00115 | 0.01240 | -0.01007 | | | | |
| C64 | 1 | 0.29561 | 0.60475 | 0.81590 | 11.00000 | 0.08355 | 0.04152 | = |
| | 0.05459 | -0.00010 | 0.00128 | -0.02308 | | | | |
| N65 | 5 | 0.31344 | 0.68797 | 0.70771 | 11.00000 | 0.03846 | 0.03072 | = |
| | 0.04952 | -0.00160 | 0.00032 | 0.00597 | | | | |
| C66 | 1 | 0.33129 | 0.72953 | 0.64125 | 11.00000 | 0.03574 | 0.02676 | = |
| | 0.05519 | 0.00406 | 0.00580 | 0.00330 | | | | |
| C67 | 1 | 0.26347 | 0.76733 | 0.70231 | 11.00000 | 0.03803 | 0.03316 | = |
| | 0.04166 | 0.01528 | 0.00868 | 0.00029 | | | | |
| N68 | 5 | 0.35122 | 0.78274 | 0.79764 | 11.00000 | 0.03387 | 0.03259 | = |
| | 0.05055 | 0.00549 | 0.00427 | 0.00218 | | | | |
| C69 | 1 | 0.24763 | 0.81583 | 0.84108 | 11.00000 | 0.05345 | 0.03305 | = |
| | 0.04570 | 0.00005 | 0.02067 | -0.00546 | | | | |
| C70 | 1 | 0.09873 | 0.81841 | 0.77077 | 11.00000 | 0.04465 | 0.03799 | = |
| | 0.06107 | 0.00794 | 0.01464 | 0.00936 | | | | |
| N71 | 5 | 0.10819 | 0.78841 | 0.68720 | 11.00000 | 0.03892 | 0.03266 | = |
| | 0.05306 | 0.00974 | 0.01063 | 0.00803 | | | | |
| C72 | 1 | 0.30218 | 0.84064 | 0.94469 | 11.00000 | 0.08091 | 0.04934 | = |
| | 0.08052 | -0.01505 | 0.02392 | -0.00661 | | | | |
| C73 | 1 | 0.22541 | 0.72388 | 0.52948 | 11.00000 | 0.04039 | 0.05583 | = |
| | 0.03295 | 0.00047 | 0.00724 | -0.00165 | | | | |
| C74 | 1 | 0.30154 | 0.68566 | 0.46508 | 11.00000 | 0.05896 | 0.05343 | = |
| | 0.05504 | -0.00576 | 0.00667 | 0.02016 | | | | |
| C75 | 1 | 0.18003 | 0.67204 | 0.36587 | 11.00000 | 0.05296 | 0.05447 | = |
| | 0.04241 | 0.00546 | 0.01355 | 0.00171 | | | | |
| O76 | 3 | 0.06782 | 0.69497 | 0.31818 | 11.00000 | 0.05552 | 0.07543 | = |
| | 0.05719 | -0.00702 | -0.00194 | 0.02108 | | | | |
| O77 | 3 | 0.22119 | 0.62976 | 0.33149 | 11.00000 | 0.08466 | 0.04267 | = |
| | 0.04376 | -0.00714 | 0.00726 | 0.00488 | | | | |
| C78 | 1 | 0.10717 | 0.61220 | 0.23887 | 11.00000 | 0.06302 | 0.09312 | = |
| | 0.07465 | -0.02449 | 0.02418 | -0.00980 | | | | |
| H611 | 2 | 10.42342 | 10.61111 | 11.10933 | 11.00000 | 0.06582 | | |
| H621 | 2 | 10.27371 | 10.54835 | 11.03412 | 11.00000 | 0.09086 | | |
| H631 | 2 | 10.20282 | 10.54235 | 10.84949 | 11.00000 | 0.08585 | | |
| H641 | 2 | 10.26600 | 10.60396 | 10.74163 | 11.00000 | 0.07058 | | |
| H661 | 2 | 10.45616 | 10.73494 | 10.63683 | 11.00000 | 0.04658 | | |
| H701 | 2 | 10.00528 | 10.83765 | 10.77749 | 11.00000 | 0.05724 | | |
| H721 | 2 | 10.20390 | 10.85662 | 10.96784 | 11.00000 | 0.10482 | | |
| H722 | 2 | 10.39143 | 10.86250 | 10.93477 | 11.00000 | 0.10500 | | |
| H723 | 2 | 10.34863 | 10.81975 | 11.00178 | 11.00000 | 0.10479 | | |
| H731 | 2 | 10.22647 | 10.75279 | 10.49048 | 11.00000 | 0.05050 | | |
| H732 | 2 | 10.10462 | 10.71635 | 10.53573 | 11.00000 | 0.05107 | | |
| H741 | 2 | 10.41143 | 10.69632 | 10.44327 | 11.00000 | 0.06599 | | |
| H742 | 2 | 10.32279 | 10.65905 | 10.51273 | 11.00000 | 0.06616 | | |
| H571 | 2 | 10.73613 | 10.67093 | 10.88928 | 11.00000 | 0.04893 | | |
| H531 | 2 | 10.89874 | 10.79871 | 10.96543 | 11.00000 | 0.05990 | | |
| H541 | 2 | 10.63029 | 10.82681 | 10.87790 | 11.00000 | 0.05285 | | |
| H161 | 2 | 10.54702 | 10.51731 | 10.19609 | 11.00000 | 0.04687 | | |
| H201 | 2 | 11.03302 | 10.40374 | 10.33036 | 11.00000 | 0.03977 | | |
| H221 | 2 | 10.90306 | 10.37871 | 10.51025 | 11.00000 | 0.06107 | | |
| H222 | 2 | 10.77354 | 10.41394 | 10.54853 | 11.00000 | 0.06102 | | |
| H223 | 2 | 10.70245 | 10.37370 | 10.47387 | 11.00000 | 0.06087 | | |
| H231 | 2 | 10.71028 | 10.56434 | 10.08666 | 11.00000 | 0.05487 | | |
| H232 | 2 | 10.87494 | 10.53365 | 10.12431 | 11.00000 | 0.05471 | | |
| H241 | 2 | 10.56546 | 10.49241 | 10.01723 | 11.00000 | 0.06095 | | |
| H242 | 2 | 10.75795 | 10.47323 | 10.02815 | 11.00000 | 0.06099 | | |
| H111 | 2 | 10.74728 | 10.61186 | 10.71244 | 11.00000 | 0.06882 | | |
| H121 | 2 | 10.81997 | 10.68398 | 10.66349 | 11.00000 | 0.06182 | | |

# Fig. 5D

| | | | | | | |
|---|---|---|---|---|---|---|
| H131 | 2 | 10.79812 | 10.70154 | 10.48020 | 11.00000 | 0.05215 |
| H141 | 2 | 10.72939 | 10.64544 | 10.35226 | 11.00000 | 0.04595 |
| H71 | 2 | 10.35042 | 10.56684 | 10.46668 | 11.00000 | 0.04408 |
| H31 | 2 | 10.21444 | 10.43638 | 10.50355 | 11.00000 | 0.04223 |
| H41 | 2 | 10.44931 | 10.41280 | 10.42055 | 11.00000 | 0.04056 |
| H891 | 2 | 10.44977 | 10.41481 | 9.93226 | 11.00000 | 0.09285 |
| H881 | 2 | 10.39917 | 10.39332 | 9.75106 | 11.00000 | 0.09266 |
| H871 | 2 | 10.12372 | 10.38356 | 9.66972 | 11.00000 | 0.10194 |
| H861 | 2 | 9.88808 | 10.39388 | 9.76390 | 11.00000 | 0.11607 |
| H851 | 2 | 9.94416 | 10.41466 | 9.94909 | 11.00000 | 0.08904 |
| H951 | 2 | 10.86472 | 10.76918 | 10.35546 | 11.00000 | 0.06580 |
| H961 | 2 | 10.78321 | 10.73544 | 10.18942 | 11.00000 | 0.10497 |
| H971 | 2 | 10.48493 | 10.74055 | 10.10914 | 11.00000 | 0.10604 |
| H981 | 2 | 10.28646 | 10.77378 | 10.20054 | 11.00000 | 0.08719 |
| H991 | 2 | 10.37377 | 10.80653 | 10.37249 | 11.00000 | 0.07037 |
| H781 | 2 | 10.14480 | 10.58182 | 10.22240 | 11.00000 | 0.11588 |
| H782 | 2 | 10.11102 | 10.63197 | 10.17669 | 11.00000 | 0.11581 |
| H783 | 2 | 9.98883 | 10.61082 | 10.25546 | 11.00000 | 0.11600 |
| H711 | 2 | 10.01359 | 10.78308 | 10.62464 | 11.00000 | 0.05205 |
| H211 | 2 | 10.98261 | 10.46785 | 10.19729 | 11.00000 | 0.04161 |
| H281 | 2 | 10.62358 | 10.47180 | 9.67092 | 11.00000 | 0.11566 |
| H282 | 2 | 10.59036 | 10.52501 | 9.70225 | 11.00000 | 0.11566 |
| H283 | 2 | 10.79029 | 10.50514 | 9.71088 | 11.00000 | 0.11566 |

## Fig. 6A

```
TITL 1142055     Compound CNS7056 form 2
CELL  0.71073   8.921 11.154 25.834  90.000  90.000  90.000
ZERR    4 0.0001 0.0002 0.0004 0.0000 0.0000 0.0000
LATT  -1
SYMM  X+0.500,-Y+0.500,-Z
SYMM  -X,Y+0.500,-Z+0.500
SYMM  -X+0.500,-Y,Z+0.500
SFAC C       2.3100  20.8439   1.0200  10.2075   1.5886   0.5687   0.8650 =
            51.6512   0.2156   0.0033   0.0016     1.15   0.7700  12.0110
SFAC H       0.4930  10.5109   0.3229  26.1257   0.1402   3.1424   0.0408 =
            57.7998   0.0030   0.0000   0.0000     0.06   0.3200   1.0079
SFAC BR     17.1789   2.1723   5.2358  16.5796   5.6377   0.2609   3.9851 =
            41.4328   2.9557  -0.2901   2.4595  1000.00   1.1000  79.9040
SFAC N      12.2126   0.0057   3.1322   9.8933   2.0125  28.9975   1.1663 =
             0.5826 -11.5290   0.0061   0.0033     1.96   0.7700  14.0067
SFAC O       3.0485  13.2771   2.2868   5.7011   1.5463   0.3239   0.8670 =
            32.9089   0.2508   0.0106   0.0060     3.25   0.7700  15.9994
SFAC S       6.9053   1.4679   5.2034  22.2151   1.4379   0.2536   1.5863 =
            56.1720   0.8669   0.1246   0.1234    53.20   1.1100  32.0660
UNIT  108. 100.   4.  16.  20.   4.

BR1        3  -0.04819  -0.10880  -0.27710  11.00000   0.07032   0.03277 =
      0.03090   0.00144  -0.01238  -0.02224
C2         1  -0.15018  -0.21830  -0.32054  11.00000   0.02777   0.02177 =
      0.02345  -0.00009  -0.00209  -0.00471
C3         1  -0.17401  -0.18875  -0.37205  11.00000   0.02963   0.01861 =
      0.02702   0.00623   0.00188  -0.00107
C4         1  -0.24491  -0.26965  -0.40362  11.00000   0.02825   0.02442 =
      0.01718   0.00327   0.00106  -0.00145
C5         1  -0.29275  -0.37943  -0.38401  11.00000   0.02223   0.01822 =
      0.01875  -0.00067   0.00141   0.00066
C6         1  -0.27139  -0.40894  -0.33163  11.00000   0.02028   0.01967 =
      0.01926   0.00182   0.00105  -0.00153
C7         1  -0.20042  -0.32532  -0.29979  11.00000   0.02809   0.02763 =
      0.01685   0.00206   0.00190  -0.00055
C8         1  -0.32197  -0.52600  -0.30927  11.00000   0.01670   0.02233 =
      0.01945   0.00135  -0.00476  -0.00144
C9         1  -0.39853  -0.52353  -0.25770  11.00000   0.01623   0.02317 =
      0.01584   0.00259  -0.00384  -0.00281
N10        4  -0.46099  -0.41943  -0.24363  11.00000   0.02251   0.02613 =
      0.02353  -0.00189   0.00408   0.00155
C11        1  -0.52777  -0.41652  -0.19697  11.00000   0.02617   0.03441 =
      0.02357  -0.00451   0.00365   0.00346
C12        1  -0.53610  -0.51390  -0.16425  11.00000   0.02740   0.04329 =
      0.02040  -0.00335   0.00652  -0.00779
C13        1  -0.47518  -0.62062  -0.17997  11.00000   0.03584   0.03200 =
      0.02405   0.00767   0.00645  -0.00687
C14        1  -0.40334  -0.62685  -0.22730  11.00000   0.02879   0.02223 =
      0.02565   0.00090   0.00272  -0.00057
N15        4  -0.30040  -0.62781  -0.33049  11.00000   0.02151   0.02416 =
      0.01713   0.00287  -0.00002   0.00182
C16        1  -0.21928  -0.62991  -0.38036  11.00000   0.02330   0.02286 =
      0.01602   0.00057   0.00417   0.00450
```

# Fig. 6B

```
C17        1  -0.32510  -0.57975  -0.41920  11.00000    0.02824    0.02308 =
     0.01704  -0.00121   0.00336  -0.00285
N18        4  -0.36294  -0.46298  -0.41818  11.00000    0.02482    0.02037 =
     0.01483   0.00150  -0.00070   0.00079
C19        1  -0.46920  -0.44117  -0.45641  11.00000    0.03022    0.02725 =
     0.01634   0.00325   0.00039  -0.00224
C20        1  -0.49445  -0.54753  -0.47911  11.00000    0.03071    0.03401 =
     0.01669   0.00110  -0.00174  -0.00215
N21        4  -0.40440  -0.63226  -0.45591  11.00000    0.03619    0.02354 =
     0.02146  -0.00463   0.00147  -0.00154
C22        1  -0.54310  -0.32298  -0.46595  11.00000    0.03636    0.03429 =
     0.03074   0.00778  -0.00982  -0.00011
C23        1  -0.15995  -0.75547  -0.39193  11.00000    0.03430    0.02640 =
     0.01793  -0.00359   0.00177   0.00554
C24        1  -0.06166  -0.79435  -0.34621  11.00000    0.04707    0.03881 =
     0.02350   0.00041   0.00034   0.01530
C25        1   0.06625  -0.87542  -0.35603  11.00000    0.03182    0.02650 =
     0.01948   0.00340  -0.00125  -0.00016
O26        5   0.17233  -0.88334  -0.32760  11.00000    0.03778    0.06570 =
     0.03313  -0.01160  -0.01173   0.00417
O27        5   0.05245  -0.94265  -0.39885  11.00000    0.03130    0.03874 =
     0.02467  -0.00799  -0.00330   0.01418
C28        1   0.17574  -1.02443  -0.40865  11.00000    0.05622    0.08123 =
     0.03697  -0.01153  -0.00496   0.04396
S80        6  -0.94275  -0.52899  -0.49624  11.00000    0.03340    0.02679 =
     0.02442   0.00000   0.00210  -0.00075
O81        5  -0.83867  -0.47114  -0.53020  11.00000    0.05118    0.08336 =
     0.03575   0.02297  -0.00622  -0.02476
O82        5  -1.08156  -0.46260  -0.49186  11.00000    0.04015    0.07788 =
     0.05503  -0.01022  -0.00539   0.01721
O83        5  -0.97025  -0.65272  -0.50726  11.00000    0.13945    0.03230 =
     0.06071  -0.01467   0.01447  -0.00725
C84        1  -0.86288  -0.52210  -0.43343  11.00000    0.02735    0.05893 =
     0.02832   0.01509   0.00686  -0.00534
C85        1  -0.87781  -0.41462  -0.40588  11.00000    0.03763    0.08695 =
     0.03855  -0.01799   0.00427  -0.00754
C86        1  -0.81420  -0.39965  -0.35764  11.00000    0.05438    0.16315 =
     0.04455  -0.02905   0.00147  -0.02905
C87        1  -0.73766  -0.49241  -0.33773  11.00000    0.06202    0.20226 =
     0.06481   0.03510  -0.02105  -0.05062
C88        1  -0.71885  -0.60444  -0.36221  11.00000    0.04217    0.17120 =
     0.11388   0.10762  -0.01320  -0.03729
C89        1  -0.78500  -0.61610  -0.41251  11.00000    0.03725    0.08786 =
     0.07642   0.05538  -0.00772  -0.01074
H891       2   9.22557   9.31210   9.56883  11.00000    0.08027
H881       2   9.33331   9.33306   9.65289  11.00000    0.13097
H851       2   9.06867   9.64846   9.57936  11.00000    0.06577
H861       2   9.17563   9.67239   9.66111  11.00000    0.10509
H161       2   9.86530   9.42517   9.62245  11.00000    0.02469
H111       2   9.42959   9.65626   9.81326  11.00000    0.03383
H121       2   9.41618   9.49292   9.86839  11.00000    0.03606
H131       2   9.51614   9.31066   9.84059  11.00000    0.03697
H141       2   9.64103   9.30191   9.76144  11.00000    0.03108
H231       2   9.89972   9.24922   9.57680  11.00000    0.03066
H232       2   9.75764   9.18723   9.60372  11.00000    0.03099
H241       2   9.87585   9.16237   9.67759  11.00000    0.04434
H242       2   9.97980   9.27746   9.67100  11.00000    0.04489
```

Fig. 6C

| | | | | | | |
|---|---|---|---|---|---|---|
| H281 | 2 | 10.15353 | 8.92912 | 9.56085 | 11.00000 | 0.08666 |
| H282 | 2 | 10.18989 | 8.92278 | 9.62053 | 11.00000 | 0.08723 |
| H283 | 2 | 10.26566 | 9.02166 | 9.58620 | 11.00000 | 0.08710 |
| H201 | 2 | 9.44027 | 9.43682 | 9.49457 | 11.00000 | 0.03327 |
| H221 | 2 | 9.36727 | 9.66624 | 9.51370 | 11.00000 | 0.05146 |
| H222 | 2 | 9.52479 | 9.72860 | 9.51527 | 11.00000 | 0.05104 |
| H223 | 2 | 9.43193 | 9.71611 | 9.56601 | 11.00000 | 0.05131 |
| H41 | 2 | 9.73983 | 9.74902 | 9.56204 | 11.00000 | 0.02807 |
| H31 | 2 | 9.85823 | 9.88568 | 9.61518 | 11.00000 | 0.03001 |
| H71 | 2 | 9.81367 | 9.65791 | 9.73490 | 11.00000 | 0.02870 |
| H871 | 2 | 9.30621 | 9.51762 | 9.69480 | 11.00000 | 0.13226 |
| H211 | 2 | 9.59801 | 9.29339 | 9.53630 | 11.00000 | 0.03270 |

# Fig. 7A

| | | |
|---|---|---|
| S80 | O81 | 1.454(5)Å |
| S80 | O83 | 1.432(6)Å |
| C84 | C85 | 1.376(12)Å |
| C85 | C86 | 1.408(14)Å |
| C86 | C87 | 1.360(16)Å |
| C87 | C88 | 1.310(15)Å |
| C88 | C89 | 1.386(14)Å |
| C89 | H891 | 0.932Å |
| S90 | O92 | 1.454(6)Å |
| S90 | C94 | 1.793(8)Å |
| C94 | C99 | 1.354(11)Å |
| C95 | H951 | 0.938Å |
| C96 | H961 | 0.934Å |
| C97 | H971 | 0.924Å |
| C98 | H981 | 0.927Å |
| Br1 | C2 | 1.886(6)Å |
| C2 | C7 | 1.381(9)Å |
| C3 | H31 | 0.928Å |
| C4 | H41 | 0.937Å |
| C5 | N18 | 1.454(8)Å |
| C6 | C8 | 1.498(9)Å |
| C8 | C9 | 1.500(9)Å |
| C9 | N10 | 1.343(9)Å |
| N10 | C11 | 1.345(10)Å |
| C11 | H111 | 0.933Å |
| C12 | H121 | 0.927Å |
| C13 | H131 | 0.918Å |
| N15 | C16 | 1.492(9)Å |
| C16 | C23 | 1.511(9)Å |
| C17 | N18 | 1.352(8)Å |
| N18 | C19 | 1.400(8)Å |
| C19 | C22 | 1.496(9)Å |
| C20 | H201 | 0.927Å |
| C22 | H221 | 0.958Å |
| C22 | H223 | 0.953Å |
| C23 | H231 | 0.962Å |
| C24 | C25 | 1.470(11)Å |
| C24 | H242 | 0.962Å |
| C25 | O27 | 1.354(10)Å |
| C28 | H281 | 1.000Å |
| C28 | H283 | 1.000Å |
| C52 | C53 | 1.366(11)Å |
| C53 | C54 | 1.404(11)Å |
| C54 | C55 | 1.383(10)Å |
| C55 | C56 | 1.414(9)Å |
| C56 | C57 | 1.396(9)Å |
| C57 | H571 | 0.925Å |
| C58 | N65 | 1.254(8)Å |
| C59 | C64 | 1.391(10)Å |
| C61 | C62 | 1.386(14)Å |
| C62 | C63 | 1.355(15)Å |

| | | |
|---|---|---|
| S80 | O82 | 1.468(5)Å |
| S80 | C84 | 1.784(7)Å |
| C84 | C89 | 1.318(12)Å |
| C85 | H851 | 0.927Å |
| C86 | H861 | 0.936Å |
| C87 | H871 | 0.934Å |
| C88 | H881 | 0.935Å |
| S90 | O91 | 1.459(5)Å |
| S90 | O93 | 1.431(5)Å |
| C94 | C95 | 1.383(11)Å |
| C95 | C96 | 1.356(13)Å |
| C96 | C97 | 1.428(17)Å |
| C97 | C98 | 1.323(15)Å |
| C98 | C99 | 1.409(13)Å |
| C99 | H991 | 0.924Å |
| C2 | C3 | 1.382(9)Å |
| C3 | C4 | 1.358(10)Å |
| C4 | C5 | 1.388(9)Å |
| C5 | C6 | 1.398(9)Å |
| C6 | C7 | 1.394(9)Å |
| C7 | H71 | 0.926Å |
| C8 | N15 | 1.274(8)Å |
| C9 | C14 | 1.386(9)Å |
| C11 | C12 | 1.379(11)Å |
| C12 | C13 | 1.375(11)Å |
| C13 | C14 | 1.351(10)Å |
| C14 | H141 | 0.921Å |
| C16 | C17 | 1.500(9)Å |
| C16 | H161 | 0.988Å |
| C17 | N21 | 1.315(8)Å |
| C19 | C20 | 1.344(9)Å |
| C20 | N21 | 1.376(8)Å |
| N21 | H211 | 1.000Å |
| C22 | H222 | 0.950Å |
| C23 | C24 | 1.536(11)Å |
| C23 | H232 | 0.969Å |
| C24 | H241 | 0.971Å |
| C25 | O26 | 1.202(10)Å |
| O27 | C28 | 1.445(10)Å |
| C28 | H282 | 1.000Å |
| Br51 | C52 | 1.886(7)Å |
| C52 | C57 | 1.412(10)Å |
| C53 | H531 | 0.927Å |
| C54 | H541 | 0.921Å |
| C55 | N68 | 1.427(9)Å |
| C56 | C58 | 1.489(9)Å |
| C58 | C59 | 1.530(10)Å |
| C59 | N60 | 1.314(9)Å |
| N60 | C61 | 1.372(10)Å |
| C61 | H611 | 0.918Å |
| C62 | H621 | 0.928Å |

Fig. 7B

| | | |
|---|---|---|
| C63 | C64 | 1.378(13)Å |
| C64 | H641 | 0.917Å |
| C66 | C67 | 1.474(9)Å |
| C66 | H661 | 0.982Å |
| C67 | N71 | 1.334(8)Å |
| C69 | C70 | 1.343(11)Å |
| C70 | N71 | 1.366(10)Å |
| N71 | H711 | 1.000Å |
| C72 | H722 | 0.958Å |
| C73 | C74 | 1.535(10)Å |
| C73 | H732 | 0.967Å |
| C74 | H741 | 0.972Å |
| C75 | O76 | 1.185(9)Å |
| O77 | C78 | 1.440(11)Å |
| C78 | H782 | 0.966Å |

| | | |
|---|---|---|
| C63 | H631 | 0.932Å |
| N65 | C66 | 1.485(8)Å |
| C66 | C73 | 1.516(10)Å |
| C67 | N68 | 1.354(9)Å |
| N68 | C69 | 1.406(9)Å |
| C69 | C72 | 1.484(12)Å |
| C70 | H701 | 0.925Å |
| C72 | H721 | 0.964Å |
| C72 | H723 | 0.965Å |
| C73 | H731 | 0.975Å |
| C74 | C75 | 1.493(12)Å |
| C74 | H742 | 0.977Å |
| C75 | O77 | 1.360(9)Å |
| C78 | H781 | 0.965Å |
| C78 | H783 | 0.960Å |

# Fig. 8A

| | | | |
|---|---|---|---|
| O81 | S80 | O82 | 111.0(3)° |
| O82 | S80 | O83 | 114.4(4)° |
| O82 | S80 | C84 | 106.2(3)° |
| S80 | C84 | C85 | 117.7(6)° |
| C85 | C84 | C89 | 118.3(8)° |
| C84 | C85 | H851 | 119.626° |
| C85 | C86 | C87 | 118.1(10)° |
| C87 | C86 | H861 | 121.303° |
| C86 | C87 | H871 | 119.251° |
| C87 | C88 | C89 | 119.3(10)° |
| C89 | C88 | H881 | 120.264° |
| C84 | C89 | H891 | 118.485° |
| O91 | S90 | O92 | 111.7(3)° |
| O92 | S90 | O93 | 113.5(3)° |
| O92 | S90 | C94 | 105.7(3)° |
| S90 | C94 | C95 | 120.6(6)° |
| C95 | C94 | C99 | 119.3(8)° |
| C94 | C95 | H951 | 118.566° |
| C95 | C96 | C97 | 118.4(10)° |
| C97 | C96 | H961 | 121.695° |
| C96 | C97 | H971 | 119.699° |
| C97 | C98 | C99 | 120.8(9)° |
| C99 | C98 | H981 | 120.094° |
| C94 | C99 | H991 | 119.276° |
| Br1 | C2 | C3 | 121.0(5)° |
| C3 | C2 | C7 | 120.5(5)° |
| C2 | C3 | H31 | 120.203° |
| C3 | C4 | C5 | 120.6(6)° |
| C5 | C4 | H41 | 118.766° |
| C4 | C5 | N18 | 119.6(5)° |
| C5 | C6 | C7 | 117.8(6)° |
| C7 | C6 | C8 | 118.8(6)° |
| C2 | C7 | H71 | 119.721° |
| C6 | C8 | C9 | 117.5(5)° |
| C9 | C8 | N15 | 115.9(6)° |
| C8 | C9 | C14 | 121.2(6)° |
| C9 | N10 | C11 | 115.5(6)° |
| N10 | C11 | H111 | 118.526° |
| C11 | C12 | C13 | 117.4(7)° |
| C13 | C12 | H121 | 121.289° |
| C12 | C13 | H131 | 119.499° |
| C9 | C14 | C13 | 118.3(6)° |
| C13 | C14 | H141 | 121.419° |
| N15 | C16 | C17 | 105.9(5)° |
| C17 | C16 | C23 | 112.4(5)° |
| C17 | C16 | H161 | 109.539° |
| C16 | C17 | N18 | 122.7(6)° |
| N18 | C17 | N21 | 106.5(5)° |
| C5 | N18 | C19 | 127.0(5)° |
| N18 | C19 | C20 | 105.2(5)° |
| C20 | C19 | C22 | 129.4(6)° |

| | | | |
|---|---|---|---|
| O81 | S80 | O83 | 112.9(4)° |
| O81 | S80 | C84 | 105.5(3)° |
| O83 | S80 | C84 | 106.0(4)° |
| S80 | C84 | C89 | 123.6(7)° |
| C84 | C85 | C86 | 120.0(9)° |
| C86 | C85 | H851 | 120.377° |
| C85 | C86 | H861 | 120.636° |
| C86 | C87 | C88 | 121.8(10)° |
| C88 | C87 | H871 | 118.984° |
| C87 | C88 | H881 | 120.392° |
| C84 | C89 | C88 | 122.5(10)° |
| C88 | C89 | H891 | 119.061° |
| O91 | S90 | O93 | 112.8(4)° |
| O91 | S90 | C94 | 104.5(3)° |
| O93 | S90 | C94 | 108.0(3)° |
| S90 | C94 | C99 | 120.1(6)° |
| C94 | C95 | C96 | 121.6(9)° |
| C96 | C95 | H951 | 119.820° |
| C95 | C96 | H961 | 119.911° |
| C96 | C97 | C98 | 119.9(8)° |
| C98 | C97 | H971 | 120.397° |
| C97 | C98 | H981 | 119.080° |
| C94 | C99 | C98 | 119.9(9)° |
| C98 | C99 | H991 | 120.819° |
| Br1 | C2 | C7 | 118.5(5)° |
| C2 | C3 | C4 | 119.7(6)° |
| C4 | C3 | H31 | 120.109° |
| C3 | C4 | H41 | 120.600° |
| C4 | C5 | C6 | 120.6(6)° |
| C6 | C5 | N18 | 119.8(6)° |
| C5 | C6 | C8 | 123.3(6)° |
| C2 | C7 | C6 | 120.6(6)° |
| C6 | C7 | H71 | 119.679° |
| C6 | C8 | N15 | 126.6(6)° |
| C8 | C9 | N10 | 114.9(6)° |
| N10 | C9 | C14 | 123.9(6)° |
| N10 | C11 | C12 | 124.4(7)° |
| C12 | C11 | H111 | 117.061° |
| C11 | C12 | H121 | 121.279° |
| C12 | C13 | C14 | 120.4(6)° |
| C14 | C13 | H131 | 120.125° |
| C9 | C14 | H141 | 120.274° |
| C8 | N15 | C16 | 118.0(5)° |
| N15 | C16 | C23 | 109.4(5)° |
| N15 | C16 | H161 | 110.723° |
| C23 | C16 | H161 | 108.851° |
| C16 | C17 | N21 | 130.3(6)° |
| C5 | N18 | C17 | 123.1(5)° |
| C17 | N18 | C19 | 109.8(5)° |
| N18 | C19 | C22 | 125.3(6)° |
| C19 | C20 | N21 | 108.0(5)° |

## Fig. 8B

| | | | |
|---|---|---|---|
| C19 | C20 | H201 | 126.017° |
| C17 | N21 | C20 | 110.5(5)° |
| C20 | N21 | H211 | 124.681° |
| C19 | C22 | H222 | 109.778° |
| C19 | C22 | H223 | 110.905° |
| H222 | C22 | H223 | 109.018° |
| C16 | C23 | H231 | 109.392° |
| C16 | C23 | H232 | 108.378° |
| H231 | C23 | H232 | 108.825° |
| C23 | C24 | H241 | 109.968° |
| C23 | C24 | H242 | 108.195° |
| H241 | C24 | H242 | 108.752° |
| C24 | C25 | O27 | 109.4(7)° |
| C25 | O27 | C28 | 115.2(7)° |
| O27 | C28 | H282 | 109.261° |
| O27 | C28 | H283 | 109.465° |
| H282 | C28 | H283 | 109.476° |
| Br51 | C52 | C57 | 119.0(5)° |
| C52 | C53 | C54 | 118.9(7)° |
| C54 | C53 | H531 | 120.985° |
| C53 | C54 | H541 | 120.227° |
| C54 | C55 | C56 | 122.1(6)° |
| C56 | C55 | N68 | 118.5(6)° |
| C55 | C56 | C58 | 123.2(6)° |
| C52 | C57 | C56 | 120.2(7)° |
| C56 | C57 | H571 | 120.138° |
| C56 | C58 | N65 | 126.7(6)° |
| C58 | C59 | N60 | 116.3(6)° |
| N60 | C59 | C64 | 125.0(7)° |
| N60 | C61 | C62 | 121.7(8)° |
| C62 | C61 | H611 | 118.993° |
| C61 | C62 | H621 | 120.029° |
| C62 | C63 | C64 | 118.4(9)° |
| C64 | C63 | H631 | 121.124° |
| C59 | C64 | H641 | 120.844° |
| C58 | N65 | C66 | 118.2(6)° |
| N65 | C66 | C73 | 109.7(5)° |
| N65 | C66 | H661 | 109.122° |
| C73 | C66 | H661 | 112.017° |
| C66 | C67 | N71 | 130.3(7)° |
| C55 | N68 | C67 | 122.5(6)° |
| C67 | N68 | C69 | 108.7(6)° |
| N68 | C69 | C72 | 124.0(7)° |
| C69 | C70 | N71 | 109.1(6)° |
| N71 | C70 | H701 | 125.502° |
| C67 | N71 | H711 | 125.400° |
| C69 | C72 | H721 | 110.667° |
| H721 | C72 | H722 | 108.539° |
| H721 | C72 | H723 | 108.455° |
| C66 | C73 | C74 | 111.0(6)° |

| | | | |
|---|---|---|---|
| N21 | C20 | H201 | 126.026° |
| C17 | N21 | H211 | 124.840° |
| C19 | C22 | H221 | 109.508° |
| H221 | C22 | H222 | 108.808° |
| H221 | C22 | H223 | 108.786° |
| C16 | C23 | C24 | 112.3(6)° |
| C24 | C23 | H231 | 108.812° |
| C24 | C23 | H232 | 109.105° |
| C23 | C24 | C25 | 114.3(7)° |
| C25 | C24 | H241 | 110.030° |
| C25 | C24 | H242 | 105.346° |
| C24 | C25 | O26 | 126.4(7)° |
| O26 | C25 | O27 | 123.9(7)° |
| O27 | C28 | H281 | 109.674° |
| H281 | C28 | H282 | 109.475° |
| H281 | C28 | H283 | 109.476° |
| Br51 | C52 | C53 | 119.3(6)° |
| C53 | C52 | C57 | 121.7(7)° |
| C52 | C53 | H531 | 120.141° |
| C53 | C54 | C55 | 119.8(7)° |
| C55 | C54 | H541 | 120.000° |
| C54 | C55 | N68 | 119.4(6)° |
| C55 | C56 | C57 | 117.2(6)° |
| C57 | C56 | C58 | 119.5(6)° |
| C52 | C57 | H571 | 119.709° |
| C56 | C58 | C59 | 116.5(6)° |
| C59 | C58 | N65 | 116.8(6)° |
| C58 | C59 | C64 | 118.5(7)° |
| C59 | N60 | C61 | 116.1(7)° |
| N60 | C61 | H611 | 119.342° |
| C61 | C62 | C63 | 120.6(8)° |
| C63 | C62 | H621 | 119.353° |
| C62 | C63 | H631 | 120.452° |
| C59 | C64 | C63 | 118.1(8)° |
| C63 | C64 | H641 | 121.057° |
| N65 | C66 | C67 | 105.4(5)° |
| C67 | C66 | C73 | 111.5(6)° |
| C67 | C66 | H661 | 108.890° |
| C66 | C67 | N68 | 121.8(6)° |
| N68 | C67 | N71 | 107.4(6)° |
| C55 | N68 | C69 | 128.7(6)° |
| N68 | C69 | C70 | 105.5(6)° |
| C70 | C69 | C72 | 130.5(7)° |
| C69 | C70 | H701 | 125.444° |
| C67 | N71 | C70 | 109.2(6)° |
| C70 | N71 | H711 | 125.366° |
| C69 | C72 | H722 | 109.838° |
| C69 | C72 | H723 | 110.831° |
| H722 | C72 | H723 | 108.445° |
| C66 | C73 | H731 | 108.535° |

**Fig. 8C**

| | | | |
|---|---|---|---|
| C74 | C73 | H731 | 110.248° |
| C74 | C73 | H732 | 108.249° |
| C73 | C74 | C75 | 112.4(6)° |
| C75 | C74 | H741 | 109.125° |
| C75 | C74 | H742 | 108.578° |
| C74 | C75 | O76 | 126.2(7)° |
| O76 | C75 | O77 | 123.0(7)° |
| O77 | C78 | H781 | 109.214° |
| H781 | C78 | H782 | 109.923° |
| H781 | C78 | H783 | 109.026° |

| | | | |
|---|---|---|---|
| C66 | C73 | H732 | 110.751° |
| H731 | C73 | H732 | 108.042° |
| C73 | C74 | H741 | 108.496° |
| C73 | C74 | H742 | 108.155° |
| H741 | C74 | H742 | 110.035° |
| C74 | C75 | O77 | 110.7(7)° |
| C75 | O77 | C78 | 115.6(7)° |
| O77 | C78 | H782 | 109.848° |
| O77 | C78 | H783 | 109.687° |
| H782 | C78 | H783 | 109.127° |

# Fig. 9

| | | |
|---|---|---|
| Br1 | C2 | 1.892(3)Å |
| C2 | C7 | 1.383(5)Å |
| C3 | H31 | 0.938Å |
| C4 | H41 | 0.921Å |
| C5 | N18 | 1.428(4)Å |
| C6 | C8 | 1.497(4)Å |
| C8 | C9 | 1.497(4)Å |
| C9 | N10 | 1.338(4)Å |
| N10 | C11 | 1.345(4)Å |
| C11 | H111 | 0.935Å |
| C12 | H121 | 0.948Å |
| C13 | H131 | 0.936Å |
| N15 | C16 | 1.478(4)Å |
| C16 | C23 | 1.527(5)Å |
| C17 | N18 | 1.346(4)Å |
| N18 | C19 | 1.391(4)Å |
| C19 | C22 | 1.494(5)Å |
| C20 | H201 | 0.912Å |
| C22 | H221 | 0.965Å |
| C22 | H223 | 0.960Å |
| C23 | H231 | 0.969Å |
| C24 | C25 | 1.478(5)Å |
| C24 | H242 | 0.988Å |
| C25 | O27 | 1.342(4)Å |
| C28 | H281 | 0.964Å |
| C28 | H283 | 0.962Å |
| S80 | O82 | 1.447(3)Å |
| S80 | C84 | 1.774(4)Å |
| C84 | C89 | 1.369(7)Å |
| C85 | H851 | 0.932Å |
| C86 | H861 | 0.943Å |
| C87 | H871 | 0.934Å |
| C88 | H881 | 0.925Å |

| | | |
|---|---|---|
| C2 | C3 | 1.387(5)Å |
| C3 | C4 | 1.371(5)Å |
| C4 | C5 | 1.392(5)Å |
| C5 | C6 | 1.406(4)Å |
| C6 | C7 | 1.395(5)Å |
| C7 | H71 | 0.924Å |
| C8 | N15 | 1.276(4)Å |
| C9 | C14 | 1.395(5)Å |
| C11 | C12 | 1.378(5)Å |
| C12 | C13 | 1.370(5)Å |
| C13 | C14 | 1.382(5)Å |
| C14 | H141 | 0.934Å |
| C16 | C17 | 1.487(5)Å |
| C16 | H161 | 0.976Å |
| C17 | N21 | 1.320(4)Å |
| C19 | C20 | 1.342(5)Å |
| C20 | N21 | 1.378(5)Å |
| N21 | H211 | 0.854Å |
| C22 | H222 | 0.966Å |
| C23 | C24 | 1.534(5)Å |
| C23 | H232 | 0.981Å |
| C24 | H241 | 0.960Å |
| C25 | O26 | 1.201(4)Å |
| O27 | C28 | 1.451(5)Å |
| C28 | H282 | 0.965Å |
| S80 | O81 | 1.431(3)Å |
| S80 | O83 | 1.430(3)Å |
| C84 | C85 | 1.400(7)Å |
| C85 | C86 | 1.380(7)Å |
| C86 | C87 | 1.342(13)Å |
| C87 | C88 | 1.410(13)Å |
| C88 | C89 | 1.433(10)Å |
| C89 | H891 | 0.940Å |

# Fig. 10

| | | | |
|---|---|---|---|
| Br1 | C2 | C3 | 119.3(3)° |
| C3 | C2 | C7 | 121.8(3)° |
| C2 | C3 | H31 | 120.033° |
| C3 | C4 | C5 | 120.3(3)° |
| C5 | C4 | H41 | 120.261° |
| C4 | C5 | N18 | 118.9(3)° |
| C5 | C6 | C7 | 118.2(3)° |
| C7 | C6 | C8 | 119.5(3)° |
| C2 | C7 | H71 | 120.432° |
| C6 | C8 | C9 | 117.7(3)° |
| C9 | C8 | N15 | 117.9(3)° |
| C8 | C9 | C14 | 120.0(3)° |
| C9 | N10 | C11 | 116.7(3)° |
| N10 | C11 | H111 | 117.041° |
| C11 | C12 | C13 | 118.8(3)° |
| C13 | C12 | H121 | 120.783° |
| C12 | C13 | H131 | 120.694° |
| C9 | C14 | C13 | 118.1(3)° |
| C13 | C14 | H141 | 120.983° |
| N15 | C16 | C17 | 105.7(3)° |
| C17 | C16 | C23 | 115.7(3)° |
| C17 | C16 | H161 | 107.726° |
| C16 | C17 | N18 | 120.7(3)° |
| N18 | C17 | N21 | 108.0(3)° |
| C5 | N18 | C19 | 128.6(3)° |
| N18 | C19 | C20 | 105.7(3)° |
| C20 | C19 | C22 | 129.3(3)° |
| C19 | C20 | H201 | 127.007° |
| C17 | N21 | C20 | 108.7(3)° |
| C20 | N21 | H211 | 125.351° |
| C19 | C22 | H222 | 109.368° |
| C19 | C22 | H223 | 111.184° |
| H222 | C22 | H223 | 107.885° |
| C16 | C23 | H231 | 107.712° |
| C16 | C23 | H232 | 111.123° |
| H231 | C23 | H232 | 110.583° |
| C23 | C24 | H241 | 107.661° |
| C23 | C24 | H242 | 109.365° |
| H241 | C24 | H242 | 109.671° |
| C24 | C25 | O27 | 114.4(3)° |
| C25 | O27 | C28 | 115.2(3)° |
| O27 | C28 | H282 | 110.269° |
| O27 | C28 | H283 | 108.681° |
| H282 | C28 | H283 | 108.963° |
| O81 | S80 | O83 | 115.1(2)° |
| O81 | S80 | C84 | 106.30(18)° |
| O83 | S80 | C84 | 107.0(2)° |
| S80 | C84 | C89 | 122.1(4)° |
| C84 | C85 | C86 | 121.6(6)° |
| C86 | C85 | H851 | 119.275° |
| C85 | C86 | H861 | 121.859° |
| C86 | C87 | C88 | 124.9(7)° |
| C88 | C87 | H871 | 117.376° |
| C87 | C88 | H881 | 122.592° |
| C84 | C89 | C88 | 119.8(8)° |
| C88 | C89 | H891 | 120.078° |

| | | | |
|---|---|---|---|
| Br1 | C2 | C7 | 118.9(3)° |
| C2 | C3 | C4 | 119.0(3)° |
| C4 | C3 | H31 | 120.959° |
| C3 | C4 | H41 | 119.485° |
| C4 | C5 | C6 | 121.0(3)° |
| C6 | C5 | N18 | 120.1(3)° |
| C5 | C6 | C8 | 122.3(3)° |
| C2 | C7 | C6 | 119.7(3)° |
| C6 | C7 | H71 | 119.874° |
| C6 | C8 | N15 | 124.4(3)° |
| C8 | C9 | N10 | 116.6(3)° |
| N10 | C9 | C14 | 123.4(3)° |
| N10 | C11 | C12 | 123.7(3)° |
| C12 | C11 | H111 | 119.278° |
| C11 | C12 | H121 | 120.443° |
| C12 | C13 | C14 | 119.3(3)° |
| C14 | C13 | H131 | 119.952° |
| C9 | C14 | H141 | 120.942° |
| C8 | N15 | C16 | 117.6(3)° |
| N15 | C16 | C23 | 110.8(3)° |
| N15 | C16 | H161 | 107.681° |
| C23 | C16 | H161 | 108.910° |
| C16 | C17 | N21 | 131.2(3)° |
| C5 | N18 | C17 | 122.3(3)° |
| C17 | N18 | C19 | 109.0(3)° |
| N18 | C19 | C22 | 124.9(3)° |
| C19 | C20 | N21 | 108.6(3)° |
| N21 | C20 | H201 | 124.433° |
| C17 | N21 | H211 | 125.926° |
| C19 | C22 | H221 | 110.223° |
| H221 | C22 | H222 | 108.664° |
| H221 | C22 | H223 | 109.452° |
| C16 | C23 | C24 | 107.9(3)° |
| C24 | C23 | H231 | 110.073° |
| C24 | C23 | H232 | 109.430° |
| C23 | C24 | C25 | 118.8(3)° |
| C25 | C24 | H241 | 104.516° |
| C25 | C24 | H242 | 106.503° |
| C24 | C25 | O26 | 123.3(3)° |
| O26 | C25 | O27 | 122.4(3)° |
| O27 | C28 | H281 | 108.952° |
| H281 | C28 | H282 | 109.738° |
| H281 | C28 | H283 | 110.225° |
| O81 | S80 | O82 | 111.9(2)° |
| O82 | S80 | O83 | 111.2(3)° |
| O82 | S80 | C84 | 104.5(2)° |
| S80 | C84 | C85 | 117.6(4)° |
| C85 | C84 | C89 | 120.2(5)° |
| C84 | C85 | H851 | 119.148° |
| C85 | C86 | C87 | 117.5(8)° |
| C87 | C86 | H861 | 120.606° |
| C86 | C87 | H871 | 117.763° |
| C87 | C88 | C89 | 116.0(7)° |
| C89 | C88 | H881 | 121.435° |
| C84 | C89 | H891 | 120.080° |
| | | | |

# Fig. 11

**Stability Data, CNS 7056 for Injection, 26 mg (Lot A01P310)**

| Product Name: CNS 7056 for Injection | Date Manufactured: July 2009 | | | Container Closure: Type I, Glass Vials, 20 mL; Bromobutyl Rubber Stopper; and Aluminum Skirt | | | | |
|---|---|---|---|---|---|---|---|---|
| Lot: A01P310 | | | | | | | | |
| | | | Storage Condition: 25 °C/60% RH | | | | | |
| Tests[a] | Acceptance Criteria[a] | Time (Months) | | | | | | |
| | | 0 | 1 | 3 | 6 | 9 | 12 | 18 |
| Appearance of Lyophilized Product | White to off white lyophilized powder. | Complies | Complies | Complies | Complies | Complies | Complies | Complies |
| Appearance of Reconstituted Product | A clear, colorless to pale yellow solution, free from visible particulates. | Clear, colorless solution, essentially free from particulates. | Clear, very pale yellow solution, essentially free from particulates. | Clear, very pale yellow solution, essentially free from particulates | Clear, very pale yellow solution, essentially free from particulates | Clear, very pale yellow solution, essentially free from particulates | Clear, very pale yellow solution, free from particulates | Clear, very pale yellow solution, essentially free from particulates |
| Time to Reconstitute (min) | NMT 2 min | 1–2 secs | 1–2 secs | 1–2 secs | 1–2 secs | 1–2 secs | 1–2 secs | 1–2 secs |
| Osmolality | 290 ± 30 mOsmol/kg | 281 | — | 279 | 295 | — | 283 | — |
| pH on Reconstitution | 2.9 to 3.5 | 3.3 | 3.2 | 3.2 | 3.2 | 3.2 | 3.1 | 3.2 |
| Moisture Karl Fischer (% w/w) | NMT 1.5 | 0.16 | 0.32 | 0.38 | 0.45 | 0.44 | 0.33 | 0.54 |
| CNS 7056 Vial Content (mg/vial) | 24.1 to 28.0 mg/vial | 26.5 | 26.1 | 26.0 | 27.0 | 26.4 | 26.0 | 26.3 |
| | 92.5 to 107.5% nominal amount | 102.0 | 100.5 | 99.9 | 103.9 | 101.4 | 100.0 | 101.0 |
| Concentration on Reconstitution (mg/mL) | 4.5 to 5.5 (Upright) | 4.9 | 4.9 | 4.8 | 5.1 | 4.8 | 4.9 | 4.9 |
| | 4.5 to 5.5 (Inverted) | 4.8 | 4.9 | 4.8 | 5.0 | 4.9 | 5.0 | 4.8 |
| Subvisible particles | ≥10 µm report per vial<br>≥25 µm report per vial | ≥10 µm:79<br>≥25 µm:1 | — | — | ≥10 µm:80<br>≥25 µm:2 | — | ≥10 µm:344<br>≥25 µm:6 | — |

# Fig. 12

**Stability Data, CNS 7056 for Injection, 26 mg (Lot A01P310) continued**

| **Product Name:** CNS 7056 for Injection | | **Date Manufactured: July 2009** | | | **Container Closure: Type I, Glass Vials, 20 mL; Bromobutyl Rubber Stopper; and Aluminum Skirt** | | | |
|---|---|---|---|---|---|---|---|---|
| **Lot: A01P310** | | | | | | | **Drug Substance Lot: 11084** | |
| | | | | | **Storage Condition: 25 °C/60% RH** | | | |
| **Tests[a]** | **Acceptance Criteria[a]** | **Time (Months)** | | | | | | |
| | | **0** | **1** | **3** | **6** | **9** | **12** | **18** |
| Related Substances (Area %): | | | | | | | | |
| CNS 7054X | NMT 1.5% | 0.06 | 0.10 | 0.09 | 0.14 | 0.13 | 0.14 | 0.17 |
| RRT 0.67 | NMT 0.4% | 0.03 | 0.04 | 0.03 | 0.03 | 0.03 | 0.03 | 0.03 |
| RRT 0.70 | NMT 0.4% | 0.06 | 0.05 | 0.06 | 0.05 | 0.06 | 0.06 | 0.05 |
| RRT 0.92 | NMT 0.4% | 0.11 | 0.12 | 0.11 | 0.11 | 0.11 | 0.10 | 0.11 |
| RRT 1.31 | NMT 0.4% | 0.23 | 0.24 | 0.24 | 0.24 | 0.25 | 0.24 | 0.24 |
| RRT 1.82 | NMT 0.4% | 0.03 | <LOQ | <LOQ | 0.03 | 0.03 | 0.03 | <LOQ |
| Total | NMT 2.0% | 0.52 | 0.55 | 0.53 | 0.60 | 0.61 | 0.60 | 0.60 |
| Chiral (%R) | NMT 0.5% R-enantiomer | 0.27 | — | 0.28 | 0.28 | — | 0.25 | — |
| Endotoxins USP <85> | NMT 0.5 EU/mg | <0.2 EU/mg | — | — | — | — | <0.2 EU/mg | — |
| Sterility USP <71> | Complies with test | Complies | — | — | — | — | Complies | — |

[a] = These tests and acceptance criteria are based on a previous specification.

— = Not performed.

Fig. 13

**Accelerated Stability Data, CNS 7056 for Injection, 26 mg (Lot A01P310)**

| | | |
|---|---|---|
| **Product Name:** CNS 7056 for Injection | **Date Manufactured: July 2009** | **Container Closure: Type I, Glass Vials, 20 mL; Bromobutyl Rubber Stopper; and Aluminum Skirt** |
| **Lot: A01P310** | | **Drug Substance Lot: 11084** |
| | | **Storage Condition: 40 °C/75% RH** |

| Tests[a] | Acceptance Criteria[a] | Time (Months) 0 | 1 | 3 | 6 |
|---|---|---|---|---|---|
| Appearance of Lyophilized Product | White to off white lyophilized powder. | Complies | Complies | Complies | Complies |
| Appearance of Reconstituted Product | A clear, colorless to pale yellow solution, free from visible particulates. | Clear, colorless solution, essentially free from particulates. | Clear, very pale yellow solution, essentially free from particulates. | Clear, very pale yellow solution, essentially free from particulates. | Clear, very pale yellow solution, 1 vial contained small fiber |
| Time to Reconstitute (min) | NMT 2 min | 1–2 secs | 1–2 secs | 1-2 secs | 1-2 secs |
| Osmolality | 290 ± 30 mOsmol/kg | 281 | - | 279 | 295 |
| pH on Reconstitution | 2.9 to 3.5 | 3.3 | 3.2 | 3.2 | 3.2 |
| Moisture Karl Fischer (% w/w) | NMT 1.5 | 0.16 | 0.32 | 0.37 | 0.59 |
| CNS 7056 Vial Content (mg/vial) | 24.1 to 28.0 mg/vial | 26.5 | 26.3 | 26.3 | 26.0 |
| | 92.5 to 107.5% nominal amount | 102.0 | 101.0 | 101.3 | 99.8 |
| Concentration on Reconstitution (mg/mL) | 4.5 to 5.5 (Upright) | 4.9 | 4.9 | 5.0 | 5.2 |
| | 4.5 to 5.5 (Inverted) | 4.8 | 4.8 | 4.8 | 5.1 |
| Subvisible particles | ≥10 µm report per vial<br>≥25 µm report per vial | ≥10 µm:79<br>≥25 µm:1 | — | — | — |

# Fig. 14

**Accelerated Stability Data, CNS 7056 for Injection, 26 mg (Lot A01P310)**

| | | | | | |
|---|---|---|---|---|---|
| **Product Name:** **CNS 7056 for Injection** | | | **Container Closure: Type I, Glass Vials, 20 mL; Bromobutyl Rubber Stopper; and Aluminum Skirt** | | |
| **Lot: A01P310** | | | | | |
| | | | **Storage Condition: 40 °C/75% RH** | | |
| **Tests** [a] | **Acceptance Criteria** [a] | **Time (Months)** | | | |
| | | **0** | **1** | **3** | **6** |
| Related Substances (Area %): | | | | | |
| CNS 7054X | NMT 1.5% | 0.06 | 0.14 | 0.18 | 0.33 |
| RRT 0.67 | NMT 0.4% | 0.03 | 0.03 | 0.03 | 0.03 |
| RRT 0.70 | NMT 0.4% | 0.06 | 0.05 | 0.05 | 0.05 |
| RRT 0.92 | NMT 0.4% | 0.11 | 0.11 | 0.11 | 0.11 |
| RRT 1.31 | NMT 0.4% | 0.23 | 0.24 | 0.25 | 0.25 |
| RRT 1.82 | NMT 0.4% | 0.03 | <LOQ | <LOQ | <LOQ |
| Total | NMT 2.0% | 0.52 | 0.57 | 0.62 | 0.77 |
| Chiral (%R) | NMT 0.5% R-enantiomer | 0.27 | — | 0.28 | 0.28 |
| Endotoxins USP <85> | NMT 0.5 EU/mg | <0.2 EU/mg | — | — | — |
| Sterility USP <71> | Complies with test | Complies | — | — | — |

[a] = These tests and acceptance criteria are based on a previous specification.

— = Not performed.

# Fig. 15

**Long-Term Stability Data, CNS 7056 for Injection, 26 mg (Lot P310-01 (B))**

| | | | | | | | | |
|---|---|---|---|---|---|---|---|---|
| **Product Name:** CNS 7056 for Injection | **Date Manufactured: May 2009** | | **Container Closure: Type I, Glass Vials, 20 mL; Bromobutyl Rubber Stopper; and Aluminum Skirt** | | | | | |
| **Lot: P310-01 (B)** | | | | | **Drug Substance Lot: 11084** | | | |
| | | | **Storage Condition: 25 °C/60% RH** | | | | | |
| **Tests**[a] | **Acceptance Criteria**[a] | **Time (Months)** | | | | | | |
| | | **0** | **1** | **3** | **6** | **9** | **12** | **18** |
| Appearance of Lyophilized Product | White to off white lyophilized powder. | Complies | Complies | Complies | Complies | Complies | Complies | Complies |
| Appearance of Reconstituted Product | A clear, colorless to pale yellow solution, free from visible particulates. | Very pale yellow, clear solution essentially free from particulates with several fibers not thought to be product related. | Clear, virtually colorless to very pale yellow solution, free from visible particulates. Several fibers visible not thought to be product related. | Very pale yellow, clear solution essentially free from particulates with several fibers not thought to be product related. | Very pale yellow, clear solution essentially free from particulates with several fibers not thought to be product related. | Very pale yellow, clear solution essentially free from particulates with several fibers not thought to be product related. | Very pale yellow, clear solution essentially free from particulates with several fibers not thought to be product related. | Very pale yellow, clear solution essentially free from particulates with several fibers not thought to be product related |
| Time to Reconstitute (Min) | NMT 2 min | 1–2 secs | 1–2 secs | 1–2 secs | 1–2 secs | 1–2 secs | 1–2 secs | 1–2 secs |
| Osmolality | 290 ± 30 mOsmol/kg | 289 | 280 | 277 | 281 | 282 | 286 | 278 |
| pH on Reconstitution | 2.9 to 3.5 | 3.1 | 3.2 | 3.2 | 3.2 | 3.1 | 3.2 | 3.1 |
| Moisture Karl Fischer (% w/w) | NMT 1.5 | 0.39 | 0.60 | 0.62 | 0.64 | 0.55 | 0.76 | 0.86 |
| CNS 7056 Vial Content (mg/vial) | 24.1 to 28.0 mg/vial | 26.4 | 26.2 | 26.1 | 26.1 | 26.4 | 26.6 | 26.4 |

# Fig. 16

| Long-Term Stability Data, CNS 7056 for Injection, 26 mg (Lot P310-01 (B)) | | | | | | | | |
|---|---|---|---|---|---|---|---|---|
| **Product Name:** CNS 7056 for Injection | | **Date Manufactured: May 2009** | | **Container Closure: Type I, Glass Vials, 20 mL; Bromobutyl Rubber Stopper; and Aluminum Skirt** | | | | |
| **Lot: P310-01 (B)** | | | | | | **Drug Substance Lot: 11084** | | |
| | | | | **Storage Condition: 25 °C/60% RH** | | | | |
| **Tests**[a] | **Acceptance Criteria**[a] | **Time (Months)** | | | | | | |
| | | **0** | **1** | **3** | **6** | **9** | **12** | **18** |
| | 92.5 to 107.5% nominal amount | 101.7 | 100.6 | 100.5 | 100.5 | 101.5 | 102.2 | 101.4 |
| Concentration on Reconstitution (mg/mL) | 4.5 to 5.5 (Upright) | 5.0 | 5.0 | 4.9 | 4.9 | 5.1 | 4.9 | 5.1 |
| | 4.5 to 5.5 (Inverted) | 5.0 | 4.9 | 4.9 | 4.9 | 5.1 | 4.9 | 5.0 |
| Related Substances (Area %): | | | | | | | | |
| CNS 7054X | NMT 1.5% | 0.06 | 0.07 | 0.13 | 0.14 | 0.16 | 0.18 | 0.19 |
| RRT 0.68 | NMT 0.40% | 0.03 | 0.03 | 0.03 | 0.03 | 0.03 | 0.03 | 0.04 |
| RRT 0.70 | NMT 0.40% | 0.06 | 0.06 | 0.06 | 0.06 | 0.06 | 0.06 | 0.06 |
| RRT 0.92 | NMT 0.40% | 0.11 | 0.12 | 0.12 | 0.12 | 0.10 | 0.11 | 0.11 |
| RRT 0.94 | NMT 0.40% | ND | 0.03 | ND | ND | ND | ND | ND |
| RRT 1.31 | NMT 0.40% | 0.24 | 0.24 | 0.24 | 0.25 | 0.24 | 0.25 | 0.24 |
| RRT 1.85 | NMT 0.40% | <LOQ | 0.03 | <LOQ | <LOQ | <LOQ | 0.03 | 0.03 |
| Total | NMT 2.0% | 0.50% | 0.58% | 0.58 | 0.60 | 0.59 | 0.66 | 0.67 |
| Chiral (%R) | NMT 0.5% R-enantiomer | 0.25% | — | — | 0.30 | — | 0.3 | — |
| [a] = tests and acceptance criteria are based on a previous specification. | | | | | | | | |
| — = Not performed. | | | | | | | | |

Fig. 17

| Accelerated Stability Data, CNS 7056 for Injection, 26 mg (Lot P310-01 (B)) | | | | | | | |
|---|---|---|---|---|---|---|---|
| **Product Name:** CNS 7056 for Injection | | **Container Closure: Type I, Glass Vials, 20 mL; Bromobutyl Rubber Stopper; and Aluminum Skirt** | | | | | |
| **Lot: P310-01 (B)** | | | | | **Drug Substance Lot: 11084** | | |
| | | **Storage Condition: 40 °C/75% RH** | | | | | |
| **Tests**[a] | **Acceptance Criteria**[a] | **Time (Months)** | | | | | |
| | | **0** | **1** | **3** | **6** | **9** | **12** |
| Appearance of Lyophilized Product | White to off white lyophilized powder. | Complies | Complies | Complies | Complies | Complies | Complies |
| Appearance of Reconstituted Product | A clear, colorless to pale yellow solution, free from visible particulates. | Very pale yellow, clear solution essentially free from particulates with several fibers not thought to be product related. | Clear, virtually colorless to very pale yellow solution free from visible particulates. Several fibers visible not thought to be product related. | Very pale yellow, clear solution essentially free from particulates with several fibers not thought to be product related. | Very pale yellow, clear solution essentially free from particulates with several fibers not thought to be product related. | Very pale yellow, clear solution essentially free from particulates with several fibers not thought to be product related. | Very pale yellow, clear solution essentially free from particulates with several fibers not thought to be product related. |
| Time to Reconstitute (min) | NMT 2 min | 1–2 secs | 1–2 secs | 1–2 secs | 1–2 secs | 1–2 secs | 1–2 secs |
| Osmolality | 290 ± 30 mOsmol/kg | 289 | 281 | 274 | 281 | 284 | 281 |
| pH on Reconstitution | 2.9 to 3.5 | 3.1 | 3.3 | 3.2 | 3.1 | 3.1 | 3.2 |
| Moisture Karl Fischer (% w/w) | NMT 1.5 | 0.39 | 0.51 | 0.58 | 0.84 | 0.82 | 0.82 |
| CNS 7056 Vial Content (mg/vial) | 24.1 to 28.0 mg/vial | 26.4 | 26.0 | 26.3 | 26.3 | 26.2 | 26.5 |
| | 92.5 to 107.5% nominal amount | 101.7 | 100.0 | 101.3 | 101.0 | 100.6 | 102.0 |

# Fig. 18

| Accelerated Stability Data, CNS 7056 for Injection, 26 mg (Lot P310-01 (B)) | | | | | | | |
|---|---|---|---|---|---|---|---|
| **Product Name:** CNS 7056 for Injection | | **Container Closure: Type I, Glass Vials, 20 mL; Bromobutyl Rubber Stopper; and Aluminum Skirt** | | | | | |
| **Lot: P310-01 (B)** | | | | | | | |
| | | **Storage Condition: 40 °C/75% RH** | | | **Drug Substance Lot: 11084** | | |
| **Tests** [a] | **Acceptance Criteria** [a] | **Time (Months)** | | | | | |
| | | **0** | **1** | **3** | **6** | **9** | **12** |
| Concentration on Reconstitution (mg/mL) | 4.5 to 5.5 (Upright) | 5.0 | 4.9 | 4.9 | 4.8 | 5.0 | 4.9 |
| | 4.5 to 5.5 (Inverted) | 5.0 | 4.9 | 4.9 | 4.9 | 5.0 | 4.9 |
| Related Substances (Area %): | | | | | | | |
| RRT 0.51 | NMT 0.4% | <LOQ | <LOQ | 0.03 | <LOQ | <LOQ | <LOQ |
| CNS 7054X | NMT 1.5% | 0.06 | 0.11 | 0.22 | 0.36 | 0.45 | 0.68 |
| RRT 0.63 | NMT 0.4% | <LOQ | <LOQ | 0.03 | 0.03 | <LOQ | <LOQ |
| RRT 0.68 | NMT 0.4% | 0.03 | 0.03 | 0.03 | 0.03 | 0.03 | 0.03 |
| RRT 0.70 | NMT 0.4% | 0.06 | 0.06 | 0.06 | 0.06 | 0.06 | 0.06 |
| RRT 0.92 | NMT 0.4% | 0.11 | 0.12 | 0.12 | 0.12 | 0.11 | 0.11 |
| RRT 0.94 | NMT 0.4% | ND | 0.03 | ND | ND | ND | ND |
| RRT 1.31 | NMT 0.4% | 0.24 | 0.25 | 0.24 | 0.25 | 0.25 | 0.25 |
| RRT 1.85 | NMT 0.4% | <LOQ | 0.03 | <LOQ | 0.03 | <LOQ | 0.03 |
| Total | NMT 2.0% | 0.50 | 0.63 | 0.73 | 0.88 | 0.90 | 1.16 |
| Chiral (%R) | NMT 0.5% R-enantiomer | 0.25 | — | 0.3 | 0.3 | — | 0.3 |
| [a] = | These tests and acceptance criteria are based on a previous specification. | | | | | | |
| — = | Not performed. | | | | | | |

Fig. 19

**Long-Term Stability Data, CNS 7056 for Injection, 25 mg (026CNS27)**

| Product Name: CNS 7056 | | Container Closure: Type I Glass Vials, 20 mL; Bromobutyl Rubber Stopper; and Aluminum Skirt | |
|---|---|---|---|
| Lot: 026CNS27 | | | Drug Substance Lot: SOL12621/5 |
| | | Storage Condition: 25 °C/60% RH | |

| Test | Acceptance Criteria | Time (Months) 0 | 1 | 2 | 3 | 6 | 9 | 12 | 18 |
|---|---|---|---|---|---|---|---|---|---|
| Appearance of Lyophilized Product | Record | White lyophilized plug with shrinkage. Although no layering evident, the bottom of the plug (2-3 mm) appeared to be slightly more shrunken | As initial | As initial | As initial | As initial | As initial | As initial | As initial |
| Completeness of Solution | A clear, colorless solution, free from visible particulates | A clear, colorless solution, free from visible particulates. | As initial | — | As initial | As initial | As initial | As initial | As initial |
| Time to Reconstitute (min) | NMT 2 min | <1 | <1 | — | <1 | <1 | <1 | <1 | <1 |
| pH on Reconstitution | 2.9 to 3.5 | 3.1 | 3.1 | — | 3.1 | 3.1 | 3.1 | 3.2 | 3.1 |
| Moisture Karl Fischer (% w/w) | NMT 1.5 | 0.12 | 0.20 | 0.30 | 0.34 | 0.42 | 0.48 | 0.40 | 0.59 |
| CNS 7056 Vial Content (mg/vial) | 23.1 to 26.9 mg/vial | 25.4 | 25.2 | 24.8 | 25.8 | 25.9 | 25.5 | 25.4 | 25.2 |
| | 92.5 to 107.5% nominal amount | 101.6 | 100.8 | 99.2 | 103.2 | 103.6 | 102.0 | 101.6 | 100.8 |
| Concentration on Reconstitution (mg/mL) | 4.5 to 5.5 | 4.6 | — | — | 4.7 | 4.7 | 4.6 | 4.8 | 4.9 |

# Fig. 20

| **Long-Term Stability Data, CNS 7056 for Injection, 25 mg (026CNS27)** | | | | | | | | | |
|---|---|---|---|---|---|---|---|---|---|
| **Product Name:** CNS 7056 | | **Container Closure: Type I Glass Vials, 20 mL; Bromobutyl Rubber Stopper; and Aluminum Skirt** | | | | | | | |
| **Lot: 026CNS27** | | | | | **Drug Substance Lot: SOL12621/5** | | | | |
| | | **Storage Condition: 25 °C/60% RH** | | | | | | | |
| **Test** | **Acceptance Criteria** | **Time (Months)** | | | | | | | |
| | | **0** | **1** | **2** | **3** | **6** | **9** | **12** | **18** |
| Related Substances (Area %): | | | | | | | | | |
| CNS 7054X | NMT 1.5% | 1.12 | 1.12 | 1.12 | 1.14 | 1.14 | 1.13 | 1.18 | 1.20 |
| RRT 0.68 | NMT 0.4% | 0.09 | 0.09 | 0.09 | 0.08 | 0.08 | 0.10 | 0.09 | 0.10 |
| RRT 0.93 | NMT 0.4% | 0.13 | 0.13 | 0.12 | 0.14 | 0.13 | 0.12 | 0.10 | 0.16 |
| RRT 0.94 | NMT 0.4% | 0.07 | 0.09 | 0.08 | 0.12 | 0.07 | *a* | 0.09 | *a* |
| RRT 1.31 | NMT 0.4% | 0.24 | 0.23 | 0.24 | 0.23 | 0.22 | 0.23 | 0.22 | 0.23 |
| Total | NMT 2.0% | 1.65 | 1.66 | 1.65 | 1.71 | 1.64 | 1.58 | 1.68 | 1.69 |
| Chiral (% R) | NMT 0.5% R-enantiomer | 0.16 | 0.17 | — | 0.16 | 0.16 | 0.15 | 0.15 | 0.17 |
| *a* = Peaks at RRT 0.94 not resolved from CNS 7056 peak. | | | | | | | | | |
| — = Not performed. | | | | | | | | | |

# Fig. 21

**Accelerated Stability Data, CNS 7056 for Injection, 25 mg (Lot 026CNS27)**

| | | | | | | |
|---|---|---|---|---|---|---|
| **Product Name:** CNS 7056 | | **Container Closure: Type I Glass Vials, 20 mL; Bromobutyl Rubber Stopper; and Aluminum Skirt** | | | | |
| **Lot: 026CNS27** | | | | | | |
| **Bulk Batch Size: 800 mL** | | **Storage Condition: 40 °C/75% RH** | | | **Drug Substance Lot: SOL12621/5** | |
| **Test** | **Acceptance Criteria** | **Time (Months)** | | | | |
| | | **0** | **1** | **2** | **3** | **6** |
| Appearance of Lyophilized Product | Record | White lyophilized plug with shrinkage. Although no layering evident, the bottom of the plug (2-3 mm) appeared to be slightly more shrunken | As initial | As initial | As initial | As initial |
| Completeness of Solution | A clear, colorless solution, free from visible particulates | A clear, colorless solution, free from visible particulates. | As initial | — | As initial | As initial |
| Time to Reconstitute (min) | NMT 2 min | <1 | <1 | — | <1 | <1 |
| pH on Reconstitution | 2.9 to 3.5 | 3.1 | 3.1 | — | 3.1 | 3.1 |
| Moisture Karl Fischer (% w/w) | NMT 1.5 | 0.12 | 0.28 | 0.46 | 0.54 | 0.64 |
| CNS 7056 Vial Content (mg/vial) | 23.1 to 26.9 mg/vial | 25.4 | 25.4 | 24.8 | 24.8 | 25.1 |
| | 92.5 to 107.5% nominal amount | 101.6 | 101.6 | 99.2 | 99.2 | 100.4 |
| Concentration on Reconstitution (mg/mL) | 4.5 to 5.5 | 4.6 | — | — | 4.7 | 4.9 |

Fig. 22

**Accelerated Stability Data, CNS 7056 for Injection, 25 mg (Lot 026CNS27)**

| | | | | | | |
|---|---|---|---|---|---|---|
| **Product Name:** CNS 7056 | | **Container Closure: Type I Glass Vials, 20 mL; Bromobutyl Rubber Stopper; and Aluminum Skirt** | | | | |
| **Lot: 026CNS27** | | | | **Drug Substance Lot: SOL12621/5** | | |
| **Bulk Batch Size: 800 mL** | | **Storage Condition: 40 °C/75% RH** | | | | |
| **Test** | **Acceptance Criteria** | **Time (Months)** | | | | |
| | | **0** | **1** | **2** | **3** | **6** |
| Related Substances (Area %): | | | | | | |
| CNS 7054X | NMT 1.5% | 1.12 | 1.16 | 1.18 | 1.21 | 1.32 |
| RRT 0.68 | NMT 0.4% | 0.09 | 0.09 | 0.09 | 0.08 | 0.08 |
| RRT 0.93 | NMT 0.4% | 0.13 | 0.13 | 0.12 | 0.14 | 0.13 |
| RRT 0.94 | NMT 0.4% | 0.07 | 0.09 | 0.09 | 0.11 | 0.07 |
| RRT 1.31 | NMT 0.4% | 0.24 | 0.23 | 0.24 | 0.23 | 0.22 |
| Total | NMT 2.0% | 1.65 | 1.70 | 1.72 | 1.77 | 1.82 |
| Chiral (% R) | NMT 0.5% R-enantiomer | 0.16 | 0.17 | — | 0.16 | 0.16 |

— = Not performed.

## Fig. 23

**Long-Term Stability Data, CNS 7056 for Injection, 25 mg (Lot G384)**

**Product Name:** CNS 7056

**Lot:** G384

**Container Closure:** Type I Glass Vials, 20 mL; Bromobutyl Rubber Stopper; and Aluminum Skirt

**Drug Substance Lot:** SOL12621/5

**Storage Condition:** 25 °C/60% RH

| Test | Acceptance Criteria | Time (Months) 0 | 1 | 2 | 3 | 6 | 9 | 12 | 18 |
|---|---|---|---|---|---|---|---|---|---|
| Appearance of Lyophilized Product | Record | White lyophilized plug with shrinkage. Although no splitting evident, the bottom of the plug (2-3 mm) appeared to be slightly more shrunken. | As initial | As initial | As initial | As initial | As initial | As initial | As initial |
| Completeness of Solution | A clear, colorless solution, free from visible particulates | A clear, colorless solution, free from visible particulates. | As initial | As initial | As initial | As initial | As initial | As initial | As initial |
| Time to Reconstitute (min) | NMT 2 min | $<1$ | $<1$ | $<1$ | $<1$ | $<1$ | $<1$ | $<1$ | $<1$ |
| pH on Reconstitution | 2.9 to 3.5 | 3.3 | 3.3 | 3.4 | 3.3 | 3.3 | 3.3 | 3.3 | 3.3 |
| Moisture Karl Fischer (% w/w) | NMT 1.5 | 0.10 | 0.22 | 0.30 | 0.31 | 0.43 | 0.50 | 0.45 | 0.60 |

# Fig. 24

| Long-Term Stability Data, CNS 7056 for Injection, 25 mg (Lot G384) | | | | | | | | | |
|---|---|---|---|---|---|---|---|---|---|
| Product Name: CNS 7056 | | Container Closure: Type I Glass Vials, 20 mL; Bromobutyl Rubber Stopper; and Aluminum Skirt | | | | | | | |
| Lot: G384 | | | | | Drug Substance Lot: SOL12621/5 | | | | |
| | | Storage Condition: 25 °C/60% RH | | | | | | | |
| Test | Acceptance Criteria | Time (Months) 0 | 1 | 2 | 3 | 6 | 9 | 12 | 18 |
| CNS 7056 Vial Content (mg/vial) | 23.1 to 26.9 mg/vial | 23.4 | 23.7 | 23.8 | 23.7 | 23.7 | 23.6 | 23.8 | 23.8 |
| | 92.5 to 107.5% nominal amount | 93.6 | 94.8 | 95.2 | 94.8 | 94.8 | 94.4 | 95.2 | 95.2 |
| Concentration on Reconstitution (mg/mL) | 4.5 to 5.5 | 4.4[a] | — | 4.5 | — | 4.8 | — | 4.6 | 4.5 |
| Related Substances (Area %): | | | | | | | | | |
| CNS 7054X | NMT 1.5% | 1.08 | 1.10 | 1.10 | 1.11 | 1.11 | 1.14 | 1.13 | 1.16 |
| RRT 0.68 | NMT 0.4% | 0.09 | 0.09 | 0.09 | 0.09 | 0.10 | 0.10 | 0.08 | 0.08 |
| RRT 0.93 | NMT 0.4% | 0.12 | 0.12 | 0.14 | 0.14 | 0.12 | 0.12 | 0.13 | 0.18 |
| RRT 0.94 | NMT 0.4% | 0.07 | 0.09 | 0.10 | 0.10 | [b] | [b] | <LOQ | [c] |
| RRT 1.31 | NMT 0.4% | 0.23 | 0.24 | 0.24 | 0.24 | 0.24 | 0.24 | 0.22 | 0.23 |
| Total | NMT 2.0% | 1.59 | 1.64 | 1.67 | 1.68 | 1.57 | 1.60 | 1.56 | 1.65 |
| Chiral (% R) | NMT 0.5% R-enantiomer | 0.16 | 0.16 | — | 0.16 | 0.17 | 0.15 | 0.14 | 0.16 |

[a] = See Section 7.2.13.1 for discussion.
[b] = Peak at RRT 0.94 not resolved from CNS 7056.
[c] = Peak not detected. Appeared to be unresolved from previous peak.
— = Not performed.

# Fig. 25

| Accelerated Stability Data, CNS 7056 for Injection, 25 mg (Lot G384) | | | | | | |
|---|---|---|---|---|---|---|
| **Product Name:** CNS 7056 | | | **Container Closure: Type I Glass Vials, 20 mL; Bromobutyl Rubber Stopper; and Aluminum Skirt** | | | |
| **Lot: G384** | | | | | **Drug Substance Lot: SOL12621/5** | |
| | | | **Storage Condition: 40 °C/75% RH** | | | |
| **Test** | **Acceptance Criteria** | **Time (Months)** | | | | |
| | | **0** | **1** | **2** | **3** | **6** |
| Appearance of Lyophilized Product | Record | White lyophilized plug with shrinkage. Although no splitting evident, the bottom of the plug (2-3 mm) appeared to be slightly more shrunken | As initial | As initial | As initial | As initial |
| Completeness of Solution | A clear, colorless solution, free from visible particulates | A clear, colorless solution, free from visible particulates. | As initial | As initial | As initial | As initial |
| Time to Reconstitute (min) | NMT 2 min | <1 | <1 | <1 | <1 | <1 |
| pH on Reconstitution | 2.9 to 3.5 | 3.3 | 3.3 | 3.4 | 3.4 | 3.3 |
| Moisture Karl Fischer (% w/w) | NMT 1.5 | 0.10 | 0.48 | 0.44 | 0.50 | 0.69 |
| CNS 7056 Vial Content (mg/vial) | 23.1 to 26.9 mg/vial | 23.4 | 23.6 | 23.8 | 23.7 | 23.7 |
| | 92.5 to 107.5% nominal amount | 93.6 | 94.4 | 95.2 | 94.8 | 94.8 |
| Concentration on Reconstitution (mg/mL) | 4.5 to 5.5 | 4.4[a] | — | 4.5 | — | 4.7 |

# Fig. 26

**Accelerated Stability Data, CNS 7056 for Injection, 25 mg (Lot G384)**

| | | | | | | |
|---|---|---|---|---|---|---|
| **Product Name:** CNS 7056 | | **Container Closure: Type I Glass Vials, 20 mL; Bromobutyl Rubber Stopper; and Aluminum Skirt** | | | | |
| **Lot: G384** | | | | **Drug Substance Lot: SOL12621/5** | | |
| | | **Storage Condition: 40 °C/75% RH** | | | | |
| **Test** | **Acceptance Criteria** | **Time (Months)** | | | | |
| | | 0 | 1 | 2 | 3 | 6 |
| Related Substances (Area %): | | | | | | |
| CNS 7054X | NMT 1.5% | 1.08 | 1.14 | 1.14 | 1.18 | 1.28 |
| RRT 0.68 | NMT 0.4% | 0.09 | 0.08 | 0.08 | 0.08 | 0.10 |
| RRT 0.93 | NMT 0.4% | 0.12 | 0.12 | 0.14 | 0.14 | 0.12 |
| RRT 0.94 | NMT 0.4% | 0.07 | 0.09 | 0.12 | 0.10 | [b] |
| RRT 1.31 | NMT 0.4% | 0.23 | 0.24 | 0.23 | 0.24 | 0.23 |
| Total | NMT 2.0% | 1.59 | 1.67 | 1.71 | 1.74 | 1.73 |
| Chiral (% R) | NMT 0.5% R-enantiomer | 0.16 | 0.14 | — | 0.16 | 0.16 |

[a] = See Section 7.2.13.1 for discussion.
[b] = Peak at RRT 0.94 not resolved from CNS 7056.
— = Not performed.

# Fig. 27

| Long-Term Stability Data, CNS 7056 for Injection, 23 mg (Lot P02308) | | | | | | | | | | |
|---|---|---|---|---|---|---|---|---|---|---|
| Product Name: CNS 7056 | | | Container Closure: Type I Glass Vials, 20 mL; Bromobutyl Rubber Stopper; and Aluminum Skirt | | | | | | | |
| Lot: P02308 | | | | | | | Drug Substance Lot: 11084 | | | |
| | | Storage Condition: 25 °C/60% RH | | | | | | | | |
| Test | Acceptance Criteria | Time (Months) | | | | | | | | |
| | | 0 | 1 | 3 | 6 | 9 | 12 | 18 | 24 | 36 |
| Appearance of Lyophilized Product | White to off-white lyophilized plug | Off-white lyophilized plug | As initial | As initial | As initial | As initial | As initial | As initial | As initial | As initial |
| Completeness of Solution | A clear, colorless to pale yellow solution, free from visible particulates | All vials - a clear , very pale yellow solution, free from visible particulates | As initial | As initial | As initial | As initial | As initial | As initial | As initial | As initial |
| Time to Reconstitute (min) | NMT 2 min | <1 | <1 | <1 | <1 | <1 | <1 | <1 | <1 | <1 |
| Subvisible Particulates | ≥10 µm: report | 66 | 65 | — | 93 | — | 24 | 16 | 13 | 40 |
| | ≥25 µm: report | 1 | 0 | — | <1 | — | <1 | <1 | 0 | <1 |
| Osmolality (mOsm/kg) | 290 ± 30 | 280 | 283 | 292 | 288 | 289 | 287 | 282 | 284 | 288 |
| pH on Reconstitution | 2.9 to 3.5 | 3.1 | 3.1 | 3.1 | 3.2 | 3.2 | 3.1 | 3.2 | 3.1 | 3.1 |
| Moisture Karl Fischer (% w/w) | NMT 1.5 | 0.27 | 0.27 | 0.36 | 0.50 | 0.47 | 0.46 | 0.62 | 0.59 | 0.68 |
| CNS 7056 Vial Content (mg/vial) | 21.3 to 24.7 mg/vial | 23.6 | 23.6 | 23.4 | 23.6 | 23.6 | 23.5 | 23.5 | 23.4 | 23.4 |
| | 92.5 to 107.5% nominal amount | 102.6 | 102.6 | 101.7 | 102.6 | 102.3 | 102.2 | 102.1 | 101.5 | 101.6 |

## Fig. 28

| Long-Term Stability Data, CNS 7056 for Injection, 23 mg (Lot P02308) | | | | | | | | | | |
|---|---|---|---|---|---|---|---|---|---|---|
| Product Name: CNS 7056 | | | | | Container Closure: Type I Glass Vials, 20 mL; Bromobutyl Rubber Stopper; and Aluminum Skirt | | | | | |
| Lot: P02308 | | | | | | | Drug Substance Lot: 11084 | | | |
| | | | Storage Condition: 25 °C/60% RH | | | | | | | |
| Test | Acceptance Criteria | Time (Months) | | | | | | | | |
| | | 0 | 1 | 3 | 6 | 9 | 12 | 18 | 24 | 36 |
| Test | Acceptance Criteria | Time (Months) | | | | | | | | |
| | | 0 | 1 | 3 | 6 | 9 | 12 | 18 | 24 | 36 |
| Concentration on Reconstitution (mg/mL) | 4.5 to 5.5 | 4.9 | 5.1 | 5.0 | 5.0 | 5.1 | 5.0 | 5.1 | 5.1 | 5.0 |
| Related Substances (Area %): | | | | | | | | | | |
| CNS 7054X | NMT 1.5% | 0.07 | 0.10 | 0.11 | 0.13 | 0.16 | 0.16 | 0.20 | 0.23 | 0.29 |
| RRT 0.67 | NMT 0.4% | 0.03 | 0.03 | 0.04 | 0.03 | 0.03 | 0.03 | 0.03 | 0.03 | 0.03 |
| RRT 0.71 | NMT 0.4% | 0.06 | 0.06 | 0.06 | 0.06 | 0.05 | 0.05 | 0.06 | 0.06 | 0.06 |
| RRT 0.92 | NMT 0.4% | 0.12 | 0.12 | 0.12 | 0.11 | 0.12 | 0.12 | 0.11 | 0.11 | 0.11 |
| RRT 0.96 | NMT 0.4% | 0.12 | <LOQ | 0.10 | 0.04 | 0.09 | 0.10 | 0.04 | 0.03 | 0.03 |
| RRT 1.31 | NMT 0.4% | 0.25 | 0.27 | 0.25 | 0.25 | 0.25 | 0.26 | 0.26 | 0.25 | 0.25 |
| RRT 1.80 | NMT 0.4% | <LOQ | <LOQ | <LOQ | 0.03 | <LOQ | <LOQ | <LOQ | 0.03 | 0.03 |
| Total | NMT 2.0% | 0.65 | 0.58 | 0.68 | 0.65 | 0.70 | 0.72 | 0.70 | 0.74 | 0.80 |
| Chiral (% R) | NMT 0.5% R-enantiomer | 0.26 | 0.27 | — | 0.27 | — | 0.27 | 0.27 | 0.26 | 0.28 |
| Sterility | Complies | Complies | — | — | — | — | Complies | - | Complies | Complies |
| — = | Not performed. | | | | | | | | | |

## Fig. 29

| Accelerated Stability Data, CNS 7056 for Injection, 23 mg (Lot P02308) | | | | | | | | |
|---|---|---|---|---|---|---|---|---|
| Product Name: CNS 7056 | | Container Closure: Type I Glass Vials, 20 mL; Bromobutyl Rubber Stopper; and Aluminum Skirt | | | | | | |
| Lot: P02308 | | | | Drug Substance Lot: 11084 | | | | |
| | | Storage Condition: 40 °C/75% RH | | | | | | |
| Test | Acceptance Criteria | Time (Months) | | | | | | |
| | | 0 | 1 | 3 | 6 | 9 | 12 |
| Appearance of Lyophilized Product | White to off-white lyophilized plug | Off-white lyophilized plug. | As initial | As initial | As initial | As initial | As initial |
| Completeness of Solution | A clear, colorless to pale yellow solution, free from visible particulates | A clear, very pale yellow solution free from visible particulates. | As initial | As initial | As initial | As initial | As initial |
| Time to Reconstitute (min) | NMT 2 min | <1 | <1 | <1 | <1 | <1 | <1 |
| Subvisible Particulates | ≥10 µm: report | 66 | 76 | 12 | 74 | — | 20 |
| | ≥25 µm: report | 1 | <1 | 0 | <1 | — | 0 |
| pH on Reconstitution | 2.9 to 3.5 | 3.1 | 3.1 | 3.1 | 3.2 | 3.2 | 3.1 |
| Osmolality (mOsm/kg) | 290 ± 30 | 280 | 289 | 291 | 286 | 290 | 288 |
| Moisture Karl Fischer (% w/w) | NMT 1.5 | 0.27 | 0.40 | 0.58 | 0.67 | 0.78 | 0.86 |
| CNS 7056 Vial Content (mg/vial) | 21.3 to 24.7 mg/vial | 23.6 | 23.6 | 23.4 | 23.5 | 23.4 | 23.2 |
| | 92.5 to 107.5% nominal amount | 102.6 | 102.6 | 101.7 | 102.2 | 101.7 | 101.0 |

## Fig. 30

| Accelerated Stability Data, CNS 7056 for Injection, 23 mg (Lot P02308) | | | | | | | |
|---|---|---|---|---|---|---|---|
| Product Name: CNS 7056 | | Container Closure: Type I Glass Vials, 20 mL; Bromobutyl Rubber Stopper; and Aluminum Skirt | | | | | |
| Lot: P02308 | | | | Drug Substance Lot: 11084 | | | |
| | | Storage Condition: 40 °C/75% RH | | | | | |
| Test | Acceptance Criteria | Time (Months) | | | | | |
| | | 0 | 1 | 3 | 6 | 9 | 12 |
| Concentration on Reconstitution (mg/mL) | 4.5 to 5.5 | 4.9 | 5.2 | 5.0 | 5.0 | 5.1 | 5.1 |
| Related Substances (Area %): | | | | | | | |
| CNS 7054X | NMT 1.5% | 0.07 | 0.12 | 0.20 | 0.32 | 0.41 | 0.54 |
| RRT 0.67 | NMT 0.4% | 0.03 | 0.03 | 0.04 | 0.03 | 0.03 | 0.03 |
| RRT 0.71 | NMT 0.4% | 0.06 | 0.07 | 0.05 | 0.06 | 0.05 | 0.05 |
| RRT 0.92 | NMT 0.4% | 0.12 | 0.12 | 0.12 | 0.11 | 0.12 | 0.12 |
| RRT 0.96 | NMT 0.4% | 0.12 | 0.03 | 0.10 | 0.05 | 0.10 | 0.09 |
| RRT 1.31 | NMT 0.4% | 0.25 | 0.27 | 0.27 | 0.28 | 0.29 | 0.29 |
| RRT 1.80 | NMT 0.4% | <LOQ | <LOQ | <LOQ | 0.03 | <LOQ | <LOQ |
| Total | NMT 2.0% | 0.65 | 0.64 | 0.78 | 0.88 | 1.00 | 1.12 |
| Chiral (% R) | NMT 0.5% R-enantiomer | 0.26 | 0.27 | 0.27 | 0.27 | — | 0.27 |
| Sterility | Complies | Complies | — | — | — | — | — |

— = Not performed.

**Fig. 31**

**Long-Term Stability Data, CNS 7056 for Injection, 25 mg (Lot 025CNS27)**

| Product Name: CNS 7056 | | Container Closure: Type I Glass Vials, 20 mL; Bromobutyl Rubber Stopper; and Aluminum Skirt | | | | | | | | |
|---|---|---|---|---|---|---|---|---|---|---|
| Lot: 025CNS27 | | | | Drug Substance Lot: SOL12621/5 | | | | | | |
| | | Storage Condition: 25 °C/60% RH | | | | | | | | |
| **Test** | **Acceptance Criteria** | **Plug** | **Time (Months)** 0 | 1 | 2 | 3 | 6 | 9 | 12 | 18 |
| Appearance of Lyophilized Product | Record | Unsplit | White lyophilized plug with shrinkage. | As initial | — | — | — | — | — | — |
| | | Split | White lyophilized plug with shrinkage. 2 distinct layers evident. | As initial | As initial | As initial | As initial | As initial | As initial[a] | White lyophilized plug with shrinkage. |
| Completeness of Solution | A clear, colorless solution, free from visible particulates | | The bulk of the plug dissolved rapidly. The resultant solution was clear, colorless, and free from visible particulates. | As initial | As initial | As initial | As initial | As initial | As initial | As initial |
| Time to Reconstitute (Min) | NMT 2 min | Unsplit | <1 | <1 | — | — | — | <1[b] | <1 | <1[b] |
| | | Split | <1 | <1 | <1 | <1 | <1 | | <1 | |
| pH on Reconstitution | 2.9 to 3.5 | Unsplit | 3.1 | — | — | — | — | 3.1[b] | 3.1 | 3.2[b] |
| | | Split | 3.2 | 3.1 | 3.2 | 3.1 | 3.2 | | 3.1 | |

Fig. 32

| Long-Term Stability Data, CNS 7056 for Injection, 25 mg (Lot 025CNS27) | | | | | | | | | | |
|---|---|---|---|---|---|---|---|---|---|---|
| Product Name: CNS 7056 | | | Container Closure: Type I Glass Vials, 20 mL; Bromobutyl Rubber Stopper; and Aluminum Skirt | | | | | | | |
| Lot: 025CNS27 | | | | | Drug Substance Lot: SOL12621/5 | | | | | |
| | | | Storage Condition: 25 °C/60% RH | | | | | | | |
| Test | Acceptance Criteria | Plug | Time (Months) | | | | | | | |
| | | | 0 | 1 | 2 | 3 | 6 | 9 | 12 | 18 |
| Moisture Karl Fischer (% w/w) | NMT 1.5 | Unsplit | 0.36 | 0.31 | — | — | — | 0.79[b] | 0.76 | 0.62[b] |
| | | Split | 0.83 | 0.78 | 0.86 | 0.87 | 0.87 | | | |
| CNS 7056 Vial Content (mg/vial) | 23.1 to 26.9 mg/vial | Unsplit | 24.6 | 25.0 | 24.7 | 24.4 | 24.7 | 24.8 | 25.0 | 25.0 |
| | 92.5 to 107.5% nominal amount | | 98.5 | 100.0 | 98.7 | 97.6 | 98.7 | 99.2 | 100.0 | 100.0 |
| Concentration on Reconstitution (mg/mL) | 4.5 to 5.5 | Unsplit | 4.6 | — | — | 4.6 | 4.7 | 4.8 | 4.8 | 4.7 |
| Related Substances (Area %): | | | | | | | | | | |
| CNS 7054X | NMT 1.5% | Unsplit | 1.08 | 1.09 | — | — | — | — | — | — |
| RRT 0.68 | NMT 0.4% | | 0.09 | 0.09 | — | — | — | — | — | — |
| RRT 0.93 | NMT 0.4% | | 0.12 | 0.13 | — | — | — | — | — | — |
| RRT 0.94 | NMT 0.4% | | 0.07 | 0.08 | — | — | — | — | — | — |
| RRT 1.31 | NMT 0.4% | | 0.22 | 0.24 | — | — | — | — | — | — |
| Total | NMT 2.0% | | 1.58 | 1.628 | — | — | — | — | — | — |

Fig. 33

**Long-Term Stability Data, CNS 7056 for Injection, 25 mg (Lot 025CNS27)**

Product Name: CNS 7056

Container Closure: Type I Glass Vials, 20 mL; Bromobutyl Rubber Stopper; and Aluminum Skirt

Lot: 025CNS27

Drug Substance Lot: SOL12621/5

Storage Condition: 25 °C/60% RH

| Test | Acceptance Criteria | Plug | Time (Months) 0 | 1 | 2 | 3 | 6 | 9 | 12 | 18 |
|---|---|---|---|---|---|---|---|---|---|---|
| Related Substances (Area %): | | | | | | | | | | |
| CNS 7054X | NMT 1.5% | Split | 1.08 | 1.09 | 1.12 | 1.14 | 1.15 | 1.20 | 1.20 | 1.16 |
| RRT 0.68 | NMT 0.4% | | 0.09 | 0.09 | 0.11 | 0.08 | 0.09 | 0.08 | 0.10 | 0.10 |
| RRT 0.93 | NMT 0.4% | | 0.12 | 0.13 | 0.20 | 0.12 | 0.14 | 0.12 | 0.12 | 0.13 |
| RRT 0.94 | NMT 0.4% | | 0.07 | 0.08 | c | 0.09 | 0.09 | d | d | <0.05 |
| RRT 1.31 | NMT 0.4% | | 0.22 | 0.23 | 0.24 | 0.24 | 0.24 | 0.23 | 0.24 | 0.24 |
| Total | NMT 2.0% | | 1.58 | 1.62 | 1.67 | 1.67 | 1.71 | 1.63 | 1.66 | 1.63 |
| Chiral (% R) | NMT 0.5% R-enantiomer | Unsplit | 0.16 | 0.16 | — | 0.12 | 0.15 | 0.15 | 0.15 | 0.16 |

a = Two distinct layers evident in only 9 of 11 vials.
b = At 39 and 78 weeks, it is not known whether the vials used for the analysis were unsplit or split.
c = Peaks at RRT 0.93 and 0.94 are not resolved; total area remains the same.
d = Peak at RRT 0.94 not resolved from CNS 7056 peak.
— = Not performed.

## Fig. 34

**Accelerated Stability Data, CNS for Injection, 25 mg (Lot 025CNS27)**

**Product Name:** CNS 7056

**Lot:** 025CNS27

**Container Closure:** Type I Glass Vials, 20 mL; Bromobutyl Rubber Stopper; and Aluminum Skirt

**Drug Substance Lot:** SOL12621/5

**Storage Condition:** 40 °C/75% RH

| Test | Acceptance Criteria | Plug | Time (Months) 0 | 1 | 2 | 3 | 6 | 9 | 12 |
|---|---|---|---|---|---|---|---|---|---|
| Appearance of Lyophilized Product | Record | Unsplit | White lyophilized plug with shrinkage. | As initial | As initial | As initial | As initial | As initial | As initial |
| | | Split | White lyophilized plug with shrinkage. 2 distinct layers evident. | As initial | As initial | As initial | As initial | As initial | White lyophilized plug with shrinkage. |
| Completeness of Solution | A clear, colorless solution, free from visible particulates | Unsplit | The bulk of the plug dissolved rapidly. The resultant solution was clear, colorless and free from visible particulates. | As initial | As initial | As initial | As initial | As initial | As initial |
| Time to Reconstitute (min) | NMT 2 min | Unsplit | <1 | <1 | — | — | — | <1[a] | <1[a] |
| | | Split | <1 | <1 | <1 | <1 | <1 | | |
| pH on Reconstitution | 2.9 to 3.5 | Unsplit | 3.1 | 3.2 | — | — | — | 3.1[a] | 3.1[a] |
| | | Split | 3.2 | 3.1 | 3.3 | 3.1 | 3.2 | | |

# Fig. 35

| Accelerated Stability Data, CNS for Injection, 25 mg (Lot 025CNS27) | | | | | | | | | |
|---|---|---|---|---|---|---|---|---|---|
| Product Name: CNS 7056 | | | Container Closure: Type I Glass Vials, 20 mL; Bromobutyl Rubber Stopper; and Aluminum Skirt | | | | | | |
| Lot: 025CNS27 | | | | | Drug Substance Lot: SOL12621/5 | | | | |
| | | | Storage Condition: 40 °C/75% RH | | | | | | |
| Test | Acceptance Criteria | Plug | Time (Months) | | | | | | |
| | | | 0 | 1 | 2 | 3 | 6 | 9 | 12 |
| Moisture Karl Fischer (% w/w) | NMT 1.5 | Unsplit | 0.36 | 0.47 | — | — | — | 1.24[a] | 0.82 |
| | | Split | 0.83 | 1.18 | 1.00 | 0.78 | 1.11 | | |
| CNS 7056 Vial Content (mg/vial) | 23.1 to 26.9 mg/vial | Unsplit | 24.6 | 24.6 | 24.4 | 24.1 | 24.6 | 24.3 | 24.6 |
| | 92.5 to 107.5% nominal amount | | 98.5 | 98.4 | 97.5 | 96.4 | 98.3 | 97.0 | 98.4 |

# Fig. 36

| Accelerated Stability Data, CNS for Injection, 25 mg (Lot 025CNS27) | | | | | | | | | |
|---|---|---|---|---|---|---|---|---|---|
| Product Name: CNS 7056 | | | Container Closure: Type I Glass Vials, 20 mL; Bromobutyl Rubber Stopper; and Aluminum Skirt | | | | | | |
| Lot: 025CNS27 | | | Storage Condition: 40 °C/75% RH | | Drug Substance Lot: SOL12621/5 | | | | |
| Test | Acceptance Criteria | Plug | Time (Months) | | | | | | |
| | | | 0 | 1 | 2 | 3 | 6 | 9 | 12 |
| Conc. on Reconst. (mg/mL) | 4.5 to 5.5 | Unsplit | 4.6 | 4.6 | 4.5 | 4.6 | 4.6 | 4.9 | [b] |
| Rel. subst. (Area %) | | | | | | | | | |
| CNS 7054X | NMT 1.5% | Unsplit | 1.08 | 1.15 | — | — | — | —[c] | — |
| RRT 0.68 | NMT 0.4% | | 0.09 | 0.09 | — | — | — | —[c] | — |
| RRT 0.93 | NMT 0.4% | | 0.12 | 0.13 | — | — | — | —[c] | — |
| RRT 0.94 | NMT 0.4% | | 0.07 | 0.08 | — | — | — | —[c] | — |
| RRT 1.31 | NMT 0.4% | | 0.22 | 0.24 | — | — | — | —[c] | — |
| Total | NMT 2.0% | | 1.58 | 1.67 | — | — | — | —[c] | — |
| Rel. Sub. (Area %): | | | | | | | | | |
| CNS 7054X | NMT 1.5% | Split | 1.08 | 1.15 | 1.24 | 1.25 | 1.36 | 1.40 | 1.51 |
| RRT 0.68 | NMT 0.4% | | 0.09 | 0.09 | 0.11 | 0.08 | 0.09 | 0.08 | 0.10 |
| RRT 0.93 | NMT 0.4% | | 0.12 | 0.13 | 0.20 | 0.12 | 0.13 | 0.11 | 0.12 |
| RRT 0.94 | NMT 0.4% | | 0.07 | 0.08 | [d] | 0.09 | 0.10 | [d] | [d] |
| RRT 1.31 | NMT 0.4% | | 0.22 | 0.24 | 0.24 | 0.23 | 0.24 | 0.23 | 0.23 |
| Total | NMT 2.0% | | 1.58 | 1.69 | 1.79 | 1.77 | 1.92 | 1.82 | 1.96 |
| Chiral (% R) | NMT 0.5% R-enantiomer | Unsplit | 0.16 | 0.16 | — | 0.12 | 0.16 | 0.15 | 0.15 |

[a] = It is not known whether the vials used for the analysis were unsplit or split.
[b] = Not done due to shortage of vials stored at 40 °C/75% RH.
[c] = It is not known whether the vials used for the analysis were unsplit or split; aligned with split data for ease of comparison.
[d] = Peaks at RRT 0.93 and 0.94 were not resolved.
— = Not performed.

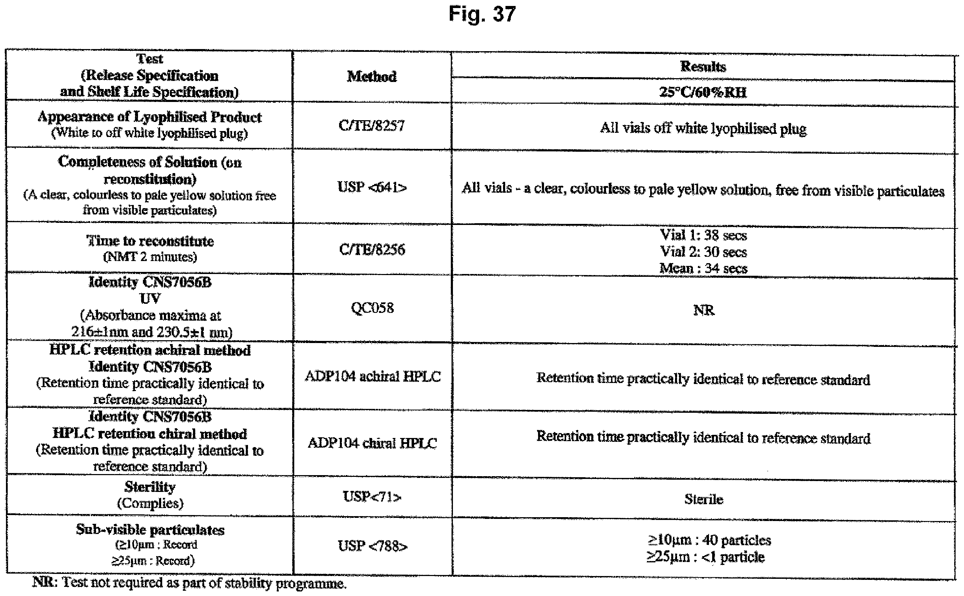

# Fig. 37

| Test (Release Specification and Shelf Life Specification) | Method | Results |
|---|---|---|
| | | 25°C/60%RH |
| **Appearance of Lyophilised Product** (White to off white lyophilised plug) | C/TE/8257 | All vials off white lyophilised plug |
| **Completeness of Solution (on reconstitution)** (A clear, colourless to pale yellow solution free from visible particulates) | USP <641> | All vials - a clear, colourless to pale yellow solution, free from visible particulates |
| **Time to reconstitute** (NMT 2 minutes) | C/TE/8256 | Vial 1: 38 secs<br>Vial 2: 30 secs<br>Mean : 34 secs |
| **Identity CNS7056B UV** (Absorbance maxima at 216±1nm and 230.5±1 nm) | QC058 | NR |
| **HPLC retention achiral method Identity CNS7056B** (Retention time practically identical to reference standard) | ADP104 achiral HPLC | Retention time practically identical to reference standard |
| **Identity CNS7056B HPLC retention chiral method** (Retention time practically identical to reference standard) | ADP104 chiral HPLC | Retention time practically identical to reference standard |
| **Sterility** (Complies) | USP<71> | Sterile |
| **Sub-visible particulates** (≥10μm : Record ≥25μm : Record) | USP <788> | ≥10μm : 40 particles<br>≥25μm : <1 particle |

**NR:** Test not required as part of stability programme.

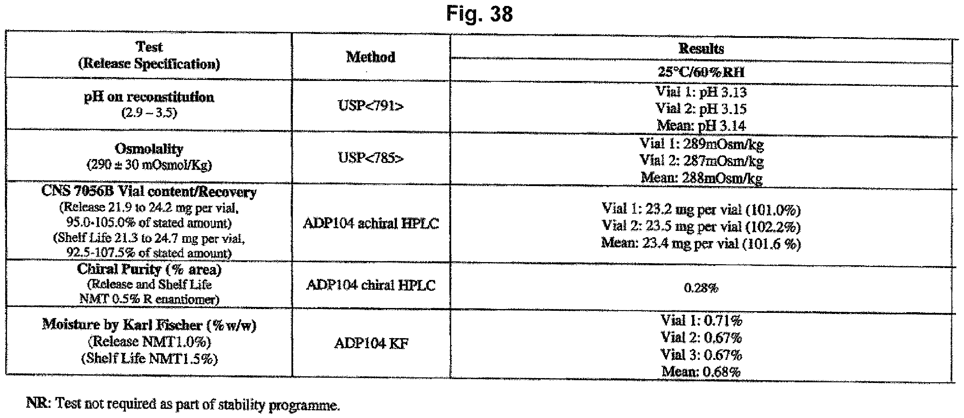

## Fig. 38

| **Test** (Release Specification) | **Method** | **Results** 25°C/60%RH |
|---|---|---|
| **pH on reconstitution** (2.9 – 3.5) | USP<791> | Vial 1: pH 3.13<br>Vial 2: pH 3.15<br>Mean: pH 3.14 |
| **Osmolality** (290 ± 30 mOsmol/Kg) | USP<785> | Vial 1: 289mOsm/kg<br>Vial 2: 287mOsm/kg<br>Mean: 288mOsm/kg |
| **CNS 7056B Vial content/Recovery** (Release 21.9 to 24.2 mg per vial, 95.0-105.0% of stated amount) (Shelf Life 21.3 to 24.7 mg per vial, 92.5-107.5% of stated amount) | ADP104 achiral HPLC | Vial 1: 23.2 mg per vial (101.0%)<br>Vial 2: 23.5 mg per vial (102.2%)<br>Mean: 23.4 mg per vial (101.6 %) |
| **Chiral Purity (% area)** (Release and Shelf Life NMT 0.5% R enantiomer) | ADP104 chiral HPLC | 0.28% |
| **Moisture by Karl Fischer (%w/w)** (Release NMT1.0%) (Shelf Life NMT1.5%) | ADP104 KF | Vial 1: 0.71%<br>Vial 2: 0.67%<br>Vial 3: 0.67%<br>Mean: 0.68% |

**NR:** Test not required as part of stability programme.

Fig. 39

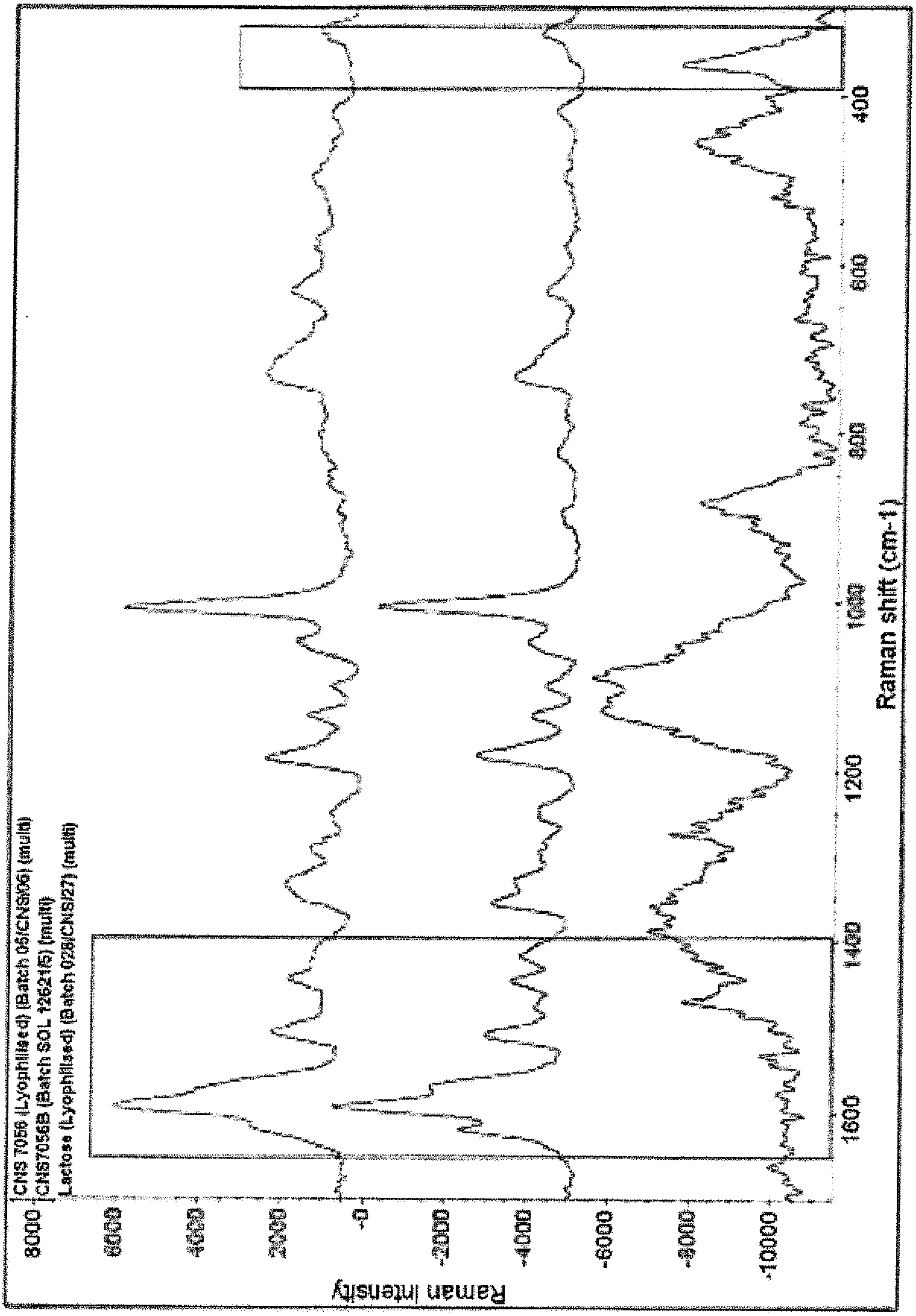
CNS 7056 (Lyophilised) (Batch 05/CNS/06) (multi)
CNS7056B (Batch SOL 1262I/5) (multi)
Lactose (Lyophilised) (Batch 028/CNS/27) (multi)
Raman Intensity
8000
6000
4000
2000
0
-2000
-4000
-6000
-8000
-10000
1600
1400
1200
1000
800
600
400
Raman shift (cm-1)

Fig. 40

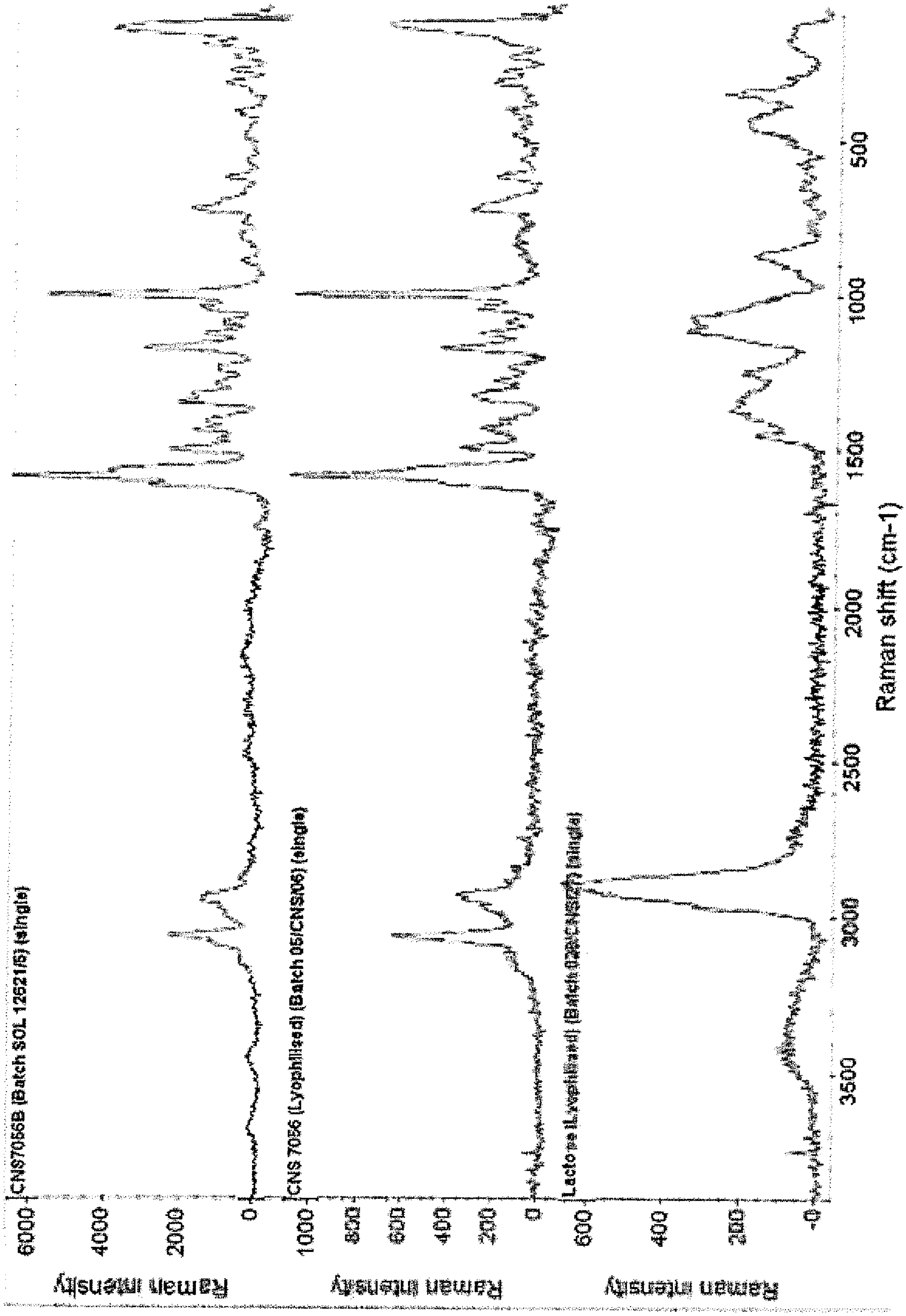
CNS7056B (Batch SOL 12521/5) (single)
CNS 7056 (Lyophilised) (Batch 05/CNS/05) (single)
Raman intensity
Raman intensity
Raman intensity
Raman shift (cm-1)

Fig. 41

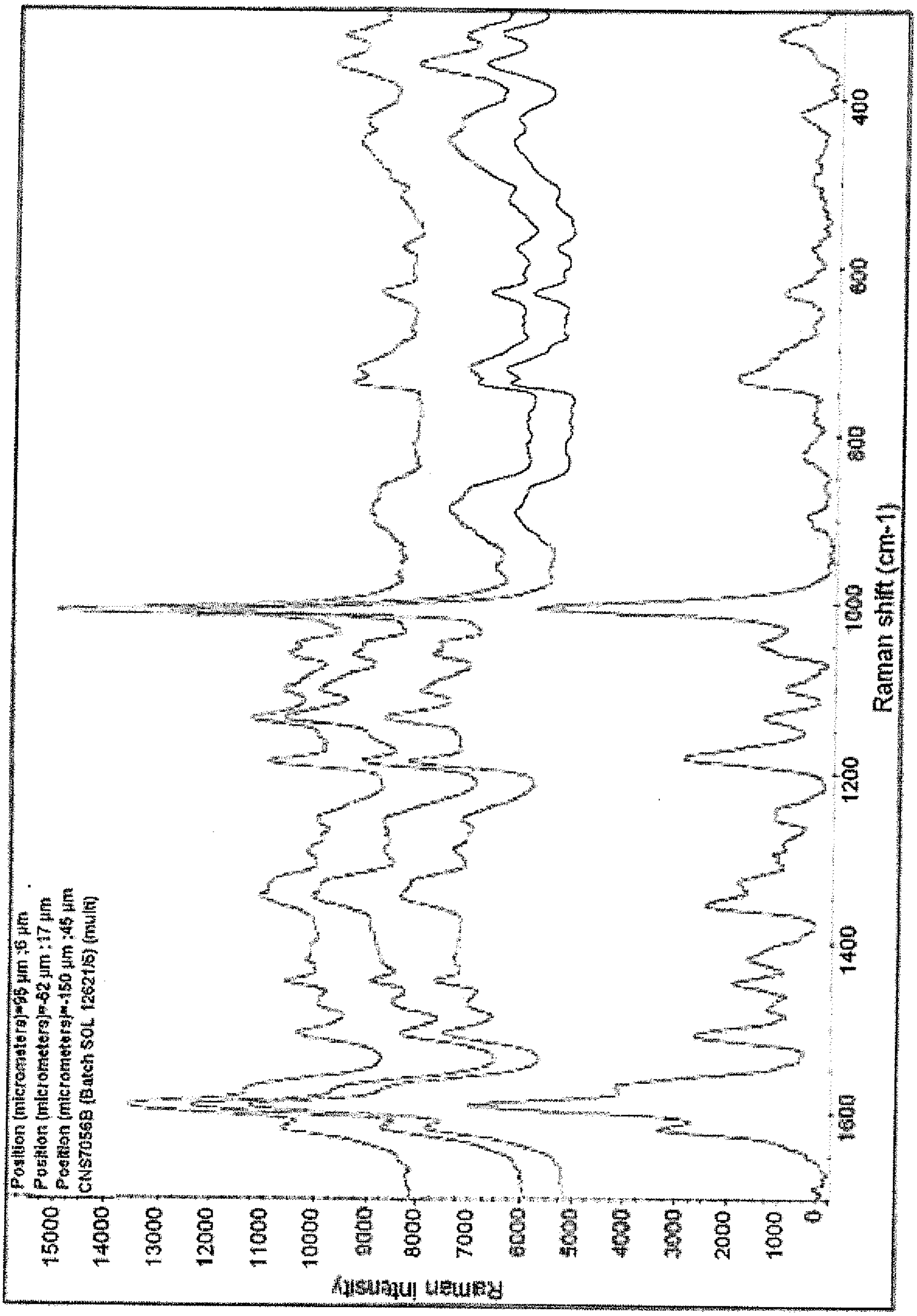
Position (micrometers)=95 µm ;6 µm
Position (micrometers)=-52 µm ;17 µm
Position (micrometers)=-150 µm ;45 µm
CNS7056B (Batch SOL 12621/5) (mulls)
Raman intensity
15000
14000
13000
12000
11000
10000
9000
8000
7000
6000
5000
4000
3000
2000
1000
0
1600
1400
1200
1000
800
600
400
Raman shift (cm-1)

# Figure 42

**Initial**

| Test Parameters | Specification | 12PM529-8-2 | 12PM529-8-1 | PM0237/12 | 12PM529-9-1 |
|---|---|---|---|---|---|
| Appearance | White to off white powder | White powder | White powder | White powder | White powder |
| Assay (mg/vial) | 24.94 – 27.66 | 25.97 | 26.11 | 26.25 | N/A |
| % Assay | 95%-105% | 98.73% | 99.29% | 99.79% | 98.75% |
| Total Impurities | 2% | 0.73% | 0.67% | 0.48% | 0.97% |
| Impurities Above 0.045% | N/A | 0.43% | 0.39% | 0.32% | 0.76% |
| Moisture | 1.5%w/w | 0.24% | 2.87% | 0.34% | 2.98% |
| Reconstitution Time | 60 seconds | 38.5s | 30.5s | 34.5s | - |
| Appearance after reconstitution time | A clear colourless to pale yellow solution free from visible particulates | clear very pale yellow solution | clear pale yellow solution | Clear colourless solution | - |
| XRPD | Assess whether crystalline material is present | No crystalline material detected | No crystalline material detected | No crystalline material detected | No crystalline material detected |

# Figure 43

4 Weeks

| Test Parameters | Specification | 12PM529-8-2 (40°C/75%RH) | 12PM529-8-2 (55°C) | PM0237/12 (40°C/75%RH) | PM0237/12 (55°C) | 12PM529-9-1 (40°C/75%RH) | 12PM529-9-1 (55°C) |
|---|---|---|---|---|---|---|---|
| Appearance | White to off white powder | White Colour free flowing powder | White Colour free flowing powder | White Colour not free flowing powder | White Colour not free flowing powder | Off white colour free flowing powder | Light grey colour free flowing powder |
| Assay (mg/vial) | 24.94 – 27.66 | 25.64mg/vial | 25.72mg/vial | 25.65mg/vial | 25.72mg/vial | N/A | N/A |
| % Assay | 95%-105% | 102.56% | 102.88% | 102.61% | 102.88% | 95.51% | 96.25% |
| Total Impurities | 2% | 0.92% | 0.99% | 0.61% | 0.71% | 2.56% | 1.94% |
| Impurities Above 0.045% | N/A | 0.69% | 0.65% | 0.40% | 0.53% | 2.35% | 1.71% |
| Moisture | 1.5%w/w | 0.25% | 0.36% | 0.25% | 0.23% | - | - |
| Reconstitution Time | 60 seconds | 42.5s | 35.0s | 35.0s | 37.5s | - | - |
| Appearance after reconstitution time | A clear colourless to pale yellow solution free from visible particulates | Clear very pale yellow solution, free from visual particulates | Clear very pale yellow solution, free from visual particulates | Clear colourless solution free from visual particulates | Clear colourless solution free from visual particulates | - | - |
| XRPD | Assess whether crystalline material is present | No crystalline material detected | No crystalline material detected | No crystalline material detected | No crystalline material detected | - | - |

# Figure 44

**13 Weeks**

| Test Parameters | Specification | 12PM529-8-2 (40°C/75%RH) | 12PM529-8-1 (40°C/75%RH) | PM0237/12 (40°C/75%RH) | 12PM529-9-1 (40°C/75%RH) |
|---|---|---|---|---|---|
| Appearance | White to off white powder | White Colour free flowing powder | White Colour free flowing powder | White Colour not free flowing powder | Light beige colour free flowing powder |
| Assay (mg/vial) | 24.94 – 27.66 | 25.69mg/vial | 23.92mg/vial | 25.48mg/vial | N/A |
| % Assay | 95%-105% | 102.76% | 95.67% | 101.92% | 95.35% |
| Total Impurities | 2% | 0.78% | 1.33% | 0.57% | 3.35% |
| Impurities Above 0.045% | N/A | 0.49% | 0.98% | 0.41% | 3.08% |
| Moisture | 1.5%w/w | 0.28% | 2.96% | 0.28% | - |
| Reconstitution Time | 60 seconds | 32.5s | 37.5s | 37.5s | - |
| Appearance after reconstitution time | A clear colourless to pale yellow solution free from visible particulates | Clear very pale yellow solution, free from visual particulates | Clear colourless solution free from visual particulates | Clear colourless solution free from visual particulates | - |
| XRPD | Assess whether crystalline material is present | No crystalline material detected | No crystalline material detected | No crystalline material detected | - |

# Figure 45

| L10R5 | | | | | |
|---|---|---|---|---|---|
| **Test** | **Time 0** | **1 month 40°C±2°C / 75%±5% RH** | **3 months 40°C±2°C / 75%±5% RH** | **3 months 25°C±2°C / 60%±5% RH** | **1 month 55°C** |
| **Appearance of the lyophilisate** | Off white cohesive cake, bright surface, some vials show cracks | Off white cohesive cake, bright surface, some vials show cracks | Off white cohesive cake, bright surface, some vials show cracks | Off white cohesive cake, bright surface, some vials show cracks | Off white cohesive cake, bright surface, some vials show cracks |
| **Reconstitution time (V=4 mL)** | 30 s | 35 s | 72 s | 63 s | 38 s |
| **Appearance of reconstitutedsolution** | Clear, pale yellow solution free of visible particles | Clear, pale yellow solution free of visible particles | Clear, pale yellow solution free of visible particles | Clear, pale yellow solution free of visible particles | Clear, pale yellow solution free of visible particles |
| **Osmolality** | 303 mOsm/Kg | - | - | - | - |
| **Moisturecontent** | 0.29%<br>(0.30%; 0.28%; 0.29%) | **0.69%**<br>(0.69%; 0.64%; 0.75%) | **0.89%**<br>(0.93%; 0.86%; 0.89%) | **0.56%**<br>(0.62%; 0.51%; 0.53%) | **1.02%**<br>(1.12%; 0.98%; 0.95%) |
| **HPLC content/assay** | 20.4 mg/vial<br>102% | 20.2 mg/vial<br>101% | 20.1 mg/vial<br>101% | 20.3 mg/vial<br>102% | 20.2 mg/vial<br>101% |
| **HPLC sum of RS & RS details** | Total RS = 0.52%<br>CNS7054X = 0.06%<br>RRT 0.66 = 0.04%<br>RRT 0.69 = 0.05%<br>RRT 0.92 = 0.11%<br>RRT 1.32 = 0.22%<br>RRT 1.92 =0.03% | **Total RS = 0.60%**<br>**CNS7054X =0,13%**<br>RRT 0.66 = 0,03%<br>RRT 0.68 = 0,05%<br>RRT 0.92 = 0,11%<br>**RRT 1.31 = 0,25%**<br>RRT 1.91 = 0,03% | **Total RS = 0.74%**<br>**CNS7054X = 0.28%**<br>RRT 0.66 =0.03%<br>RRT 0.69 = 0.05%<br>RRT 0.92 = 0.11%<br>RRT 1.31 = 0.24%<br>RRT 1.91 =0.03% | Total RS = 0.57%<br>**CNS7054X = 0.12%**<br>RRT 0.66 =0.04%<br>RRT 0.69 = 0.05%<br>RRT 0.92 = 0.10%<br>RRT 1.31 = 0.23%<br>RRT 1.91 =0.03% | **Total RS = 0.93%**<br>**CNS7054X = 0.53%**<br>RRT 0.66 =0.03%<br>RRT 0.67 = 0.04%<br>RRT 0.92 = 0.10%<br>RRT 1.31 = 0.23%<br>RRT 1.93 <LOQ |

# Figure 46

| L10R10 | | | |
|---|---|---|---|
| **Test** | **Time 0** | **1 month 40°C±2°C / 75%±5% RH** | **1 month 55°C** |
| **Appearance of the lyophilisate** | Off-white cohesive cake, bright surface, irregular edge all around the cake | Off-white cohesive cake, bright surface, irregular edge all around the cake | Off-white cohesive cake, bright surface, irregular edge all around the cake |
| **Reconstitution time (V=8 mL)** | 50 s | 45 s | 38s |
| **Appearance of reconstitutedsolution** | Clear, pale yellow solution free of visible particles | Clear, pale yellow solution free of visible particles | Clear, pale yellow solution free of visible particles |
| **Osmolality** | 616 mOsm/kg | - | - |
| **Moisturecontent** | 1.22%<br>(1.29%; 1.16%) | **0.71%**<br>(0.70%; 0.73%) | 1.23%<br>(**1.71%***; 0.74%) |
| **HPLC content/assay** | 39.7 mg/vial<br>99% | 39.1 mg/vial<br>98% | 39.4 mg/vial<br>99% |
| **HPLC sum of RS & RS details** | Total RS = 0.57%<br>CNS7054X = 0.05%<br>RRT 0.66 = 0.05%<br>RRT 0.69 = 0.07%<br>RRT 0.92 = 0.13%<br>RRT 1.32 = 0.25%<br>RRT 1.92=0.03% | Total RS = 0.61%<br>**CNS7054X =0,13%**<br>RRT 0.66 = 0,04%<br>RRT 0.68 = 0,05%<br>**RRT 0.92 = 0,11%**<br>RRT 1.31 =0,25%<br>RRT 1.92=0,03% | **Total RS = 0.84%**<br>**CNS7054X = 0.42%**<br>RRT 0.67 = 0.03%<br>RRT 0.71 = 0.05%<br>RRT 0.92 = 0.10%<br>RRT 1.32 = 0.24%<br>RRT 1.97<LOQ (**) |

(*) Sample partially melt

(**)Slight shift of the RRT, this RS has to be compared with RRT 1.92

# Figure 47

| L10R5S87 | | | | | |
|---|---|---|---|---|---|
| **Test** | **Time 0** | **1 month 40°C±2°C / 75%±5% RH** | **3 months 40°C±2°C / 75%±5% RH** | **3 months 25°C±2°C / 60%±5% RH** | **1 month 55°C** |
| **Appearance of the lyophilisate** | Off white cohesive cake, bright surface, some vials show irregular edge only on a small part of the cake | Off white cohesive cake, bright surface, some vials show irregular edge only on a small part of the cake | Off white cohesive cake, bright surface, some vials show irregular edge only on a small part of the cake | Off white cohesive cake, bright surface, some vials show irregular edge only on a small part of the cake | Off white cohesive cake, bright surface, some vials show irregular edge only on a small part of the cake |
| **Reconstitution time (V=4 mL)** | 30 s | 38 s | 68 s | 41 s | 40 s |
| **Appearance of reconstitutedsolution** | Clear, pale yellow solution free of visible particles | Clear, pale yellow solution free of visible particles | Clear, pale yellow solution free of visible particles | Clear, pale yellow solution free of visible particles | Clear, pale yellow solution free of visible particles |
| **Osmolality** | 303 mOsm/Kg | - | | | - |
| **Moisturecontent** | 0.56%<br>(0.76%; 0.48%; 0.46%) | **0.75%**<br>(0.78%; 0.73%; 0.76%) | **0.92%**<br>(0.87%; 1..07%; 0.80%) | 0.64%<br>(0.74%; 0.57%; 0.61%) | **1.03%**<br>(1.01%; 1.08%; 1.00%) |
| **HPLC content/assay** | 20.7 mg/vial<br>104% | 20.2 mg/vial<br>101% | 20.2 mg/vial<br>101% | 20.2 mg/vial<br>101% | 20.2 mg/vial<br>101% |
| **HPLC sum of RS & RS details** | Total RS = 0.55%<br>CNS7054X = 0.06%<br>RRT 0.66 = 0.04%<br>RRT 0.69 = 0.06%<br>RRT 0.92 = 0.13%<br>RRT 1.32 = 0.23%<br>RRT 1.92=0.03% | Total RS = 0.61%<br>**CNS7054X = 0,16%**<br>RRT 0.66 =0,03%<br>RRT 0.68 = 0,05%<br>RRT 0.92 = 0,11%<br>RRT 1.31 = 0,25%<br>RRT 1.92= 0,03% | **Total RS = 0.74%**<br>**CNS7054X = 0.28%**<br>RRT 0.66 =0.03%<br>RRT 0.69 = 0.05%<br>RRT 0.92 = 0.11%<br>RRT 1.31 = 0.24%<br>RRT 1.91 =0.04% | Total RS = 0.57%<br>**CNS7054X = 0.12%**<br>RRT 0.66 =0.03%<br>RRT 0.69 = 0.05%<br>RRT 0.92 = 0.10%<br>RRT 1.31 = 0.24%<br>RRT 1.91 =0.03% | **Total RS = 1.10%**<br>**CNS7054X = 0.69%**<br>RRT 0.67 = 0.03%<br>RRT 0.71 = 0.05%<br>RRT 0.92 = 0.10%<br>RRT 1.32 = 0.23%<br>RRT 1.97 <LOQ(*) |

(*) Slight shift of the RRT, this RS has to be compared with RRT 1.92

# Figure 48

| L20R5 (*) | | | | | |
|---|---|---|---|---|---|
| **Test** | **Time 0** | **1 month 40°C±2°C / 75%±5% RH** | **3 months 40°C±2°C / 75%±5% RH** | **3 months 25°C±2°C / 60%±5% RH** | **1 month 55°C** |
| **Appearance of the lyophilisate** | Off white cohesive cake, bright surface | Off white cohesive cake, bright surface (**some cakes were found to be shrunk**) | Off white cohesive cake, bright surface (**some cakes were found to be shrunk**) | Off white cohesive cake, bright surface | Off white cohesive cake, bright surface (**some vials were found to be charred**) |
| **Reconstitution time (V=5 mL)** | 30 s | **34 s** | 68 s | 59 s | 32 s |
| **Appearance of reconstitutedsolution** | Clear, pale yellow solution free of visible particles | Clear, pale yellow solution free of visible particles | Clear, pale yellow solution free of visible particles | Clear, pale yellow solution free of visible particles | Clear, pale yellow solution free of visible particles (the charred cake was unsoluble) |
| **Osmolality** | - | - | - | - | - |
| **Moisturecontent** | 0.22% (0.19%;0.23%;0.22%) | 0.37% (0.36%; 0.37%; **5.40%****) | **0.76%** (0.44%; 1.07%(■■)) | **0.63%** (1.01%; **4.85%(■)**; 0.26%) | **0.53%** (0.52%; 0.52%; 0.55%) |
| **HPLC content/assay** | 25.5 mg/vial<br>98% | 26.9 mg/vial<br>103% | 25.8 mg/vial<br>99% | 25.6 mg/vial<br>98% | 25.6 mg/vial<br>98% |
| **HPLC sum of RS & RS details** | Total RS = 0.58%<br>CNS7054X = 0.07%<br>RRT 0.66 = 0.05%<br>RRT 0.69 = 0.06%<br>RRT 0.92 = 0.12%<br>RRT 1.32 = 0.24%<br>RRT 1.92=0.03% | Total RS = 0.69%<br>CNS7054X =0,25%<br>RRT 0.66 = 0,03%<br>RRT 0.68 =0,05%<br>RRT 0.92 =0,11%<br>RRT 1.32 = 0,24%<br>RRT 1.92=0,03% | **Total RS = 1.02%**<br>**CNS7054X = 0.51%**<br>**RRT 0.61 = 0.03%**<br>RRT 0.66 =0.03%<br>RRT 0.69 = 0.05%<br>RRT 0.92 = 0.11%<br>RRT 1.31 = 0.23%<br>RRT 1.91 =0.03%<br>**RRT 2.07 = 0.03%** | **Total RS = 1.60%**<br>**CNS7054X = 1.11%**<br>**RRT 0.61 = 0.03%**<br>RRT 0.66 =0.04%<br>RRT 0.69 = 0.05%<br>RRT 0.92 = 0.11%<br>RRT 1.31 = 0.23%<br>RRT 1.91 =0.03%<br>**RRT 2.07 < LOQ** | **Total RS = 1.47%**<br>**CNS7054X = 1.03%**<br>RRT 0.64 = 0.03%<br>**RRT 0.67 = 0.03%**<br>RRT 0.71 = 0.05%<br>RRT 0.92 = 0.10%<br>RRT 1.32= 0.23%<br>RRT 1.97 < LOQ (•) |

(*) vials from clinical batch A01P310. Vials rejected after visual inspection;(**) Moisture content of a shrunk cake. This result wasn't considered in the average one as the other two results refer to cakes whose appearance didn't change from time 0. (***) Sample with a shrunk cake.(•)Slight shift of the RRT, this RS has to be compared with RRT 1.92; (■) result not included in the average; (■■) Only two vials available for the analysis, the other vials were found to be shrunk.

## Figure 49

| L4M110R5 | | | |
|---|---|---|---|
| **Test** | **Time 0** | **1 month 40°C±2°C / 75%±5% RH** | **1 month 55°C** |
| **Appearance of the lyophilisate** | Off white cohesive cake, bright surface, some vials show cracks | Off white cohesive cake, bright surface, some vials show cracks | **Yellowish shrunk cake** |
| **Reconstitution time (V=4 mL)** | 40 s | 25 s | 31 s |
| **Appearance of reconstitutedsolution** | Clear, pale yellow solution free of visible particles | Clear, pale yellow solution free of visible particles | Clear, pale yellow solution free of visible particles |
| **Osmolality** | 292 mOsm/Kg | - | - |
| **Moisturecontent** | 0.33% (0.34%; 0.33%) | **0.69%** (0.73%; 0.65%) | **1.05%** (1.10%; 1.01%) |
| **HPLC content/assay** | 20.6 mg/vial 103% | 20.3 mg/vial 102% | 20.2 mg/vial 101% |
| **HPLC sum of RS & RS details** | Total RS = 0.56%<br>CNS7054X = 0.07%<br>RRT 0.66 = 0.04%<br>RRT 0.69 = 0.06%<br>RRT 0.92 = 0.13%<br>RRT 1.32 = 0.23%<br>RRT 1.92=0.03% | **Total RS = 0.66%**<br>**CNS7054X =0,20%**<br>RRT 0.66 = 0,03%<br>RRT 0.68 = 0,04%<br>RRT 0.92 = 0,11%<br>RRT 1.32 = 0,25%<br>RRT 1.92=0,03% | **Total RS = 1.33%**<br>**RRT 0,36 = 0.03%**<br>**CNS7054X = 0.90%**<br>RRT 0.67 = 0.03%<br>RRT 0.71 = 0.05%<br>RRT 0.92 = 0.10%<br>RRT 1.32= 0.24%<br>RRT 1.97 <LOQ (*) |
| **L4M110R10** | | | |
| **Test** | **Time 0** | **1 month 40°C±2°C / 75%±5% RH** | **1 month 55°C** |
| **Appearance of the lyophilisate** | Off-white cohesive cake, irregular edge all around the cake | Off-white cohesive cake, irregular edge all around the cake | **Yellowish shrunk cake** |
| **Reconstitution time (V=8 mL)** | 50 s | 81 s | 65 s |
| **Appearance of reconstitutedsolution** | Clear, pale yellow solution free of visible particles | Clear, pale yellow solution free of visible particles | Clear, pale yellow solution free of visible particles |
| **Osmolality** | 596 mOsm/Kg | - | - |
| **Moisturecontent** | 0.53% (0.54%; 0.52%) | **0.95%** (0.79%; 1.12%) | **0.98%** (0.95%; 1.01%) |
| **HPLC content/assay** | 41.0 mg/vial 103% | 40.1 mg/vial 100% | **39.7 mg/vial 99%** |
| **HPLC sum of RS & RS details** | Total RS = 0.55%<br>CNS7054X = 0.06%<br>RRT 0.66 = 0.04%<br>RRT 0.69 = 0.06%<br>RRT 0.92 = 0.12%<br>RRT 1.32 = 0.24%<br>RRT 1.92 = 0.03% | **Total RS = 0.61%**<br>**CNS7054X =0,16%**<br>RRT 0.66 =0,03%<br>RRT 0.68 =0,06%<br>RRT 0.92 = 0,11%<br>RRT 1.32 = 0,25%<br>RRT 1.92 <LOQ | **Total RS = 1.34%**<br>**RRT 0.34 = 0.03%**<br>**RRT 0.36 = 0.03%**<br>**CNS7054X = 0.88%**<br>RRT 0.67 = 0.03%<br>RRT 0.71 = 0.06%<br>RRT 0.92 = 0.10%<br>RRT 1.32 = 0.24%<br>RRT 1.97<LOQ (*) |

# Figure 50

| L2M110R5 | | | |
|---|---|---|---|
| **Test** | **Time 0** | **1 month 40°C±2°C / 75%±5% RH** | **1 month 55°C** |
| **Appearance of the lyophilisate** | Off white cohesive cake, surface less brighter than the other formulations, some vials show irregular edge all around the cake | Off-white cohesive cake **further shrunk**, irregular edge all around the cake, surface less brighter than the other formulations | **Yellowish shrunk cake** |
| **Reconstitution time (V=4 mL)** | 50 s | 43 s | 45 s |
| **Appearance of reconstitutedsolution** | Clear, pale yellow solution free of visible particles | Clear, pale yellow solution free of visible particles | Clear, pale yellow solution free of visible particles |
| **Osmolality** | 329 mOsm/Kg | - | - |
| **Moisturecontent** | 0.34% (0.31%; 0.36%) | **0.78%** (0.82%; 0.73%) | **0.83%** (0.73%; 0.92%) |
| **HPLC content/assay** | 20.5 mg/vial 103% | 20.2 mg/vial 101% | **19.9 mg/vial** **100%** |
| **HPLC sum of RS & RS details** | Total RS = 0.58%<br>CNS7054X = 0.08%<br>RRT 0.66 = 0.05%<br>RRT 0.69 = 0.06%<br>RRT 0.92 = 0.13%<br>RRT 1.32 = 0.24%<br>RRT 1.92 = 0.03% | **Total RS = 0.68%**<br>**CNS7054X =0,22%**<br>**RRT 0,61 = 0.03%**<br>RRT 0.66 = 0,03%<br>RRT 0.68 = 0,05%<br>RRT 0.92 = 0,11%<br>RRT 1.32 = 0,23%<br>RRT 1.92 = 0,03% | **Total RS = 1.76%**<br>**RRT 0,34 = 0.05%**<br>**RRT 0,36 = 0.05%**<br>**CNS7054X = 1.21%**<br>**RRT 0.64 <LOQ**<br>RRT 0.67 = 0.03%<br>RRT 0.71 = 0.06%<br>RRT 0.92 = 0.10%<br>RRT 1.32 = 0.24%<br>**RRT 1.46 = 0.03%**<br>RRT 1.97<LOQ (*) |

## Figure 51

| L2M110R10 | | | |
|---|---|---|---|
| **Test** | **Time 0** | **1 month 40°C±2°C / 75%±5% RH** | **1 month 55°C** |
| **Appearance of the lyophilisate** | Off-white cohesive cake, bright surface, irregular edge all around the cake, shrunk cake, surface less brighter than the other formulations | Off-white cohesive cake **further shrunk**, irregular edge all around the cake, surface less brighter than the other formulations | **Yellowish shrunk cake** |
| **Recon. time (V=8 mL)** | 62 s | 81 s | 54 s |
| **Appearance of recon solution** | Clear, pale yellow solution free of visible particles | Clear, pale yellow solution free of visible particles | Clear, pale yellow solution free of visible particles |
| **Osmolality** | 648 mOsm/Kg | - | - |
| **Moisturecontent** | 1.03% (1.16%; 0.91%) | 1.00% (0.92%; 1.08%) | 0.95% (0.96%; 0.94%) |
| **HPLC content/assay** | 40.6 mg/vial 102% | 40.5 mg/vial 101% | 39.8 mg/vial 100% |
| **HPLC sum of RS & RS details** | Total RS = 0.58%%<br>CNS7054X = 0.07%<br>RRT 0.66 = 0.04%<br>RRT 0.69 = 0.07%<br>RRT 0.92 = 0.12%<br>RRT 1.32 = 0.25%<br>RRT 1.92 = 0.03% | **Total RS = 0.72%**<br>**CNS7054X = 0,25%**<br>**RRT 0,61 = 0,03%**<br>RRT 0.66 = 0,03%<br>RRT 0.68 = 0,05%<br>RRT 0.92 = 0,11%<br>RRT 1.32 = 0,25%<br>RRT 1.92 < LOQ | **Total RS = 2.23%**<br>**RRT 0,34 = 0.04%**<br>**RRT 0,36 = 0.05%**<br>**CNS7054X = 1.65%**<br>RRT 0.64 = 0.03%<br>RRT 0.67 = 0.03%<br>RRT 0.71 = 0.07%<br>RRT 0.92 = 0.10%<br>RRT 1.32 = 0.24%<br>RRT 1.97<LOQ (*) |

(*)Slight shift of the RRT, this RS has to be compared with RRT 1.92

Figure 52

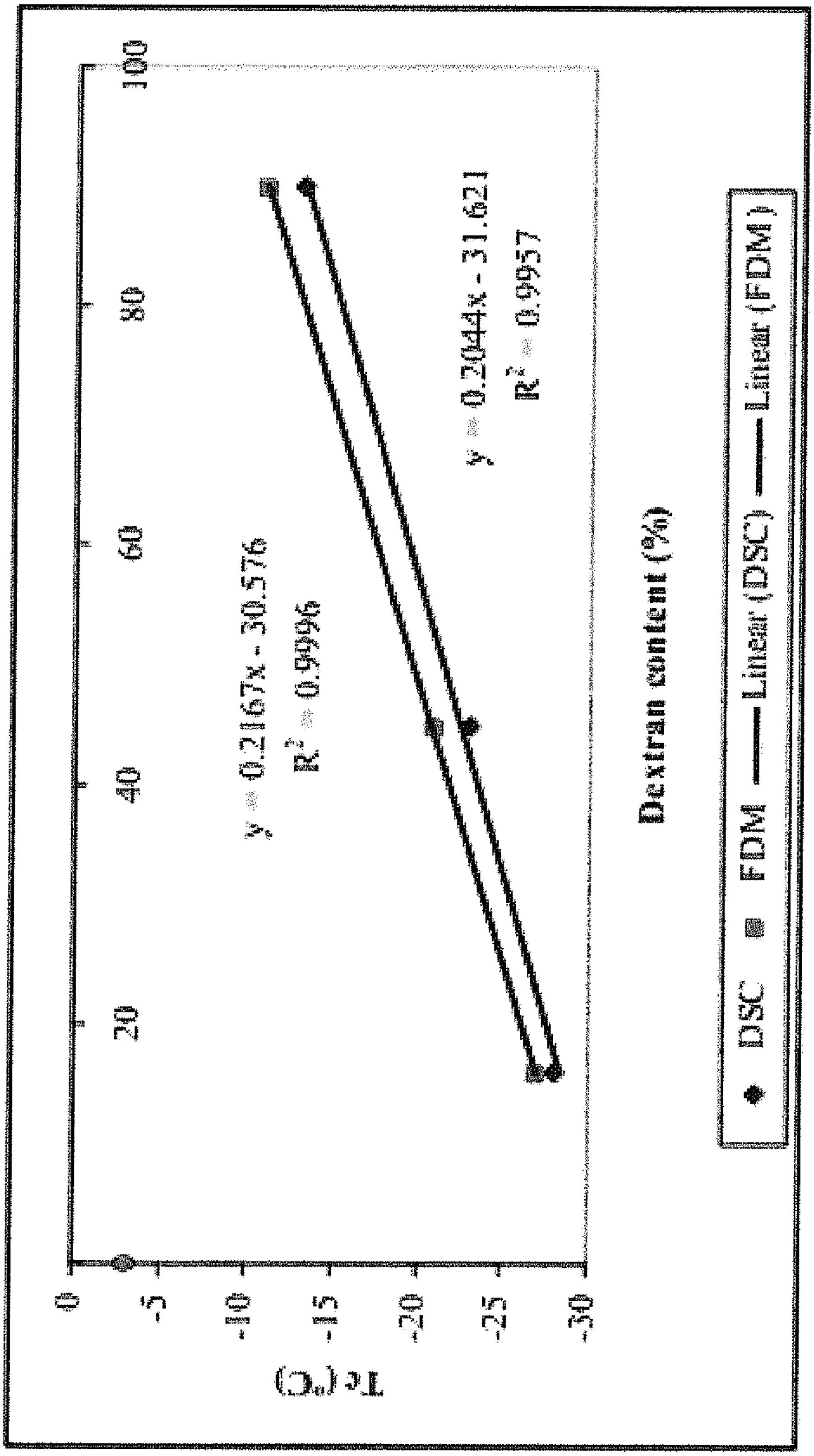

y = 0.2167x - 30.576
R² = 0.9996
y = 0.2044x - 31.621
R² = 0.9957
Tc (ºC)
0
-5
-10
-15
-20
-25
-30
20
40
60
80
100
Dextran content (%)
DSC
FDM
Linear (DSC)
Linear (FDM)

Figure 53

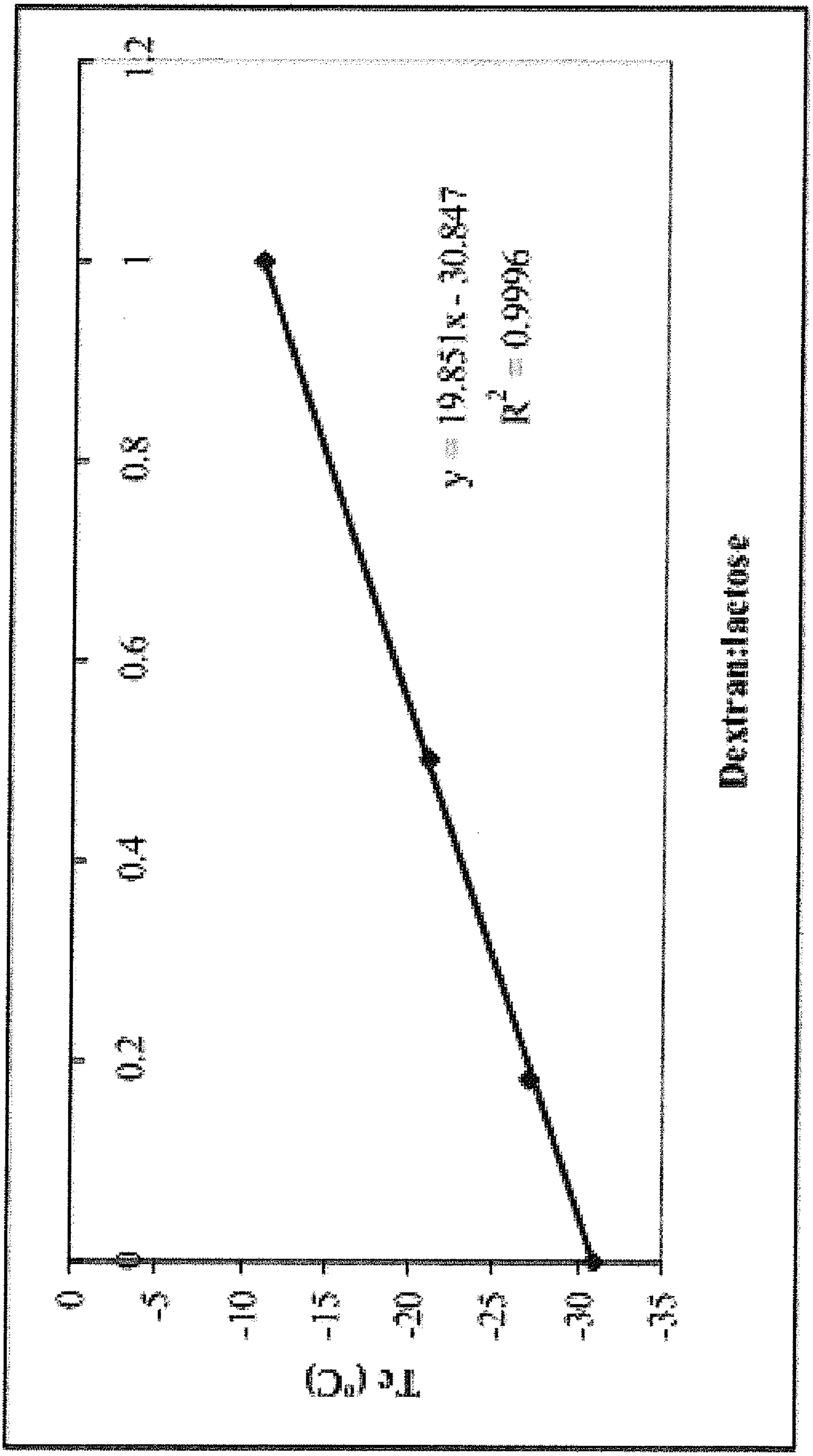
y = 19.851x - 30.847
$R^2$ = 0.9996
Te (°C)
0
-5
-10
-15
-20
-25
-30
-35
0
0.2
0.4
0.6
0.8
1
1.2
Dextran:lactose

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9623790 A
- WO 9620941 A
- US 5665718 A
- WO 200069836 A1
- WO 2008007071 A1
- WO 2008007081 A1
- WO 2013029431 A1


### Non-patent literature cited in the description


- **GOODMAN ; GILMAN.** The Pharmacological Basis of Therapeutics. Pergamon Press, 1990, 303-304, 346-358
- **W. HERING et al.** *Anesthesiology,* 1996, vol. 189, 85
- **J. DINGEMANSE et al.** *Br. J. Anaesth,* 1997, vol. 79, 567-574
- **E. MANGHISI ; A. SALIMBEMI.** *Boll. Chim. Farm.,* 1974, vol. 113, 642-644
- **W. A. KHAN ; P. SINGH.** *Org. Prep. Proc. Int.,* 1978, vol. 10, 105-111
- **J. B. HESTER, JR et al.** *J. Med. Chem.,* 1980, vol. 23, 643-647
- **T. M. BAUER et al.** *Lancet,* 1995, vol. 346, 145-7
- **A. SHAFER.** *Crit Care Med,* 1998, vol. 26, 947-956
- *Critical Reviews in Therapeutic Drug Carrier Systems,* 1997, vol. 14 (1), 1-104